# EUROPEAN PATENT APPLICATION

(11) **EP 1 690 538 A1**
(43) Date of publication of application: **16.08.2006**
(21) Application number: 04819801.4
(22) Date of filing: 29.11.2004
(51) Int. Cl.: A61K 31/42, A61K 31/423, A61K 31/4245, A61K 31/427, A61K 31/428, A61K 31/435, A61K 31/4427, A61K 31/501, A61K 31/502, A61K 31/506, A61K 31/536, A61K 31/5377, A61K 31/5395, A61P 1/04, A61P 1/18, A61P 15/00, A61P 17/04, A61P 17/06, A61P 19/02, A61P 19/10, A61P 25/16

(54) **ISOXAZOLE DERIVATIVE HAVING AGONISTIC ACTIVITY AGAINST PEROXISOME PROLIFERATOR-ACTIVATED RECEPTOR**

(30) Priority: 02.12.2003 JP 2003403274; 16.04.2004 JP 2004121635; 07.06.2004 JP 2004167941; 29.10.2004 JP 2004316251
(71) Applicant: SHIONOGI & CO., LTD., Osaka-shi, Osaka 541-0045 (JP)
(72) Inventor: FUKUI, Yoshikazu c/o Shionogi & Co., Ltd., Osaka-shi, Osaka 5530002 (JP); SASATANI, Takashi c/o Shionogi & Co., Ltd., Osaka-shi, Osaka 5530002 (JP); MATSUMURA, Ken-ichi c/o Shionogi & Co., Ltd., Osaka-shi, Osaka 5530002 (JP); ISHIZUKA, Natsuki c/o Shionogi & Co., Ltd., Osaka-shi, Osaka 5530002 (JP); YANO, Toshisada c/o Shionogi & Co., Ltd., Osaka-shi, Osaka 5530002 (JP); KANDA, Yasuhiko c/o Shionogi & Co., Ltd., Osaka-shi, Osaka 5530002 (JP); CHOMEI, Nobuo c/o Shionogi & Co., Ltd., Osaka-shi, Osaka 5530002 (JP)
(74) Representative: Vossius & Partner
(86) International application number: PCT/JP2004/017706
(87) International publication number: WO 2005/054213

(57) **Abstract**

A compound of formula (I) : 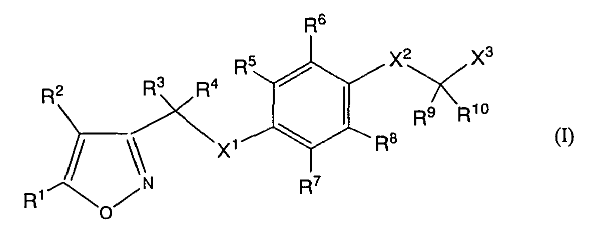 (wherein
R¹-R¹⁰ are each independently hydrogen, halogen, optionally substituted lower alkyl or the like, X¹ is -O-, -S-, -NR¹¹- (wherein R¹¹ is hydrogen, lower alkyl or the like), -CR¹²R¹³CO-, -(CR¹²R¹³)mO-, -O(CR¹²R¹³)m- (wherein R¹² and R¹³ are each independently hydrogen or lower alkyl and m is a integer between 1 and 3) or the like,
X² is a bond, -O-, -S-, -NR¹⁴- (wherein R¹⁴ is hydrogen, lower alkyl or the like, R¹⁴ and R⁶ can be taken together with the neighboring atom to form a ring) or -CR¹⁵R¹⁶⁻(wherein R¹⁵ and R¹⁶ are each independently hydrogen or lower alkyl, R¹⁵ and R⁶ or R¹⁰ can be taken together with the neighboring carbon atom to form a ring, R¹⁶ and R⁹ can be joined together to form a bond), X³ is COOR¹⁷, C( = NR¹⁷)NR¹⁸OR¹⁹ or the like), a pharmaceutically acceptable salt or a solvate thereof.

## Description

### Field of the Invention

The present invention relates to new compounds which have an agonist activity of a peroxisome proliferator-activated receptor (referred to below as PPAR) and which are useful as a medicine.

### Background of the Art

Peroxisome proliferators which proliferate an intracellular granule, peroxisome, are thought as important controlling elements of lipid metabolism. A nuclear receptor PPAR which is activated by the peroxisome proliferator has turned out to be a multifunctional receptor concerning incretion, metabolism, inflammation or the like. Therefore, the ligand is thought to be able to apply as various medicines and the number of researches is recently increasing.

The subtype genes of PPARs are found from various animal organs and formed a family. In mammals, PPARs are classified into three subtypes of PPARα, PPARδ (also referred to as PPARβ) and PPARγ.

The drugs of the fibrate group used as an antihyperlipemic drug are thought to show the activity by PPARα activation-mediated transcriptional enhancement of the gene group which improves serum lipid. Additionally, it is suggested that PPARα may relate to bone metabolism and expression of the activity of non-steroidal anti-inflammatory drugs.

The thiazolidindion compounds, which are improving drugs for insulin resistance, are ligands of PPARγ. As these compounds show hypoglycemic action, hypolipidemic action, adipocyte differentiation-inducing action or the like, PPARγ agonists are expected to develop as therapeutic agents for diabetes, hyperlipidemia, obesity or the like. Furthermore, PPARγ agonists are expected to be therapeutic agents for chronic pancreatitis, inflammatory colitis, glomerulosclerosis, Alzheimer's disease, psoriasis, parkinsonism, Basedow's disease, chronic rheumatoid arthritis, cancer (breast cancer, colonic cancer, prostatic cancer or the like), sterility or the like.

It was reported that transgenic mice in which PPARδ is overexpressed specifically in adipocyte were difficult to get fat or the like. Therefore, PPARδ agonists can be used as an antiobestic drug or an antidiabetic drug. Additionally, PPARδ, agonists are suggested the possibility as therapeutic agents for colonic cancer, osteoporosis, sterility, psoriasis, multiple sclerosis or the like.

Based on these findings, PPAR agonists are expected to be useful for treatment or prevention of hyperlipidemia, diabetes, hyperglycosemia, insulin resistance, obesity, arteriosclerosis, atherosclerosis, hypertension, syndrome X, inflammation, allergic disease (inflammatory colitis, chronic rheumatoid arthritis, chronic pancreatitis, multiple sclerosis, glomerulosclerosis, psoriasis or the like), osteoporosis, sterility, cancer, Alzheimer's disease, parkinsonism, Basedow's disease, or the like (Non-Patent Document 1).

Patent Document 1 and Patent Document 2 disclosed various compounds, with PPAR agonist activity, for example, isoxazole compounds. However, compounds having isoxazole skeleton and phenoxyacetic acid, phenylthio acetic acid or phenylamino acetic acid skeleton such as compounds of the present invention were not disclosed. Furthermore, isoxazole compounds in Patent Document 2 have substituents on isoxazole in the different position compared to compounds of the present invention. Additionally, although PPARα and (or) PPARγ agonist activity of the compounds were recognized, no data of PPARδ agonist activity was disclosed. Furthermore, there was no data of isoxazole compounds even about PPARα or γ agonist activity. In a word, the PPAR agonist activity was not recognized.

Although Patent Document 3 disclosed isoxazole compounds, the compounds have substituents on isoxazole in the different position compared to compounds of the present invention. Furthermore, it was disclosed that the compounds are as ligands of FXR NR1H4 receptor and useful for hypercholesterolemia or hyperlipidemia. However, the PPAR agonist activity was not disclosed.

Although Patent Document 4 disclosed isoxazole compounds, the compounds have substituents on isoxazole in the different position compared to compounds of the present invention. Additionally, it was disclosed that the compounds are useful for arteriosclerosis or hypertension. However, the PPAR, agonist activity was not disclosed.

Patent Document 5 and 6 disclosed thiazole compounds, oxazole compounds and imidazole compounds with PPARδ agonist activity. However, isoxazole compounds were not suggested.

Patent Document 7 disclosed isoxazole compounds with cinnamic acid at the terminal position. It was disclosed that the compounds have thyroid receptor antagonist activity. However, the PPAR agonist activity was not disclosed.

Patent Document 8 disclosed isoxazole compounds. The disclosed compounds have hydrogen on the isoxazole ring when they have phenoxy acetic acid at the terminal position. Therefore, they are different from compounds of the present invention. The data of agonist activity of PPARα and PPARδ were disclosed.
Patent Document 1: WO99/11255
Patent Document 2: WO99/58510
Patent Document 3: WO03/15771
Patent Document 4: EP0558062
Patent Document 5: WO01/00603
Patent Document 6: WO02/14291
Patent Document 7: WO01/36365
Patent Document 8: WO03/084916
Non-Patent Document 1: Current Medicinal Chemistry, 2003, Vol. 10, 267-280

### Disclosure of Invention

### Problems to be solved by the Invention

The objection of the present invention is to provide good PPAR agonists.

### Means for Solving the Problem

The present inventors have intensively studied to synthesize new good PPAR agonists as below. Compounds which have hydrogen at the 4 position of isoxazole and phenoxyacetic acid at the terminal are disclosed in Patent Document 8. However, the present inventors found that PPAR transcription activity of compounds, of which the hydrogen at the 4 position is substituted for the other substituent such as methyl, is greatly improved compared to the compounds before substitution. Furthermore, the inventors found that compounds, of which phenoxyacetic acid at the terminal is substituted for cinnamic acid, have the weaker drug metabolism enzyme inhibition than the compounds before substitution.

The present invention is,
(1) A compound of the formula (I): 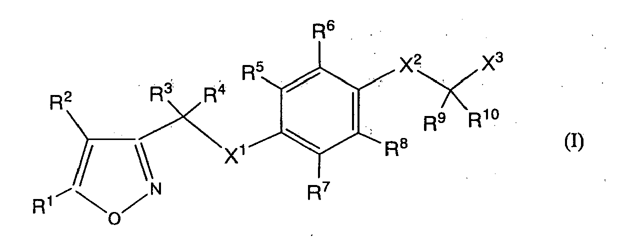 (wherein
   R¹ is halogen, hydroxy, optionally substituted lower alkyl, optionally substituted lower alkenyl, optionally substituted lower alkynyl, optionally substituted lower alkoxy, carboxy, optionally substituted lower alkoxycarbonyl, optionally substituted lower alkylthio, optionally substituted acyl, optionally substituted amino, optionally substituted carbamoyl, optionally substituted thiocarbamoyl, optionally substituted carbamoyloxy, optionally substituted thiocarbamoyloxy, optionally substituted hydrazinocarbonyl, optionally substituted lower alkylsulfonyloxy, optionally substituted arylsulfonyloxy, optionally substituted aryl, optionally substituted aryloxy, optionally substituted arylthio or optionally substituted heterocycle,
   R² is hydrogen, halogen, hydroxy, optionally substituted lower alkyl, optionally substituted lower alkenyl, optionally substituted lower alkynyl, optionally substituted lower alkoxy, carboxy, optionally substituted lower alkoxycarbonyl, optionally substituted lower alkylthio, optionally substituted acyl, optionally substituted amino, optionally substituted carbamoyl, optionally substituted thiocarbamoyl, optionally substituted carbamoyloxy, optionally substituted thiocarbamoyloxy, optionally substituted hydrazinocarbonyl, optionally substituted lower alkylsulfonyloxy, optionally substituted arylsulfonyloxy, optionally substituted aryl, optionally substituted aryloxy, optionally substituted arylthio or optionally substituted heterocycle,
   R³ and R⁴ are each independently hydrogen, halogen, optionally substituted lower alkyl, optionally substituted lower alkenyl, optionally substituted lower alkynyl, optionally substituted aryl or optionally substituted heterocycle,
   R⁵, R⁶, R⁷ and R⁸ are each independently hydrogen, halogen, hydroxy, optionally substituted lower alkyl, optionally substituted lower alkenyl, optionally substituted lower alkynyl, optionally substituted lower alkoxy, optionally substituted lower alkylthio, optionally substituted acyl, optionally substituted amino, optionally substituted aryl, optionally substituted atyloxy; optionally substituted arylthio or optionally substituted heterocycle,
   R⁹ and R¹⁰ are each independently hydrogen, halogen, cyano, optionally substituted lower alkyl, optionally substituted lower alkoxy, optionally substituted amino or optionally substituted aryl,
   X¹ is -O-, -S-, -NR¹¹- (wherein R¹¹ is hydrogen; optionally substituted lower alkyl, optionally substituted acyl, optionally substituted lower alkylsulfonyl or optionally substituted arylsulfonyl), -CR¹²R¹³CO-, -(CR¹²R¹³)mO-, -(CR¹²R¹³)mS- or -O(CR¹²R¹³)m- (wherein R¹² and R¹³ are each independently hydrogen or lower alkyl and m is an integer between 1 and 3),
   X² is a bond, -O-, -S-, -SO-, -SO₂-, -CR²⁶=CR²⁷- (wherein R²⁶ and R²⁷ are each independently hydrogen or lower alkyl), -NR¹⁴- (wherein R¹⁴ is hydrogen, optionally substituted lower alkyl, optionally substituted acyl, optionally substituted lower alkylsulfonyl or optionally substituted arylsulfonyl), -CR¹⁵R¹⁶- (wherein R¹⁵ and R¹⁶ are each independently hydrogen or lower alkyl) or -COCR²⁴R²⁵- (wherein R²⁴ and R²⁵ are each independently hydrogen or lower alkyl), and
   X³ is COOR¹⁷, C(=NR¹⁷)NR¹⁸OR¹⁹, 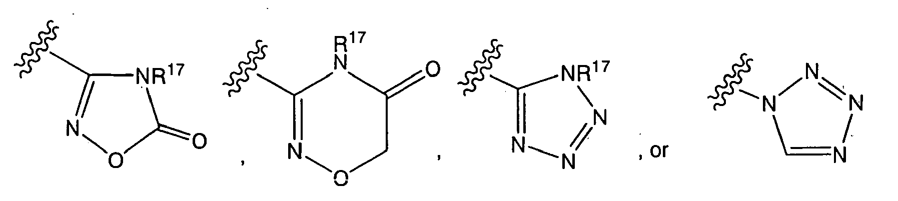 (wherein R¹⁷ - R¹⁹ are each independently hydrogen or lower alkyl),
   provided that,
   R⁶ and R¹⁴ can be taken together with the neighboring atom to form a ring,
   R⁶, R⁹ and R¹⁰ can be taken together with the neighboring carbon atom to form a ring;
   R⁶ and R⁹ can be taken together with the neighboring carbon atom to form a ring,
   R⁶, R¹⁵ and R¹⁶ can be taken together with the neighboring carbon atom to form a ring, R⁶ and R²⁴ can be taken together with the neighboring carbon atom to form a ring,
   R⁹ and R¹⁶ can be joined together to form a bond,
   R⁹ and R¹⁰ can be taken together to form a ring,
   R⁹ and R²⁵ can be joined together to form a bond,
   R⁹, R¹⁰ and R¹⁵ can be taken together with the neighboring carbon atom to form a ring, R¹⁰ and R¹⁵ can be joined together to form a bond, and
   R¹⁰ and R¹⁵ can be taken together with the neighboring carbon atom to form a ring) (provided that, a compound wherein R¹ is an unsubstituted lower alkyl, R⁵ and R⁷ are bromo and X¹ is -O-, a compound wherein R¹ is an unsubstituted lower alkyl and X² is -CH₂- and a compound wherein R² is hydrogen and X² is -O- are excluded.),
   a pharmaceutically acceptable salt or a solvate thereof.
(2) The compound of (1) wherein R¹ is halogen, optionally substituted lower alkyl, optionally substituted aryl or optionally substituted heterocycle, a pharmaceutically acceptable salt or a solvate thereof.
(3) The compound of (1) wherein R² is halogen, optionally substituted lower alkyl, optionally substituted lower alkenyl, optionally substituted alkynyl, optionally substituted lower alkoxy, optionally substituted acyl, optionally substituted carbamoyl, optionally substituted aryl or optionally substituted arylthio, a pharmaceutically acceptable salt or a solvate thereof.
(4) The compound of (1) wherein R² is hydrogen, halogen, optionally substituted lower alkyl, optionally substituted lower alkenyl, optionally substituted alkynyl, optionally substituted lower alkoxy, optionally substituted acyl, optionally substituted carbamoyl, optionally substituted aryl or optionally substituted arylthio, a pharmaceutically acceptable salt or a solvate thereof.
(5) The compound of (1) wherein R³ and R⁴ are each independently hydrogen, lower alkyl or optionally substituted aryl, a pharmaceutically acceptable salt or a solvate thereof.
(6) The compound of (1) wherein R⁵, R⁶, R⁷ and R⁸ are each independently hydrogen, halogen, optionally substituted lower alkyl or optionally substituted lower alkoxy, provided that,
   R⁶ and R¹⁴ can be taken together with the neighboring atom to form a ring;
   R⁶, R⁹ and R¹⁰ can be taken together with the neighboring carbon atom to form a ring,
   R⁶ and R⁹ can be taken together with the neighboring carbon atom to form a ring,
   R⁶, R¹⁵ and R¹⁶ can be taken together with the neighboring carbon atom to form a ring, and R⁶ and R²⁴ can be taken together with the neighboring carbon atom to form a ring,
   a pharmaceutically acceptable salt or a solvate thereof.
(7) The compound of (1) wherein R⁹ and R¹⁰ are each independently hydrogen, halogen, cyano, optionally substituted lower alkyl or optionally substituted lower alkoxy, provided that,
   R⁹, R¹⁰ and R⁶ can be taken together with the neighboring carbon atom to form a ring,
   R⁹ and R⁶ can be taken together with the neighboring carbon atom to form a ring,
   R⁹ and R¹⁶ can be joined together to form a bond,
   R⁹ and R¹⁰ can be taken together to form a ring,
   R⁹ and R²⁵ can be joined together to form a bond,
   R⁹, R¹⁰ and R¹⁵ can be taken together with the neighboring carbon atom to form a ring, R¹⁰ and R¹⁵ can be joined together to form a bond, and
   R¹⁰ and R¹⁵ can be taken together with the neighboring carbon atom to form a ring,
   a pharmaceutically acceptable salt or a solvate thereof.
(8) The compound of (1) wherein X¹ is O, S, NR¹¹ (wherein R¹¹ is hydrogen or optionally substituted lower alkyl) or CH₂CO, a pharmaceutically acceptable salt or a solvate thereof.
(9) The compound of (1) wherein X³ is COOR¹⁷ (wherein R¹⁷ is hydrogen or lower alkyl), a pharmaceutically acceptable salt or a solvate thereof.
(10) The compound of (1) wherein R¹ is lower alkyl, optionally substituted aryl (the substituent is halogen, optionally substituted lower alkyl or optionally substituted lower alkoxy) or heterocycle,
   R² is hydrogen, halogen, optionally substituted lower alkyl (the substituent is halogen, hydroxy, optionally substituted lower alkoxy, lower alkylamino, optionally substituted imino, lower alkylsulfonyl, optionally substituted aryl or heterocycle), optionally substituted lower alkynyl (the substituent is aryl), optionally substituted lower alkoxy (the substituent is halogen), alkoxycarbonyl, acyl, carbamoyl, optionally substituted aryl (the substituent is optionally substituted lower alkyl or optionally substituted lower alkoxy) or arylthio,
   R³ and R⁴ are each independently, hydrogen, lower alkyl or optionally substituted aryl (the substituent is halogen),
   R⁵, R⁶, R⁷ and R⁸ are each independently, hydrogen, halogen, optionally substituted lower alkyl (the substituent is halogen) or optionally substituted lower alkoxy (the substituent is halogen),
   R⁹ and R¹⁰ are each independently hydrogen, halogen, cyano, lower alkyl or lower alkoxy,
   X¹ is O, S, NH or CH₂CO, and
   X³ is COOR¹⁷, C(=NR¹⁷)NR¹⁸OR¹⁹, 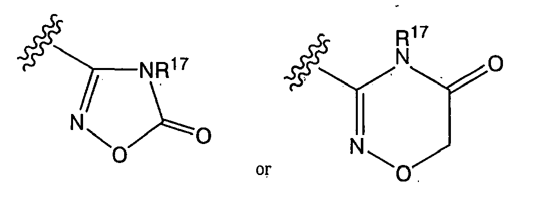 (wherein R¹⁷ - R¹⁹ are each independently hydrogen or lower alkyl),
   provided that,
   R⁶ and R¹⁴ can be taken together with the neighboring atom to form a ring,
   R⁶, R⁹ and R¹⁰ can be taken together with the neighboring carbon atom to form a ring,
   R⁶ and R⁹ can be taken together with the neighboring carbon atom to form a ring,
   R⁶, R¹⁵ and R¹⁶ can be taken together with the neighboring carbon atom to form a ring,
   R⁶ and R²⁴ can be taken together with the neighboring carbon atom to form a ring,
   R⁹ and R¹⁶ can be joined together to form a bond,
   R⁹ and R¹⁰ can be taken together to form a ring,
   R⁹ and R²⁵ can be joined together to form a bond,
   R9, R¹⁰ and R¹⁵ can be taken together with the neighboring carbon atom to form a ring,
   R¹⁰ and R¹⁵ can be joined together to form a bond, and
   R¹⁰ and R¹⁵ can be taken together with the neighboring carbon atom to form a ring,
   a pharmaceutically acceptable salt or a solvate thereof.
(11) The compound of any one of (1) - (10) wherein X² is a bond, -O-, -SO-, -SO₂- or -CR²⁶=CR²⁷- (wherein R²⁶ and R²⁷ are each independently hydrogen or lower alkyl), a pharmaceutically acceptable salt or a solvate thereof.
(12) The compound of any one of (1) - (10) wherein X² is -CR¹⁵R¹⁶- (wherein R¹⁵ is hydrogen or lower alkyl and R¹⁶ and R⁹ are joined together to form a bond or wherein R¹⁶ and R⁹ are joined together to form a bond and R¹⁵ and R¹⁰ are joined together to form a bond), a pharmaceutically acceptable salt or a solvate thereof.
(13) The compound of any one of (1) - (10) wherein X² is -NR¹⁴- (wherein R¹⁴ is hydrogen, lower alkyl, acyl or lower alkylsulfonyl or wherein R¹⁴ and R⁶ are taken together with the neighboring atom to form a ring), -CR¹⁵R¹⁶- (wherein R¹⁵, R¹⁶ and R⁶ are taken together with the neighboring carbon atom to form a ring, wherein R⁹, R¹⁰ and R¹⁵ can be taken together with the neighboring carbon atom to form a ring or wherein R¹⁵ and R¹⁰ are taken together with the neighboring carbon atom to form a ring and R¹⁶ and R⁹ are joined together to form a bond) or -COCR²⁴R²⁵- (wherein R²⁴ and R⁶ are taken together with the neighboring carbon atom to form a ring and R²⁵ and R⁹ are joined together to form a bond), a pharmaceutically acceptable salt or a solvate thereof.
(14) The compound of (1) wherein R² is halogen, hydroxy, optionally substituted lower alkyl, optionally substituted lower alkenyl,' optionally substituted lower alkynyl, optionally substituted lower alkoxy, carboxy, optionally substituted lower alkoxycarbonyl, optionally substituted lower alkylthio, optionally substituted acyl, optionally substituted amino, optionally substituted carbamoyl, optionally substituted thiocarbamoyl, optionally substituted carbamoyloxy, optionally substituted thiocarbamoyloxy, optionally substituted hydrazinocarbonyl, optionally substituted lower alkylsulfonyloxy, optionally substituted arylsulfonyloxy, optionally substituted aryl, optionally substituted aryloxy, optionally substituted arylthio or optionally substituted heterocycle,
   R⁹ and R¹⁰ are each independently hydrogen,
   X¹ is -O-, -S-, -(CR¹²R¹³)mO- or -(CR¹²R¹³)mS- (wherein R¹² and R¹³ are each independently hydrogen or lower alkyl and m is an integer between 1 and 3),
   X² is -O-, and
   X³ is COOR¹⁷ (wherein R¹⁷ is hydrogen or lower alkyl),
   a pharmaceutically acceptable salt or a solvate thereof.
(15) The compound of (1) wherein R⁹ and R¹⁶ are joined together to form a bond,
   R¹⁰ is hydrogen, halogen, lower alkyl, lower alkoxy or cyano,
   X¹ is -O-, -S-, -(CR¹²R¹³)mO- or -(CR¹²R¹³)mS- (wherein R¹² and R¹³ are each independently hydrogen or lower alkyl and m is an integer between 1 and 3),
   X² is -CR¹⁵R¹⁶- (wherein R¹⁵ is hydrogen or lower alkyl and R¹⁶ and R⁹ are joined together to form a bond), and
   X³ is COOR¹⁷ (wherein R¹⁷ is hydrogen or lower alkyl), a pharmaceutically acceptable salt or a solvate thereof.
(16) The compound of (1) wherein R¹ is halogen, a substituted lower alkyl, optionally substituted aryl or optionally substituted heterocycle,
   R⁹ and R¹⁰ are each independently hydrogen or lower alkyl,
   X¹ is -O-, -S-, -(CR¹²R¹³)mO- or -(CR¹²R¹³)mS- (wherein R¹² and R¹³ are each independently hydrogen or lower alkyl and m is an integer between 1 and 3),
   X² is a bond or -CR¹⁵R¹⁶- (wherein R¹⁵ and R¹⁶ are each independently hydrogen or lower alkyl), and
   X³ is COOR¹⁷ (wherein R¹⁷ is hydrogen or lower alkyl), a pharmaceutically acceptable salt or a solvate thereof.
(17) The compound of (1) wherein R⁹ and R¹⁰ are each independently hydrogen,
   X¹ is -O- or -S-,
   X² is -NR¹⁴- (wherein R¹⁴ and R⁶ are taken together with the neighboring atom to form a ring), -CR¹⁵R¹⁶- (wherein R¹⁵, R¹⁶ and R⁶ are taken together with the neighboring carbon atom to form a ring), or -COCR²⁴R²⁵- (wherein R²⁴ and R⁶ are taken together with the neighboring carbon atom to form a ring and R²⁵ and R⁹ are joined together to form a bond), and
   X³ is COOR¹⁷ (wherein R¹⁷ is hydrogen or lower alkyl), a pharmaceutically acceptable salt or a solvate thereof.
(18) The compound of (1) wherein R⁹ and R¹⁶ are joined together to form a bond,
   X¹ is -O- or -S-,
   X² is -CR¹⁵R¹⁶- (wherein R¹⁵ and R¹⁰ are taken together with the neighboring carbon atom to form a ring and R¹⁶ and R⁹ are joined together to form a bond or wherein R⁹, R¹⁰ and R¹⁵ are taken together with the neighboring carbon atom to form a ring), and
   X³ is COOR¹⁷ (wherein R¹⁷ is hydrogen or lower alkyl), a pharmaceutically acceptable salt or a solvate thereof.
(19) The compound of (1) wherein R⁹ and R¹⁰ are taken together to form a ring,
   X¹ is -O- or -S-,
   X² is a bond or -CR¹⁵R¹⁶- (wherein R¹⁵ and R¹⁶ are each independently hydrogen or lower alkyl), and
   X³ is COOR¹⁷ (wherein R¹⁷ is hydrogen or lower alkyl), a pharmaceutically acceptable salt or a solvate thereof.
(20) A compound of the formula: 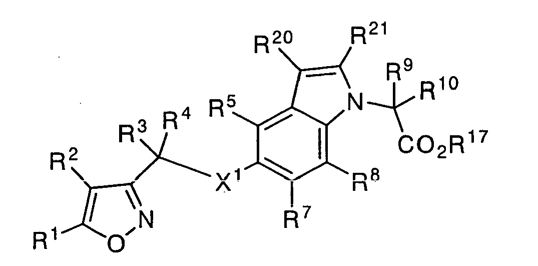 (wherein
   R¹ is halogen, hydroxy, optionally substituted lower alkyl, optionally substituted lower alkenyl, optionally substituted lower alkynyl, optionally substituted lower alkoxy, carboxy, optionally substituted lower alkoxycarbonyl, optionally substituted lower alkylthio, optionally substituted acyl, optionally substituted amino, optionally substituted carbamoyl, optionally substituted thiocarbamoyl, optionally substituted carbamoyloxy, optionally substituted thiocarbamoyloxy, optionally substituted hydrazinocarbonyl, optionally substituted lower alkylsulfonyloxy, optionally substituted arylsulfonyloxy, optionally substituted aryl, optionally substituted aryloxy, optionally substituted arylthio or optionally substituted heterocycle,
   R² is hydrogen, halogen, hydroxy, optionally substituted lower alkyl, optionally substituted lower alkenyl, optionally substituted lower alkynyl, optionally substituted lower alkoxy, carboxy, optionally substituted lower alkoxycarbonyl, optionally substituted lower alkylthio, optionally substituted acyl, optionally substituted amino, optionally substituted carbamoyl, optionally substituted thiocarbamoyl, optionally substituted carbamoyloxy, optionally substituted thiocarbamoyloxy, optionally substituted hydrazinocarbonyl, optionally substituted lower alkylsulfonyloxy, optionally substituted arylsulfonyloxy, optionally substituted aryl, optionally substituted aryloxy, optionally substituted arylthio or optionally substituted heterocycle,
   R³ and R⁴ are each independently, hydrogen, halogen, optionally substituted lower alkyl, optionally substituted lower alkenyl, optionally substituted lower alkynyl, optionally substituted aryl or optionally substituted heterocycle,
   R⁵, R⁷ and R⁸ are each independently hydrogen, halogen, hydroxy, optionally substituted lower alkyl, optionally substituted lower alkenyl, optionally substituted lower alkynyl, optionally substituted lower alkoxy, optionally substituted lower alkylthio, optionally substituted acyl, optionally substituted amino, optionally substituted aryl, optionally substituted aryloxy, optionally substituted arylthio or optionally substituted heterocycle,
   R⁹ and R¹⁰ are each independently hydrogen, halogen, cyano, optionally substituted lower alkyl, optionally substituted lower alkoxy, optionally substituted amino or optionally substituted aryl,
   R²⁰ and R²¹ are each independently hydrogen, halogen; hydroxy, cyano, optionally substituted lower alkyl, optionally substituted lower alkenyl, optionally substituted lower alkynyl, optionally substituted lower alkoxy, optionally substituted lower alkylthio, optionally substituted acyl, optionally substituted amino, optionally substituted imino, optionally substituted aryl, optionally substituted aryloxy, optionally substituted arylthio or optionally substituted heterocycle,
   X¹ is -O-, -S-, -NR¹¹- (wherein R¹¹ is hydrogen, optionally substituted lower alkyl, optionally substituted acyl, optionally substituted lower alkylsulfonyl or optionally substituted arylsulfonyl), -CR¹²R¹³CO-, -(CR¹²R¹³)mO-, -(CR¹²R¹³)mS- or -O(CR¹²R¹³)m-(wherein R¹² and R¹³ are each independently hydrogen or lower alkyl and m is an integer between 1 and 3), and
   R¹⁷ is hydrogen or lower alkyl), a pharmaceutically acceptable salt or a solvate thereof.
(21) The compound of (20) wherein R¹ is optionally substituted aryl,
   R² is optionally substituted lower alkyl,
   R³ and R⁴ are each independently hydrogen or optionally substituted aryl,
   R⁵, R⁷ and R⁸ are each independently hydrogen, optionally substituted lower alkyl or optionally substituted lower alkoxy,
   R⁹ and R¹⁰ are each independently hydrogen or optionally substituted lower alkyl,
   R²⁰ and R²¹ are each independently hydrogen, cyano, optionally substituted lower alkyl or optionally substituted lower alkoxy, and
   X¹ is -O- or -S-,
   a pharmaceutically acceptable salt or a solvate thereof.
(22) A compound of the formula: 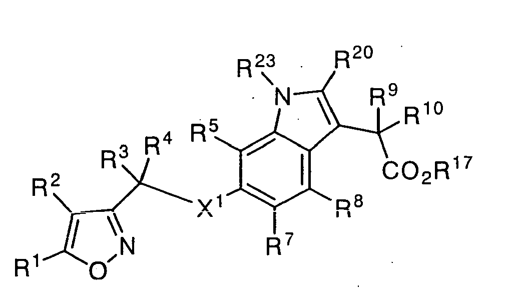 (wherein
   R¹ is halogen, hydroxy, optionally substituted lower alkyl, optionally substituted lower alkenyl, optionally substituted lower alkynyl, optionally substituted lower alkoxy, carboxy, optionally substituted lower alkoxycarbonyl, optionally substituted lower alkylthio, optionally substituted acyl, optionally substituted amino, optionally substituted carbamoyl, optionally substituted thiocarbamoyl, optionally substituted carbamoyloxy, optionally substituted thiocarbamoyloxy, optionally substituted hydrazinocarbonyl, optionally substituted lower alkylsulfonyloxy, optionally substituted arylsulfonyloxy, optionally substituted aryl, optionally substituted aryloxy, optionally substituted arylthio or optionally substituted heterocycle,
   R² is hydrogen, halogen, hydroxy, optionally substituted lower alkyl, optionally substituted lower alkenyl, optionally substituted lower alkynyl, optionally substituted lower alkoxy, carboxy, optionally substituted lower alkoxycarbonyl, optionally substituted lower alkylthio, optionally substituted acyl, optionally substituted amino, optionally substituted carbamoyl, optionally substituted thiocarbamoyl, optionally substituted carbamoyloxy, optionally substituted thiocarbamoyloxy, optionally substituted hydrazinocarbonyl, optionally substituted lower alkylsulfonyloxy, optionally substituted arylsulfonyloxy, optionally substituted aryl, optionally substituted aryloxy, optionally substituted arylthio or optionally substituted heterocycle,
   R³ and R⁴ are each independently hydrogen, halogen, optionally substituted lower alkyl, optionally substituted lower alkenyl, optionally substituted lower alkynyl, optionally substituted aryl or optionally substituted heterocycle,
   R⁵, R⁷, R⁸ and R²⁰ are each independently hydrogen, halogen, hydroxy, optionally substituted lower alkyl, optionally substituted lower alkenyl, optionally substituted lower alkynyl, optionally substituted lower alkoxy, optionally substituted lower alkylthio, optionally substituted acyl, optionally substituted amino, optionally substituted aryl, optionally substituted aryloxy, optionally substituted arylthio or optionally substituted heterocycle,
   R²³ is hydrogen, optionally substituted lower alkyl, optionally substituted lower alkenyl, optionally substituted lower alkynyl, optionally substituted acyl, optionally substituted lower alkylsulfonyl or optionally substituted arylsulfonyl, optionally substituted amino, optionally substituted aryl or optionally substituted heterocycle,
   R⁹ and R¹⁰ are each independently hydrogen, halogen, cyano, optionally substituted lower alkyl, optionally substituted lower alkoxy, optionally substituted amino or optionally substituted aryl,
   X¹ is -O-, -S-, -NR¹¹- (wherein R¹¹ is hydrogen, optionally substituted lower alkyl, optionally substituted acyl, optionally substituted lower alkylsulfonyl or optionally substituted arylsulfonyl), -CR¹²R¹³CO-, -(CR¹²R¹³)mO-, -(CR¹²R¹³)mS- or -O(CR¹²R¹³)m-(wherein R¹² and R¹³ are each independently hydrogen or lower alkyl and m is an integer between 1 and 3), and
   R¹⁷ is hydrogen or lower alkyl),
   a pharmaceutically acceptable salt or a solvate thereof.
(23) The compound of (22) wherein R¹ is optionally substituted aryl,
   R² is optionally substituted lower alkyl,
   R³ and R⁴ are hydrogen,
   R⁵, R⁷ and R⁸ are hydrogen,
   R⁹ and R¹⁰ are each independently hydrogen or optionally substituted lower alkyl;
   R²⁰ and R²³ are each independently hydrogen or optionally substituted lower alkyl, and
   X¹ is -O- or -S-, a pharmaceutically acceptable salt or a solvate thereof.
(24) A compound of the formula: 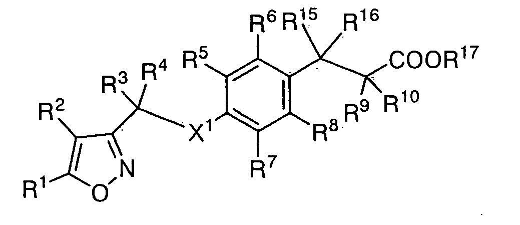 (wherein
   R¹ is halogen, hydroxy, optionally substituted lower alkyl, optionally substituted lower alkenyl, optionally substituted lower alkynyl, optionally substituted lower alkoxy, carboxy, optionally substituted lower alkoxycarbonyl, optionally substituted lower alkylthio, optionally substituted acyl, optionally substituted, amino, optionally substituted carbamoyl, optionally substituted thiocarbamoyl, optionally substituted carbamoyloxy, optionally substituted thiocarbamoyloxy, optionally substituted hydrazinocarbonyl, optionally substituted lower alkylsulfonyloxy, optionally substituted arylsulfonyloxy, optionally substituted aryl, optionally substituted aryloxy, optionally substituted arylthio or optionally substituted heterocycle,
   R² is hydrogen, halogen, hydroxy, optionally substituted lower alkyl, optionally substituted lower alkenyl, optionally substituted lower alkynyl, optionally substituted lower alkoxy, carboxy, optionally substituted lower alkoxycarbonyl, optionally substituted lower alkylthio, optionally substituted acyl, optionally substituted amino, optionally substituted carbamoyl, optionally substituted thiocarbamoyl, optionally substituted carbamoyloxy, optionally substituted thiocarbamoyloxy, optionally substituted hydrazinocarbonyl, optionally substituted lower alkylsulfonyloxy, optionally substituted arylsulfonyloxy, optionally substituted aryl, optionally substituted aryloxy, optionally substituted arylthio or optionally substituted heterocycle,
   R³ and R⁴ are each independently hydrogen, halogen, optionally substituted lower alkyl, optionally substituted lower alkenyl, optionally substituted lower alkynyl, optionally substituted aryl or optionally substituted heterocycle,
   R⁵, R⁶, R⁷ and R⁸ are each independently hydrogen, halogen, hydroxy, optionally substituted lower alkyl, optionally substituted lower alkenyl, optionally substituted lower alkynyl, optionally substituted lower alkoxy, optionally substituted lower alkylthio, optionally substituted acyl, optionally substituted amino, optionally substituted aryl, optionally substituted aryloxy, optionally substituted arylthio or optionally substituted heterocycle,
   R⁹ and R¹⁰ are hydrogen,
   X¹ is -O-, -S-, -NR¹¹- (wherein R¹¹ is hydrogen, optionally substituted lower alkyl, optionally substituted acyl, optionally substituted lower alkylsulfonyl or optionally substituted arylsulfonyl), -CR¹²R¹³CO-, -(CR¹²R¹³)mO-, -(CR¹²R¹³)mS- or -O(CR¹²R¹³)m-(wherein R¹² and R¹³ are each independently hydrogen or lower alkyl and m is an integer between 1 and 3),
   R¹⁵ is lower alkyl,
   R¹⁶ is hydrogen, and
   R¹⁷ is hydrogen or lower alkyl)
   a pharmaceutically acceptable salt or a solvate thereof.
(25) The compound of (24) wherein R¹ is optionally substituted aryl,
   R² is optionally substituted lower alkyl,
   R³ and R⁴ are hydrogen,
   R⁵, R⁶, R⁷ and R⁸ are each independently hydrogen, halogen, optionally substituted lower alkyl or optionally substituted lower alkoxy, and
   X¹ is -O- or -S-,
   a pharmaceutically acceptable salt or a solvate thereof.
(26) A pharmaceutical composition comprising a compound, a pharmaceutically acceptable salt or a solvate thereof of any one of (1) - (25),
(27) A pharmaceutical composition as peroxisome proliferator-activated receptors agonists, which comprises a compound, a pliarmaceutically acceptable salt or a solvate thereof of any one of (1) - (25) as active ingredient.

Furthermore, the present invention includes the below.
(X1) A compound of the formula (I): 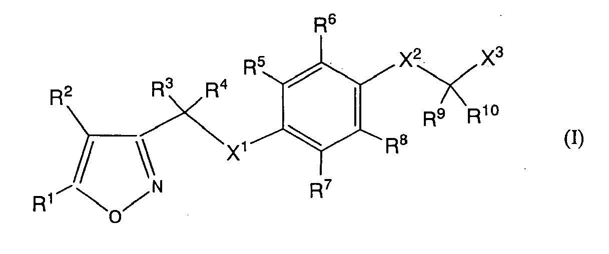 (wherein
   R¹ and R² are each independently hydrogen, halogen, hydroxy, optionally substituted lower alkyl, optionally substituted lower alkenyl, optionally substituted lower alkynyl, optionally substituted lower alkoxy, carboxy, optionally substituted lower alkoxycarbonyl; optionally substituted lower alkylthio, optionally substituted acyl, optionally substituted amino, optionally substituted carbamoyl, optionally substituted thiocarbamoyl, optionally substituted carbamoyloxy, optionally substituted thiocarbamoyloxy, optionally substituted hydrazinocarbonyl, optionally substituted lower alkylsulfonyloxy, optionally substituted arylsulfonyloxy, optionally substituted aryl, optionally substituted aryloxy, optionally substituted arylthio or optionally substituted heterocycle,
   R³ and R⁴ are each independently, hydrogen, halogen, optionally substituted lower alkyl, optionally substituted lower alkenyl, optionally substituted lower alkynyl, optionally substituted aryl or optionally substituted heterocycle,
   R⁵, R⁶, R⁷ and R⁸ are each independently hydrogen, halogen, hydroxy, optionally substituted lower alkyl, optionally substituted lower alkenyl, optionally substituted lower alkynyl, optionally substituted lower alkoxy, optionally substituted lower alkylthio, optionally substituted acyl, optionally substituted amino, optionally substituted aryl, optionally substituted aryloxy, optionally substituted arylthio or optionally substituted heterocycle,
   R⁹ and R¹⁰ are each independently hydrogen, halogen, optionally substituted lower alkyl, optionally substituted lower alkoxy, optionally substituted amino or optionally substituted aryl, R⁹ and R¹⁶ can be joined together to form a bond,
   X¹ is -O-, -S-, -NR¹¹- (wherein R¹¹ is hydrogen, optionally substituted lower alkyl, optionally substituted acyl, optionally substituted lower alkylsulfonyl or optionally substituted arylsulfonyl), -CR¹²R¹³CO-, -(CR¹²R¹³)mO- or -O(CR¹²R¹³)m- (wherein R¹² and R¹³ are each independently hydrogen or lower alkyl and m is an integer between 1 and 3),
   X² is a bond, -O-, -S-, -NR¹⁴- (wherein R¹⁴ is hydrogen, optionally substituted lower alkyl, optionally substituted acyl, optionally substituted lower alkylsulfonyl or optionally substituted arylsulfonyl) or -CR¹⁵R¹⁶- (wherein R¹⁵ and R¹⁶ are each independently hydrogen or lower alkyl, R¹⁶ and R⁹ can be joined together to form a bond), and
   X³ is COOR¹⁷, C(=NR¹⁷)NR¹⁸OR¹⁹, 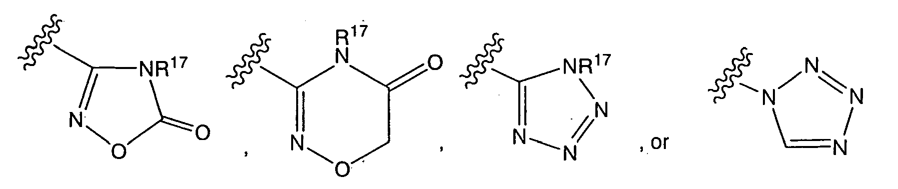 (wherein R¹⁷ - R¹⁹ are each independently hydrogen or lower alkyl))
   a prodrug, a pharmaceutically acceptable salt or a solvate thereof.
(X2) The compound of (X1) wherein R¹ is halogen, optionally substituted lower alkyl, optiorially substituted aryl or optionally substituted heterocycle, a prodrug, a pharmaceutically acceptable salt or a solvate thereof.
(X3) The compound of (X1) wherein R² is hydrogen, halogen, optionally substituted lower alkyl, optionally substituted lower alkenyl, optionally substituted alkynyl, optionally substituted lower alkoxy, optionally substituted acyl, optionally substituted aryl or optionally substituted arylthio, a prodrug, a pharmaceutically acceptable salt or a solvate thereof.
(X4) The compound of (X1) wherein R³ and R⁴ are hydrogen, a prodrug, a pharmaceutically acceptable salt or a solvate thereof.
(X5) The compound of (X1) wherein R⁵ and R⁶ are each independently hydrogen, halogen, optionally substituted lower alkyl or optionally substituted lower alkoxy and R⁷ and R⁸ are hydrogen, a prodrug, a pharmaceutically acceptable salt or a solvate thereof.
(X6) The compound of (X1) wherein R⁹ and R¹⁰ are hydrogen, a prodrug, a pharmaceutically acceptable salt or a solvate thereof.
(X7) The compound of (X1) wherein X¹ is O, S, NR¹¹ (wherein R¹¹ is hydrogen or optionally substituted lower alkyl) or CH₂CO, a prodrug, a pharmaceutically acceptable salt or a solvate thereof.
(X8) The compound of (X1) wherein X² is a bond or O; a prodrug, a pharmaceutically acceptable salt or a solvate thereof.
(X9) The compound of (X1) wherein X³ is carboxy, a prodrug, a pharmaceutically acceptable salt or a solvate thereof.
(X10) A pharmaceutical composition comprising a compound, a pharmaceutically acceptable salt or a solvate thereof of any one of (X1) - (X9).
(X11) A pharmaceutical composition as peroxisome proliferator-activated receptors agonists, which comprises a compound, a pharmaceutically acceptable salt or a solvate thereof of any one of (X1) - (X9) as active ingredient.
(preferably provided that, a compound wherein X³ is COOR¹⁷, X² is -CR¹⁵R¹⁶⁻, and R¹⁶ is hydrogen or lower alkyl is excluded from the above compounds.)

Furthermore, the present invention provides a method for PPAR activation characterized by administrating the above compound, a pharmaceutically acceptable salt or a solvate thereof. In details, it is the treatment method and/or prevention method for hyperlipidemia, diabetes, obesity, arteriosclerosis, atherosclerosis, hyperglycemia and/or syndrome X.

As the other embodiment, the present invention provides the medicine for PPAR activation. In details, it is use of a compound (I), a pharmaceutically acceptable salt or a solvate thereof to produce medicines for treatment and/or prevention for hyperlipidemia, diabetes, obesity, arteriosclerosis, atherosclerosis, hyperglycemia and/or syndrome X.

### The effect of the invention

As the following test results show, compounds of the present invention have PPAR agonist activity and are very useful as medicine and especially medicine for treatment and/or prevention for hyperlipidemia, diabetes, obesity, arteriosclerosis, atherosclerosis, hyperglycemia and/or syndrome X.

### Best Mode for Carrying out the Invention

The term "halogen" in the present specification means fluorine, chlorine, bromine or iodine. Especially, fluorine or chlorine is preferable.

The term "lower alkyl" means a C1-C10, preferably C1-C6 and more preferably C1-C3 straight or branched alkyl group, for example, methyl, ethyl, n-propyl, isopropyl, n-butyl, isobutyl, sec-buthyl, tert-butyl, n-pentyl, isopentyl, neopentyl, hexyl, isohexyl, n-heptyl, isoheptyl, n-octyl, isooctyl, n-nonyl, n-decyl or the like.

The term "lower alkenyl" means C2-C10 having one or more double bonds at optional positions, preferably C2-C6 and more preferably C2-C4 straight or branched alkenyl having one or more double bonds. For example, it is vinyl, propenyl, isopropenyl, butenyl, isobutenyl, prenyl, butadienyl, pentenyl, isopentenyl, pentadienyl, hexenyl, isohexenyl, hexadienyl, heptenyl, octenyl, nonenyl, decenyl or the like.

The term "lower alkynyl" means C2-C10, preferably C2-C6 and more preferably C2-C4 straight or branched alkynyl, for example, ethynyl, propynyl, butynyl, pentynyl, hexynyl, heptynyl, octynyl, nonynyl, decenyl or the like. These have one or more triple bonds at optional positions and can have double bonds.

A substituent of "optionally substituted lower alkyl", "optionally substituted lower alkenyl" or "optionally substituted lower alkynyl" is halogen, hydroxy, optionally substituted lower alkoxy, amino, lower alkylamino, arylainino, heterocycleamino, acylamino, lower alkoxycarbonylamino, mercapto, lower alkylthio, acyl, acyloxy, optionally substituted imino, carboxy, lower alkoxycarbonyl, carbamoyl, lower alkyl carbamoyl, thiocarbamoyl, lower alkylthiocarbamoyl, carbamoyloxy, lower alkylcarbamoyloxy, thiocarbamoyloxy, lower alkylthiocarbamoyloxy, sulfamoyl, lower alkylsulfamoyl, lower alkylsulfonyl, lower alkylsulfonyloxy, cyano, nitro, cycloalkyl, cycloalkyloxy, optionally substituted aryl, optionally substituted aryloxy, optionally substituted arylthio, optionally substituted aryl lower alkoxy, optionally substituted arylsulfonyloxy or optionally substituted heterocycle (wherein a substituent is halogen, hydroxy, lower alkyl, halogeno lower alkyl, hydroxy lower alkyl, lower alkenyl, lower alkoxy, aryl lower alkoxy, halogeno lower alkoxy, carboxy, lower alkoxycarbonyl, carbamoyl, lower alkylcarbamoyl, arylcarbamoyl, acylamino, mercapto, lower alkylthio, amino, lower alkylamino, acyl, acyloxy, cyano, nitro; phenyl, heterocycle or the like). They can be substituted at optional positions with one ore more substituents selected from the above.

A substituent of "optionally substituted lower alkyl", "optionally substituted lower alkenyl", "optionally substituted lower alkynyl" or the like is preferably morpholino, piperidino, piperazino, furyl, thienyl or pyridyl.

Lower alkyl part of "halogeno lower alkyl", "hydroxy lower alkyl", "lower alkoxy", "halogeno lower alkoxy", "aryl lower alkoxy", "hydroxy lower alkoxy", lower alkylamino", "lower alkylthio", "lower alkylsulfonyl", "lower alkylsulfonyloxy", "lower alkyl carbamoyl", "lower alkylthio carbamoyl", "lower alkyl carbamoyloxy", "lower alkylthio carbamoyloxy", "lower alkyl sulfamoyl", "lower alkoxycarbonyl" or "lower alkoxycarbonyl amino" is same as the above "lower alkyl".

A substituent of "optionally substituted lower alkoxy", "optionally substituted lower alkoxycarbonyl", "optionally substituted lower alkylthio", "optionally substituted lower alkylsulfonyloxy" or "optionally substituted imino" is same as a substituent of the above "optionally substituted lower alkyl".

The term "acyl" includes (a) C1-C10, more preferably C1-C6 and most preferably C1-C3 straight or branched alkylcarbonyl or alkenyl carbonyl, (b) C4-C9 and preferably C4-C7 cycloalkylcarbonyl, (c) C7-C11 arylcarbonyl or (d) formyl. For example, it is formyl, acetyl, propionyl, butyryl, isobutyryl, valeryl, pivaloyl, hexanoyl, acryloyl, propioloyl, methacryloyl, crotonoyl, cyclopropyl carbonyl, cyclohexyl carbonyl, cyclooctyl carbonyl, benzoyl or the like.

Acyl part of "acyl amino" or "acyloxy" is same as the above "acyl".

A substituent of "optionally substituted acyl" is same as a substituent of the above "optionally substituted lower alkyl". Furthermore, cycloalkyl carbonyl and aryl carbonyl can be substituted with lower alkyl, halogeno lower alkyl, hydroxy lower alkyl, lower alkenyl, halogeno lower alkenyl and/or hydroxy lower alkenyl.

A substituent of "optionally substituted amino" is same as the above "optionally substituted lower alkyl". Furthermore, "optionally substituted amino" can be substituted with lower alkyl, halogeno lower alkyl, hydroxy lower alkyl, lower alkenyl, halogeno lower alkenyl and/or hydroxy lower alkenyl.

A substituent of "optionally substituted carbamoyl", "optionally substituted thiocarbamoyl", "optionally substituted carbamoyloxy", "optionally substituted thiocarbamoyloxy" or "optionally substituted hydrazinocarbonyl" is same as the above "optionally substituted lower alkyl".

The term "cycloalkyl" includes C3-C8 and preferably C5 or C6 cyclic alkyl. For example, it is cyclopropyl, cyclobutyl, cyclopentyl, cyclohexyl, cycloheptyl, cycloctyl or the like.

"Aryl" includes phenyl, naphthyl, anthryl, phenanthryl or the like. Additionally, it includes aryl, which is condensed with the other non-aromatic hydrocarbon ring, for example, indanyl, indenyl, biphenylyl, acenaphthenyl, fluorenyl or the like. In case that aryl is condensed with the other non-aromatic hydrocarbon ring, bonds can be attached to any of the rings. The preferable example of aryl is phenyl.

A substituent of "optionally substituted aryl" is same as a substituent of the above "optionally substituted lower alkyl" as long as there is not a special provision. Furthermore, it can be substituted with lower alkyl, halogeno lower alkyl, hydroxy lower alkyl, lower alkenyl, halogeno lower alkenyl, hydroxy lower alkenyl, alkylenedioxy and/or oxo.

Aryl part of "aryloxy", "arylthio", "aryl lower alkoxy", "aryl amino" or "arylsulfonyloxy" is same as the above "aryl".

A substituent of "optionally substituted aryloxy", "optionally substituted arylthio" or "optionally substituted arylsulfonyloxy" is same as a substituent of the above "optionally substituted aryl" as long as there is not a special provision.

"Heterocycle" includes heterocycle having 1 or more hetero atom(s) selected from O, S and N in a ring, for example, 5-6 membered heteroaryl such as pyrrolyl, imidazolyl, pyrazolyl, pyridyl, pyridazinyl, pyrimidinyl, pyradinyl, triazolyl, triazinyl, tetrazolyl, isoxazolyl, oxazolyl, oxadiazolyl, isothiazolyl, thiazolyl, thiadiazolyl, furyl, thienyl or the like; bicyclic condensed heterocycle such as indolyl, isoindolyl, indazolyl, indolizinyl, quinolyl, isoquinolyl, cinnolinyl, phthalazinyl, quinazolinyl, naphthyridinyl, quinoxalinyl, prinyl, pteridinyl, benzopyranyl, benzimidazolyl, benzisoxazolyl, berizoxazolyl; benzoxadiazolyl, benzoisothiazolyl, benzothiazolyl, benzothiadiazolyl, benzofuryl, isobenzofuryl, benzothienyl, benzotriazolyl, imidazopyridyl, triazolopyridyl, imidazothiazolyl, pyradino pyridazinyl, quinazolinyl, tetrahydroquinolyl, tetrahydrobenzothienyl or the like; tricyclic condensed heterocycle such as carbazolyl, acridinyl, xanthenyl, phenothiazinyl, phenoxathiinyl, phenoxazinyl, dibenzofuryl or the like; non-aromatic heterocycle such as indolinyl, dioxanyl, thiiranyl, oxyranyl, oxathiolanyl, azetidinyl, thianyl, pyrrolidinyl, pyrrolinyl, imidazolidinyl, imidazolinyl, pyrazolidinyl, pyrazolinyl, piperidyl, piperidino, piperazinyl, piperidino, morpholinyl, morpholino, oxadiadinyl, dihydropyridyl or the like. In case that heterocycle is a condensed ring, the bonds can be attached to any of the rings.

As "heterocycle" for R¹ and R², pyridyl, morpholino or piperazino or piperidino is preferred.

A substituent of "optionally substituted heterocycle" is same as the above "optionally substituted aryl".

Heterocycle part of "heterocycle amino" is same as the above "heterocycle".

"R⁶ and R¹⁴ can be taken together with the neighboring atom to form a ring" or "R¹⁴ and R⁶ can be taken together with the neighboring atom to form a ring" means that R¹⁴ and R⁶ form a 4-7 membered ring having 1-3 hetero atom(s) which is condensed to benzene ring of formula (I). The preferable example of condensed heterocycle with benzene ring is optionally substituted bicyclic heterocycle, for example, indole, benzimidazole, 1H-indazole, 2,3-dihydroindole, 1,2,3,4-tetrahydroquinoline, 2,3-dihydro1,4-benzoxazin, 2,3-dihydrobenzthiazole, 2,3-dihydrobenzoxazole, 1,2-dihydroquinoline, 1,4-dihydroquinoline or the like. The substituent of "optionally substituted bicyclic heterocycle" is the same substituent as a substituent on benzene ring of formula (I) or oxo group. The substituent is, for example, halogen, hydroxy, optionally substituted lower alkyl, optionally substituted lower alkenyl, optionally substituted lower alkynyl, optionally substituted lower alkoxy, optionally substituted lower alkylthio, optionally substituted acyl, optionally substituted amino, optionally substituted aryl, optionally substituted aryloxy, optionally substituted aryl thio, optionally substituted heterocycle or oxo. As the substituent of heterocycle condensed to benzene ring, oxo, halogen, hydroxy, optionally substituted lower alkoxy, optionally substituted lower alkylthio or optionally substituted lower alkyl is especially preferable.

The preferable example of "optionally substituted heterocycle" is, 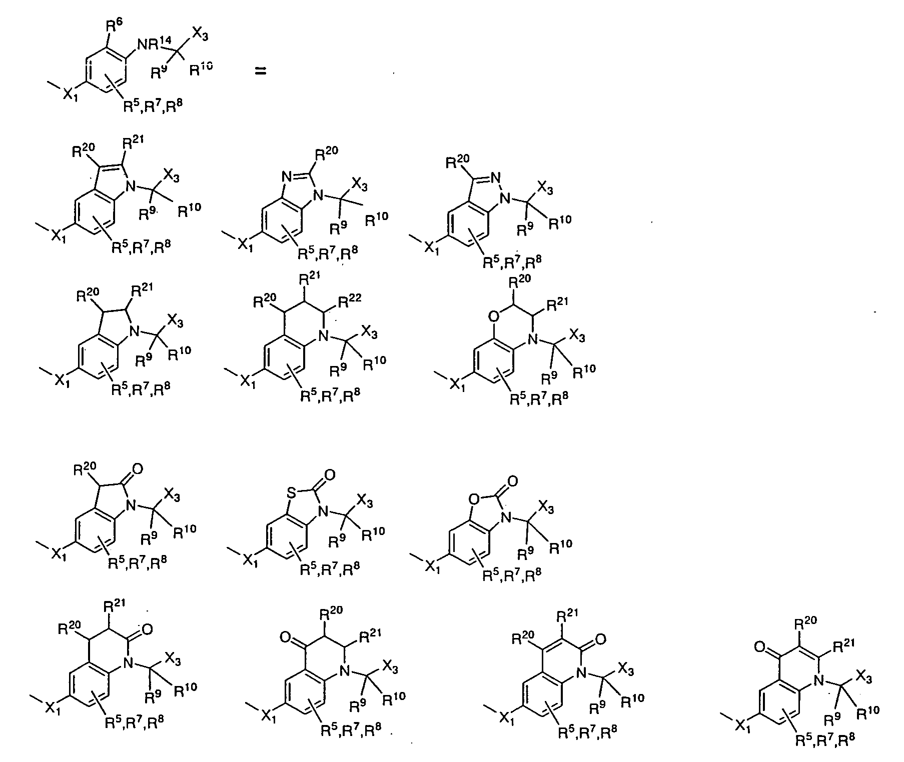 (wherein
R⁵, R⁷, R⁸ are each independently hydrogen, halogen, hydroxy, optionally substituted lower alkyl, optionally substituted lower alkenyl, optionally substituted lower alkynyl, optionally substituted lower alkoxy, optionally substituted lower alkylthio, optionally substituted acyl, optionally substituted amino, optionally substituted aryl, optionally substituted aryloxy, optionally substituted arylthio or optionally substituted heterocycle,
R⁹ and R¹⁰ are each independently hydrogen, halogen, cyano, optionally substituted lower alkyl, optionally substituted lower alkoxy, optionally substituted amino or optionally substituted aryl,
R²⁰ - R²² are each independently hydrogen, halogen, hydroxy, cyano, optionally substituted lower alkyl, optionally substituted lower alkenyl, optionally substituted lower alkynyl, optionally substituted lower alkoxy, optionally substituted lower alkylthio, optionally substituted acyl, optionally substituted amino, optionally substituted imino, optionally substituted aryl, optionally substituted aryloxy, optionally substituted arylthio or optionally substituted heterocycle,
X¹ is -O-, -S-, -NR¹¹- (wherein R¹¹ is hydrogen, optionally substituted lower alkyl, optionally substituted acyl, optionally substituted lower alkylsulfonyl or optionally substituted arylsulfonyl), -CR¹²R¹³CO-, -(CR¹²R¹³)mO-, -(CR¹²R¹³)mS- or -O(CR¹²R¹³)m- (wherein R¹² and R¹³ are each independently hydrogen or lower alkyl, m is an integer between 1 and 3) (-O- or -S- is preferable and -S- is especially preferable),
X³ is COOR¹⁷ (wherein R¹⁷ is hydrogen or lower alkyl)).

"R⁶, R⁹ and R¹⁰ can be taken together with the neighboring carbon atom to form a ring" or "R⁹, R¹⁰ and R⁶ can be taken together with the neighboring carbon atom to form a ring" means that R⁶, R⁹ and R¹⁰ form a 4-7 membered ring having 0-3 hetero atom(s) which is condensed to benzene ring of formula (I). The preferable example of condensed ring with benzene ring is optionally substituted C8-C11 carbon ring (especially optionally substituted naphthalene) or optionally substituted bicyclic heterocycle. For example, it is indole, benzothiophene, benzofuran, benzoisoxazole, 1H-indazole, naphthalene, quinazoline, isoquinoline, 2H-chromene, 1,4-dihydronaphthalene, 1,2,3,4-tetrahydronaphthalene or the like. The substituent of "optionally substituted C8-C11 carbon ring (especially optionally substituted naphthalene)" or "optionally substituted bicyclic heterocycle" is the same substituent as a substituent on benzene ring of formula (I) or oxo group. The substituent is, for example, halogen, hydroxy, optionally substituted lower alkyl, optionally substituted lower alkenyl, optionally substituted lower alkynyl, optionally substituted lower alkoxy, optionally substituted lower alkylthio, optionally substituted acyl, optionally substituted amino, optionally substituted aryl, optionally substituted aryloxy, optionally substituted aryl thio, optionally substituted heterocycle or oxo. Especially, the substituent on heterocycle condensed to benzene ring is oxo, halogen, hydroxy, optionally substituted lower alkoxy or optionally substituted lower alkylthio. Optionally substituted lower alkyl is preferable.

The preferable example of "optionally substituted C8-C11 carbon ring (especially optionally substituted naphthalene)" or "optionally substituted bicyclic heterocycle" is, 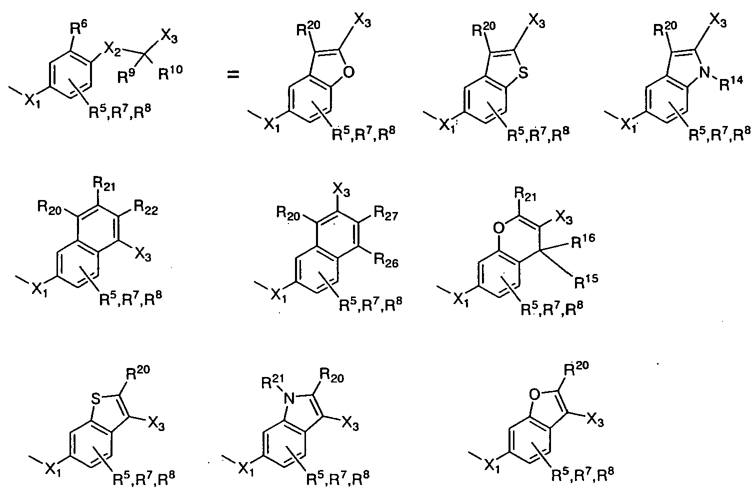 (wherein
R⁵, R⁷, R⁸ and R²⁰-R²² are each independently hydrogen, halogen, hydroxy, optionally substituted lower alkyl, optionally substituted lower alkenyl, optionally substituted lower alkynyl, optionally substituted lower alkoxy, optionally substituted lower alkylthio, optionally substituted acyl, optionally substituted amino, optionally substituted aryl, optionally substituted aryloxy, optionally substituted arylthio or optionally substituted heterocycle,
X¹ is -O-, -S-, -NR¹¹- (wherein R¹¹ is hydrogen, optionally substituted lower alkyl, optionally substituted acyl, optionally substituted lower alkylsulfonyl or optionally substituted arylsulfonyl), -CR¹²R¹³CO-, -(CR¹²R¹³)mO-, -(CR¹²R¹³)mS- or -O(CR¹²R¹³)m- (wherein R¹² and R¹³ are each independently hydrogen or lower alkyl and m is an integer between 1 and 3) (-O- or -S- is preferable and -S- is especially preferable),
R¹⁴ is hydrogen, optionally substituted lower alkyl, optionally substituted acyl, optionally substituted lower alkylsulfonyl or optionally substituted arylsulfonyl,
R¹⁵, R¹⁶, R²⁶ and R²⁷ are each independently hydrogen or lower alkyl,
X³ is COOR¹⁷ (wherein R¹⁷ is hydrogen or lower alkyl)).
"R⁶ and R⁹ can be taken together with the neighboring carbon atom to form a ring" or "R⁹ and R⁶ can be taken together with the neighboring carbon atom to form a ring" means that R⁶ and R⁹ form a 4-7 membered ring having 0-3 hetero atom(s) which is condensed to benzene ring of formula (I). The preferable example of condensed heterocycle with benzene ring is optionally substituted C8-C11 carbon ring (especially optionally substituted naphthalene) or optionally substituted bicyclic heterocycle. The substituent of "optionally substituted C8-C11 carbon ring (especially optionally substituted naphthalene)" or "optionally substituted bicyclic heterocycle" is the same substituent as a substituent on benzene ring of formula (I) or oxo group. The substituent is, for example, halogen, hydroxy, optionally substituted lower alkyl, optionally substituted lower alkenyl, optionally substituted lower alkynyl, optionally substituted lower alkoxy, optionally substituted lower alkylthio, optionally substituted acyl, optionally substituted amino, optionally substituted aryl, optionally substituted aryloxy, optionally substituted aryl thio, optionally substituted heterocycle or oxo. As the substituent of heterocycle condensed to benzene ring, oxo, halogen, hydroxy, optionally substituted lower alkoxy, optionally substituted lower alkylthio or optionally substituted lower alkyl is especially preferable.

The preferable example of "optionally substituted C8-C11 carbon ring (especially optionally substituted naphthalene)" or "optionally substituted bicyclic heterocycle" is, 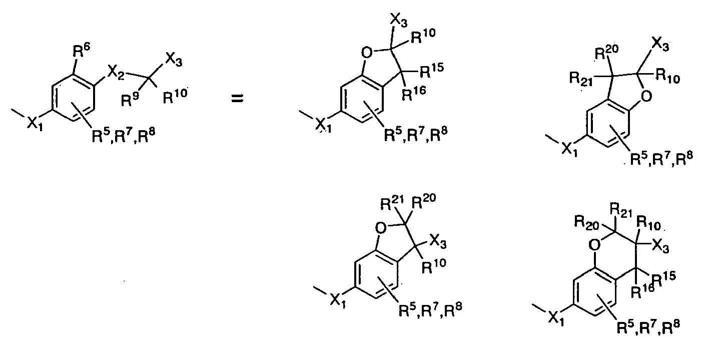 (wherein
R⁵, R⁷, R⁸, R²⁰ and R²¹ are each independently hydrogen, halogen, hydroxy, optionally substituted lower alkyl, optionally substituted lower alkenyl, optionally substituted lower alkynyl, optionally substituted lower alkoxy, optionally substituted lower alkylthio, optionally substituted acyl, optionally substituted amino, optionally substituted aryl, optionally substituted aryloxy, optionally substituted arylthio or optionally substituted heterocycle,
R¹⁰ is hydrogen, halogen, cyano, optionally substituted lower alkyl, optionally substituted lower alkoxy, optionally substituted amino or optionally substituted aryl, X¹ is -O-, -S-, -NR¹¹- (wherein R¹¹ is hydrogen, optionally substituted lower alkyl, optionally substituted acyl, optionally substituted lower alkylsulfonyl or optionally substituted arylsulfonyl), -CR¹²R¹³CO-, -(CR¹²R¹³)mO-, -(CR¹²R¹³)mS- or -O(CR¹²R¹³)m- (wherein R¹² and R¹³ are each independently hydrogen or lower alkyl, m is an integer between 1 and 3) (-O- or -S- is preferable and -S- is especially preferable),
R¹⁵ and R¹⁶ are each independently hydrogen or lower alkyl,
X³ is COOR¹⁷ (wherein R¹⁷ is hydrogen or lower alkyl)).

"R⁶, R¹⁵ and R¹⁶ can be taken together with the neighboring carbon atom to form a ring" or "R¹⁵, R¹⁶ and R⁶ can be taken together with the neighboring carbon atom to form a ring" means that R⁶, R¹⁵ and R¹⁶ form a 4-7 membered ring having 0-3 hetero atom(s) which is condensed to benzene ring of formula (I). The preferable example of condensed heterocycle with benzene ring is optionally substituted C8-C11 carbon ring (especially, optionally substituted naphthalene) or optionally substituted bicyclic heterocycle. For example, it is indole, benzothiophene, benzofuran, benzoisoxazole, 1H-indazole, naphthalene, quinazoline, isoquinoline, 2H-chromene, 1,4-dihydronaphthalene, 1,2,3,4-tetrahydronaphthalene or the like. The substituent of "optionally substituted C8-C11 carbon ring (especially optionally substituted naphthalene)" or "optionally substituted bicyclic heterocycle" is same substituent as a substituent on benzene ring of formula (I) or oxo group. The substituent is, for example, halogen, hydroxy, optionally substituted lower alkyl, optionally substituted lower alkenyl, optionally substituted lower alkynyl, optionally substituted lower alkoxy, optionally substituted lower alkylthio, optionally substituted acyl, optionally substituted amino, optionally substituted aryl, optionally substituted aryloxy, optionally substituted aryl thio, optionally substituted heterocycle or oxo. As the substituent on heterocycle condensed to benzene ring, oxo, halogen, hydroxy, optionally substituted lower alkoxy, optionally substituted lower alkylthio or optionally substituted lower alkyl is especially preferable.

The preferable example of "optionally substituted C8-C11 carbon ring (especially, optionally substituted naphthalene)" or "optionally substituted bicyclic heterocycle" is, 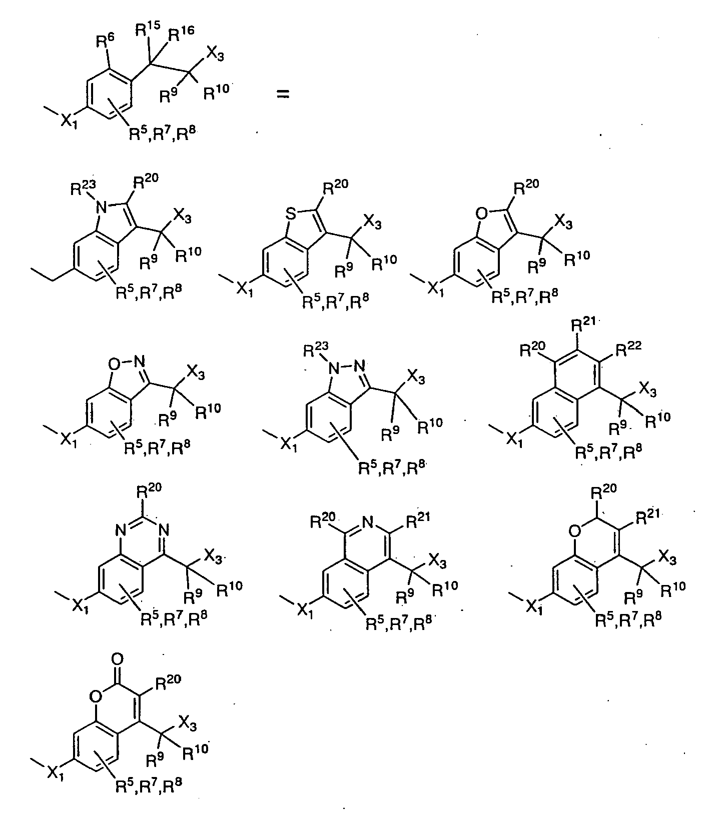 (wherein
R⁵, R⁷, R⁸ and R²⁰-R²² are each independently hydrogen, halogen, hydroxy, optionally substituted lower alkyl; optionally substituted lower alkenyl, optionally substituted lower alkynyl, optionally substituted lower alkoxy, optionally substituted lower alkylthio, optionally substituted acyl, optionally substituted amino, optionally substituted aryl, optionally substituted aryloxy, optionally substituted arylthio or optionally substituted heterocycle,
R⁹ and R¹⁰ are each independently hydrogen, halogen, cyano, optionally substituted lower alkyl, optionally substituted lower alkoxy, optionally substituted amino or optionally substituted aryl,
R²³ is hydrogen, optionally substituted lower alkyl, optionally substituted lower alkenyl, optionally substituted lower alkynyl, optionally substituted acyl, optionally substituted lower alkylsulfonyl or optionally substituted arylsulfonyl, optionally substituted amino, optionally substituted aryl or optionally substituted heterocycle,
X¹ is -O-, -S-, -NR¹¹- (wherein R¹¹ is hydrogen, optionally substituted lower alkyl, optionally substituted acyl, optionally substituted lower alkylsulfonyl or optionally substituted arylsulfonyl), -CR¹²R¹³CO-, -(CR¹²R¹³)mO-, -(CR¹²R¹³)mS- or -O(CR¹²R¹³)m- (wherein R¹² and R¹³ are each independently hydrogen or lower alkyl and m is an integer between 1 and 3) (-O- or -S- is preferable and -S- is especially preferable),
X³ is COOR¹⁷ (wherein R¹⁷ is hydrogen or lower alkyl)).

"R⁶ and R²⁴ can be taken together with the neighboring carbon atom to form a ring" or "R²⁴ and R⁶ can be taken together with the neighboring carbon atom to form a ring" means that R⁶ and R²⁴ form a 4-7 membered ring having 0-3 hetero atom(s) which is condensed to benzene ring of formula (I). The preferable example of condensed heterocycle with benzene ring is optionally substituted C8-C11 carbon ring or optionally substituted bicyclic heterocycle. The substituent of "optionally substituted C8-C11 carbon ring" or "optionally substituted bicyclic heterocycle" is the same substituent as a substituent on benzene ring of formula (I) or oxo group. The substituent is, for example, halogen, hydroxy, optionally substituted lower alkyl, optionally substituted lower alkenyl, optionally substituted lower alkynyl, optionally substituted lower alkoxy, optionally substituted lower alkylthio, optionally substituted acyl, optionally substituted amino, optionally substituted aryl, optionally substituted aryloxy, optionally substituted aryl thio, optionally substituted heterocycle or oxo. As the substituent of heterocycle condensed to benzene ring, oxo; halogen, hydroxy, optionally substituted lower alkoxy, optionally substituted lower alkylthio or optionally substituted lower alkyl is especially preferable.

The preferable examples of "optionally substituted C8-C11 carbon ring" or "optionally substituted bicyclic heterocycle" is, 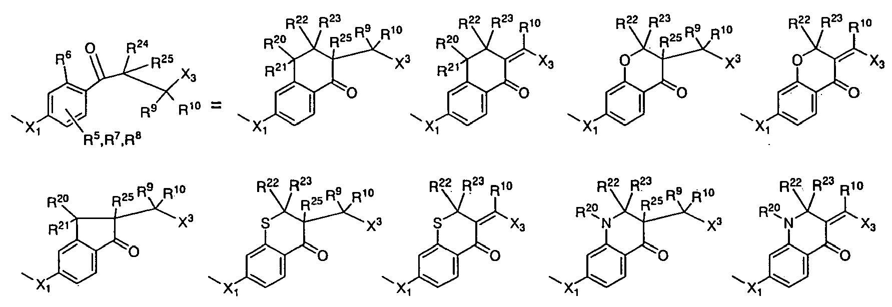 (wherein
R⁵, R⁷, R⁸ and R²⁰-R²³ are each independently hydrogen, halogen, hydroxy, optionally substituted lower alkyl, optionally substituted lower alkenyl, optionally substituted lower alkynyl, optionally substituted lower alkoxy, optionally substituted lower alkylthio, optionally substituted acyl, optionally substituted amino, optionally substituted aryl, optionally substituted aryloxy, optionally substituted arylthio or optionally substituted heterocycle,
R⁹, R¹⁰ and R²⁵ are each independently hydrogen, halogen, cyano, optionally substituted lower alkyl, optionally substituted lower alkoxy, optionally substituted amino or optionally substituted aryl,
X¹ is -O-, -S-, -NR¹¹- (wherein R¹¹ is hydrogen, optionally substituted lower alkyl, optionally substituted acyl, optionally substituted lower alkylsulfonyl or optionally substituted arylsulfonyl), -CR¹²R¹³CO-, -(CR¹²R¹³)mO-, -(CR¹²R¹³)mS- or -O(CR¹²R¹³)m- (wherein R¹² and R¹³ are each independently hydrogen or lower alkyl and m is an integer between 1 and 3) (-O- or -S- is preferable and -S- is especially preferable),
X³ is COOR¹⁷ (wherein R¹⁷ is hydrogen or lower alkyl)).

"R⁹ and R²⁵ can be joined together to form a bond" or "R²⁵ and R⁹ can be joined together to form a bond" means 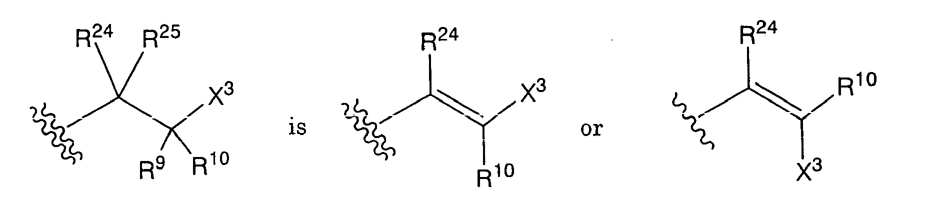 (wherein
R¹⁰ and R²⁴ are each independently hydrogen, halogen, cyano, optionally substituted lower alkyl, optionally substituted lower alkoxy, optionally substituted amino or optionally substituted aryl, and
X³ is COOR¹⁷ (wherein R¹⁷ is hydrogen or lower alkyl)).

"R⁹ and R¹⁰ can be taken together to form a ring" means that R⁹ and R¹⁰ form a 3-7 membered ring with 0-3 hetero atom(s). The preferable example of the ring is optionally substituted C3-C7 carbon monocycle or optionally substituted hetero monocycle. It is, for example, cycloalkane (cyclopropane, cyclobutane, cyclopentane, cyelohexane or cycloheptane), oxan or the like. The substituent of "optionally substituted C3-C7 carbon monocycle (especially optionally substituted three-membered ring)" or "optionally substituted hetero monocycle" is the same substituent as a substituent on benzene ring of formula (I). The substituent is, for example, halogen, hydroxy, optionally substituted lower alkyl, optionally substituted lower alkenyl, optionally substituted lower alkynyl, optionally substituted lower alkoxy, optionally substituted lower alkylthio, optionally substituted acyl, optionally substituted amino, optionally substituted aryl, optionally substituted aryloxy, optionally substituted aryl thio, optionally substituted heterocycle or oxo. Halogen, hydroxy, optionally substituted lower alkoxy, optionally substituted lower alkylthio or optionally substituted lower alkyl is especially preferable.

The preferable example of "optionally substituted C3-C7 carbon monocycle (especially optionally substituted three-membered ring)" or "optionally substituted hetero monocycle" is 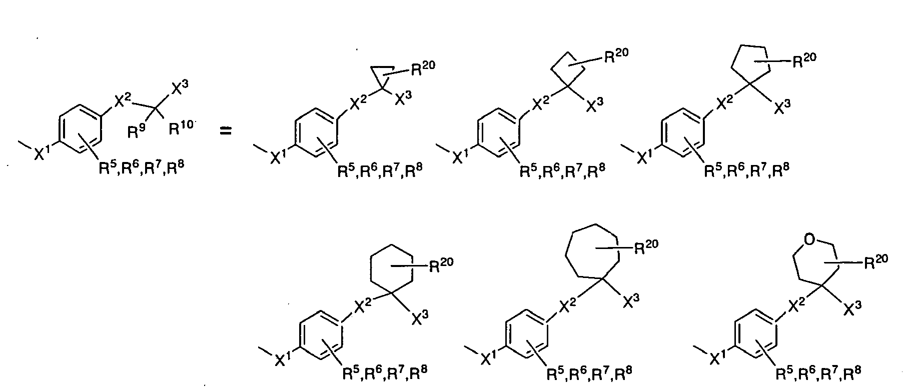 (wherein
R⁵, R⁶, R⁷, R⁸ and R²⁰ are each independently hydrogen, halogen, hydroxy, optionally substituted lower alkyl, optionally substituted lower alkenyl, optionally substituted lower alkynyl, optionally substituted lower alkoxy, optionally substituted lower alkylthio, optionally substituted acyl, optionally substituted amino, optionally substituted aryl, optionally substituted aryloxy, optionally substituted arylthio or optionally substituted heterocycle,
X¹ is -O-, -S-, -NR¹¹- (wherein R¹¹ is hydrogen, optionally substituted lower alkyl, optionally substituted acyl, optionally substituted lower alkylsulfonyl or optionally substituted arylsulfonyl), -CR¹²R¹³CO-, -(CR¹²R¹³)mO-, -(CR¹²R¹³)mS- or -O(CR¹²R¹³)m- (wherein R¹² and R¹³ are each independently hydrogen or lower alkyl and m is an integer between 1 and 3) (-O- or -S- is preferable and -S- is especially preferable),
X² is a bond, -O-, -S-, -SO-, -SO₂-, -C=C-, -NR¹⁴- (wherein R¹⁴ is hydrogen, optionally substituted lower alkyl, optionally substituted acyl, optionally substituted lower alkylsulfonyl or optionally substituted arylsulfonyl), -CR¹⁵R¹⁶- (wherein R¹⁵ and R¹⁶ are each independently hydrogen or lower alkyl) or -COCR²³R²⁴⁻ (wherein R²³ and R²⁴ are each independently hydrogen or lower alkyl) and
X³ is COOR¹⁷ (wherein R¹⁷ is hydrogen or lower alkyl).

"R¹⁰ and R¹⁵ can be taken together with the neighboring carbon atom to form a ring" or "R¹⁵ and R¹⁰ can be taken together with the neighboring carbon atom to form a ring" means that R¹⁵ and R¹⁰ form a 4-7 membered ring having 0-3 heteroatom. The preferable example of the ring is optionally substituted C3-C7 carbon monocycle or optionally substituted hetero monocycle. It is, for example, thiophene, pyrimidine, furan, pyridine, imidazole, isothiazole, isoxazole, pyridazine, pyrazine, thiazole, oxazole or the like.

The case that R¹⁶ and R⁹ are joined together to form a bond or the case that R⁹, R¹⁰ and R¹⁵ can be taken together with the neighboring carbon atom to form a ring is especially preferable. The substituent of "optionally substituted C3-C7 carbon monocycle" or "optionally substituted hetero monocycle" is same as a substituent on benzene ring of formula (I). The substituent is, for example, halogen, hydroxy, optionally substituted lower alkyl, optionally substituted lower alkenyl, optionally substituted lower alkynyl, optionally substituted lower alkoxy, optionally substituted lower alkylthio, optionally substituted acyl, optionally substituted amino, optionally substituted aryl, optionally substituted aryloxy, optionally substituted aryl thio, optionally substituted heterocycle or oxo. Halogen, hydroxy, optionally substituted lower alkoxy, optionally substituted lower alkylthio or optionally substituted lower alkyl is especially preferable.

The preferable example of "optionally substituted C3-C7 carbon monocycle (especially optionally substituted phenyl)" or "optionally substituted hetero monocycle" is, 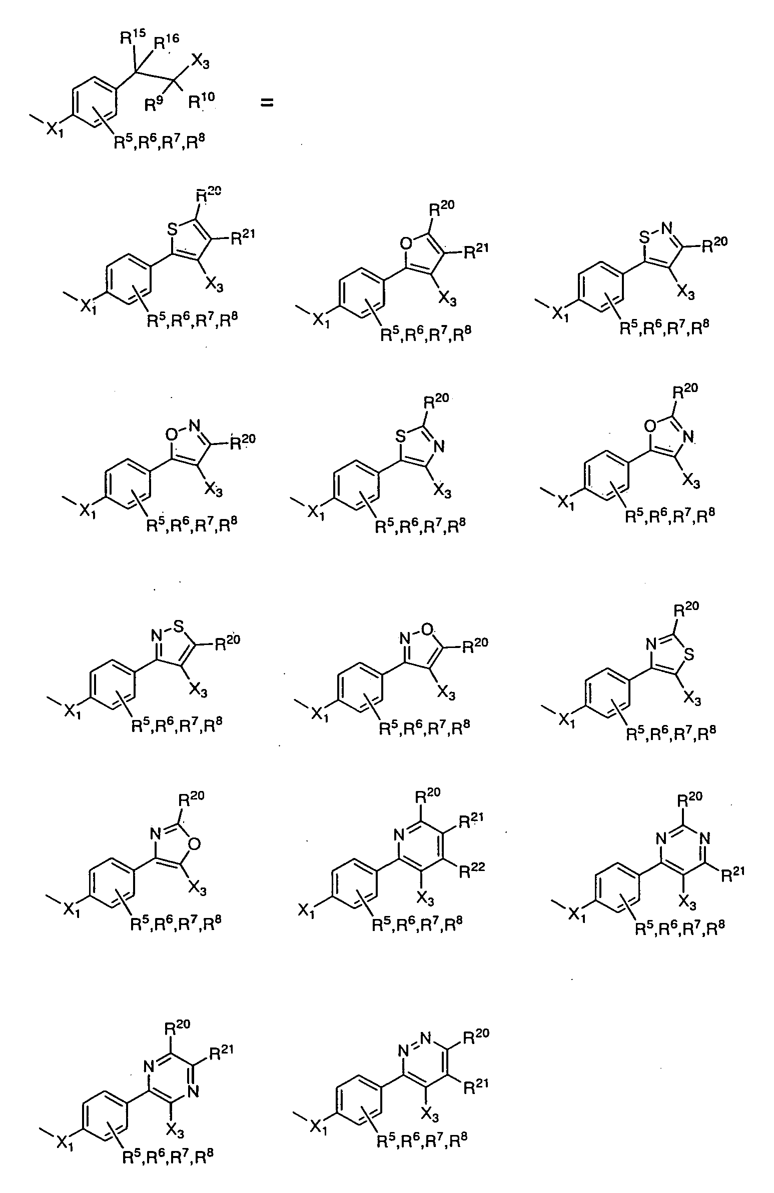 (wherein
R⁵, R⁶, R⁷, R⁸, R²⁰-R²² are each independently hydrogen, halogen, hydroxy, optionally substituted lower alkyl, optionally substituted lower alkenyl, optionally substituted lower alkynyl, optionally substituted lower alkoxy, optionally substituted lower alkylthio, optionally substituted acyl, optionally substituted amino, optionally substituted aryl, optionally substituted aryloxy, optionally substituted arylthio or optionally substituted heterocycle,
X¹ is -O-, -S-, -NR¹¹- (wherein R¹¹ is hydrogen, optionally substituted lower alkyl, optionally substituted acyl, optionally substituted lower alkylsulfonyl or optionally substituted arylsulfonyl), -CR¹²R¹³CO-, -(CR¹²R¹³)mO-, -(CR¹²R¹³)mS- or -O(CR¹²R¹³)m- (wherein R¹² and R¹³ are each independently hydrogen or lower alkyl and m is an integer between 1 and 3) (-O- or -S- is preferable and -S- is especially preferable),
X³ is COOR¹⁷ (wherein R¹⁷ is hydrogen or lower alkyl)).

"R⁹ and R¹⁶ can be joined together to form a bond" or "R¹⁶ and R⁹ can be joined together to form a bond" means 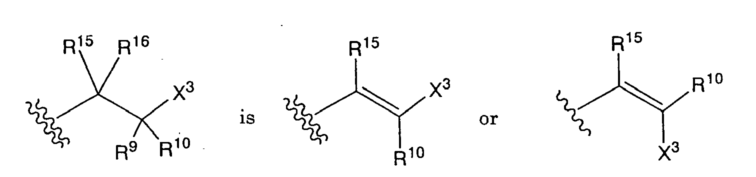 (wherein
R¹⁰ and R¹⁵ are each independently hydrogen, halogen, cyano, optionally substituted lower alkyl, optionally substituted lower alkoxy, optionally substituted amino or optionally substituted aryl, and
X³ is COOR¹⁷ (wherein R¹⁷ is hydrogen or lower alkyl)).

"R¹⁶ and R⁹ are taken together to form a bond and R¹⁵ and R¹⁰ are taken together to form a bond" means that 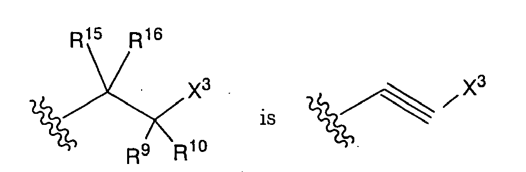 (wherein X³ is COOR¹⁷ (wherein R¹⁷ is hydrogen or lower alkyl)).

A compound of the present invention includes pharmaceutically acceptable salts, which can produce each compound. "A pharmaceutically acceptable salt" includes for example, salts of inorganic acid such as hydrochloric acid, sulfuric acid, nitric acid, phosphoric acid or the like; salts of organic acid such as paratoluenesulfonic acid, methanesulfonic acid, oxalic acid, citric acid or the like; salts of organic salt group such as ammonium, trimethylammonium or triethylammonium; salts of alkali metal such as sodium or potassium; alkaline-earth metal salts such as calcium, magnesium or the like.

A compound of the present invention includes a solvate thereof and can be coordinate any number of solvent molecules to a compound (I). Preferred is hydrate.

When a compound of the present invention (I) has an asymmetric carbon atom, it contained racemic body and all stereoisomers (a diastereoisomer, an antipode or the like). When a compound of the present invention (I) has a double bond and there is geometrical isomer at a substituent position of double bond, it includes both type of the isomers.

Compound (I) of the present invention can be synthesized, for example, by the following methods.

### (Method 1) Synthesis of compound (Ia) (X¹ = O, (CR¹²R¹³)mO, O(CR¹²R¹³)m)

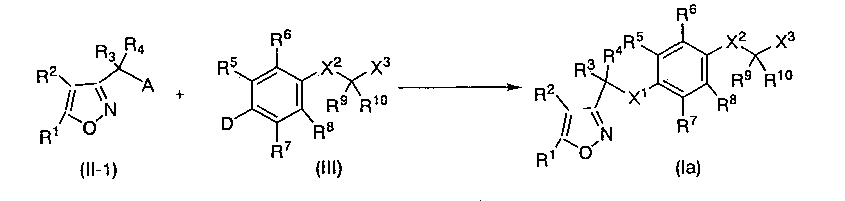

(wherein the one of A and D is OH and another is (CR¹²R¹³)mOH or both A and D are OH, and the other signs are the same meanings as the above.)

Compound (II-1) and compound (III) are subject to Mitsunobu reaction to obtain compound (Ia). Mitsunobu reaction can be performed by a well-known method and preferably performed in a solvent of N,N-dimethyl formamide, dimethyl sulfoxide, aromatic hydrocarbon group (for example, toluene, benzene, xylene or the like), saturated hydrocarbon group (for example, cyelohexane, hexane or the like), halogenated hydrocarbon group (for example, dichloromethane, 1,2-dichloroethane or the like), ether group (for example, tetrahydrofuran, dioxane or the like), ketone group (for example, acetone, methyl ethylketone or the like), nitryl group (for example, acetonitrile or the like), water, a mixed solvent thereof or the like under the presence of azodicarboxylate, amide (diethylazodicarboxylate or the like) or phosphine group such as triphenylphosphine or the like at -30 °C - 150 °C and preferably at 0 °C - 100 °C for 0.5 - 90 hours.

As compound (II-1) and compound (III), well known compounds and compounds, which are lead from well-known compounds by usual methods, can be used.

### (Method 2) Synthesis of compound (Ib) (X¹ = O, S or NR¹¹)

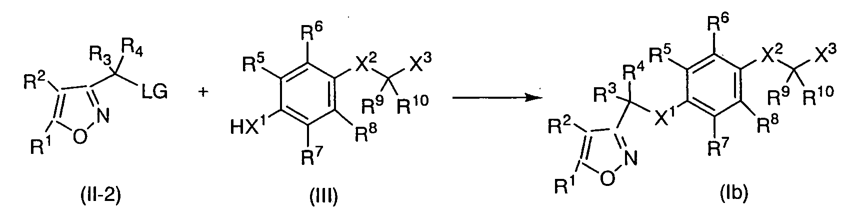

(wherein LG is a leaving group such as halogen, lower alkylsulfonyloxy or the like and the other signs are the same meanings as the above)

Compound (Ib) can be synthesized by reacting compound (II-2) and compound (III). The reaction can be performed in an appropriate solvent under the presence of base at -10 - 180 °C and preferably at 0 - 150 °C for 0.5 - 90 hours. As the solvent, the same solvent described in the above method 1 can be used. The base is, for example, metal hydride (for example, sodium hydride, potassium hydride or the like), metal hydroxide (for example, sodium hydroxide, potassium hydroxide, calcium hydroxide, barium hydroxide or the like), metal carbonate (for example, sodium carbonate, potassium carbonate, calcium carbonate, cesium carbonate or the like), metal alkoxide (for example, sodium methoxide, sodium ethoxide, Potassium tert-butoxide or the like), sodium hydrogen carbonate, metallic sodium, organic amine (triethylamine , DBU or the like) or the like.

As compound (II-2) and compound (III), well known compounds and compounds, which is lead from well-known compounds by usual methods, can be used.

### (Method 3) Syntheses of compound (Ic) (X¹ = CR¹²R¹³CO)

Compound (Ic) can be synthesized by the following route. 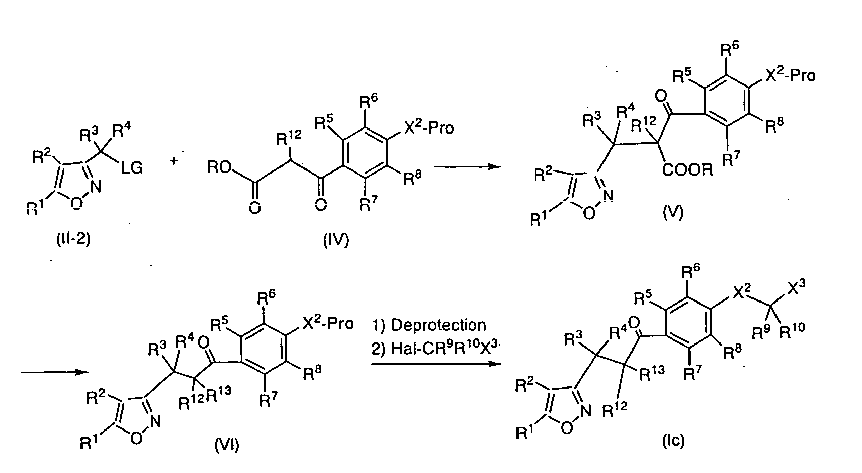 (wherein X² is O, S or NR¹⁴, R is lower alkyl, LG is a leaving group such as halogen, lower alkylsulfonyl or the like, Hal is halogen, Pro is protecting group and the other signs are the same meanings as the above.)

Compound (II-3) and compound (IV) are subject to addition reaction to give compound (V). The reaction can be performed preferably in an appropriate solvent under the presence of base at - 50°C - 150°C and preferably at 20°C - 100°C for 0.5 - 60 hours. The solvent described in the above method 1 can be used as the solvent, and the base described in the above method 2 can be used as the base.

Next, compound (V) is treated with acid to give compound (VI). The reaction can be performed by using the acid such as hydrochloric acid, sulfuric acid in a solvent such as acetic acid, water or the like or without any solvent at 0 °C - 180 °C and preferably at 20°C - 150 °C for 0.5-90 hours. A target compound wherein R¹³ is hydrogen can be obtained in this process. A target compound wherein R¹³ is optionally substituted lower alkyl can be obtained by alkylating with the usual method in an appropriate step, after this process or after the next process or the like.

Finally, phenol compound obtained by deprotection of compound (VI) and a halogen compound are reacted to give target compound (Ic). Deprotection can be performed by the usual method. The reaction can be performed with correspond halogen compound having CR⁹R¹⁰X³ group under the presence of the base in an appropriate solvent at -10 - 180 °C and preferably at 0 - 150 °C for 0.5 - 90 hours. The solvent described in the above method 1 can be used as the solvent. The base described in the above method 2 can be used as the base. As compound (II-3) and compound (VI), well known compounds and compounds, which is lead from well-known compounds by usual methods, can be used.

### (Method 4) Syntheses of compound (Id) (X³ = C( = NH)NHOH)

Compound (Id) is synthesized by the following method. 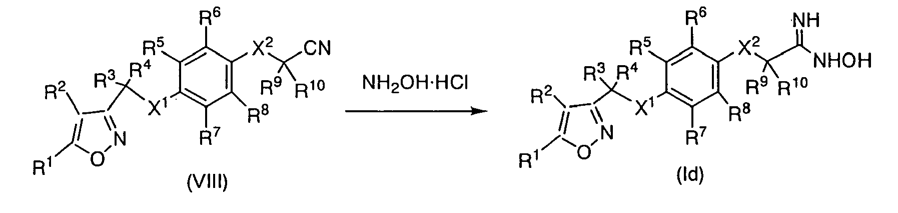 (wherein each sign is the same meanings as the above)

Compound (VIII) is reacted with hydroxylamine to give a target compound (Id). The reaction can be performed in an appropriate solvent at 0 °C - 150 °C and preferably at 20 °C - 100 °C for 0.5 -90 hours. The solvent described in the above method 1 can be used as the solvent. The base described in the above method 2 can be used as the base.

As compound (VIII), well known compounds and compounds, which is lead from well-known compounds by usual methods, can be used.

### (Method 5) Syntheses of compound (Ie) (X³ = oxadiazolon)

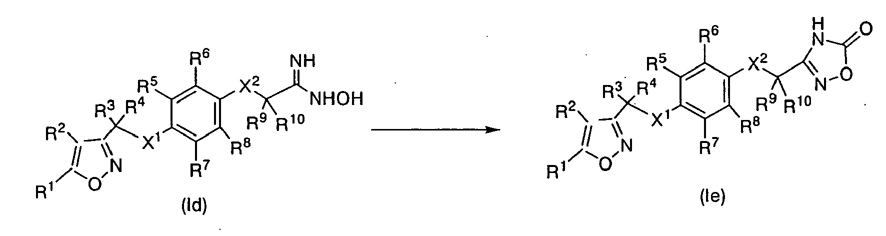

(wherein each sign are the same meanings as the above.)

Compound (Id) obtained in the above method 4 is reacted with CDI, phosgene, triphosgene or the like to give a target compound (Ie). The reaction can be performed in an appropriate solvent at -30 °C - 150 °C and preferably at 0 °C - 100 °C for 0.5 - 90 hours. The solvent described in the above method 1 can be used as a solvent. The base described in the above method 2 can be used as the base.

The target oxadiazolon compound (Ie) substituted with R¹⁷ is obtained by following method. A compound wherein R¹⁷ is H is synthesized by the above method, followed by introducing an appropriate subsistent by the usual method to give target compound.

### (Method 6) Syntheses of compound (If) (X³ = oxadiadinon)

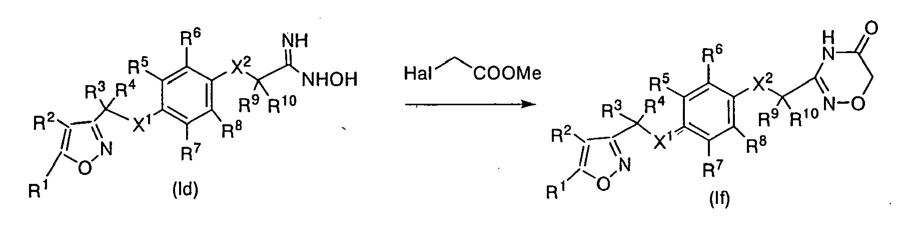

(wherein each sign is the same meanings as the above.)

Compound (Id) obtained in the above method 4 and a halogen compound are reacted to give target compound (If). The reaction can be performed in an appropriate solvent at -30 °C - 150 °C and preferably at 0 °C - 100 °C for 0.5 - 90 hours reaction. The solvent described in the above method 1 can be used as the solvent. The base described in the above method 2 can be used as the base.

### (Method 7) Syntheses of compound (Ig) (X¹ = O, S or NR¹¹)

Compound (Ig) is synthesized by the following route. 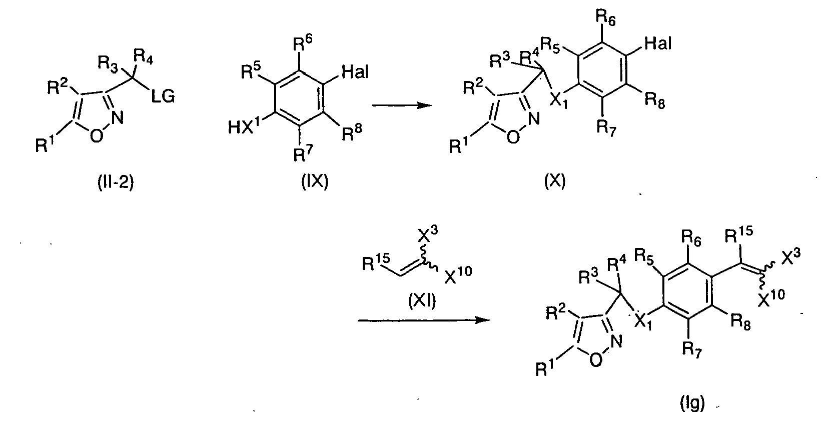 (wherein each sign is the same meanings as the above.)

Compound (II-2) and compound (IX) are subject to an addition reaction to give compound (X). The reaction can be performed preferably in an appropriate solvent under the presence of the base at -50 °C - 150 °C and preferably at 20 °C - 100 °C for 0.5-60 hours. The solvent described in the above method 1 as the solvent and the base described in the above method 2 as the base can be used.

Next, compound (X) is subject to coupling reaction with compound (XI) to give compound (Ig). The reaction can be performed preferably in an appropriate solvent under the presence of the base and palladium catalyst at -50 °C - 200 °C and preferably at 20 °C - 150 °C for 0.5-60 hours. The solvent described in the above method 1 can be used as the solvent, and the base described in the above method 2 can be used as the base. As a palladium catalyst, various palladium catalysts can be used and preferably it is combination of tris (bisbenzylidene acetone) dipalladium and tri-o-tolylphosphine, a combination of palladium acetate and triphenylphosphine or the like.

As compound (II-2), compound (IX) and compound (XI), well known compounds and compounds, which is lead from well-known compounds by usual methods, can be used.

When the compound obtained by the above any method is ester, i.e. X³ = COOR¹⁷, this compound is hydrolyze by the usual method to give carboxylic acid, i.e. X³ = COOH.

If necessary, at an appropriate step in the above method for producing, any substituent can be transform to a different substituent by the well-known organic synthesized reaction.

For example, when the compound has halogen, it is reacted with alcohol in a solvent such as DMF, tetrahydrofuran or the like under the presence of base such as sodium hydride, potassium hydride or the like and deacid reagent such as alkali metal hydroxide, alkali metal hydrogencarbonate, alkali metal carbonate, organic base or the like at -20 °C- 100 °C to give compound whose substituent is transformed to lower alkoxy.

When the compound has hydroxy, it is reacted with oxidizing agent such as pyridinium dichromate, Jones reagent, manganese dioxide, potassium permanganate, ruthenium tetroxide or the like in a solvent such as dimethyl formamide, tetrahydrofuran, dichioromethane, benzene, acetone or the like to give a compound whose substituent is transformed to carboxy.

If necessary, after amino or hydroxy of a compound is protected by the usual method at an appropriate step, it is subjected to the reaction and then deprotected by treatment with acid or base at an appropriate step.

As an amino protecting group, phthalimide, lower alkoxycarbonyl, lower alkenyloxy carbonyl, halogeno alkoxycarbonyl, aryl lower alkoxycarbonyl, trialkyl silyl, lower alkylsulfonyl, halogeno lower alkylsulfonyl, arylsulfonyl, lower alkylcarbonyl, arylcarbonyl or the like can be used.

As a hydroxy protecting group, alkyl (t-butyl or the like), aralkyl (triphenylmethyl or benzyl), trialkyl silyl (t-butyldimethylsilyl, triisopropyl silyl or the like), alkyldiarylsilyl (t-butyldiphenylsilyl or the like), triaralkylsilyl (tribenzylsilyl or the like), alkoxyalkyl (methoxymethyl, 1-ethoxyethyl, 1-methyl 1-methoxyethyl or the like), alkoxyalkoxyalkyl (methoxyethoxymethyl or the like), alkylthioalkyl (methylthiomethyl or the like), tetrahydropyranyl (tetrahydropyran-2-yl, 4-methoxytetrahydropyran-4-yl or the like), tetrahydrothiopyranyl (tetrahydrothiopyran-2-yl or the like), tetrahydrofuranyl (tetrahydrofuran-2-yl or the like), tetrahydrothio furanyl (tetrahydrothio furan-2-yl or the like), aralkyloxyalkyl (benzyloxymethyl or the like) alkylsulfonyl, acyl, p-toluenesulfonyl or the like can be used.

Deprotection reaction is accomplished in a solvent such as tetrahydrofuran, dimethylformamide, diethylether, dichloromethane, toluene, benzene, xylene, cyelohexane, hexane, chloroform, ethyl acetate, butyl acetate, pentane, heptane, dioxane, acetone, acetonitrile or a mixed solvent thereof, by using base such as hydrazine, pyridine, sodium hydroxide, potassium hydroxide or the like or acid such as hydrochloric acid, trifluoroacetic acid, hydrofluoric acid or the like.

Preferable compounds in compounds of the present invention are followings.
1) A compound wherein the part (A part) of formula: 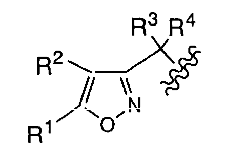 is the one of the followings,

**Table 1**

| | | | | | |
|---|---|---|---|---|---|
| 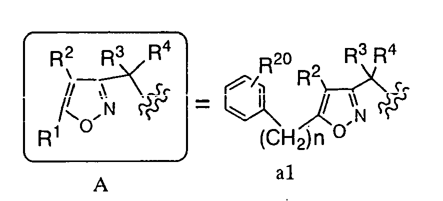 | | | | | |

| A Part No. | Type | R20 | n | R2 | R3,R4 |
|---|---|---|---|---|---|
| A1 | a1 | 4-Cl | 0 | H | H,H |
| A2 | a1 | 4-Cl | 0 | H | Me,Me |
| A3 | a1 | 4-Cl | 0 | H | Et,Et |
| A4 | a1 | 4-Cl | 0 | H | H.Et |
| A5 | a1 | 4-Cl | 0 | H | H,Ph |
| A6 | a1 | 4-Cl | 0 | H | H,C6H4-4-F |
| A7 | a1 | 4-Cl | 0 | Me | H,H |
| A8 | a1 | 4-Cl | 0 | Me | Me,Me |
| A9 | a1 | 4-Cl | 0 | Me | Et,Et |
| A10 | a1 | 4-Cl | 0 | Me | H.Et |
| A11 | a1 | 4-Cl | 0 | Me | H,Ph |
| A12 | a1 | 4-Cl | 0 | Me | H,C6H4-4-F |
| A13 | a1 | 4-Cl | 0 | OMe | H,H |
| A14 | a1 | 4-Cl | 0 | OMe | Me,Me |
| A15 | a1 | 4-Cl | 0 | OMe | Et,Et |
| A16 | a1 | 4-Cl | 0 | OMe | H.Et |
| A17 | a1 | 4-Cl | 0 | OMe | H,Ph |
| A18 | a1 | 4-Cl | 0 | OMe | H,C6H4-4-F |
| A19 | a1 | 4-Cl | 0 | CH2OH | H,H |
| A20 | a1 | 4-Cl | 0 | CH2OH | H,C6H4-4-F |
| A21 | a1 | 4-Cl | 0 | CH2OMe | H,H |
| A22 | a1 | 4-Cl | 0 | CH2OMe | Me,Me |
| A23 | a1 | 4-Cl | 0 | CH2OMe | Et,Et |
| A24 | a1 | 4-Cl | 0 | CH2OMe | H.Et |
| A25 | a1 | 4-Cl | 0 | CH2OMe | H,Ph |
| A26 | a1 | 4-Cl | 0 | CH2OMe | H,C6H4-4-F |
| A27 | a1 | 4-Cl | 0 | CF3 | H,H |
| A28 | a1 | 4-Cl | 0 | CF3 | Me,Me |
| A29 | a1 | 4-Cl | 0 | CF3 | Et,Et |
| A30 | a1 | 4-Cl | 0 | CF3 | H.Et |
| A31 | a1 | 4-Cl | 0 | CF3 | H,Ph |
| A32 | a1 | 4-Cl | 0 | CF3 | H,C6H4-4-F |
| A33 | a1 | 4-Cl | 0 | CH2OPh | H,H |

**Table 2**

| | | | | | |
|---|---|---|---|---|---|
| A34 | a1 | 4-Cl | 0 | CH2OPh | H,C6H4-4-F |
| A35 | a1 | 4-Cl | 0 | CH2OCH2Ph | H,H |
| A36 | a1 | 4-Ci | 0 | CH2OCH2Ph | H,C6H4-4-F |
| A37 | a1 | 4-Cl | 0 | CH2-morpholino | H,H |
| A38 | a1 | 4-Cl | 0 | CH2-morpholino | Me,Me |
| A39 | a1 | 4-Cl | 0 | CH2-morpholino | Et,Et |
| A40 | a1 | 4-Cl | 0 | CH2-morpholino | H.Et |
| A41 | a1 | 4-Cl | 0 | CH2-morpholino | H,Ph |
| A42 | a1 | 4-Cl | 0 | CH2-morpholino | H,C6H4-4-F |
| A43 | a1 | 4-Cl | 0 | CH2NHBu | H,H |
| A44 | a1 | 4-Cl | 0 | CH2NHBu | H,C6H4-4-F |
| A45 | a1 | 4-Cl | 0 | C≡CPh | H,H |
| A46 | a1 | 4-Cl | 0 | C≡CPh | H,C6H4-4-F |
| A47 | a1 | 4-Cl | 0 | Ph | H,H |
| A48 | a1 | 4-Cl | 0 | Ph | H,C6H4-4-F |
| A49 | a1 | 4-Cl | 0 | C6H4-4-CF3 | H,H |
| A50 | a1 | 4-Cl | 0 | C6H4-4-CF3 | H,C6H4-4-F |
| A51 | a1 | 4-Cl | 0 | C6H4-3-CF3 | H,H |
| A52 | a1 | 4-Cl | 0 | C6H4-3-CF3 | H,C6H4-4-F |
| A53 | a1 | 4-Cl | 0 | C6H4-4-OH | H,H |
| A54 | a1 | 4-Cl | 0 | C6H4-4-OH | H,C6H4-4-F |
| A55 | a1 | 4-Cl | 0 | CH2Ph | H,H |
| A56 | a1 | 4-Cl | 0 | CH2Ph | H,C6H4-4-F |
| A57 | a1 | 4-Cl | 0 | CH2C6H4-4-CF3 | H,H |
| A58 | a1 | 4-Cl | 0 | CH2C6H4-4-CF3 | Me,Me |
| A59 | a1 | 4-Cl | 0 | CH2C6H4-4-CF3 | Et,Et |
| A60 | a1 | 4-Cl | 0 | CH2C6H4-4-CF3 | H.Et |
| A61 | a1 | 4-Cl | 0 | CH2C6H4-4-CF3 | H,Ph |
| A62 | a1 | 4-Cl | 0 | CH2C6H4-4-CF3 | H,C6H4-4-F |
| A63 | a1 | 4-Cl | 0 | CH2C6H4-4-OCF3 | H,H |
| A64 | a1 | 4-Cl | 0 | CH2C6H4-4-OCF3 | H,C6H4-4-F |
| A65 | a1 | 4-Cl | 0 | CH2C6H4-4-Ph | H,H |
| A66 | a1 | 4-Cl | 0 | CH2C6H4-4-Ph | H,C6H4-4-F |
| A67 | a1 | 4-Cl | 0 | CH2C6H4-2-Cl | H,H |
| A68 | a1 | 4-Cl | 0 | CH2C6H4-2-Cl | H,C6H4-4-F |
| A69 | a1 | 4-Cl | 0 | (CH2)2Ph | H,H |
| A70 | a1 | 4-Cl | 0 | (CH2)2Ph | H,C6H4-4-F |
| A71 | a1 | 4-Cl | 0 | SPh | H,H |
| A72 | a1 | 4-Cl | 0 | SPh | H,C6H4-4-F |
| A73 | a1 | 4-Cl | 0 | NH2 | H,H |
| A74 | a1 | 4-Cl | 0 | NH2 | H,C6H4-4-F |
| A75 | a1 | 4-Cl | 0 | NHMe | H,H |
| A76 | a1 | 4-Cl | 0 | NHMe | H,C6H4-4-F |
| A77 | a1 | 4-Cl | 0 | CH2-piperazino-Ph | H,H |

**Table 3**

| | | | | | |
|---|---|---|---|---|---|
| A78 | a1 | 4-Cl | 0 | CH2-piperazino-Ph | H,C6H4-4-F |
| A79 | a1 | 4-Cl | 0 | CH2-piperidino | H,H |
| A80 | a1 | 4-Cl | 0 | CH2-piperidino | H,C6H4-4-F |
| A81 | a1 | 4-Cl | 0 | OCH2Ph | H,H |
| A82 | a1 | 4-Cl | 0 | OCH2Ph | H,C6H4-4-F |
| A83 | a1 | 4-Cl | 0 | Ac | H,H |
| A84 | a1 | 4-Cl | 0 | Ac | H,C6H4-4-F |
| A85 | a1 | 4-Cl | 0 | CONH2 | H,H |
| A86 | a1 | 4-Cl | 0 | CONH2 | H,C6H4-4-F |
| A87 | a1 | 4-Cl | 0 | CSNH2 | H,H |
| A88 | a1 | 4-Cl | 0 | CSNH2 | H,C6H4-4-F |
| A89 | a1 | 4-Cl | 0 | OCONH2 | H,H |
| A90 | a1 | 4-Cl | 0 | OCONH2 | H,C6H4-4-F |
| A91 | a1 | 4-Cl | 0 | OCSNH2 | H,H |
| A92 | a1 | 4-Cl | 0 | OCSNH2 | H,C6H4-4-F |
| A93 | a1 | 4-Cl | 0 | OSO2Me | H,H |
| A94 | a1 | 4-Cl | 0 | OSO2Me | H,C6H4-4-F |
| A95 | a1 | 4-Cl | 0 | OSO2Ph | H,H |
| A96 | a1 | 4-Cl | 0 | OSO2Ph | H,C6H4-4-F |
| A97 | a1 | 4-Cl | 0 | I | H,H |
| A98 | a1 | 4-Cl | 0 | I | H,C6H4-4-F |
| A99 | a1 | 4-Cl | 1 | H | H,H |
| A100 | a1 | 4-Cl | 1 | H | Me,Me |
| A101 | a1 | 4-Cl | 1 | H | Et,Et |
| A102 | a1 | 4-Cl | 1 | H | H.Et |
| A103 | a1 | 4-Cl | 1 | H | H,Ph |
| A104 | a1 | 4-Cl | 1 | H | H,C6H4-4-F |
| A105 | a1 | 4-Cl | 1 | Me | H,H |
| A106 | a1 | 4-Cl | 1 | Me | Me,Me |
| A107 | a1 | 4-Cl | 1 | Me | Et,Et |
| A108 | a1 | 4-Cl | 1 | Me | H.Et |
| A109 | a1 | 4-Cl | 1 | Me | H,Ph |
| A110 | a1 | 4-Cl | 1 | Me | H,C6H4-4-F |
| A111 | a1 | 4-Cl | 1 | OMe | H,H |
| A112 | a1 | 4-Cl | 1 | OMe | Me,Me |
| A113 | a1 | 4-Cl | 1 | OMe | Et,Et |
| A114 | a1 | 4-Cl | 1 | OMe | H.Et |
| A115 | a1 | 4-Cl | 1 | OMe | H,Ph |
| A116 | a1 | 4-Cl | 1 | OMe | H,C6H4-4-F |
| A117 | a1 | 4-Cl | 1 | CH2OH | H,H |
| A118 | a1 | 4-Cl | 1 | CH2OH | H,C6H4-4-F |
| A119 | a1 | 4-Cl | 1 | CH2OMe | H,H |
| A120 | a1 | 4-Cl | 1 | CH2OMe | Me,Me |
| A121 | a1 | 4-Cl | 1 | CH2OMe | Et,Et |

**Table 4**

| | | | | | |
|---|---|---|---|---|---|
| A122 | a1 | 4-Cl | 1 | CH2OMe | H.Et |
| A123 | a1 | 4-Cl | 1 | CH2OMe | H,Ph |
| A124 | a1 | 4-Cl | 1 | CH2OMe | H,C6H4-4-F |
| A125 | a1 | 4-Cl | 1 | CF3 | H,H |
| A126 | a1 | 4-Cl | 1 | CF3 | Me,Me |
| A127 | a1 | 4-Cl | 1 | CF3 | Et,Et |
| A128 | a1 | 4-Cl | 1 | CF3 | H.Et |
| A129 | a1 | 4-Cl | 1 | CF3 | H,Ph |
| A130 | a1 | 4-Cl | 1 | CF3 | H,C6H4-4-F |
| A131 | a1 | 4-Cl | 1 | CH2OPh | H,H |
| A132 | a1 | 4-Cl | 1 | CH2OPh | H,C6H4-4-F |
| A133 | a1 | 4-Cl | 1 | CH2OCH2Ph | H,H |
| A134 | a1 | 4-Cl | 1 | CH2OCH2Ph | H,C6H4-4-F |
| A135 | a1 | 4-Cl | 1 | CH2-morpholino | H,H |
| A136 | a1 | 4-Cl | 1 | CH2-morpholino | Me,Me |
| A137 | a1 | 4-Cl | 1 | CH2-morpholino | Et,Et |
| A138 | a1 | 4-Cl | 1 | CH2-morpholino | H.Et |
| A139 | a1 | 4-Cl | 1 | CH2-morpholino | H,Ph |
| A140 | a1 | 4-Cl | 1 | CH2-morpholino | H,C6H4-4-F |
| A141 | a1 | 4-Cl | 1 | CH2NHBu | H,H |
| A142 | a1 | 4-Cl | 1 | CH2NHBu | H,C6H4-4-F |
| A143 | a1 | 4-Cl | 1 | C≡CPh | H,H |
| A144 | a1 | 4-Cl | 1 | C≡CPh | H,C6H4-4-F |
| A145 | a1 | 4-Cl | 1 | Ph | H,H |
| A146 | a1 | 4-Cl | 1 | Ph | H,C6H4-4-F |
| A147 | a1 | 4-Cl | 1 | C6H4-4-CF3 | H,H |
| A148 | a1 | 4-Cl | 1 | C6H4-4-CF3 | H,C6H4-4-F |
| A149 | a1 | 4-Cl | 1 | C6H4-3-CF3 | H,H |
| A150 | a1 | 4-Cl | 1 | C6H4-3-CF3 | H,C6H4-4-F |
| A151 | a1 | 4-Cl | 1 | C6H4-4-OH | H,H |
| A152 | a1 | 4-Cl | 1 | C6H4-4-OH | H,C6H4-4-F |
| A153 | a1 | 4-Cl | 1 | CH2Ph | H,H |
| A154 | a1 | 4-Cl | 1 | CH2Ph | H,C6H4-4-F |
| A155 | a1 | 4-Cl | 1 | CH2C6H4-4-CF3 | H,H |
| A156 | a1 | 4-Cl | 1 | CH2C6H4-4-CF3 | Me,Me |
| A157 | a1 | 4-Cl | 1 | CH2C6H4-4-CF3 | Et,Et |
| A158 | a1 | 4-Cl | 1 | CH2C6H4-4-CF3 | H.Et |
| A159 | a1 | 4-Cl | 1 | CH2C6H4-4-CF3 | H,Ph |
| A160 | a1 | 4-Cl | 1 | CH2C6H4-4-CF3 | H,C6H4-4-F |
| A161 | a1 | 4-Cl | 1 | CH2C6H4-4-OCF3 | H,H |
| A162 | a1 | 4-Cl | 1 | CH2C6H4-4-OCF3 | H,C6H4-4-F |
| A163 | a1 | 4-Cl | 1 | CH2C6H4-4-Ph | H,H |
| A164 | a1 | 4-Cl | 1 | CH2C6H4-4-Ph | H,C6H4-4-F |
| A165 | a1 | 4-Cl | 1 | CH2C6H4-2-Cl | H,H |

**Table 5**

| | | | | | |
|---|---|---|---|---|---|
| A166 | a1 | 4-Cl | 1 | CH2C6H4-2-Cl | H,C6H4-4-F |
| A167 | a1 | 4-Cl | 1 | (CH2)2Ph | H,H |
| A168 | a1 | 4-Cl | 1 | (CH2)2Ph | H,C6H4-4-F |
| A169 | a1 | 4-Cl | 1 | SPh | H,H |
| A170 | a1 | 4-Cl | 1 | SPh | H,C6H4-4-F |
| A171 | a1 | 4-Cl | 1 | NH2 | H,H |
| A172 | a1 | 4-Cl | 1 | NH2 | H,C6H4-4-F |
| A173 | a1 | 4-Cl | 1 | NHMe | H,H |
| A174 | a1 | 4-Cl | 1 | NHMe | H,C6H4-4-F |
| A175 | a1 | 4-Cl | 1 | CH2-piperazino-Ph | H,H |
| A176 | a1 | 4-Cl | 1 | CH2-piperazino-Ph | H,C6H4-4-F |
| A177 | a1 | 4-Cl | 1 | CH2-piperidino | H,H |
| A178 | a1 | 4-Cl | 1 | CH2-piperidino | H,C6H4-4-F |
| A179 | a1 | 4-Cl | 1 | OCH2Ph | H,H |
| A180 | a1 | 4-Cl | 1 | OCH2Ph | H,C6H4-4-F |
| A181 | a1 | 4-Cl | 1 | Ac | H,H |
| A182 | a1 | 4-Cl | 1 | Ac | H,C6H4-4-F |
| A183 | a1 | 4-Cl | 1 | CONH2 | H,H |
| A184 | a1 | 4-Cl | 1 | CONH2 | H,C6H4-4-F |
| A185 | a1 | 4-Cl | 1 | CSNH2 | H,H |
| A186 | a1 | 4-Cl | 1 | CSNH2 | H,C6H4-4-F |
| A187 | a1 | 4-Cl | 1 | OCONH2 | H,H |
| A188 | a1 | 4-Cl | 1 | OCONH2 | H,C6H4-4-F |
| A189 | a1 | 4-Cl | 1 | OCSNH2 | H,H |
| A190 | a1 | 4-Cl | 1 | OCSNH2 | H,C6H4-4-F |
| A191 | a1 | 4-Cl | 1 | OSO2Me | H,H |
| A192 | a1 | 4-Cl | 1 | OSO2Me | H,C6H4-4-F |
| A193 | a1 | 4-Cl | 1 | OSO2Ph | H,H |
| A194 | a1 | 4-Cl | 1 | OSO2Ph | H,C6H4-4-F |
| A195 | a1 | 4-Cl | 1 | I | H,H |
| A196 | a1 | 4-Cl | 1 | I | H,C6H4-4-F |
| A197 | a1 | 4-Cl | 2 | H | H,H |
| A198 | a1 | 4-Cl | 2 | H | Me,Me |
| A199 | a1 | 4-Cl | 2 | H | Et,Et |
| A200 | a1 | 4-Cl | 2 | H | H.Et |
| A201 | a1 | 4-Cl | 2 | H | H,Ph |
| A202 | a1 | 4-Cl | 2 | H | H,C6H4-4-F |
| A203 | a1 | 4-Cl | 2 | Me | H,H |
| A204 | a1 | 4-Cl | 2 | Me | Me,Me |
| A205 | a1 | 4-Cl | 2 | Me | Et,Et |
| A206 | a1 | 4-Cl | 2 | Me | H.Et |
| A207 | a1 | 4-Cl | 2 | Me | H,Ph |
| A208 | a1 | 4-Cl | 2 | Me | H,C6H4-4-F |
| A209 | a1 | 4-Cl | 2 | OMe | H,H |

**Table 6**

| | | | | | |
|---|---|---|---|---|---|
| A210 | a1 | 4-Cl | 2 | OMe | Me,Me |
| A211 | a1 | 4-Cl | 2 | OMe | Et,Et |
| A212 | a 1 | 4-Cl | 2 | OMe | H.Et |
| A213 | a1 | 4-Cl | 2 | OMe | H,Ph |
| A214 | a1 | 4-Cl | 2 | OMe | H,C6H4-4-F |
| A215 | a1 | 4-Cl | 2 | CH2OH | H,H |
| A216 | a1 | 4-Cl | 2 | CH2OH | H,C6H4-4-F |
| A217 | a1 | 4-Cl | 2 | CH2OMe | H,H |
| A218 | a1 | 4-Cl | 2 | CH2OMe | Me,Me |
| A219 | a1 | 4-Cl | 2 | CH2OMe | Et,Et |
| A220 | a1 | 4-Cl | 2 | CH2OMe | H.Et |
| A221 | a1 | 4-Cl | 2 | CH2OMe | H,Ph |
| A222 | a1 | 4-Cl | 2 | CH2OMe | H,C6H4-4-F |
| A223 | a1 | 4-Cl | 2 | CF3 | H,H |
| A224 | a1 | 4-Cl | 2 | CF3 | Me,Me |
| A225 | a1 | 4-Cl | 2 | CF3 | Et,Et |
| A226 | a1 | 4-Cl | 2 | CF3 | H.Et |
| A227 | a1 | 4-Cl | 2 | CF3 | H,Ph |
| A228 | a1 | 4-Cl | 2 | CF3 | H,C6H4-4-F |
| A229 | a1 | 4-Cl | 2 | CH2OPh | H,H |
| A230 | a1 | 4-Cl | 2 | CH2OPh | H,C6H4-4-F |
| A231 | a1 | 4-Cl | 2 | CH2-OCH2Ph | H,H |
| A232 | a1 | 4-Cl | 2 | CH2OCH2Ph | H,C6H4-4-F |
| A233 | a1 | 4-Cl | 2 | CH2-morpholino | H,H |
| A234 | a1 | 4-Cl | 2 | CH2-morpholino | Me,Me |
| A235 | a1 | 4-Cl | 2 | CH2-morpholino | Et,Et |
| A236 | a1 | 4-Cl | 2 | CH2-morpholino | H.Et |
| A237 | a1 | 4-Cl | 2 | CH2-morpholino | H,Ph |
| A238 | a1 | 4-Cl | 2 | CH2-morpholino | H,C6H4-4-F |
| A239 | a1 | 4-Cl | 2 | CH2NHBu | H,H |
| A240 | a1 | 4-Cl | 2 | CH2NHBu | H,C6H4-4-F |
| A241 | a1 | 4-Cl | 2 | C≡CPh | H,H |
| A242 | a1 | 4-Cl | 2 | C≡CPh | H,C6H4-4-F |
| A243 | a1 | 4-Cl | 2 | Ph | H,H |
| A244 | a1 | 4-Cl | 2 | Ph | H,C6H4-4-F |
| A245 | a1 | 4-Cl | 2 | C6H4-4-CF3 | H,H |
| A246 | a1 | 4-Cl | 2 | C6H4-4-CF3 | H,C6H4-4-F |
| A247 | a1 | 4-Cl | 2 | C6H4-3-CF3 | H,H |
| A248 | a1 | 4-Cl | 2 | C6H4-3-CF3 | H,C6H4-4-F |
| A249 | a1 | 4-Cl | 2 | C6H4-4-OH | H,H |
| A250 | a1 | 4-Cl | 2 | C6H4-4-OH | H,C6H4-4-F |
| A251 | a1 | 4-Cl | 2 | CH2Ph | H,H |
| A252 | a1 | 4-Cl | 2 | CH2Ph | H,C6H4-4-F |
| A253 | a1 | 4-Cl | 2 | CH2C6H4-4-CF3 | H,H |

**Table 7**

| | | | | | |
|---|---|---|---|---|---|
| A254 | a1 | 4-Cl | 2 | CH2C6H4-4-CF3 | Me,Me |
| A255 | a1 | 4-Cl | 2 | CH2C6H4-4-CF3 | Et,Et |
| A256 | a1 | 4-Cl | 2 | CH2C6H4-4-CF3 | H.Et |
| A257 | a1 | 4-Cl | 2 | CH2C6H4-4-CF3 | H,Ph |
| A258 | a1 | 4-Cl | 2 | CH2C6H4-4-CF3 | H,C6H4-4-F |
| A259 | a1 | 4-Cl | 2 | CH2C6H4-4-OCF3 | H,H |
| A260 | a1 | 4-Cl | 2 | CH2C6H4-4-OCF3 | H,C6H4-4-F |
| A261 | a1 | 4-Cl | 2 | CH2C6H4-4-Ph | H,H |
| A262 | a1 | 4-Cl | 2 | CH2C6H4-4-Ph | H,C6H4-4-F |
| A263 | a1 | 4-Cl | 2 | CH2C6H4-2-Cl | H,H |
| A264 | a1 | 4-Cl | 2 | CH2C6H4-2-Cl | H,C6H4-4-F |
| A265 | a1 | 4-Cl | 2 | (CH2)2Ph | H,H |
| A266 | a1 | 4-Cl | 2 | (CH2)2Ph | H,C6H4-4-F |
| A267 | a1 | 4-Cl | 2 | SPh | H,H |
| A268 | a1 | 4-Cl | 2 | SPh | H,C6H4-4-F |
| A269 | a1 | 4-Cl | 2 | NH2 | H,H |
| A270 | a1 | 4-Cl | 2 | NH2 | H,C6H4-4-F |
| A271 | a1 | 4-Cl | 2 | NHMe | H,H |
| A272 | a1 | 4-Cl | 2 | NHMe | H,C6H4-4-F |
| A273 | a1 | 4-Cl | 2 | CH2-piperazino-Ph | H,H |
| A274 | a1 | 4-Cl | 2 | CH2-piperazino-Ph | H,C6H4-4-F |
| A275 | a1 | 4-Cl | 2 | CH2-piperidino | H,H |
| A276 | a1 | 4-Cl | 2 | CH2-piperidino | H,C6H4-4-F |
| A277 | a1 | 4-Cl | 2 | OCH2Ph | H,H |
| A278 | a1 | 4-Cl | 2 | OCH2Ph | H,C6H4-4-F |
| A279 | a1 | 4-Cl | 2 | Ac | H,H |
| A280 | a1 | 4-Cl | 2 | Ac | H,C6H4-4-F |
| A281 | a1 | 4-Cl | 2 | CONH2 | H,H |
| A282 | a1 | 4-Cl | 2 | CONH2 | H,C6H4-4-F |
| A283 | a1 | 4-Cl | 2 | CSNH2 | H,H |
| A284 | a1 | 4-Cl | 2 | CSNH2 | H,C6H4-4-F |
| A285 | a1 | 4-Cl | 2 | OCONH2 | H,H |
| A286 | a1 | 4-Cl | 2 | OCONH2 | H,C6H4-4-F |
| A287 | a1 | 4-Cl | 2 | OCSNH2 | H,H |
| A288 | a1 | 4-Cl | 2 | OCSNH2 | H,C6H4-4-F |
| A289 | a1 | 4-Cl | 2 | OSO2Me | H,H |
| A290 | a1 | 4-Cl | 2 | OSO2Me | H,C6H4-4-F |
| A291 | a1 | 4-Cl | 2 | OSO2Ph | H,H |
| A292 | a1 | 4-Cl | 2 | OSO2Ph | H,C6H4-4-F |
| A293 | a1 | 4-Cl | 2 | I | H,H |
| A294 | a1 | 4-Cl | 2 | I | H,C6H4-4-F |
| A295 | a1 | 4-CF3 | 0 | H | H,H |
| A296 | a1 | 4-CF3 | 0 | H | Me,Me |
| A297 | a1 | 4-CF3 | 0 | H | Et,Et |

**Table 8**

| | | | | | |
|---|---|---|---|---|---|
| A298 | a1 | 4-CF3 | 0 | H | H.Et |
| A299 | a1 | 4-CF3 | 0 | H | H,Ph |
| A300 | a1 | 4-CF3 | 0 | H | H,C6H4-4-F |
| A301 | a1 | 4-CF3 | 0 | Me | H,H |
| A302 | a1 | 4-CF3 | 0 | Me | Me,Me |
| A303 | a1 | 4-CF3 | 0 | Me | Et,Et |
| A304 | a1 | 4-CF3 | 0 | Me | H.Et |
| A305 | a1 | 4-CF3 | 0 | Me | H,Ph |
| A306 | a1 | 4-CF3 | 0 | Me | H,C6H4-4-F |
| A307 | a1 | 4-CF3 | 0 | OMe | H,H |
| A308 | a1 | 4-CF3 | 0 | OMe | Me,Me |
| A309 | a1 | 4-CF3 | 0 | OMe | Et,Et |
| A310 | a1 | 4-CF3 | 0 | OMe | H.Et |
| A311 | a1 | 4-CF3 | 0 | OMe | H,Ph |
| A312 | a1 | 4-CF3 | 0 | OMe | H,C6H4-4-F |
| A313 | a1 | 4-CF3 | 0 | CH2OH | H,H |
| A314 | a1 | 4-CF3 | 0 | CH2OH | H,C6H4-4-F |
| A315 | a1 | 4-CF3 | 0 | CH2OMe | H,H |
| A316 | a1 | 4-CF3 | 0 | CH2OMe | Me,Me |
| A317 | a1 | 4-CF3 | 0 | CH2OMe | Et,Et |
| A318 | a1 | 4-CF3 | 0 | CH2OMe | H.Et |
| A319 | a1 | 4-CF3 | 0 | CH2OMe | H,Ph |
| A320 | a1 | 4-CF3 | 0 | CH2OMe | H,C6H4-4-F |
| A321 | a1 | 4-CF3 | 0 | CF3 | H,H |
| A322 | a1 | 4-CF3 | 0 | CF3 | Me,Me |
| A323 | a1 | 4-CF3 | 0 | CF3 | Et,Et |
| A324 | a1 | 4-CF3 | 0 | CF3 | H.Et |
| A325 | a1 | 4-CF3 | 0 | CF3 | H,Ph |
| A326 | a1 | 4-CF3 | 0 | CF3 | H,C6H4-4-F |
| A327 | a1 | 4-CF3 | 0 | CH2OPh | H,H |
| A328 | a1 | 4-CF3 | 0 | CH2OPh | H,C6H4-4-F |
| A329 | a1 | 4-CF3 | 0 | CH2OCH2Ph | H,H |
| A330 | a1 | 4-CF3 | 0 | CH2OCH2Ph | H,C6H4-4-F |
| A331 | a1 | 4-CF3 | 0 | CH2-morpholino | H,H |
| A332 | a1 | 4-CF3 | 0 | CH2-morpholino | Me,Me |
| A333 | a1 | 4-CF3 | 0 | CH2-morpholino | Et,Et |
| A334 | a1 | 4-CF3 | 0 | CH2-morpholino | H.Et |
| A335 | a1 | 4-CF3 | 0 | CH2-morpholino | H,Ph |
| A336 | a1 | 4-CF3 | 0 | CH2-morpholino | H,C6H4-4-F |
| A337 | a1 | 4-CF3 | 0 | CH2NHBu | H,H |
| A338 | a1 | 4-CF3 | 0 | CH2NHBu | H,C6H4-4-F |
| A339 | a1 | 4-CF3 | 0 | C≡CPh | H,H |
| A340 | a1 | 4-CF3 | 0 | C≡CPh | H,C6H4-4-F |
| A341 | a1 | 4-CF3 | 0 | Ph | H,H |

**Table 9**

| | | | | | |
|---|---|---|---|---|---|
| A342 | a1 | 4-CF3 | 0 | Ph | H,C6H4-4-F |
| A343 | a1 | 4-CF3 | 0 | C6H4-4-CF3 | H,H |
| A344 | a1 | 4-CF3 | 0 | C6H4-4-CF3 | H,C6H4-4-F |
| A345 | a1 | 4-CF3 | 0 | C6H4-3-CF3 | H,H |
| A346 | a1 | 4-CF3 | 0 | C6H4-3-CF3 | H,C6H4-4-F |
| A347 | a1 | 4-CF3 | 0 | C6H4-4-OH | H,H |
| A348 | a1 | 4-CF3 | 0 | C6H4-4-OH | H,C6H4-4-F |
| A349 | a1 | 4-CF3 | 0 | CH2Ph | H,H |
| A350 | a1 | 4-CF3 | 0 | CH2Ph | H,C6H4-4-F |
| A351 | a1 | 4-CF3 | 0 | CH2C6H4-4-CF3 | H,H |
| A352 | a1 | 4-CF3 | 0 | CH2C6H4-4-CF3 | Me,Me |
| A353 | a1 | 4-CF3 | 0 | CH2C6H4-4-CF3 | Et,Et |
| A354 | a1 | 4-CF3 | 0 | CH2C6H4-4-CF3 | H.Et |
| A355 | a1 | 4-CF3 | 0 | CH2C6H4-4-CF3 | H,Ph |
| A356 | a1 | 4-CF3 | 0 | CH2C6H4-4-CF3 | H,C6H4-4-F |
| A357 | a1 | 4-CF3 | 0 | CH2C6H4-4-OCF3 | H,H |
| A358 | a1 | 4-CF3 | 0 | CH2C6H4-4-OCF3 | H,C6H4-4-F |
| A359 | a1 | 4-CF3 | 0 | CH2C6H4-4-Ph | H,H |
| A360 | a1 | 4-CF3 | 0 | CH2C6H4-4-Ph | H,C6H4-4-F |
| A361 | a1 | 4-CF3 | 0 | CH2C6H4-2-Cl | H,H |
| A362 | a1 | 4-CF3 | 0 | CH2C6H4-2-Cl | H,C6H4-4-F |
| A363 | a1 | 4-CF3 | 0 | (CH2)2Ph | H,H |
| A364 | a1 | 4-CF3 | 0 | (CH2)2Ph | H,C6H4-4-F |
| A365 | a1 | 4-CF3 | 0 | SPh | H,H |
| A366 | a1 | 4-CF3 | 0 | SPh | H,C6H4-4-F |
| A367 | a1 | 4-CF3 | 0 | NH2 | H,H |
| A368 | a1 | 4-CF3 | 0 | NH2 | H,C6H4-4-F |
| A369 | a1 | 4-CF3 | 0 | NHMe | H,H |
| A370 | a1 | 4-CF3 | 0 | NHMe | H,C6H4-4-F |
| A371 | a1 | 4-CF3 | 0 | CH2-piperazino-Ph | H,H |
| A372 | a1 | 4-CF3 | 0 | CH2-piperazino-Ph | H,C6H4-4-F |
| A373 | a1 | 4-CF3 | 0 | CH2-piperidino | H,H |
| A374 | a1 | 4-CF3 | 0 | CH2-piperidino | H,C6H4-4-F |
| A375 | a1 | 4-CF3 | 0 | OCH2Ph | H,H |
| A376 | a1 | 4-CF3 | 0 | OCH2Ph | H,C6H4-4-F |
| A377 | a1 | 4-CF3 | 0 | Ac | H,H |
| A378 | a1 | 4-CF3 | 0 | Ac | H,C6H4-4-F |
| A379 | a1 | 4-CF3 | 0 | CONH2 | H,H |
| A380 | a1 | 4-CF3 | 0 | CONH2 | H,C6H4-4-F |
| A381 | a1 | 4-CF3 | 0 | CSNH2 | H,H |
| A382 | a1 | 4-CF3 | 0 | CSNH2 | H,C6H4-4-F |
| A383 | a1 | 4-CF3 | 0 | OCONH2 | H,H |
| A384 | a1 | 4-CF3 | 0 | OCONH2 | H,C6H4-4-F |
| A385 | a1 | 4-CF3 | 0 | OCSNH2 | H,H |

**Table 10**

| | | | | | |
|---|---|---|---|---|---|
| A386 | a1 | 4-CF3 | 0 | OCSNH2 | H,C6H4-4-F |
| A387 | a1 | 4-CF3 | 0 | OSO2Me | H,H |
| A388 | a1 | 4-CF3 | 0 | OSO2Me | H,C6H4-4-F |
| A389 | a1 | 4-CF3 | 0 | OSO2Ph | H,H |
| A390 | a1 | 4-CF3 | 0 | OSO2Ph | H,C6H4-4-F |
| A391 | a1 | 4-CF3 | 0 | I | H,H |
| A392 | a1 | 4-CF3 | 0 | I | H,C6H4-4-F |
| A393 | a1 | 4-CF3 | 1 | H | H,H |
| A394 | a1 | 4-CF3 | 1 | H | Me,Me |
| A395 | a1 | 4-CF3 | 1 | H | Et,Et |
| A396 | a1 | 4-CF3 | 1 | H | H.Et |
| A397 | a1 | 4-CF3 | 1 | H | H,Ph |
| A398 | a1 | 4-CF3 | 1 | H | H,C6H4-4-F |
| A399 | a1 | 4-CF3 | 1 | Me | H,H |
| A400 | a1 | 4-CF3 | 1 | Me | Me,Me |
| A401 | a1 | 4-CF3 | 1 | Me | Et,Et |
| A402 | a1 | 4-CF3 | 1 | Me | H.Et |
| A403 | a1 | 4-CF3 | 1 | Me | H,Ph |
| A404 | a1 | 4-CF3 | 1 | Me | H,C6H4-4-F |
| A405 | a1 | 4-CF3 | 1 | OMe | H,H |
| A406 | a1 | 4-CF3 | 1 | OMe | Me,Me |
| A407 | a1 | 4-CF3 | 1 | OMe | Et,Et |
| A408 | a1 | 4-CF3 | 1 | OMe | H.Et |
| A409 | a1 | 4-CF3 | 1 | OMe | H,Ph |
| A410 | a1 | 4-CF3 | 1 | OMe | H,C6H4-4-F |
| A411 | a1 | 4-CF3 | 1 | CH2OH | H,H |
| A412 | a1 | 4-CF3 | 1 | CH2OH | H,C6H4-4-F |
| A413 | a1 | 4-CF3 | 1 | CH2OMe | H,H |
| A414 | a1 | 4-CF3 | 1 | CH2OMe | Me,Me |
| A415 | a1 | 4-CF3 | 1 | CH2OMe | Et,Et |
| A416 | a1 | 4-CF3 | 1 | CH2OMe | H.Et |
| A417 | a1 | 4-CF3 | 1 | CH2OMe | H,Ph |
| A418 | a1 | 4-CF3 | 1 | CH2OMe | H,C6H4-4-F |
| A419 | a1 | 4-CF3 | 1 | CF3 | H,H |
| A420 | a1 | 4-CF3 | 1 | CF3 | Me,Me |
| A421 | a1 | 4-CF3 | 1 | CF3 | Et,Et |
| A422 | a1 | 4-CF3 | 1 | CF3 | H.Et |
| A423 | a1 | 4-CF3 | 1 | CF3 | H,Ph |
| A424 | a1 | 4-CF3 | 1 | CF3 | H,C6H4-4-F |
| A425 | a1 | 4-CF3 | 1 | CH2OPh | H,H |
| A426 | a1 | 4-CF3 | 1 | CH2OPh | H,C6H4-4-F |
| A427 | a1 | 4-CF3 | 1 | CH2OCH2Ph | H,H |
| A428 | a1 | 4-CF3 | 1 | CH2OCH2Ph | H,C6H4-4-F |
| A429 | a1 | 4-CF3 | 1 | CH2-morpholino | H,H |

**Table 11**

| | | | | | |
|---|---|---|---|---|---|
| A430 | a1 | 4-CF3 | 1 | CH2-morpholino | Me,Me |
| A431 | a1 | 4-CF3 | 1 | CH2-morpholino | Et,Et |
| A432 | a1 | 4-CF3 | 1 | CH2-morpholino | H.Et |
| A433 | a1 | 4-CF3 | 1 | CH2-morpholino | H,Ph |
| A434 | a1 | 4-CF3 | 1 | CH2-morpholino | H,C6H4-4-F |
| A435 | a1 | 4-CF3 | 1 | CH2NHBu | H,H |
| A436 | a1 | 4-CF3 | 1 | CH2NHBu | H,C6H4-4-F |
| A437 | a1 | 4-CF3 | 1 | C≡CPh | H,H |
| A438 | a1 | 4-CF3 | 1 | C≡CPh | H,C6H4-4-F |
| A439 | a1 | 4-CF3 | 1 | Ph | H,H |
| A440 | a1 | 4-CF3 | 1 | Ph | H,C6H4-4-F |
| A441 | a1 | 4-CF3 | 1 | C6H4-4-CF3 | H,H |
| A442 | a1 | 4-CF3 | 1 | C6H4-4-CF3 | H,C6H4-4-F |
| A443 | a1 | 4-CF3 | 1 | C6H4-3-CF3 | H,H |
| A444 | a1 | 4-CF3 | 1 | C6H4-3-CF3 | H,C6H4-4-F |
| A445 | a1 | 4-CF3 | 1 | C6H4-4-OH | H,H |
| A446 | a1 | 4-CF3 | 1 | C6H4-4-OH | H,C6H4-4-F |
| A447 | a1 | 4-CF3 | 1 | CH2Ph | H,H |
| A448 | a1 | 4-CF3 | 1 | CH2Ph | H,C6H4-4-F |
| A449 | a1 | 4-CF3 | 1 | CH2C6H4-4-CF3 | H,H |
| A450 | a1 | 4-CF3 | 1 | CH2C6H4-4-CF3 | Me,Me |
| A451 | a1 | 4-CF3 | 1 | CH2C6H4-4-CF3 | Et,Et |
| A452 | a1 | 4-CF3 | 1 | CH2C6H4-4-CF3 | H.Et |
| A453 | a1 | 4-CF3 | 1 | CH2C6H4-4-CF3 | H,Ph |
| A454 | a1 | 4-CF3 | 1 | CH2C6H4-4-CF3 | H,C6H4-4-F |
| A455 | a1 | 4-CF3 | 1 | CH2C6H4-4-OCF3 | H,H |
| A456 | a1 | 4-CF3 | 1 | CH2C6H4-4-OCF3 | H,C6H4-4-F |
| A457 | a1 | 4-CF3 | 1 | CH2C6H4-4-Ph | H,H |
| A458 | a1 | 4-CF3 | 1 | CH2C6H4-4-Ph | H,C6H4-4-F |
| A459 | a1 | 4-CF3 | 1 | CH2C6H4-2-Cl | H,H |
| A460 | a1 | 4-CF3 | 1 | CH2C6H4-2-Cl | H,C6H4-4-F |
| A461 | a1 | 4-CF3 | 1 | (CH2)2Ph | H,H |
| A462 | a1 | 4-CF3 | 1 | (CH2)2Ph | H,C6H4-4-F |
| A463 | a1 | 4-CF3 | 1 | SPh | H,H |
| A464 | a1 | 4-CF3 | 1 | SPh | H,C6H4-4-F |
| A465 | a1 | 4-CF3 | 1 | NH2 | H,H |
| A466 | a1 | 4-CF3 | 1 | NH2 | H,C6H4-4-F |
| A467 | a1 | 4-CF3 | 1 | NHMe | H,H |
| A468 | a1 | 4-CF3 | 1 | NHMe | H,C6H4-4-F |
| A469 | a1 | 4-CF3 | 1 | CH2-piperazino-Ph | H,H |
| A470 | a1 | 4-CF3 | 1 | CH2-piperazino-Ph | H,C6H4-4-F |
| A471 | a1 | 4-CF3 | 1 | CH2-piperidino | H,H |
| A472 | a1 | 4-CF3 | 1 | CH2-piperidino | H,C6H4-4-F |
| A473 | a1 | 4-CF3 | 1 | OCH2Ph | H,H |

**Table 12**

| | | | | | |
|---|---|---|---|---|---|
| A474 | a1 | 4-CF3 | 1 | OCH2Ph | H,C6H4-4-F |
| A475 | a1 | 4-CF3 | 1 | Ac | H,H |
| A476 | a1 | 4-CF3 | 1 | Ac | H,C6H4-4-F |
| A477 | a1 | 4-CF3 | 1 | CONH2 | H,H |
| A478 | a1 | 4-CF3 | 1 | CONH2 | H,C6H4-4-F |
| A479 | a1 | 4-CF3 | 1 | CSNH2 | H,H |
| A480 | a1 | 4-CF3 | 1 | CSNH2 | H,C6H4-4-F |
| A481 | a1 | 4-CF3 | 1 | OCONH2 | H,H |
| A482 | a1 | 4-CF3 | 1 | OCONH2 | H,C6H4-4-F |
| A483 | a1 | 4-CF3 | 1 | OCSNH2 | H,H |
| A484 | a1 | 4-CF3 | 1 | OCSNH2 | H,C6H4-4-F |
| A485 | a1 | 4-CF3 | 1 | OSO2Me | H,H |
| A486 | a1 | 4-CF3 | 1 | OSO2Me | H,C6H4-4-F |
| A487 | a1 | 4-CF3 | 1 | OSO2Ph | H,H |
| A488 | a1 | 4-CF3 | 1 | OSO2Ph | H,C6H4-4-F |
| A489 | a1 | 4-CF3 | 1 | I | H,H |
| A490 | a1 | 4-CF3 | 1 | I | H,C6H4-4-F |
| A491 | a1 | 4-CF3 | 2 | H | H,H |
| A492 | a1 | 4-CF3 | 2 | H | Me,Me |
| A493 | a1 | 4-CF3 | 2 | H | Et,Et |
| A494 | a1 | 4-CF3 | 2 | H | H.Et |
| A495 | a1 | 4-CF3 | 2 | H | H,Ph |
| A496 | a1 | 4-CF3 | 2 | H | H,C6H4-4-F |
| A497 | a1 | 4-CF3 | 2 | Me | H,H |
| A498 | a1 | 4-CF3 | 2 | Me | Me,Me |
| A499 | a1 | 4-CF3 | 2 | Me | Et,Et |
| A500 | a1 | 4-CF3 | 2 | Me | H.Et |
| A501 | a1 | 4-CF3 | 2 | Me | H,Ph |
| A502 | a1 | 4-CF3 | 2 | Me | H,C6H4-4-F |
| A503 | a1 | 4-CF3 | 2 | OMe | H,H |
| A504 | a1 | 4-CF3 | 2 | OMe | Me,Me |
| A505 | a1 | 4-CF3 | 2 | OMe | Et,Et |
| A506 | a1 | 4-CF3 | 2 | OMe | H.Et |
| A507 | a1 | 4-CF3 | 2 | OMe | H,Ph |
| A508 | a1 | 4-CF3 | 2 | OMe | H,C6H4-4-F |
| A509 | a1 | 4-CF3 | 2 | CH2OH | H,H |
| A510 | a1 | 4-CF3 | 2 | CH2OH | H,C6H4-4-F |
| A511 | a1 | 4-CF3 | 2 | CH2OMe | H,H |
| A512 | a1 | 4-CF3 | 2 | CH2OMe | Me,Me |
| A513 | a1 | 4-CF3 | 2 | CH2OMe | Et,Et |
| A514 | a1 | 4-CF3 | 2 | CH2OMe | H.Et |
| A515 | a1 | 4-CF3 | 2 | CH2OMe | H,Ph |
| A516 | a1 | 4-CF3 | 2 | CH2OMe | H,C6H4-4-F |
| A517 | a1 | 4-CF3 | 2 | CF3 | H,H |

**Table 13**

| | | | | | |
|---|---|---|---|---|---|
| A518 | a1 | 4-CF3 | 2 | CF3 | Me,Me |
| A519 | a1 | 4-CF3 | 2 | CF3 | Et,Et |
| A520 | a1 | 4-CF3 | 2 | CF3 | H.Et |
| A521 | a1 | 4-CF3 | 2 | CF3 | H,Ph |
| A522 | a1 | 4-CF3 | 2 | CF3 | H,C6H4-4-F |
| A523 | a1 | 4-CF3 | 2 | CH2OPh | H,H |
| A524 | a1 | 4-CF3 | 2 | CH2OPh | H,C6H4-4-F |
| A525 | a1 | 4-CF3 | 2 | CH2OCH2Ph | H,H |
| A526 | a1 | 4-CF3 | 2 | CH2OCH2Ph | H,C6H4-4-F |
| A527 | a1 | 4-CF3 | 2 | CH2-morpholino | H,H |
| A528 | a1 | 4-CF3 | 2 | CH2-morpholino | Me,Me |
| A529 | a1 | 4-CF3 | 2 | CH2-morpholino | Et,Et |
| A530 | a1 | 4-CF3 | 2 | CH2-morpholino | H.Et |
| A531 | a1 | 4-CF3 | 2 | CH2-morpholino | H,Ph |
| A532 | a1 | 4-CF3 | 2 | CH2-morpholino | H,C6H4-4-F |
| A533 | a1 | 4-CF3 | 2 | CH2NHBu | H,H |
| A534 | a1 | 4-CF3 | 2 | CH2NHBu | H,C6H4-4-F |
| A535 | a1 | 4-CF3 | 2 | C≡CPh | H,H |
| A536 | a1 | 4-CF3 | 2 | C≡CPh | H,C6H4-4-F |
| A537 | a1 | 4-CF3 | 2 | Ph | H,H |
| A538 | a1 | 4-CF3 | 2 | Ph | H,C6H4-4-F |
| A539 | a1 | 4-CF3 | 2 | C6H4-4-CF3 | H,H |
| A540 | a1 | 4-CF3 | 2 | C6H4-4-CF3 | H,C6H4-4-F |
| A541 | a1 | 4-CF3 | 2 | C6H4-3-CF3 | H,H |
| A542 | a1 | 4-CF3 | 2 | C6H4-3-CF3 | H,C6H4-4-F |
| A543 | a1 | 4-CF3 | 2 | C6H4-4-OH | H,H |
| A544 | a1 | 4-CF3 | 2 | C6H4-4-OH | H,C6H4-4-F |
| A545 | a1 | 4-CF3 | 2 | CH2Ph | H,H |
| A546 | a1 | 4-CF3 | 2 | CH2Ph | H,C6H4-4-F |
| A547 | a1 | 4-CF3 | 2 | CH2C6H4-4-CF3 | H,H |
| A548 | a1 | 4-CF3 | 2 | CH2C6H4-4-CF3 | Me,Me |
| A549 | a1 | 4-CF3 | 2 | CH2C6H4-4-CF3 | Et,Et |
| A550 | a1 | 4-CF3 | 2 | CH2C6H4-4-CF3 | H.Et |
| A551 | a1 | 4-CF3 | 2 | CH2C6H4-4-CF3 | H,Ph |
| A552 | a1 | 4-CF3 | 2 | CH2C6H4-4-CF3 | H,C6H4-4-F |
| A553 | a1 | 4-CF3 | 2 | CH2C6H4-4-OCF3 | H,H |
| A554 | a1 | 4-CF3 | 2 | CH2C6H4-4-OCF3 | H,C6H4-4-F |
| A555 | a1 | 4-CF3 | 2 | CH2C6H4-4-Ph | H,H |
| A556 | a1 | 4-CF3 | 2 | CH2C6H4-4-Ph | H,C6H4-4-F |
| A557 | a1 | 4-CF3 | 2 | CH2C6H4-2-Cl | H,H |
| A558 | a1 | 4-CF3 | 2 | CH2C6H4-2-Cl | H,C6H4-4-F |
| A559 | a1 | 4-CF3 | 2 | (CH2)2Ph | H,H |
| A560 | a1 | 4-CF3 | 2 | (CH2)2Ph | H,C6H4-4-F |
| A561 | a1 | 4-CF3 | 2 | SPh | H,H |

**Table 14**

| | | | | | |
|---|---|---|---|---|---|
| A562 | a1 | 4-CF3 | 2 | SPh | H,C6H4-4-F |
| A563 | a1 | 4-CF3 | 2 | NH2 | H,H |
| A564 | a1 | 4-CF3 | 2 | NH2 | H,C6H4-4-F |
| A565 | a1 | 4-CF3 | 2 | NHMe | H,H |
| A566 | a1 | 4-CF3 | 2 | NHMe | H,C6H4-4-F |
| A567 | a1 | 4-CF3 | 2 | CH2-piperazino-Ph | H,H |
| A568 | a1 | 4-CF3 | 2 | CH2-piperazino-Ph | H,C6H4-4-F |
| A569 | a1 | 4-CF3 | 2 | CH2-piperidino | H,H |
| A570 | a1 | 4-CF3 | 2 | CH2-piperidino | H,C6H4-4-F |
| A571 | a1 | 4-CF3 | 2 | OCH2Ph | H,H |
| A572 | a1 | 4-CF3 | 2 | OCH2Ph | H,C6H4-4-F |
| A573 | a1 | 4-CF3 | 2 | Ac | H,H |
| A574 | a1 | 4-CF3 | 2 | Ac | H,C6H4-4-F |
| A575 | a1 | 4-CF3 | 2 | CONH2 | H,H |
| A576 | a1 | 4-CF3 | 2 | CONH2 | H,C6H4-4-F |
| A577 | a1 | 4-CF3 | 2 | CSNH2 | H,H |
| A578 | a1 | 4-CF3 | 2 | CSNH2 | H,C6H4-4-F |
| A579 | a1 | 4-CF3 | 2 | OCONH2 | H,H |
| A580 | a1 | 4-CF3 | 2 | OCONH2 | H,C6H4-4-F |
| A581 | a1 | 4-CF3 | 2 | OCSNH2 | H,H |
| A582 | a1 | 4-CF3 | 2 | OCSNH2 | H,C6H4-4-F |
| A583 | a1 | 4-CF3 | 2 | OSO2Me | H,H |
| A584 | a1 | 4-CF3 | 2 | OSO2Me | H,C6H4-4-F |
| A585 | a1 | 4-CF3 | 2 | OSO2Ph | H,H |
| A586 | a1 | 4-CF3 | 2 | OSO2Ph | H,C6H4-4-F |
| A587 | a1 | 4-CF3 | 2 | I | H,H |
| A588 | a1 | 4-CF3 | 2 | I | H,C6H4-4-F |
| A589 | a1 | H | 0 | H | H,H |
| A590 | a1 | 3-F | 0 | H | Me,Me |
| A591 | a1 | 2-Me | 0 | H | Et,Et |
| A592 | a1 | 3-OMe | 0 | H | H.Et |
| A593 | a1 | 4-OH | 0 | H | H,Ph |
| A594 | a1 | 4-OMe | 0 | H | H,C6H4-4-F |
| A595 | a1 | 2-Ac | 0 | Me | H,H |
| A596 | a1 | 4-CH=CH2 | 0 | Me | Me,Me |
| A597 | a1 | 4-CF3, 3-F | 0 | Me | Et,Et |
| A598 | a1 | 4-OCF3 | 0 | Me | H.Et |
| A599 | a1 | 4-SMe | 0 | Me | H,Ph |
| A600 | a1 | 3,5-difluoro | 0 | Me | H,C6H4-4-F |
| A601 | a1 | H | 0 | OMe | H,H |
| A602 | a1 | 3-F | 0 | OMe | Me,Me |
| A603 | a1 | 2-Me | 0 | OMe | Et,Et |
| A604 | a1 | 3-OMe | 0 | OMe | H.Et |
| A605 | a1 | 4-OH | 0 | OMe | H,Ph |

**Table 15**

| | | | | | |
|---|---|---|---|---|---|
| A606 | a1 | 4-OMe | 0 | OMe | H,C6H4-4-F |
| A607 | a1 | 2-Ac | 0 | CH2OH | H,H |
| A608 | a1 | 4-CH=CH2 | 0 | CH2OH | H,C6H4-4-F |
| A609 | a1 | 4-CF3, 3-F | 0 | CH2OMe | H,H |
| A610 | a1 | 4-OCF3 | 0 | CH2OMe | Me,Me |
| A611 | a1 | 4-SMe | 0 | CH2OMe | Et,Et |
| A612 | a1 | 3,5-difluoro | 0 | CH2OMe | H.Et |
| A613 | a1 | H | 0 | CH2OMe | H,Ph |
| A614 | a1 | 3-F | 0 | CH2OMe | H,C6H4-4-F |
| A615 | a1 | 2-Me | 0 | CF3 | H,H |
| A616 | a1 | 3-OMe | 0 | CF3 | Me,Me |
| A617 | a1 | 4-OH | 0 | CF3 | Et,Et |
| A618 | a1 | 4-OMe | 0 | CF3 | H.Et |
| A619 | a1 | 2-Ac | 0 | CF3 | H,Ph |
| A620 | a1 | 4-CH=CH2 | 0 | CF3 | H,C6H4-4-F |
| A621 | a1 | 4-CF3, 3-F | 0 | CH2OPh | H,H |
| A622 | a1 | 4-OCF3 | 0 | CH2OPh | H,C6H4-4-F |
| A623 | a1 | 4-SMe | 0 | CH2OCH2Ph | H,H |
| A624 | a1 | 3,5-difluoro | 0 | CH2OCH2Ph | H,C6H4-4-F |
| A625 | a1 | H | 0 | CH2-morpholino | H,H |
| A626 | a1 | 3-F | 0 | CH2-morpholino | Me,Me |
| A627 | a1 | 2-Me | 0 | CH2-morpholino | Et,Et |
| A628 | a1 | 3-OMe | 0 | CH2-morpholino | H.Et |
| A629 | a1 | 4-OH | 0 | CH2-morpholino | H,Ph |
| A630 | a1 | 4-OMe | 0 | CH2-morpholino | H,C6H4-4-F |
| A631 | a1 | 2-Ac | 0 | CH2NHBu | H,H |
| A632 | a1 | 4-CH=CH2 | 0 | CH2NHBu | H,C6H4-4-F |
| A633 | a1 | 4-CF3, 3-F | 0 | C≡CPh | H,H |
| A634 | a1 | 4-OCF3 | 0 | C≡CPh | H,C6H4-4-F |
| A635 | a1 | 4-SMe | 0 | Ph | H,H |
| A636 | a1 | 3,5-difluoro | 0 | Ph | H,C6H4-4-F |
| A637 | a1 | H | 0 | C6H4-4-CF3 | H,H |
| A638 | a1 | 3-F | 0 | C6H4-4-CF3 | H,C6H4-4-F |
| A639 | a1 | 2-Me | 0 | C6H4-3-CF3 | H,H |
| A640 | a1 | 3-OMe | 0 | C6H4-3-CF3 | H,C6H4-4-F |
| A641 | a1 | 4-OH | 0 | C6H4-4-OH | H,H |
| A642 | a1 | 4-OMe | 0 | C6H4-4-OH | H,C6H4-4-F |
| A643 | a1 | 2-Ac | 0 | CH2Ph | H,H |
| A644 | a1 | 4-CH=CH2 | 0 | CH2Ph | H,C6H4-4-F |
| A645 | a1 | 4-CF3, 3-F | 0 | CH2C6H4-4-CF3 | H,H |
| A646 | a1 | 4-OCF3 | 0 | CH2C6H4-4-CF3 | Me,Me |
| A647 | a1 | 4-SMe | 0 | CH2C6H4-4-CF3 | Et,Et |
| A648 | a1 | 3,5-difluoro | 0 | CH2C6H4-4-CF3 | H.Et |
| A649 | a1 | H | 0 | CH2C6H4-4-CF3 | H,Ph |

**Table 16**

| | | | | | |
|---|---|---|---|---|---|
| A650 | a1 | 3-F | 0 | CH2C6H4-4-CF3 | H,C6H4-4-F |
| A651 | a1 | 2-Me | 0 | CH2C6H4-4-OCF3 | H,H |
| A652 | a1 | 3-OMe | 0 | CH2C6H4-4-OCF3 | H,C6H4-4-F |
| A653 | a1 | 4-OH | 0 | CH2C6H4-4-Ph | H,H |
| A654 | a1 | 4-OMe | 0 | CH2C6H4-4-Ph | H,C6H4-4-F |
| A655 | a1 | 2-Ac | 0 | CH2C6H4-2-Cl | H,H |
| A656 | a1 | 4-CH=CH2 | 0 | CH2C6H4-2-Cl | H,C6H4-4-F |
| A657 | a1 | 4-CF3, 3-F | 0 | (CH2)2Ph | H,H |
| A658 | a1 | 4-OCF3 | 0 | (CH2)2Ph | H,C6H4-4-F |
| A659 | a1 | 4-SMe | 0 | SPh | H,H |
| A660 | a1 | 3,5-difluoro | 0 | SPh | H,C6H4-4-F |
| A661 | a1 | H | 0 | NH2 | H,H |
| A662 | a1 | 3-F | 0 | NH2 | H,C6H4-4-F |
| A663 | a1 | 2-Me | 0 | NHMe | H,H |
| A664 | a1 | 3-OMe | 0 | NHMe | H,C6H4-4-F |
| A665 | a1 | 4-OH | 0 | CH2-piperazino-Ph | H,H |
| A666 | a1 | 4-OMe | 0 | CH2-piperazino-Ph | H,C6H4-4-F |
| A667 | a1 | 2-Ac | 0 | CH2-piperidino | H,H |
| A668 | a1 | 4-CH=CH2 | 0 | CH2-piperidino | H,C6H4-4-F |
| A669 | a1 | 4-CF3, 3-F | 0 | OCH2Ph | H,H |
| A670 | a1 | 4-OCF3 | 0 | OCH2Ph | H,C6H4-4-F |
| A671 | a1 | 4-SMe | 0 | Ac | H,H |
| A672 | a1 | 3,5-difluoro | 0 | Ac | H,C6H4-4-F |
| A673 | a1 | H | 0 | CONH2 | H,H |
| A674 | a1 | 3-F | 0 | CONH2 | H,C6H4-4-F |
| A675 | a1 | 2-Me | 0 | CSNH2 | H,H |
| A676 | a1 | 3-OMe | 0 | CSNH2 | H,C6H4-4-F |
| A677 | a1 | 4-OH | 0 | OCONH2 | H,H |
| A678 | a1 | 4-OMe | 0 | OCONH2 | H,C6H4-4-F |
| A679 | a1 | 2-Ac | 0 | OCSNH2 | H,H |
| A680 | a1 | 4-CH=CH2 | 0 | OCSNH2 | H,C6H4-4-F |
| A681 | a1 | 4-CF3, 3-F | 0 | OSO2Me | H,H |
| A682 | a1 | 4-OCF3 | 0 | OSO2Me | H,C6H4-4-F |
| A683 | a1 | 4-SMe | 0 | OSO2Ph | H,H |
| A684 | a1 | 3,5-difluoro | 0 | OSO2Ph | H,C6H4-4-F |
| A685 | a1 | H | 0 | I | H,H |
| A686 | a1 | 3-F | 0 | I | H,C6H4-4-F |
| A687 | a1 | H | 1 | H | H,H |
| A688 | a1 | 3-F | 1 | H | Me,Me |
| A689 | a1 | 2-Me | 1 | H | Et,Et |
| A690 | a1 | 3-OMe | 1 | H | H.Et |
| A691 | a1 | 4-OH | 1 | H | H,Ph |
| A692 | a1 | 4-OMe | 1 | H | H,C6H4-4-F |
| A693 | a1 | 2-Ac | 1 | Me | H,H |
| A694 | a1 | 4-CH=CH2 | 1 | Me | Me,Me |
| A695 | a1 | 4-CF3, 3-F | 1 | Me | Et,Et |

**Table 17**

| | | | | | |
|---|---|---|---|---|---|
| A696 | a1 | 4-OCF3 | 1 | Me | H.Et |
| A697 | a1 | 4-SMe | 1 | Me | H,Ph |
| A698 | a1 | 3.5-difluoro | 1 | Me | H,C6H4-4-F |
| A699 | a1 | H | 1 | OMe | H,H |
| A700 | a1 | 3-F | 1 | OMe | Me,Me |
| A701 | a1 | 2-Me | 1 | OMe | Et,Et |
| A702 | a1 | 3-OMe | 1 | OMe | H.Et |
| A703 | a1 | 4-OH | 1 | OMe | H,Ph |
| A704 | a1 | 4-OMe | 1 | OMe | H,C6H4-4-F |
| A705 | a1 | 2-Ac | 1 | CH2OH | H,H |
| A706 | a1 | 4-CH=CH2 | 1 | CH2OH | H,C6H4-4-F |
| A707 | a1 | 4-CF3, 3-F | 1 | CH2OMe | H,H |
| A708 | a1 | 4-OCF3 | 1 | CH2OMe | Me,Me |
| A709 | a1 | 4-SMe | 1 | CH2OMe | Et,Et |
| A710 | a1 | 3,5-difluoro | 1 | CH2OMe | H.Et |
| A711 | a1 | H | 1 | CH2OMe | H,Ph |
| A712 | a1 | 3-F | 1 | CH2OMe | H,C6H4-4-F |
| A713 | a1 | 2-Me | 1 | CF3 | H,H |
| A714 | a1 | 3-OMe | 1 | CF3 | Me,Me |
| A715 | a1 | 4-OH | 1 | CF3 | Et,Et |
| A716 | a1 | 4-OMe | 1 | CF3 | H.Et |
| A717 | a1 | 2-Ac | 1 | CF3 | H,Ph |
| A718 | a1 | 4-CH=CH2 | 1 | CF3 | H,C6H4-4-F |
| A719 | a1 | 4-CF3, 3-F | 1 | CH2OPh | H,H |
| A720 | a1 | 4-OCF3 | 1 | CH2OPh | H,C6H4-4-F |
| A721 | a1 | 4-SMe | 1 | CH2OCH2Ph | H,H |
| A722 | a1 | 3,5-difluoro | 1 | CH2OCH2Ph | H,C6H4-4-F |
| A723 | a1 | H | 1 | CH2-morpholino | H,H |
| A724 | a1 | 3-F | 1 | CH2-morpholino | Me,Me |
| A725 | a1 | 2-Me | 1 | CH2-morpholino | Et,Et |
| A726 | a1 | 3-OMe | 1 | CH2-morpholino | H.Et |
| A727 | a1 | 4-OH | 1 | CH2-morpholino | H,Ph |
| A728 | a1 | 4-OMe | 1 | CH2-morpholino | H,C6H4-4-F |
| A729 | a1 | 2-Ac | 1 | CH2NHBu | H,H |
| A730 | a1 | 4-CH=CH2 | 1 | CH2NHBu | H,C6H4-4-F |
| A731 | a1 | 4-CF3, 3-F | 1 | C≡CPh | H,H |
| A732 | a1 | 4-OCF3 | 1 | C≡CPh | H,C6H4-4-F |
| A733 | a1 | 4-SMe | 1 | Ph | H,H |
| A734 | a1 | 3,5-difluoro | 1 | Ph | H,C6H4-4-F |
| A735 | a1 | H | 2 | C6H4-4-CF3 | H,H |
| A736 | a1 | 3-F | 2 | C6H4-4-CF3 | H,C6H4-4-F |
| A737 | a1 | 2-Me | 2 | C6H4-3-CF3 | H,H |
| A738 | a1 | 3-OMe | 2 | C6H4-3-CF3 | H;C6H4-4-F |
| A739 | a1 | 4-OH | 2 | C6H4-4-OH | H,H |
| A740 | a1 | 4-OMe | 2 | C6H4-4-OH | H,C6H4-4-F |
| A741 | a1 | 2-Ac | 2 | CH2Ph | H,H |

**Table 18**

| | | | | | |
|---|---|---|---|---|---|
| A742 | a1 | 4-CH=CH2 | 2 | CH2Ph | H,C6H4-4-F |
| A743 | a1 | 4-CF3, 3-F | 2 | CH2C6H4-4-CF3 | H,H |
| A744 | a1 | 4-OCF3 | 2 | CH2C6H4-4CF3 | Me,Me |
| A745 | a1 | 4-SMe | 2 | CH2C6H4-4-CF3 | Et,Et |
| A746 | a1 | 3,5-difluoro | 2 | CH2C6H4-4-CF3 | H.Et |
| A747 | a1 | H | 2 | CH2C6H4-4-CF3 | H,Ph |
| A748 | a1 | 3-F | 2 | CH2C6H4-4-CF3 | H,C6H4-4-F |
| A749 | a1 | 2-Me | 2 | CH2C6H4-4-OCF3 | H,H |
| A750 | a1 | 3-OMe | 2 | CH2C6H4-4-OCF3 | H,C6H4-4-F |
| A751 | a1 | 4-OH | 2 | CH2C6H4-4-Ph | H,H |
| A752 | a1 | 4-OMe | 2 | CH2C6H4-4-Ph | H,C6H4-4-F |
| A753 | a1 | 2-Ac | 2 | CH2C6H4-2-Cl | H,H |
| A754 | a1 | 4-CH=CH2 | 2 | CH2C6H4-2-Cl | H,C6H4-4-F |
| A755 | a1 | 4-CF3, 3-F | 2 | (CH2)2Ph | H,H |
| A756 | a1 | 4-OCF3 | 2 | (CH2)2Ph | H,C6H4-4-F |
| A757 | a1 | 4-SMe | 2 | SPh | H,H |
| A758 | a1 | 3,5-difluoro | 2 | SPh | H,C6H4-4-F |
| A759 | a1 | H | 2 | NH2 | H,H |
| A760 | a1 | 3-F | 2 | NH2 | H,C6H4-4-F |
| A761 | a1 | 2-Me | 2 | NHMe | H,H |
| A762 | a1 | 3-OMe | 2 | NHMe | H,C6H4-4-F |
| A763 | a1 | 4-OH | 2 | CH2-piperazino-Ph | H,H |
| A764 | a1 | 4-OMe | 2 | CH2-piperazino-Ph | H,C6H4-4-F |
| A765 | a1 | 2-Ac | 2 | CH2-piperidino | H,H |
| A766 | a1 | 4-CH=CH2 | 2 | CH2-piperidino | H,C6H4-4-F |
| A767 | a1 | 4-CF3, 3-F | 2 | OCH2Ph | H,H |
| A768 | a1 | 4-OCF3 | 2 | OCH2Ph | H,C6H4-4-F |
| A769 | a1 | 4-SMe | 2 | Ac | H,H |
| A770 | a1 | 3,5-difluoro | 2 | Ac | H,C6H4-4-F |
| A771 | a1 | H | 2 | CONH2 | H,H |
| A772 | a1 | 3-F | 2 | CONH2 | H,C6H4-4-F |
| A773 | a1 | 2-Me | 2 | CSNH2 | H,H |
| A774 | a1 | 3-OMe | 2 | CSNH2 | H,C6H4-4-F |
| A775 | a1 | 4-OH | 2 | OCONH2 | H,H |
| A776 | a1 | 4-OMe | 2 | OCONH2 | H,C6H4-4-F |
| A777 | a1 | 2-Ac | 2 | OCSNH2 | H,H |
| A778 | a1 | 4-CH=CH2 | 2 | OCSNH2 | H,C6H4-4-F |
| A779 | a1 | 4-CF3, 3-F | 2 | OSO2Me | H,H |
| A780 | a1 | 4-OCF3 | 2 | OSO2Me | H,C6H4-4-F |
| A781 | a1 | 4-SMe | 2 | OSO2Ph | H,H |
| A782 | a1 | 3,5-difluoro | 2 | OSO2Ph | H,C6H4-4-F |
| A783 | a1 | H | 2 | I | H,H |
| A784 | a1 | 3-F | 2 | I | H,C6H4-4-F |

**Table 19**

| | | | | |
|---|---|---|---|---|
| 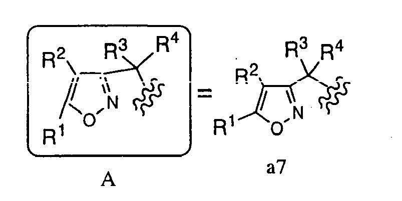 | | | | |

| A Part No. | Type | R1 | R2 | R3,R4 |
|---|---|---|---|---|
| A2353 | a7 | Me | H | H,H |
| A2354 | a7 | Me | H | Me,Me |
| A2355 | a7 | Me | H | Et,Et |
| A2356 | a7 | Me | H | H.Et |
| A2357 | a7 | Me | H | H,Ph |
| A2358 | a7 | Me | H | H,C6H4-4-F |
| A2359 | a7 | Me | Me | H,H |
| A2360 | a7 | Me | Me | Me,Me |
| A2361 | a7 | Me | Me | Et,Et |
| A2362 | a7 | Me | Me | H.Et |
| A2363 | a7 | Me | Me | H,Ph |
| A2364 | a7 | Me | Me | H,C6H4-4-F |
| A2365 | a7 | Me | CH2OMe | H,H |
| A2366 | a7 | Me | CH2OMe | Me,Me |
| A2367 | a7 | Me | CH2OMe | Et,Et |
| A2368 | a7 | Me | CH2OMe | H.Et |
| A2369 | a7 | Me | CH2OMe | H,Ph |
| A2370 | a7 | Me | CH2OMe | H,C6H4-4-F |
| A2371 | a7 | Me | CF3 | H,H |
| A2372 | a7 | Me | CF3 | Me,Me |
| A2373 | a7 | Me | CF3 | Et,Et |
| A2374 | a7 | Me | CF3 | H.Et |
| A2375 | a7 | Me | CF3 | H,Ph |
| A2376 | a7 | Me | CF3 | H,C6H4-4-F |
| A2377 | a7 | Me | CH2OH | H,H |
| A2378 | a7 | Me | CH2OH | H,C6H4-4-F |
| A2379 | a7 | Me | CH2NHBu | H,H |
| A2380 | a7 | Me | CH2NHBu | H,C6H4-4-F |
| A2381 | a7 | Me | CH2C≡CH | H,H |
| A2382 | a7 | Me | CH2C≡CH | H,C6H4-4-F |
| A2383 | a7 | Me | OMe | H,H |
| A2384 | a7 | Me | OMe | H,C6H4-4-F |
| A2385 | a7 | Me | NH2 | H,H |
| A2386 | a7 | Me | NH2 | H,C6H4-4-F |

**Table 20**

| | | | | |
|---|---|---|---|---|
| A2387 | a7 | Me | NHMe | H,H |
| A2388 | a7 | Me | NHMe | H,C6H4-4-F |
| A2389 | a7 | Me | CH2OPh | H,H |
| A2390 | a7 | Me | CH2OPh | H,C6H4-4-F |
| A2391 | a7 | Me | CH2OCH2Ph | H,H |
| A2392 | a7 | Me | CH2OCH2Ph | H,C6H4-4-F |
| A2393 | a7 | Me | CH2-morpholino | H,H |
| A2394 | a7 | Me | CH2-morpholino | H,C6H4-4-F |
| A2395 | a7 | Me | CH=CH-pyridyl | H,H |
| A2396 | a7 | Me | CH=CH-pyridyl | H,C6H4-4-F |
| A2397 | a7 | Me | C≡CPh | H,H |
| A2398 | a7 | Me | C≡CPh | H,C6H4-4-F |
| A2399 | a7 | Me | Ph | H,H |
| A2400 | a7 | Me | Ph | H,C6H4-4-F |
| A2401 | a7 | Me | C6H4-4-CF3 | H,H |
| A2402 | a7 | Me | C6H4-4-CF3 | Me,Me |
| A2403 | a7 | Me | C6H4-4-CF3 | Et,Et |
| A2404 | a7 | Me | C6H4-4-CF3 | H.Et |
| A2405 | a7 | Me | C6H4-4-CF3 | H,Ph |
| A2406 | a7 | Me | C6H4-4-CF3 | H,C6H4-4-F |
| A2407 | a7 | Me | C6H4-3-CF3 | H,H |
| A2408 | a7 | Me | C6H4-3-CF3 | H,C6H4-4-F |
| A2409 | a7 | Me | C6H4-4-OH | H,H |
| A2410 | a7 | Me | C6H4-4-OH | H,C6H4-4-F |
| A2411 | a7 | Me | CH2Ph | H,H |
| A2412 | a7 | Me | CH2Ph | H,C6H4-4-F |
| A2413 | a7 | Me | CH2C6H4-4-CF3 | H,H |
| A2414 | a7 | Me | CH2C6H4-4-CF3 | Me,Me |
| A2415 | a7 | Me | CH2C6H4-4-CF3 | Et,Et |
| A2416 | a7 | Me | CH2C6H4-4-CF3 | H.Et |
| A2417 | a7 | Me | CH2C6H4-4-CF3 | H,Ph |
| A2418 | a7 | Me | CH2C6H4-4-CF3 | H,C6H4-4-F |
| A2419 | a7 | Me | CH2C6H4-4-OCF3 | H,H |
| A2420 | a7 | Me | CH2C6H4-4-OCF3 | H,C6H4-4-F |
| A2421 | a7 | Me | CH2C6H4-4-Ph | H,H |
| A2422 | a7 | Me | CH2C6H4-4-Ph | H,C6H4-4-F |
| A2423 | a7 | Me | CH2C6H4-2-Cl | H,H |
| A2424 | a7 | Me | CH2C6H4-2-Cl | H,C6H4-4-F |
| A2425 | a7 | Me | (CH2)2Ph | H,H |
| A2426 | a7 | Me | (CH2)2Ph | H,C6H4-4-F |
| A2427 | a7 | Me | CH2-piperazino-Ph | H,H |
| A2428 | a7 | Me | CH2-piperazino-Ph | Me,Me |
| A2429 | a7 | Me | CH2-piperazino-Ph | Et,Et |
| A2430 | a7 | Me | CH2-piperazino-Ph | H.Et |

**Table 21**

| | | | | |
|---|---|---|---|---|
| A2431 | a7 | Me | CH2-piperazino-Ph | H,Ph |
| A2432 | a7 | Me | CH2-piperazino-Ph | H,C6H4-4-F |
| A2433 | a7 | Me | CH2-piperidino | H,H |
| A2434 | a7 | Me | CH2-piperidino | H,C6H4-4-F |
| A2435 | a7 | Me | SPh | H,H |
| A2436 | a7 | Me | SPh | H,C6H4-4-F |
| A2437 | a7 | Me | OCH2Ph | H,H |
| A2438 | a7 | Me | OCH2Ph | H,C6H4-4-F |
| A2439 | a7 | Me | Ac | H,H |
| A2440 | a7 | Me | Ac | H,C6H4-4-F |
| A2441 | a7 | Me | CONH2 | H,H |
| A2442 | a7 | Me | CONH2 | H,C6H4-4-F |
| A2443 | a7 | Me | CSNH2 | H,H |
| A2444 | a7 | Me | CSNH2 | H,C6H4-4-F |
| A2445 | a7 | Me | OCONH2 | H,H |
| A2446 | a7 | Me | OCONH2 | H,C6H4-4-F |
| A2447 | a7 | Me | OCSNH2 | H,H |
| A2448 | a7 | Me | OCSNH2 | H,C6H4-4-F |
| A2449 | a7 | Me | OSO2Me | H,H |
| A2450 | a7 | Me | OSO2Me | H,C6H4-4-F |
| A2451 | a7 | Me | OSO2Ph | H,H |
| A2452 | a7 | Me | OSO2Ph | H,C6H4-4-F |
| A2453 | a7 | Me | I | H,H |
| A2454 | a7 | Me | I | H,C6H4-4-F |
| A2455 | a7 | CF3 | H | H,H |
| A2456 | a7 | CF3 | H | Me,Me |
| A2457 | a7 | CF3 | H | Et,Et |
| A2458 | a7 | CF3 | H | H.Et |
| A2459 | a7 | CF3 | H | H,Ph |
| A2460 | a7 | CF3 | H | H,C6H4-4-F |
| A2461 | a7 | CF3 | Me | H,H |
| A2462 | a7 | CF3 | Me | Me,Me |
| A2463 | a7 | CF3 | Me | Et,Et |
| A2464 | a7 | CF3 | Me | H.Et |
| A2465 | a7 | CF3 | Me | H,Ph |
| A2466 | a7 | CF3 | Me | H,C6H4-4-F |
| A2467 | a7 | CF3 | CH2OMe | H,H |
| A2468 | a7 | CF3 | CH2OMe | Me,Me |
| A2469 | a7 | CF3 | CH2OMe | Et,Et |
| A2470 | a7 | CF3 | CH2OMe | H.Et |
| A2471 | a7 | CF3 | CH2OMe | H,Ph |
| A2472 | a7 | CF3 | CH2OMe | H,C6H4-4-F |
| A2473 | a7 | CF3 | CF3 | H,H |
| A2474 | a7 | CF3 | CF3 | Me,Me |

**Table 22**

| | | | | |
|---|---|---|---|---|
| A2475 | a7 | CF3 | CF3 | Et,Et |
| A2476 | a7 | CF3 | CF3 | H.Et |
| A2477 | a7 | CF3 | CF3 | H,Ph |
| A2478 | a7 | CF3 | CF3 | H,C6H4-4-F |
| A2479 | a7 | CF3 | CH2OH | H,H |
| A2480 | a7 | CF3 | CH2OH | H,C6H4-4-F |
| A2481 | a7 | CF3 | CH2NHBu | H,H |
| A2482 | a7 | CF3 | CH2NHBu | H,C6H4-4-F |
| A2483 | a7 | CF3 | CH2C≡CH | H,H |
| A2484 | a7 | CF3 | CH2C≡CH | H,C6H4-4-F |
| A2485 | a7 | CF3 | OMe | H,H |
| A2486 | a7 | CF3 | OMe | H,C6H4-4-F |
| A2487 | a7 | CF3 | NH2 | H,H |
| A2488 | a7 | CF3 | NH2 | H,C6H4-4-F |
| A2489 | a7 | CF3 | NHMe | H,H |
| A2490 | a7 | CF3 | NHMe | H,C6H4-4-F |
| A2491 | a7 | CF3 | CH2OPh | H,H |
| A2492 | a7 | CF3 | CH2OPh | H,C6H4-4-F |
| A2493 | a7 | CF3 | CH2OCH2Ph | H,H |
| A2494 | a7 | CF3 | CH2OCH2Ph | H,C6H4-4-F |
| A2495 | a7 | CF3 | CH2-morpholino | H,H |
| A2496 | a7 | CF3 | CH2-morpholino | H,C6H4-4-F |
| A2497 | a7 | CF3 | CH=CH-pyridyl | H,H |
| A2498 | a7 | CF3 | CH=CH-pyridyl | H,C6H4-4-F |
| A2499 | a7 | CF3 | C≡CPh | H,H |
| A2500 | a7 | CF3 | C≡CPh | H,C6H4-4-F |
| A2501 | a7 | CF3 | Ph | H,H |
| A2502 | a7 | CF3 | Ph | H,C6H4-4-F |
| A2503 | a7 | CF3 | C6H4-4-CF3 | H,H |
| A2504 | a7 | CF3 | C6H4-4-CF3 | Me,Me |
| A2505 | a7 | CF3 | C6H4-4-CF3 | Et,Et |
| A2506 | a7 | CF3 | C6H4-4-CF3 | H.Et |
| A2507 | a7 | CF3 | C6H4-4-CF3 | H,Ph |
| A2508 | a7 | CF3 | C6H4-4-CF3 | H,C6H4-4-F |
| A2509 | a7 | CF3 | C6H4-3-CF3 | H,H |
| A2510 | a7 | CF3 | C6H4-3-CF3 | H,C6H4-4-F |
| A2511 | a7 | CF3 | C6H4-4-OH | H,H |
| A2512 | a7 | CF3 | C6H4-4-OH | H,C6H4-4-F |
| A2513 | a7 | CF3 | CH2Ph | H,H |
| A2514 | a7 | CF3 | CH2Ph | H,C6H4-4-F |
| A2515 | a7 | CF3 | CH2C6H4-4-CF3 | H,H |
| A2516 | a7 | CF3 | CH2C6H4-4-CF3 | Me,Me |
| A2517 | a7 | CF3 | CH2C6H4-4-CF3 | Et,Et |
| A2518 | a7 | CF3 | CH2C6H4-4-CF3 | H.Et |

**Table 23**

| | | | | |
|---|---|---|---|---|
| A2519 | a7 | CF3 | CH2C6H4-4-CF3 | H,Ph |
| A2520 | a7 | CF3 | CH2C6H4-4-CF3 | H,C6H4-4-F |
| A2521 | a7 | CF3 | CH2C6H4-4-OCF3 | H,H |
| A2522 | a7 | CF3 | CH2C6H4-4-OCF3 | H,C6H4-4-F |
| A2523 | a7 | CF3 | CH2C6H4-4-Ph | H,H |
| A2524 | a7 | CF3 | CH2C6H4-4-Ph | H,C6H4-4-F |
| A2525 | a7 | CF3 | CH2C6H4-2-Cl | H,H |
| A2526 | a7 | CF3 | CH2C6H4-2-Cl | H,C6H4-4-F |
| A2527 | a7 | CF3 | (CH2)2Ph | H,H |
| A2528 | a7 | CF3 | (CH2)2Ph | H,C6H4-4-F |
| A2529 | a7 | CF3 | CH2-piperazino-Ph | H,H |
| A2530 | a7 | CF3 | CH2-piperazino-Ph | Me,Me |
| A2531 | a7 | CF3 | CH2-piperazino-Ph | Et,Et |
| A2532 | a7 | CF3 | CH2-piperazino-Ph | H.Et |
| A2533 | a7 | CF3 | CH2-piperazino-Ph | H,Ph |
| A2534 | a7 | CF3 | CH2-piperazino-Ph | H,C6H4-4-F |
| A2535 | a7 | CF3 | CH2-piperidino | H,H |
| A2536 | a7 | CF3 | CH2-piperidino | H,C6H4-4-F |
| A2537 | a7 | CF3 | SPh | H,H |
| A2538 | a7 | CF3 | SPh | H,C6H4-4-F |
| A2539 | a7 | CF3 | OCH2Ph | H,H |
| A2540 | a7 | CF3 | OCH2Ph | H,C6H4-4-F |
| A2541 | a7 | CF3 | Ac | H,H |
| A2542 | a7 | CF3 | Ac | H,C6H4-4-F |
| A2543 | a7 | CF3 | CONH2 | H,H |
| A2544 | a7 | CF3 | CONH2 | H,C6H4-4-F |
| A2545 | a7 | CF3 | CSNH2 | H,H |
| A2546 | a7 | CF3 | CSNH2 | H,C6H4-4-F |
| A2547 | a7 | CF3 | OCONH2 | H,H |
| A2548 | a7 | CF3 | OCONH2 | H,C6H4-4-F |
| A2549 | a7 | CF3 | OCSNH2 | H,H |
| A2550 | a7 | CF3 | OCSNH2 | H,C6H4-4-F |
| A2551 | a7 | CF3 | OSO2Me | H,H |
| A2552 | a7 | CF3 | OSO2Me | H,C6H4-4-F |
| A255 | a7 | CF3 | OSO2Ph | H,H |
| A2554 | a7 | CF3 | OSO2Ph | H,C6H4-4-F |
| A2555 | a7 | CF3 | I | H,H |
| A2556 | a7 | CF3 | I | H,C6H4-4-F |
| A2557 | a7 | CH=CHPh | H | H,H |
| A2558 | a7 | CH=CHPh | H | Me,Me |
| A2559 | a7 | CH=CHPh | H | Et,Et |
| A2560 | a7 | CH=CHPh | H | H.Et |
| A2561 | a7 | CH=CHPh | H | H,Ph |
| A2562 | a7 | CH=CHPh | H | H,C6H4-4-F |

**Table 24**

| | | | | |
|---|---|---|---|---|
| A2563 | a7 | CH=CHPh | Me | H,H |
| A2564 | a7 | CH=CHPh | Me | Me,Me |
| A2565 | a7 | CH=CHPh | Me | Et,Et |
| A2566 | a7 | CH=CHPh | Me | H.Et |
| A2567 | a7 | CH=CHPh | Me | H,Ph |
| A2568 | a7 | CH=CHPh | Me | H,C6H4-4-F |
| A2569 | a7 | CH=CHPh | CH2OMe | H,H |
| A2570 | a7 | CH=CHPh | CH2OMe | Me,Me |
| A2571 | a7 | CH=CHPh | CH2OMe | Et,Et |
| A2572 | a7 | CH=CHPh | CH2OMe | H.Et |
| A2573 | a7 | CH=CHPh | CH2OMe | H,Ph |
| A2574 | a7 | CH=CHPh | CH2OMe | H,C6H4-4-F |
| A2575 | a7 | CH=CHPh | CF3 | H,H |
| A2576 | a7 | CH=CHPh | CF3 | Me,Me |
| A2577 | a7 | CH=CHPh | CF3 | Et,Et |
| A2578 | a7 | CH=CHPh | CF3 | H.Et |
| A2579 | a7 | CH=CHPh | CF3 | H,Ph |
| A2580 | a7 | CH=CHPh | CF3 | H,C6H4-4-F |
| A2581 | a7 | CH=CHPh | CH2OH | H,H |
| A2582 | a7 | CH=CHPh | CH2OH | H,C6H4-4-F |
| A2583 | a7 | CH=CHPh | CH2NHBu | H,H |
| A2584 | a7 | CH=CHPh | CH2NHBu | H,C6H4-4-F |
| A2585 | a7 | CH=CHPh | CH2C≡CH | H,H |
| A2586 | a7 | CH=CHPh | CH2C≡CH | H,C6H4-4-F |
| A2587 | a7 | CH=CHPh | OMe | H,H |
| A2588 | a7 | CH=CHPh | OMe | H,C6H4-4-F |
| A2589 | a7 | CH=CHPh | NH2 | H,H |
| A2590 | a7 | CH=CHPh | NH2 | H,C6H4-4-F |
| A2591 | a7 | CH=CHPh | NHMe | H,H |
| A2592 | a7 | CH=CHPh | NHMe | H,C6H4-4-F |
| A2593 | a7 | CH=CHPh | CH2OPh | H,H |
| A2594 | a7 | CH=CHPh | CH2OPh | H,C6H4-4-F |
| A2595 | a7 | CH=CHPh | CH2OCH2Ph | H,H |
| A2596 | a7 | CH=CHPh | CH2OCH2Ph | H,C6H4-4-F |
| A2597 | a7 | CH=CHPh | CH2-morpholino | H,H |
| A2598 | a7 | CH=CHPh | CH2-morpholino | H,C6H4-4-F |
| A2599 | a7 | CH=CHPh | CH=CH-pyridyl | H,H |
| A2600 | a7 | CH=CHPh | CH=CH-pyridyl | H,C6H4-4-F |
| A2601 | a7 | CH=CHPh | C≡CPh | H,H |
| A2602 | a7 | CH=CHPh | C≡CPh | H,C6H4-4-F |
| A2603 | a7 | CH=CHPh | Ph | H,H |
| A2604 | a7 | CH=CHPh | Ph | H,C6H4-4-F |
| A2605 | a7 | CH=CHPh | C6H4-4-CF3 | H,H |
| A2606 | a7 | CH=CHPh | C6H4-4-CF3 | Me,Me |

**Table 25**

| | | | | |
|---|---|---|---|---|
| A2607 | a7 | CH=CHPh | C6H4-4-CF3 | Et,Et |
| A2608 | a7 | CH=CHPh | C6H4-4-CF3 | H.Et |
| A2609 | a7 | CH=CHPh | C6H4-4-CF3 | H,Ph |
| A2610 | a7 | CH=CHPh | C6H4-4-CF3 | H,C6H4-4-F |
| A2611 | a7 | CH=CHPh | C6H4-3-CF3 | H,H |
| A2612 | a7 | CH=CHPh | C6H4-3-CF3 | H,C6H4-4-F |
| A2613 | a7 | CH=CHPh | C6H4-4-OH | H,H |
| A2614 | a7 | CH=CHPh | C6H4-4-OH | H,C6H4-4-F |
| A2615 | a7 | CH=CHPh | CH2Ph | H,H |
| A2616 | a7 | CH=CHPh | CH2Ph | H,C6H4-4-F |
| A2617 | a7 | CH=CHPh | CH2C6H4-4-CF3 | H,H |
| A2618 | a7 | CH=CHPh | CH2C6H4-4-CF3 | Me,Me |
| A2619 | a7 | CH=CHPh | CH2C6H4-4-CF3 | Et,Et |
| A2620 | a7 | CH=CHPh | CH2C6H4-4-CF3 | H.Et |
| A2621 | a7 | CH=CHPh | CH2C6H4-4-CF3 | H,Ph |
| A2622 | a7 | CH=CHPh | CH2C6H4-4-CF3 | H,C6H4-4-F |
| A2623 | a7 | CH=CHPh | CH2C6H4-4-OCF3 | H,H |
| A2624 | a7 | CH=CHPh | CH2C6H4-4-OCF3 | H,C6H4-4-F |
| A2625 | a7 | CH=CHPh | CH2C6H4-4-Ph | H,H |
| A2626 | a7 | CH=CHPh | CH2C6H4-4-Ph | H,C6H4-4-F |
| A2627 | a7 | CH=CHPh | CH2C6H4-2-Cl | H,H |
| A2628 | a7 | CH=CHPh | CH2C6H4-2-Cl | H,C6H4-4-F |
| A2629 | a7 | CH=CHPh | (CH2)2Ph | H,H |
| A2630 | a7 | CH=CHPh | (CH2)2Ph | H,C6H4-4-F |
| A2631 | a7 | CH=CHPh | CH2-piperazino-Ph | H,H |
| A2632 | a7 | CH=CHPh | CH2-piperazino-Ph | Me,Me |
| A2633 | a7 | CH=CHPh | CH2-piperazino-Ph | Et,Et |
| A2634 | a7 | CH=CHPh | CH2-piperazino-Ph | H.Et |
| A2635 | a7 | CH=CHPh | CH2-piperazino-Ph | H,Ph |
| A2636 | a7 | CH=CHPh | CH2-piperazino-Ph | H,C6H4-4-F |
| A2637 | a7 | CH=CHPh | CH2-piperidino | H,H |
| A2638 | a7 | CH=CHPh | CH2-piperidino | H,C6H4-4-F |
| A2639 | a7 | CH=CHPh | SPh | H,H |
| A2640 | a7 | CH=CHPh | SPh | H,C6H4-4-F |
| A2641 | a7 | CH=CHPh | OCH2Ph | H,H |
| A2642 | a7 | CH=CHPh | OCH2Ph | H,C6H4-4-F |
| A2643 | a7 | CH=CHPh | Ac | H,H |
| A2644 | a7 | CH=CHPh | Ac | H,C6H4-4-F |
| A2645 | a7 | CH=CHPh | CONH2 | H,H |
| A2646 | a7 | CH=CHPh | CONH2 | H,C6H4-4-F |
| A2647 | a7 | CH=CHPh | CSNH2 | H,H |
| A2648 | a7 | CH=CHPh | CSNH2 | H,C6H4-4-F |
| A2649 | a7 | CH=CHPh | OCONH2 | H,H |
| A2650 | a7 | CH=CHPh | OCONH2 | H,C6H4-4-F |

**Table 26**

| | | | | |
|---|---|---|---|---|
| A2651 | a7 | CH=CHPh | OCSNH2 | H,H |
| A2652 | a7 | CH=CHPh | OCSNH2 | H,C6H4-4-F |
| A2653 | a 7 | CH=CHPH | OSO2Me | H,H |
| A2654 | a7 | CH=CHPh | OSO2Me | H,C6H4-4-F |
| A2655 | a7 | CH=CHPh | OSO2Ph | H,H |
| A2656 | a7 | CH=CHPh | OSO2Ph | H,C6H4-4-F |
| A2657 | a7 | CH=CHPh | I | H,H |
| A2658 | a7 | CH=CHPh | I | H,C6H4-4-F |
| A2659 | a7 | ≡CPh | H | H,H |
| A2660 | a7 | ≡CPh | H | Me,Me |
| A2661 | a7 | ≡CPh | H | Et,Et |
| A2662 | a7 | ≡CPh | H | H.Et |
| A2663 | a7 | ≡CPh | H | H,Ph |
| A2664 | a7 | ≡CPh | H | H,C6H4-4-F |
| A2665 | a7 | ≡CPh | Me | H,H |
| A2666 | a7 | ≡CPh | Me | Me,Me |
| A2667 | a7 | ≡CPh | Me | Et,Et |
| A2668 | a7 | ≡CPh | Me | H.Et |
| A2669 | a7 | ≡CPh | Me | H,Ph |
| A2670 | a7 | ≡CPh | Me | H,C6H4-4-F |
| A2671 | a7 | ≡CPh | CH2OMe | H,H |
| A2672 | a7 | ≡CPh | CH2OMe | Me,Me |
| A2673 | a7 | ≡CPh | CH2OMe | Et,Et |
| A2674 | a7 | ≡CPh | CH2OMe | H.Et |
| A2675 | a7 | ≡CPh | CH2OMe | H,Ph |
| A2676 | a7 | ≡CPh | CH2OMe | H,C6H4-4-F |
| A2677 | a7 | ≡CPh | CF3 | H,H |
| A2678 | a7 | ≡CPh | CF3 | Me,Me |
| A2679 | a7 | ≡CPh | CF3 | Et,Et |
| A2680 | a7 | ≡CPh | CF3 | H.Et |
| A2681 | a7 | ≡CPh | CF3 | H,Ph |
| A2682 | a7 | ≡CPh | CF3 | H,C6H4-4-F |
| A2683 | a7 | ≡CPh | CH2OH | H,H |
| A2684 | a7 | ≡CPh | CH2OH | H,C6H4-4-F |
| A2685 | a7 | ≡CPh | CH2NHBu | H,H |
| A2686 | a7 | ≡CPh | CH2NHBu | H,C6H4-4-F |
| A2687 | a7 | ≡CPh | CH2C≡CH | H,H |
| A2688 | a7 | ≡CPh | CH2C≡CH | H,C6H4-4-F |
| A2689 | a7 | ≡CPh | OMe | H,H |
| A2690 | a7 | ≡CPh | OMe | H,C6H4-4-F |
| A2691 | a7 | ≡CPh | NH2 | H,H |
| A2692 | a7 | ≡CPh | NH2 | H,C6H4-4-F |
| A2693 | a7 | ≡CPh | NHMe | H,H |
| A2694 | a7 | ≡CPh | NHMe | H,C6H4-4-F |

**Table 27**

| | | | | |
|---|---|---|---|---|
| A2695 | a7 | ≡CPh | CH2OPh | H,H |
| A2696 | a7 | ≡CPh | CH2OPh | H,C6H4-4-F |
| A2697 | a7 | ≡CPh | CH2OCH2Ph | H,H |
| A2698 | a7 | ≡CPh | CH2OCH2Ph | H,C6H4-4-F |
| A2699 | a7 | ≡CPh | CH2-morpholino | H,H |
| A2700 | a7 | ≡CPh | CH2-morpholino | H,C6H4-4-F |
| A2701 | a7 | ≡CPh | CH=CH-pyridyl | H,H |
| A2702 | a7 | ≡CPh | CH=CH-pyridyl | H,C6H4-4-F |
| A2703 | a7 | ≡CPh | C≡CPh | H,H |
| A2704 | a7 | ≡CPh | C≡CPh | H,C6H4-4-F |
| A2705 | a7 | ≡CPh | Ph | H,H |
| A2706 | a7 | ≡CPh | Ph | H,C6H4-4-F |
| A2707 | a7 | ≡CPh | C6H4-4-CF3 | H,H |
| A2708 | a7 | ≡CPh | C6H4-4-CF3 | Me,Me |
| A2709 | a7 | ≡CPh | C6H4-4-CF3 | Et,Et |
| A2710 | a7 | ≡CPh | C6H4-4-CF3 | H.Et |
| A2711 | a7 | ≡CPh | C6H4-4-CF3 | H,Ph |
| A2712 | a7 | ≡CPh | C6H4-4-CF3 | H,C6H4-4-F |
| A2713 | a7 | ≡CPh | C6H4-3-CF3 | H,H |
| A2714 | a7 | ≡CPh | C6H4-3-CF3 | H,C6H4-4-F |
| A2715 | a7 | ≡CPh | C6H4-4-OH | H,H |
| A2716 | a7 | ≡CPh | C6H4-4-OH | H,C6H4-4-F |
| A2717 | a7 | ≡CPh | CH2Ph | H,H |
| A2718 | a7 | ≡CPh | CH2Ph | H,C6H4-4-F |
| A2719 | a7 | ≡CPh | CH2C6H4-4-CF3 | H,H |
| A2720 | a7 | ≡CPh | CH2C6H4-4-CF3 | Me,Me |
| A2721 | a7 | ≡CPh | CH2C6H4-4-CF3 | Et,Et |
| A2722 | a7 | ≡CPh | CH2C6H4-4-CF3 | H.Et |
| A2723 | a7 | ≡CPh | CH2C6H4-4-CF3 | H,Ph |
| A2724 | a7 | ≡CPh | CH2C6H4-4-CF3 | H,C6H4-4-F |
| A2725 | a7 | ≡CPh | CH2C6H4-4-OCF3 | H,H |
| A2726 | a7 | ≡CPh | CH2C6H4-4-OCF3 | H,C6H4-4-F |
| A2727 | a7 | ≡CPh | CH2C6H4-4-Ph | H,H |
| A2728 | a7 | ≡CPh | CH2C6H4-4-Ph | H,C6H4-4-F |
| A2729 | a7 | ≡CPh | CH2C6H4-2-Cl | H,H |
| A2730 | a7 | ≡CPh | CH2C6H4-2-Cl | H,C6H4-4-F |
| A2731 | a7 | ≡CPh | (CH2)2Ph | H,H |
| A2732 | a7 | ≡CPh | (CH2)2Ph | H,C6H4-4-F |
| A2733 | a7 | ≡CPh | CH2-piperazino-Ph | H,H |
| A2734 | a7 | ≡CPh | CH2-piperazino-Ph | Me,Me |
| A2735 | a7 | ≡CPh | CH2-piperazino-Ph | Et,Et |
| A2736 | a7 | ≡CPh | CH2-piperazino-Ph | H.Et |
| A2737 | a7 | ≡CPh | CH2-piperazino-Ph | H,Ph |
| A2738 | a7 | ≡CPh | CH2-piperazino-Ph | H,C6H4-4-F |

**Table 28**

| | | | | |
|---|---|---|---|---|
| A2739 | a7 | ≡CPh | CH2-piperidino | H,H |
| A2740 | a7 | ≡CPh | CH2-piperidino | H,C6H4-4-F |
| A2741 | a7 | ≡CPh | SPh | H,H |
| A2742 | a7 | ≡CPh | SPh | H,C6H4-4-F |
| A2743 | a7 | ≡CPh | OCH2Ph | H,H |
| A2744 | a7 | ≡CPh | OCH2Ph | H,C6H4-4-F |
| A2745 | a7 | ≡CPh | Ac | H,H |
| A2746 | a7 | ≡CPh | Ac | H,C6H4-4-F |
| A2747 | a7 | ≡CPh | CONH2 | H,H |
| A2748 | a7 | ≡CPh | CONH2 | H,C6H4-4-F |
| A2749 | a7 | ≡CPh | CSNH2 | H,H |
| A2750 | a7 | ≡CPh | CSNH2 | H,C6H4-4-F |
| A2751 | a7 | ≡CPh | OCONH2 | H,H |
| A2752 | a7 | ≡CPh | OCONH2 | H,C6H4-4-F |
| A2753 | a7 | ≡CPh | OCSNH2 | H,H |
| A2754 | a7 | ≡CPh | OCSNH2 | H,C6H4-4-F |
| A2755 | a7 | ≡CPh | OSO2Me | H,H |
| A2756 | a7 | ≡CPh | OSO2Me | H,C6H4-4-F |
| A2757 | a7 | ≡CPh | OSO2Ph | H,H |
| A2758 | a7 | ≡CPh | OSO2Ph | H,C6H4-4-F |
| A2759 | a7 | ≡CPh | I | H,H |
| A2760 | a7 | ≡CPh | I | H,C6H4-4-F |
| A2762 | a7 | F | H | Me,Me |
| A2763 | a7 | Et | . H | Et,Et |
| A2764 | a7 | iBu | H | H.Et |
| A2765 | a7 | CH=CHMe | H | H,Ph |
| A2766 | a7 | OH | H | H,C6H4-4-F |
| A2767 | a7 | OEt | Me | H,H |
| A2768 | a7 | COPh | Me | Me,Me |
| A2769 | a7 | 4-pyridyl | Me | Et,Et |
| A2770 | a7 | morpholino | Me | H.Et |
| A2771 | a7 | NHiPr | Me | H,Ph |
| A2773 | a7 | F | CH2OMe | H,H |
| A2774 | a7 | Et | CH2OMe | Me,Me |
| A2775 | a7 | iBu | CH2OMe | Et,Et |
| A2776 | a7 | CH=CHMe | CH2OMe | H.Et |
| A2777 | a7 | OH | CH2OMe | H,Ph |
| A2778 | a7 | OEt | CH2OMe | H,C6H4-4-F |
| A2779 | a7 | COPh | CF3 | H,H |
| A2780 | a7 | 4-pyridyl | CF3 | Me,Me |
| A2781 | a7 | morpholino | CF3 | Et,Et |
| A2782 | a7 | NHiPr | CF3 | H.Et |
| A2784 | a7 | F | CF3 | H,C6H4-4-F |
| A2785 | a7 | Et | CH2OH | H,H |

**Table 29**

| | | | | |
|---|---|---|---|---|
| A2786 | a7 | iBu | CH2OH | H,C6H4-4-F |
| A2787 | a7 | CH=CHMe | CH2NHBu | H,H |
| A2788 | a7 | OH | CH2NHBu | H,C6H4-4-F |
| A2789 | a7 | OEt | CH2C≡CH | H,H |
| A2790 | a7 | COPh | CH2C≡CH | H,C6H4-4-F |
| A2791 | a7 | 4-pyridyl | OMe | H,H |
| A2792 | a7 | morpholino | OMe | H,C6H4-4-F |
| A2793 | a7 | NHiPr | NH2 | H,H |
| A2795 | a7 | F | NHMe | H,H |
| A2796 | a7 | Et | NHMe | H,C6H4-4-F |
| A2797 | a7 | iBu | CH2OPh | H,H |
| A2798 | a7 | CH=CHMe | CH2OPh | H,C6H4-4-F |
| A2799 | a7 | OH | CH2OCH2Ph | H,H |
| A2800 | a7 | OEt | CH2OCH2Ph | H,C6H4-4-F |
| A2801 | a7 | COPh | CH2-morpholino | H,H |
| A2802 | a7 | 4-pyridyl | CH2-morpholino | H,C6H4-4-F |
| A2803 | a7 | morpholino | CH=CH-pyridyl | H,H |
| A2804 | a7 | NHiPr | CH=CH-pyridyl | H,C6H4-4-F |
| A2806 | a7 | F | C≡CPh | H,C6H4-4-F |
| A2807 | a7 | Et | Ph | H,H |
| A2808 | a7 | iBu | Ph | H,C6H4-4-F |
| A2809 | a7 | CH=CHMe | C6H4-4-CF3 | H,H |
| A2810 | a7 | OH | C6H4-4-CF3 | Me,Me |
| A2811 | a7 | OEt | C6H4-4-CF3 | Et,Et |
| A2812 | a7 | COPh | C6H4-4-CF3 | H.Et |
| A2813 | a7 | 4-pyridyl | C6H4-4-CF3 | H,Ph |
| A2814 | a7 | morpholino | C6H4-4-CF3 | H,C6H4-4-F |
| A2815 | a7 | NHiPr | C6H4-3-CF3 | H,H |
| A2817 | a7 | F | C6H4-4-OH | H,H |
| A2818 | a7 | Et | C6H4-4-OH | H,C6H4-4-F |
| A2819 | a7 | iBu | CH2Ph | H,H |
| A2820 | a7 | CH=CHMe | CH2Ph | H,C6H4-4-F |
| A2821 | a7 | OH | CH2C6H4-4-CF3 | H,H |
| A2822 | a7 | OEt | CH2C6H4-4-CF3 | Me,Me |
| A2823 | a7 | COPh | CH2C6H4-4-CF3 | Et,Et |
| A2824 | a7 | 4-pyridyl | CH2C6H4-4-CF3 | H.Et |
| A2825 | a7 | morpholino | CH2C6H4-4-CF3 | H,Ph |
| A2826 | a7 | NHiPr | CH2C6H4-4-CF3 | H,C6H4-4-F |
| A2828 | a7 | F | CH2C6H4-4-OCF3 | H,C6H4-4-F |
| A2829 | a7 | Et | CH2C6H4-4-Ph | H,H |
| A2830 | a7 | iBu | CH2C6H4-4-Ph | H,C6H4-4-F |
| A2831 | a7 | CH=CHMe | CH2C6H4-2-Cl | H,H |
| A2832 | a7 | OH | CH2C6H4-2-Cl | H,C6H4-4-F |
| A2833 | a7 | OEt | (CH2)2Ph | H,H |

**Table 30**

| | | | | |
|---|---|---|---|---|
| A2834 | a7 | COPh | (CH2)2Ph | H,C6H4-4-F |
| A2835 | a7 | 4-pyridyl | CH2-piperazino-Ph | H,H |
| A2836 | a7 | morpholino | CH2-piperazino-Ph | Me,Me |
| A2837 | a7 | NHiPr | CH2-piperazino-Ph | Et,Et |
| A2839 | a7 | F | CH2-piperazino-Ph | H,Ph |
| A2840 | a7 | Et | CH2-piperazino-Ph | H,C6H4-4-F |
| A2841 | a7 | iBu | CH2-piperidino | H,H |
| A2842 | a7 | CH=CHMe | CH2-piperidino | H,C6H4-4-F |
| A2843 | a7 | OH | SPh | H,H |
| A2844 | a7 | OEt | SPh | H,C6H4-4-F |
| A2845 | a7 | COPh | OCH2Ph | H,H |
| A2846 | a7 | 4-pyridyl | OCH2Ph | H,C6H4-4-F |
| A2847 | a7 | morpholino | Ac | H,H |
| A2848 | a7 | NHiPr | Ac | H,C6H4-4-F |
| A2850 | a7 | F | CONH2 | H,C6H4-4-F |
| A2851 | a7 | Et | CSNH2 | H,H |
| A2852 | a7 | iBu | CSNH2 | H,C6H4-4-F |
| A2853 | a7 | CH=CHMe | OCONH2 | H,H |
| A2854 | a7 | OH | OCONH2 | H,C6H4-4-F |
| A2855 | a7 | OEt | OCSNH2 | H,H |
| A2856 | a7 | COPh | OCSNH2 | H,C6H4-4-F |
| A2857 | a7 | 4-pyridyl | OSO2Me | H,H |
| A2858 | a7 | morpholino | OSO2Me | H,C6H4-4-F |
| A2859 | a7 | NHiPr | OSO2Ph | H,H |
| A2861 | a7 | F | I | H,H |
| A2862 | a7 | Et | I | H,C6H4-4-F |
| A3385 | a7 | CH2OMe | Me | H,H |
| A3386 | a7 | CH2OMe | Me | Me,Me |
| A3387 | a7 | CH2OMe | Me | Et,Et |
| A3388 | a7 | CH2OMe | Me | H.Et |
| A3389 | a7 | CH2OMe | Me | H,Ph |
| A3390 | a7 | CH2OMe | Me | H,C6H4-4-F |
| A3397 | a7 | CH2OH | Me | H,H |
| A3552 | a7 | CH2-piperazino-Ph | CF3 | H.Et |
| A3553 | a7 | CH2-piperazino-Ph | CF3 | H,Ph |
| A3554 | a7 | CH2-piperazino-Ph | CF3 | H,C6H4-4-F |
| A3555 | a7 | CH2-piperidino | CF3 | H,H |
| A3556 | a7 | CH2-piperidino | CF3 | H,C6H4-4-F |
| A3557 | a7 | SPh | CF3 | H,H |
| A3558 | a7 | SPh | CF3 | H,C6H4-4-F |
| A3559 | a7 | OCH2Ph | CF3 | H,H |
| A3560 | a7 | OCH2Ph | CF3 | H,C6H4-4-F |
| A3561 | a7 | Ac | CF3 | H,H |
| A3562 | a7 | Ac | CF3 | H,C6H4-4-F |

**Table 31**

| | | | | |
|---|---|---|---|---|
| A3563 | a7 | CONH2 | CF3 | H,H |
| A3564 | a7 | CONH2 | CF3 | H,C6H4-4-F |
| A3565 | a7 | CSNH2 | CF3 | H,H |
| A3566 | a7 | CSNH2 | CF3 | H,C6H4-4-F |
| A3567 | a7 | OCONH2 | CF3 | H,H |
| A3568 | a7 | OCONH2 | CF3 | H,C6H4-4-F |
| A3569 | a7 | OCSNH2 | CF3 | H,H |
| A3570 | a7 | OCSNH2 | CF3 | H,C6H4-4-F |
| A3571 | a7 | OSO2Me | CF3 | H,H |
| A3572 | a7 | OSO2Me | CF3 | H,C6H4-4-F |
| A3573 | a7 | OSO2Ph | CF3 | H,H |
| A3574 | a7 | OSO2Ph | CF3 | H,C6H4-4-F |
| A3575 | a7 | I | CF3 | H,H |
| A3576 | a7 | I | CF3 | H,C6H4-4-F |
| A3627 | a7 | C6H4-4-CF3 | CH=CHPh | Et,Et |
| A3628 | a7 | C6H4-4-CF3 | CH=CHPh | H.Et |
| A3629 | a7 | C6H4-4-CF3 | CH=CHPh | H,Ph |
| A3630 | a7 | C6H4-4-CF3 | CH=CHPh | H,C6H4-4-F |
| A3631 | a7 | C6H4-3-CF3 | CH=CHPh | H,H |
| A3632 | a7 | C6H4-3-CF3 | CH=CHPh | H,C6H4-4-F |
| A3633 | a7 | C6H4-4-OH | CH=CHPh | H,H |
| A3634 | a7 | C6H4-4-OH | CH=CHPh | H,C6H4-4-F |
| A3635 | a7 | CH2Ph | CH=CHPh | H,H |
| A3636 | a7 | CH2Ph | CH=CHPh | H,C6H4-4-F |
| A3637 | a7 | CH2C6H4-4-CF3 | CH=CHPh | H,H |
| A3638 | a7 | CH2C6H4-4-CF3 | CH=CHPh | Me,Me |
| A3639 | a7 | CH2C6H4-4-CF3 | CH=CHPh | Et,Et |
| A3640 | a7 | CH2C6H4-4-CF3 | CH=CHPh | H.Et |
| A3641 | a7 | CH2C6H4-4-CF3 | CH=CHPh | H,Ph |
| A3642 | a7 | CH2C6H4-4-CF3 | CH=CHPh | H,C6H4-4-F |
| A3643 | a7 | CH2C6H4-4-OCF3 | CH=CHPh | H,H |
| A3644 | a7 | CH2C6H4-4-OCF3 | CH=CHPh | H,C6H4-4-F |
| A3645 | a7 | CH2C6H4-4-Ph | CH=CHPh | H,H |
| A3646 | a7 | CH2C6H4-4-Ph | CH=CHPh | H,C6H4-4-F |
| A3647 | a7 | CH2C6H4-2-Cl | CH=CHPh | H,H |
| A3648 | a7 | CH2C6H4-2-Cl | CH=CHPh | H,C6H4-4-F |
| A3649 | a7 | (CH2)2Ph | CH=CHPh | H,H |
| A3650 | a7 | (CH2)2Ph | . CH=CHPh | H,C6H4-4-F |
| A3651 | a7 | CH2-piperazino-Ph | CH=CHPh | H,H |
| A3652 | a7 | CH2-piperazino-Ph | CH=CHPh | Me,Me |
| A3704 | a7 | CH2OH | ≡CPh | H,C6H4-4-F |
| A3705 | a7 | CH2NHBu | ≡CPh | H,H |
| A3706 | a7 | CH2NHBu | ≡CPh | H,C6H4-4-F |
| A3707 | a7 | CH2C≡CH | ≡CPh | H,H |
| A3708 | a7 | CH2C≡CH | ≡CPh | H,C6H4-4-F |
| A3709 | a7 | OMe | ≡CPh | H,H |

**Table 32**

| | | | | |
|---|---|---|---|---|
| A3710 | a7 | OMe | ≡CPh | H,C6H4-4-F |
| A3711 | a7 | NH2 | ≡CPh | H,H |
| A3712 | a7 | NH2 | ≡CPh | H,C6H4-4-F |
| A3713 | a7 | NHMe | ≡CPh | H,H |
| A3714 | a7 | NHMe | ≡CPh | H,C6H4-4-F |
| A3715 | a7 | CH2OPh | ≡CPh | H,H |
| A3716 | a7 | CH2OPh | ≡CPh | H,C6H4-4-F |
| A3717 | a7 | CH2OCH2Ph | ≡CPh | H,H |
| A3718 | a7 | CH2OCH2Ph | ≡CPh | H,C6H4-4-F |
| A3719 | a7 | CH2-morpholino | ≡CPh | H,H |
| A3720 | a7 | CH2-morpholino | ≡CPh | H,C6H4-4-F |
| A3721 | a7 | CH=CH-pyridyl | ≡CPh | H,H |
| A3722 | a7 | CH=CH-pyridyl | ≡CPh | H,C6H4-4-F |
| A3723 | a7 | C≡CPh | ≡CPh | H,H |
| A3724 | a7 | C≡CPh | ≡CPh | H,C6H4-4-F |
| A3725 | a7 | Ph | ≡CPh | H,H |
| A3726 | a7 | Ph | ≡CPh | H,C6H4-4-F |
| A3727 | a7 | C6H4-4-CF3 | ≡CPh | H,H |
| A3728 | a7 | C6H4-4-CF3 | ≡CPh | Me,Me |
| A3806 | a7 | CH2OH | iBu | H,C6H4-4-F |
| A3807 | a7 | CH2NHBu | CH=CHMe | H,H |
| A3808 | a7 | CH2NHBu | OH | H,C6H4-4-F |
| A3809 | a7 | CH2C≡CH | OEt | H,H |
| A3810 | a7 | CH2C≡CH | COPh | H,C6H4-4-F |
| A3811 | a7 | OMe | 4-pyridyl | H,H |
| A3812 | a7 | OMe | morpholino | H,C6H4-4-F |
| A3813 | a7 | NH2 | NHiPr | H,H |
| A3814 | a7 | NH2 | H | H,C6H4-4-F |
| A3815 | a7 | NHMe | F | H,H |
| A3816 | a7 | NHMe | Et | H,C6H4-4-F |
| A3817 | a7 | CH2OPh | iBu | H,H |
| A3818 | a7 | CH2OPh | CH=CHMe | H,C6H4-4-F |
| A3819 | a7 | CH2OCH2Ph | OH | H,H |
| A3820 | a7 | CH2OCH2Ph | OEt | H,C6H4-4-F |
| A3821 | a7 | CH2-morpholino | COPh | H,H |
| A3822 | a7 | CH2-morpholino | 4-pyridyl | H,C6H4-4-F |
| A3823 | a7 | CH=CH-pyridyl | morpholino | H,H |
| A3824 | a7 | CH=CH-pyridyl | NHiPr | H,C6H4-4-F |
| A3825 | a7 | C≡CPh | H | H,H |
| A3826 | a7 | C≡CPh | F | H,C6H4-4-F |
| A3827 | a7 | Ph | Et | H,H |
| A3828 | a7 | Ph | iBu | H,C6H4-4-F |
| A3829 | a7 | C6H4-4-CF3 | CH=CHMe | H,H |
| A3830 | a7 | C6H4-4-CF3 | OH | Me,Me |

**Table 33**

| | | | | | |
|---|---|---|---|---|---|
| 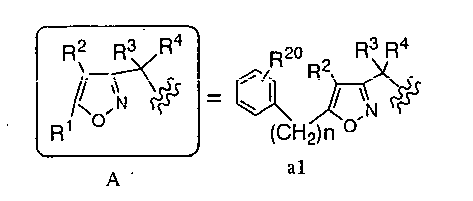 | | | | | |

| A Part No. | Type | R20 | n | R2 | R3,R4 |
|---|---|---|---|---|---|
| A3883 | a1 | 4-Cl | 0 | Me | H,4-pyridyl |
| A3884 | a1 | 4-Cl | 0 | CH2OMe | H,CH2CH=CH2 |
| A3885 | a1 | 4-Cl | 0 | CH2-morpholino | H,C≡CPh |
| A3886 | a1 | 4-CF3 | 0 | CH2C6H4-4-CF3 | H,CH=CH2 |
| A3887 | a1 | 4-CF3 | 0 | OMe | H,C6H4-4-Ph |
| A3888 | a1 | 4-CF3 | 0 | CF3 | H,CH2C≡CH |
| A3889 | a1 | 4-CF3 | 0 | Me | H,CH=CHPh |
| A3890 | a1 | 4-CF3 | 0 | CH2OMe | H,3-furyl |

2) A compound wherein the part (B part) of formula: 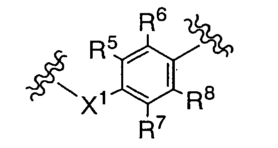 is one of the followings,

**Table 34**

| | | |
|---|---|---|
| 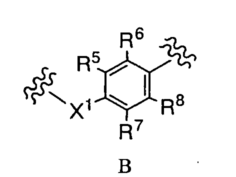 | | |

| B part No. | X1 | R5,R6,R7,R8 |
|---|---|---|
| B1 | S | H,H,H,H |
| B2 | S | H,Me,H,H |
| B3 | S | H,nPr,H,H |
| B4 | S | H,OCH2CF3,H,H |
| B5 | S | H,OH, H,H |
| B6 | S | H,OMe,H,H |
| B7 | S | H,SMe,H,H |
| B8 | S | Me,H,H,H |
| B9 | S | OMe,H,H,H |
| B10 | S | H, SPh,H,H |
| B11 | S | Me,Me,Me,Me |
| B12 | S | H,Me,H,Me |
| B13 | S | OCH2CF3,H,H,H |
| B14 | S | Cl,Cl,H,H |
| B15 | S | Cl,H,H,H |
| B16 | S | H,Cl,H,H |
| B17 | S | H,F,H,H |
| B18 | S | F,F,H,H |
| B19 | S | F,H,H,H |
| B20 | S | H,CH2CH=CH2,H,H |
| B21 | O | H,H,H,H |
| B22 | O | H,Me,H,H |
| B23 | O | H,nPr,H,H |
| B24 | O | H,OCH2CF3,H,H |
| B25 | O | H,OH, H,H |
| B26 | O | H,OMe,H,H |
| B27 | O | H,SMe,H,H |
| B28 | O | Me,H,H,H |
| B29 | O | OMe,H,H,H |
| B30 | O | Me,Me,H,H |
| B31 | O | Me,Me,Me,Me |
| B32 | O | H,OPh,H,H |
| B33 | O | OCH2CF3,H,H,H |
| B34 | O | Cl,Cl,H,H |
| B35 | O | Cl,H,H,H |
| B36 | O | H,Cl,H,H |
| B37 | O | H,F,H,H |
| B38 | O | F,F,H,H |
| B39 | O | F,H,H,H |
| B40 | O | H,CH2CH=CH2,H,H |
| B41 | CH2CO | H,H,H,H |

**Table 35**

| | | |
|---|---|---|
| B42 | CH2CO | H,Me,H,H |
| B43 | CH2CO | H,nPr,H,H |
| B44 | CH2CO | H,OCH2CF3,H,H |
| B45 | CH2CO | H,OH, H,H |
| B46 | CH2CO | H,OMe,H,H |
| B47 | CH2CO | H,SMe,H,H |
| B48 | CH2CO | Cl,H;H,H |
| B49 | CH2CO | OMe,H,H,H |
| B50 | CH2CO | Me,Me,H,H |
| B51 | CH2CO | Me,CH=CH2,Me,Me |
| B52 | CH2CO | H,Me,H,NHMe |
| B53 | CH2CO | OCH2CF3,H,H,H |
| B54 | CH2CO | Cl,Cl,H,H |
| B55 | CH2CO | Cl,H,H,H |
| B56 | CH2CO | H,F,H,H |
| B57 | CH2CO | H,CH2CH=CH2,H,H |
| B58 | NH | H,H,H,H |
| B59 | NH | H,Me,H,H |
| B60 | NH | H,nPr,H,H |
| B61 | NH | H,OCH2CF3,H,H |
| B62 | NH | H,OH, H,H |
| B63 | NH | H,OMe,H,H |
| B64 | NH | H,SMe,H,H |
| B65 | NH | Me,H,H,H |
| B66 | NH | OMe,H,H,H |
| B67 | NH | Me,CH≡CH,H,H |
| B68 | NH | Me,Me,Me,Me |
| B69 | NH | H,Ac,H,H |
| B70 | NH | OCH2CF3,H,H,H |
| B71 | NH | Cl,Cl,H,H |
| B72 | NH | Cl,H,H,H |
| B73 | NH | H,F,H,H |
| B74 | NH | H,CH2CH=CH2,H,H |
| B75 | NMe | H,H,H,H |
| B76 | NMe | H,Me,H,H |
| B77 | NMe | H,nPr,H,H |
| B78 | NMe | H,OCH2CF3,H,H |
| B79 | NMe | H,OH, H,H |
| B80 | NMe | H,OMe,H,H |
| B81 | NMe | H,SMe,H,H |
| B82 | NMe | Me,H,H,H |
| B83 | NMe | H,Ph,H,H |
| B84 | NMe | Me,Me,H,H |
| B85 | NMe | Me,Me,Me,Me |
| B86 | NMe | H,Me,H,Me |
| B87 | NMe | OCH2CF3,H,H,H |
| B88 | NMe | Cl,Cl,H,H |
| B89 | NMe | Cl,H,H,H |

**Table 36**

| | | |
|---|---|---|
| B90 | NMe | H,F,H,H |
| B91 | NMe | H,CH2CH=CH2,H,H |
| B92 | NEt | H,H,H,H |
| B93 | NMe | H,Me,H,H |
| B94 | NCH2Ph | H,nPr,H,H |
| B95 | NAc | H,OCH2CF3,H,H |
| B96 | NCOEt | H,OMe,H,H |
| B97 | NCOPh | Me,H,H,H |
| B98 | NSO2Me | H,Ph,H,H |
| B99 | NSO2Et | Me,Me,H,H |
| B100 | NSO2Ph | Me,Me,Me,Me |
| B101 | NSO2C6H4-p-Me | OCH2CF3,H,H,H |
| B102 | CH2O | H,H,H,H |
| B103 | CH2O | H,Me,H,H |
| B104 | CH2O | H,nPr,H,H |
| B105 | CH2O | H,OCH2CF3,H,H |
| B106 | CH2O | H,OH, H,H |
| B107 | CH2O | H,OMe,H,H |
| B108 | CH2O | H,Cl,H,H |
| B109 | CH2O | Me,H,H,H |
| B110 | CH2O | H,Ph,H,H |
| B111 | CH2O | Me,Me,H,H |
| B112 | CH2O | Me,Me,Me,Me |
| B113 | CH2O | H,Me,H,Me |
| B114 | CHEtO | OCH2CF3,H,H,H |
| B115 | OCH2 | H,H,H,H |
| B116 | OCH2 | H,Me,H,H |
| B117 | OCH2 | H,nPr,H,H |
| B118 | OCH2 | H,OCH2CF3,H,H |
| B119 | OCH2 | H,OH, H,H |
| B120 | OCH2 | H,OMe,H,H |
| B121 | OCH2 | H,SMe,H,H |
| B122 | OCH2 | Me,H,H,H |
| B123 | OCH2 | H,Ph,H,H |
| B124 | OCH2 | H,F,H,H |
| B125 | OCH2 | Me,Me,Me,Me |
| B126 | OCH2 | H,Me,H,Me |
| B127 | OCHMe | OCH2CF3,H,H,H |

3) A compound of the part (C part) of formula: 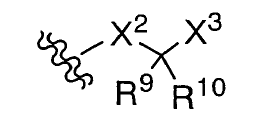 is one of the followings.

**Table 37**

| | | | | |
|---|---|---|---|---|
| 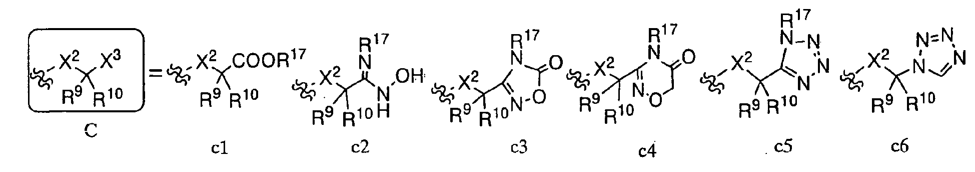 | | | | |

| C part No. | Type | X2 | R9,R10 | R17 |
|---|---|---|---|---|
| C1 | c1 | O | H,H | H |
| C2 | c1 | O | H,H | Me |
| C3 | c1 | O | Me,H | H |
| C4 | c1 | O | Me,H | Me |
| C5 | c1 | O | Et,H | H |
| C6 | c1 | O | CH2OMe,H | Me |
| C7 | c1 | O | nPr,H | |
| C8 | c1 | O | nPr,H | Me |
| C9 | c1 | O | Me,Me | H |
| C10 | c1 | O | Ph,Me | Me |
| C11 | c1 | S | H,H | H |
| C12 | c1 | S | H,H | Me |
| C13 | c1 | S | CH2Ph,H | H |
| C14 | c1 | S | Me,H | Me |
| C15 | c1 | S | Et,H | H |
| C16 | c1 | S | Et,H | Et |
| C17 | c1 | S | nPr,H | H |
| C18 | c1 | S | nPr,H | iPr |
| C19 | c1 | S | Me,Me | H |
| C20 | c1 | S | Me,Me | Me |
| C21 | c1 | NH | H,H | H |
| C22 | c1 | NH | H,H | Me |
| C23 | c1 | NH | Me,H | H |
| C24 | c1 | NH | Me,H | Me |
| C25 | c1 | NH | Et,H | H |
| C26 | c1 | NH | Et,H | Me |
| C27 | c1 | NH | nPr,H | H |
| C28 | c1 | NH | nPr,H | Me |
| C29 | c1 | NH | Me,Me | H |
| C30 | c1 | NH | Me,Me | tBu |
| C31 | c1 | NEt | H,H | H |
| C32 | c1 | NMe | H,H | Me |
| C33 | c1 | NCH2Ph | Me,H | H |
| C34 | c1 | NAc | Me,H | Me |
| C35 | c1 | NCOEt | Et,H | H |
| C36 | c1 | NCOPh | Et,H | Me |
| C37 | c1 | NSO2Me | nPr,H | H |
| C38 | c1 | NSO2Et | nPr,H | Me |
| C39 | c1 | NSO2Ph | Me,Me | H |
| C40 | c1 | NSO2C6H4-p-Me | Me,Me | Me |
| C41 | c1 | *1 | *1 | H |
| C42 | c1 | *1 | *1 | Me |
| C43 | c2 | O | H,H | H |
| C44 | c2 | Single bond | H,H | H |
| C45 | c2 | S | H,H | H |
| C46 | c2 | CH2 | H,H | H |
| C47 | c2 | NH | H,H | H |
| C48 | c2 | *1 | *1 | H |
| C49 | c3 | O | H,H | H |
| C50 | c3 | O | H,H | Me |
| C51 | c3 | O | Me,H | H |
| C52 | c3 | O | Me,H | Me |
| C53 | c3 | O | Et,H | H |

**Table 38**

| | | | | |
|---|---|---|---|---|
| C54 | c3 | O | OEt,H | Me |
| C55 | c3 | O | nPr,H | H |
| C56 | c3 | O | nPr,H | Me |
| C57 | c3 | O | Me,Me | H |
| C58 | c3 | O | Me,Me | Me |
| C59 | c3 | Single bond | H,H | H |
| C60 | c3 | Single bond | OMe,H | H |
| C61 | c3 | Single bond | Et,H | H |
| C62 | c3 | Single bond | nPr,H | H |
| C63 | c3 | Single bond | Me,Me | H |
| C64 | c3 | S | H,H | H |
| C65 | c3 | S | Ph,Me | H |
| C66 | c3 | S | Et,H | H |
| C67 | c3 | S | nPr,H | H |
| C68 | c3 | S | Me,Me | H |
| C69 | c3 | CH2 | H,H | H |
| C70 | c3 | CH2 | Me,H | H |
| C71 | c3 | CH2 | OEt,H | H |
| C72 | c3 | CH2 | nPr,H | H |
| C73 | c3 | CH2 | Me,Me | H |
| C74 | c3 | NH | H,H | H |
| C75 | c3 | NMe | OMe,H | H |
| C76 | c3 | NH | Et,H | H |
| C77 | c3 | NH | nPr,H | H |
| C78 | c3 | NMe | Me,Me | H |
| C79 | c3 | *1 | *1 | H |
| C80 | c3 | *2 | *2 | Me |
| C81 | c4 | O | H,H | H |
| C82 | c4 | Single bond | H,H | H |
| C83 | c4 | S | H,H | H |
| C84 | c4 | CH2 | H,H | H |
| C85 | c4 | NH | H,H | H |
| C86 | c4 | *1 | *1 | H |
| C87 | c5 | O | H,H | H |
| C88 | c5 | Single bond | H,H | H |
| C89 | c5 | S | H,H | H |
| C90 | c5 | CH2 | H,H | H |
| C91 | c5 | NH | H,H | H |
| C92 | c5 | *1 | *1 | H |
| C93 | c6 | O | H,H | H |
| C94 | c6 | Single bond | H,H | H |
| C95 | c6 | S | H.H | H |
| C96 | c6 | CH2 | H,H | H |
| C97 | c6 | NH | H,H | H |
| C98 | c6 | *2 | *2 | H |
| C99 | c1 | CH2 | H,H | H |
| C100 | c1 | CH2 | H,Me | H |
| C101 | c1 | CH2 | H,H | Me |
| C102 | c1 | CH2 | H,Me | Me |

| | | | | |
|---|---|---|---|---|
| 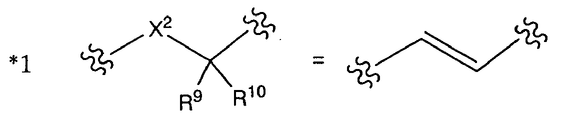 | | | | |
| 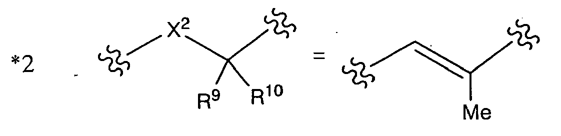 | | | | |

Concretely, a compound wherein the combination of A part, B part and C part of a compound (I) is the followings is preferable.

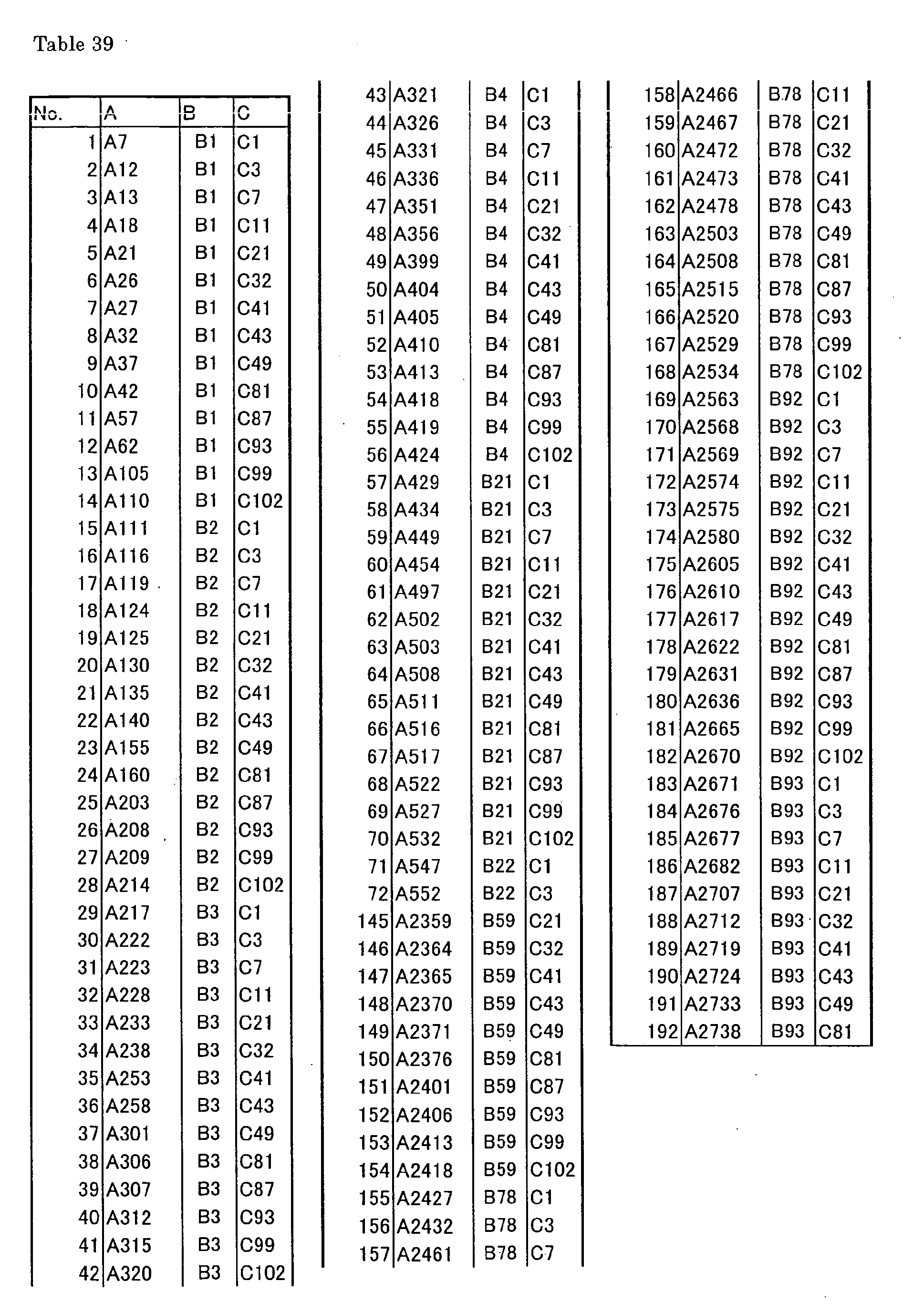

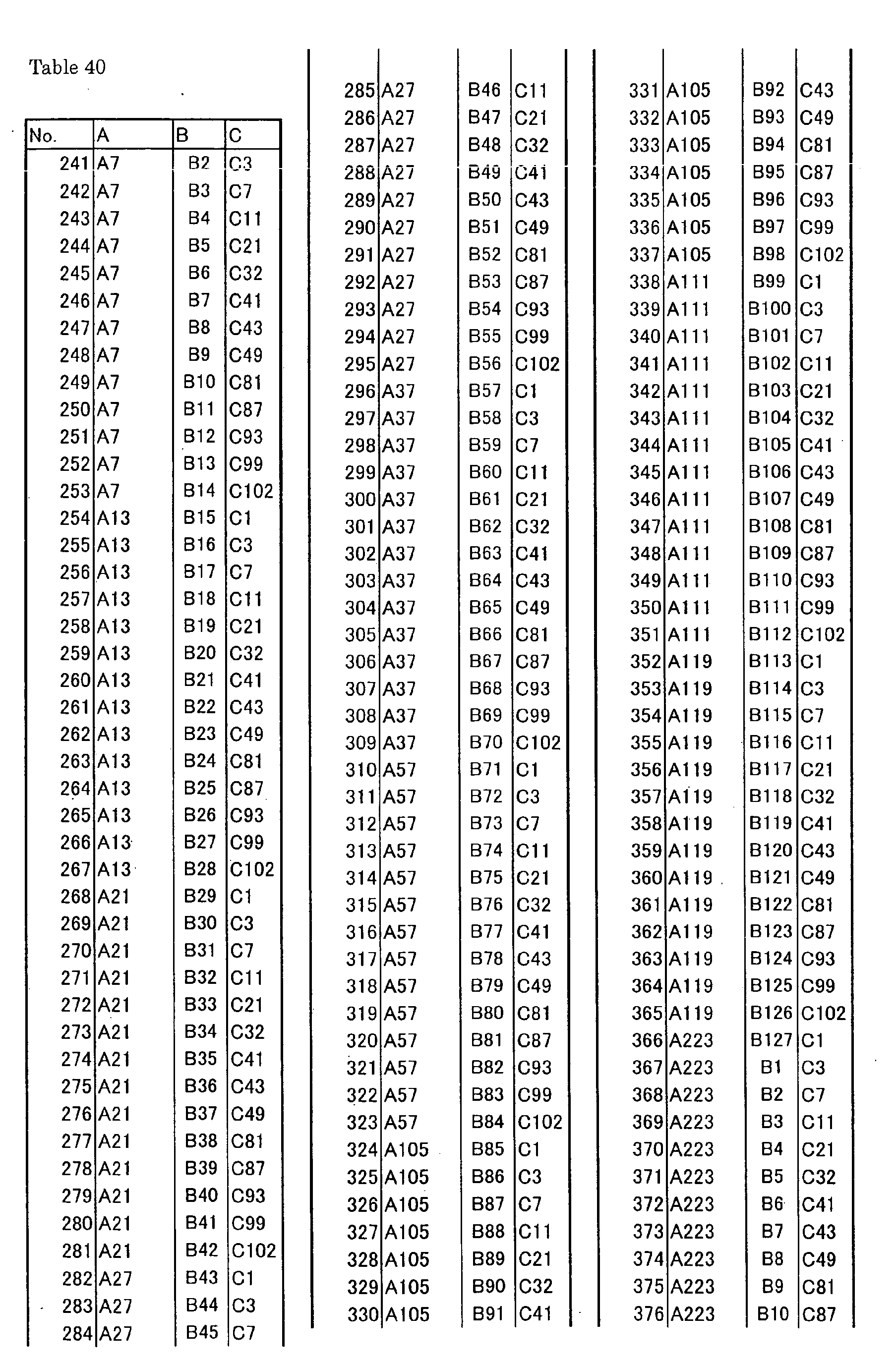

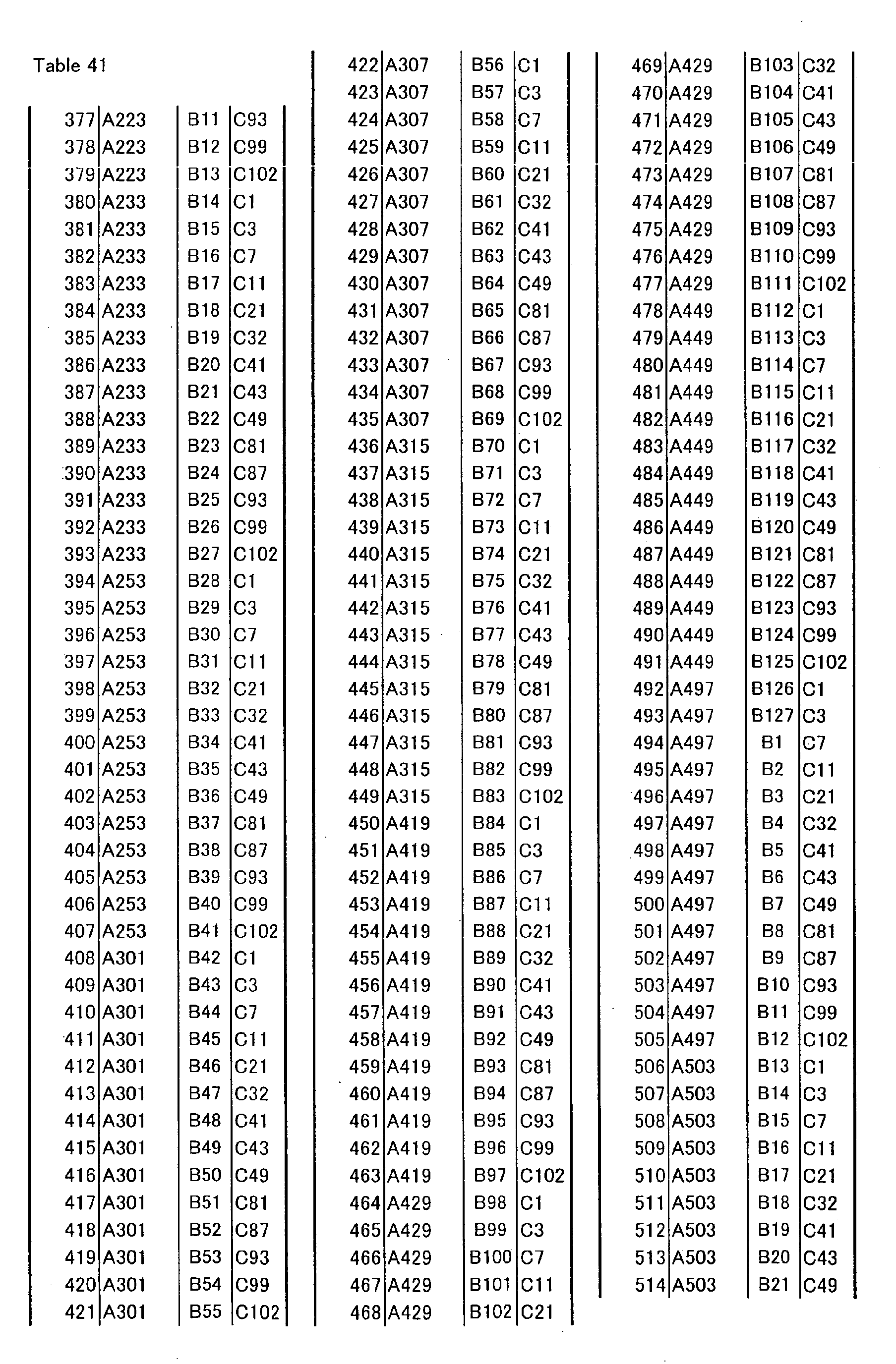

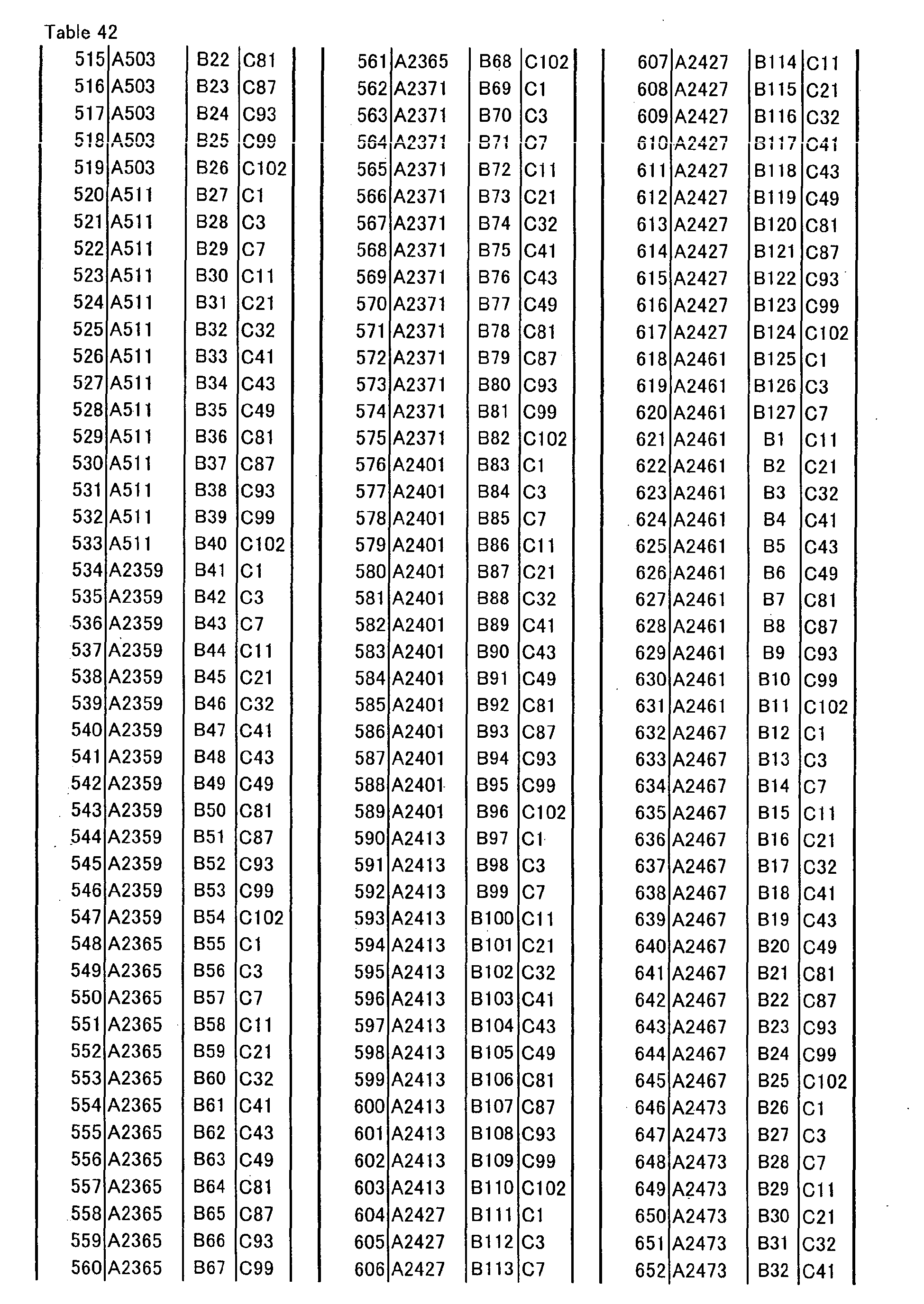

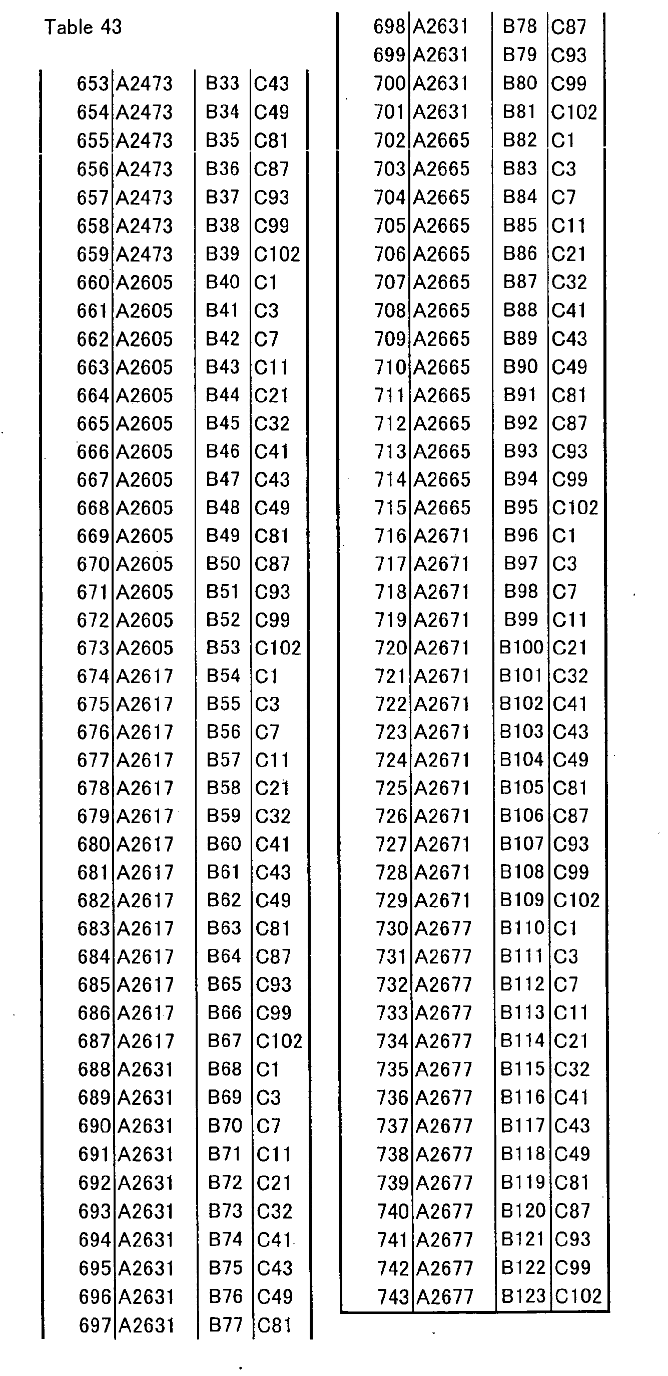

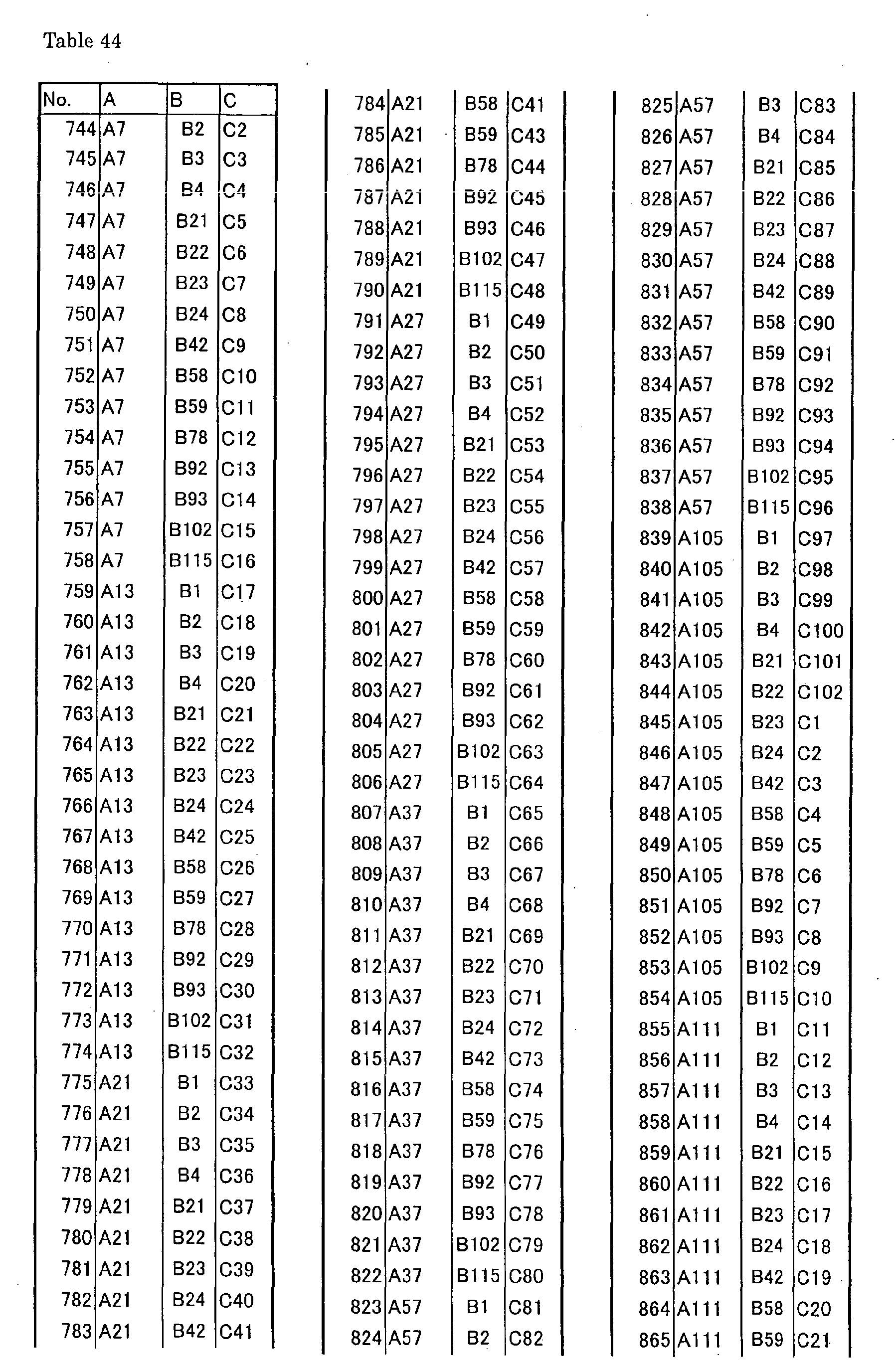

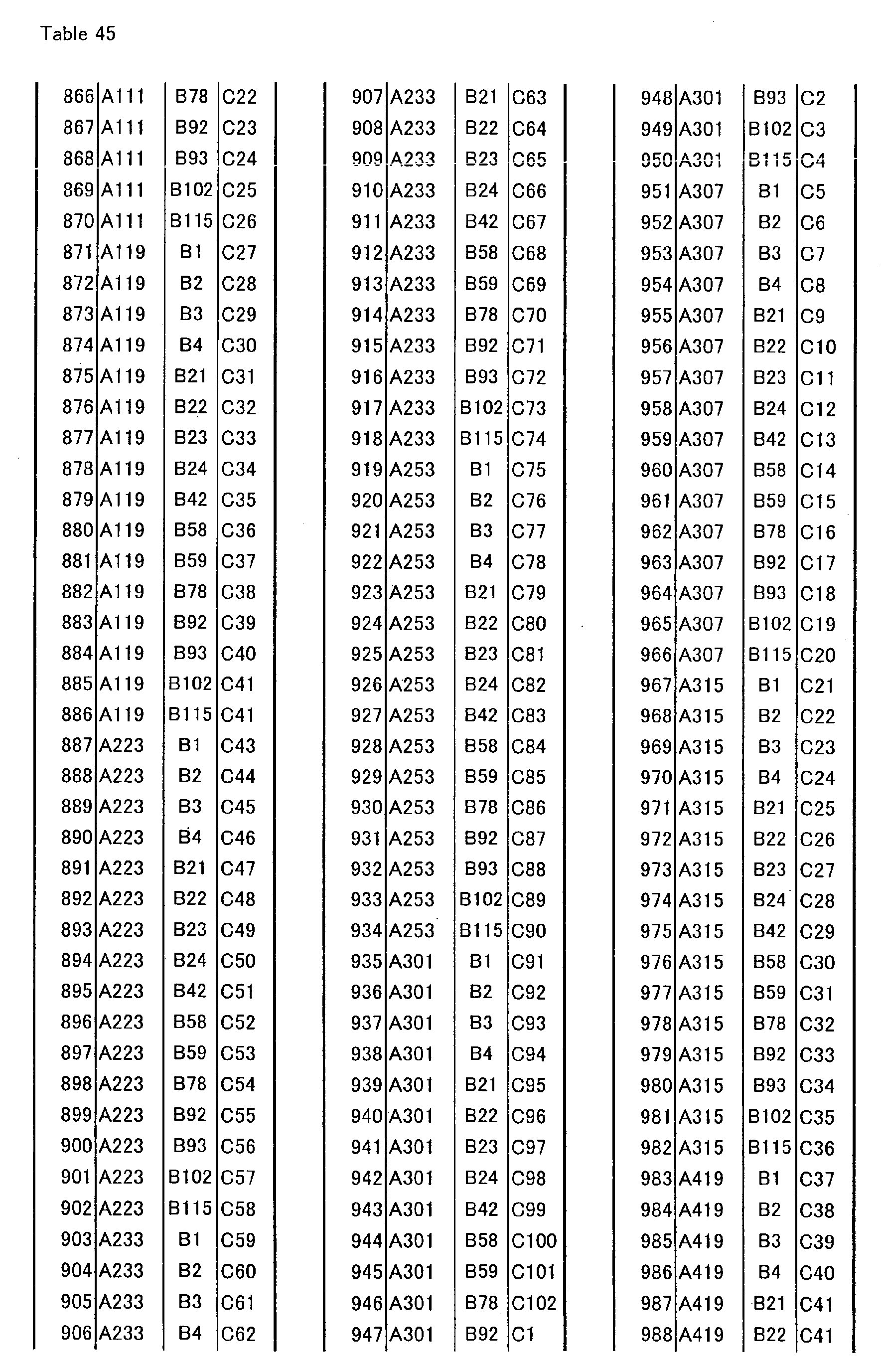

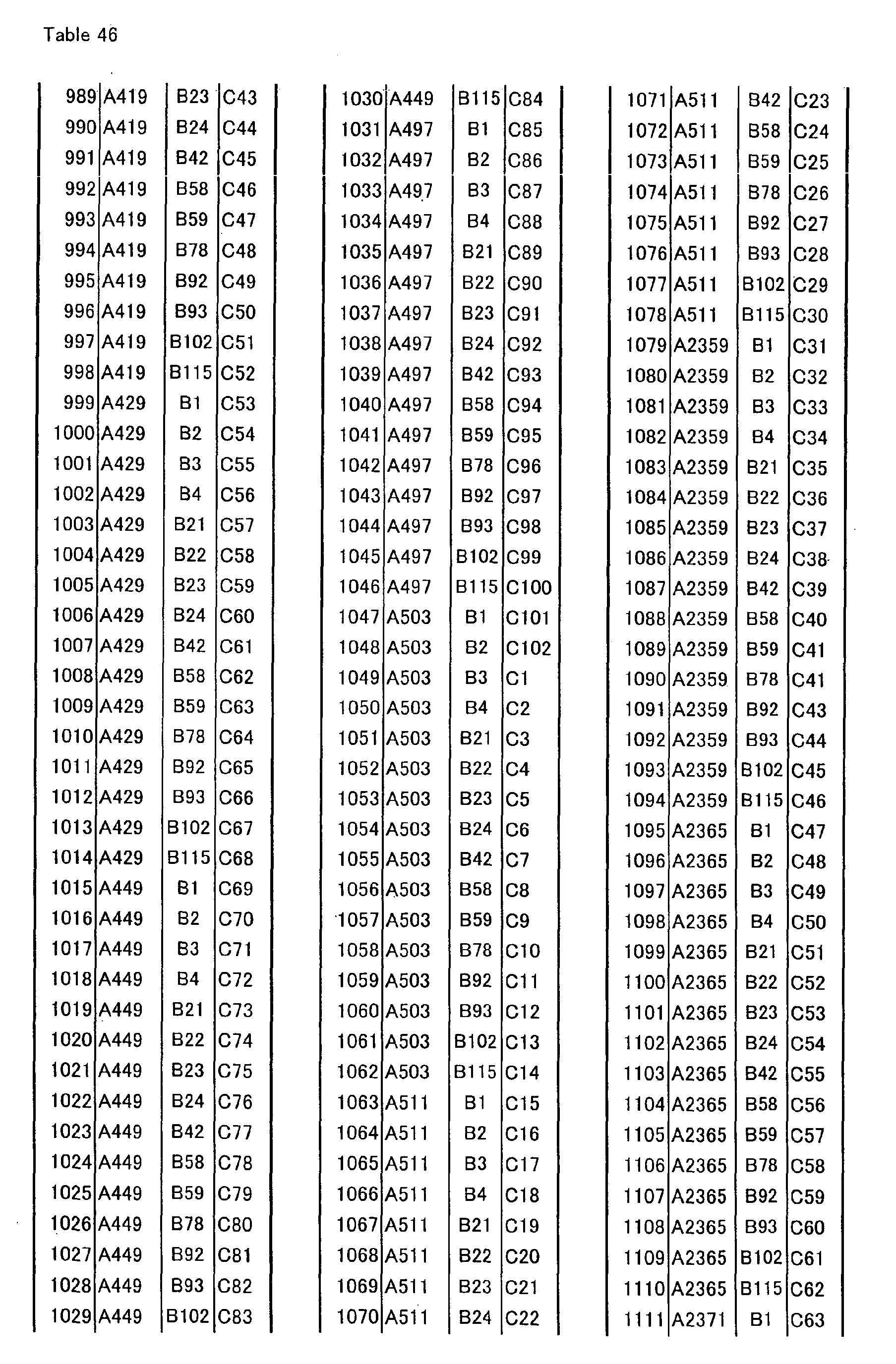

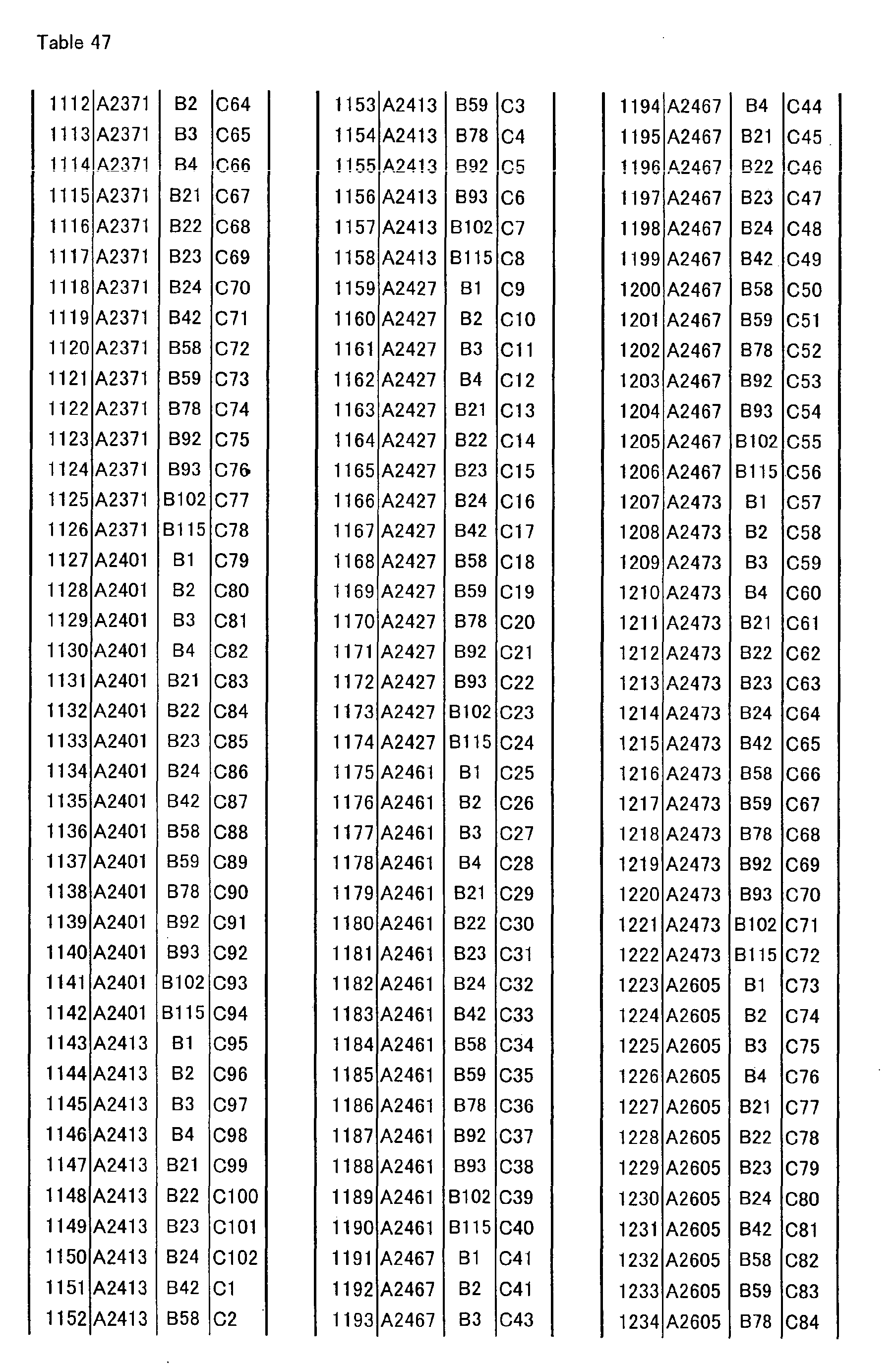

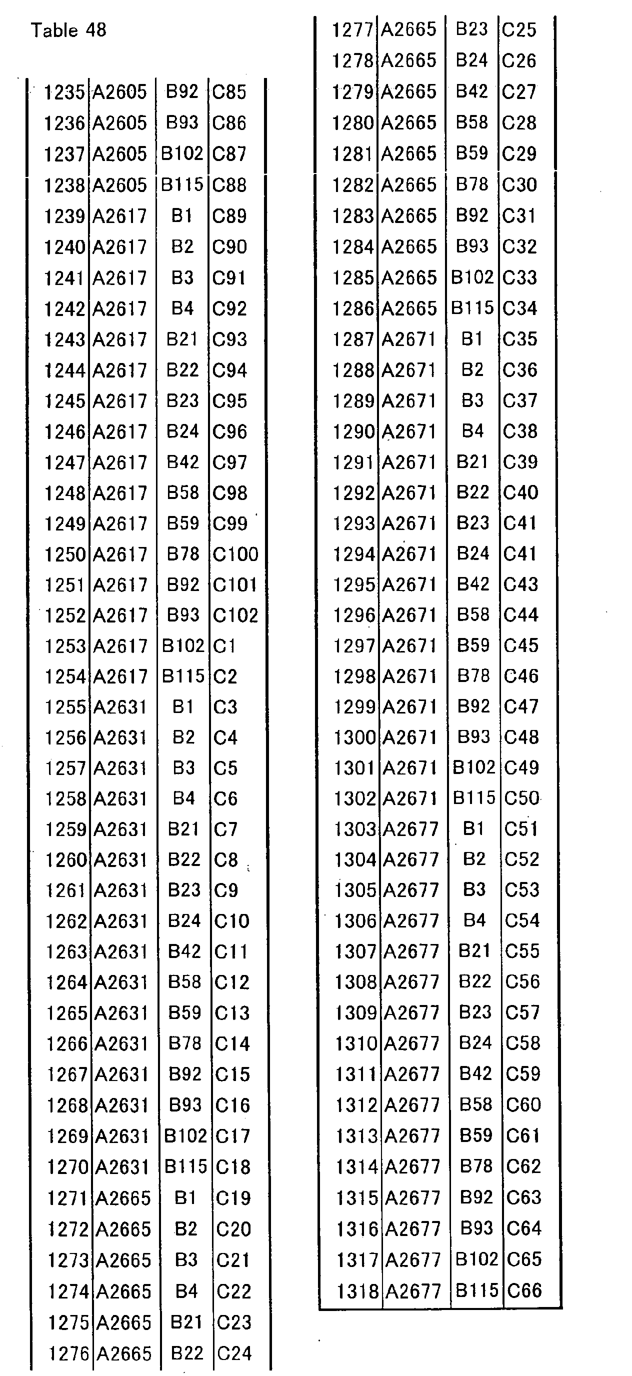

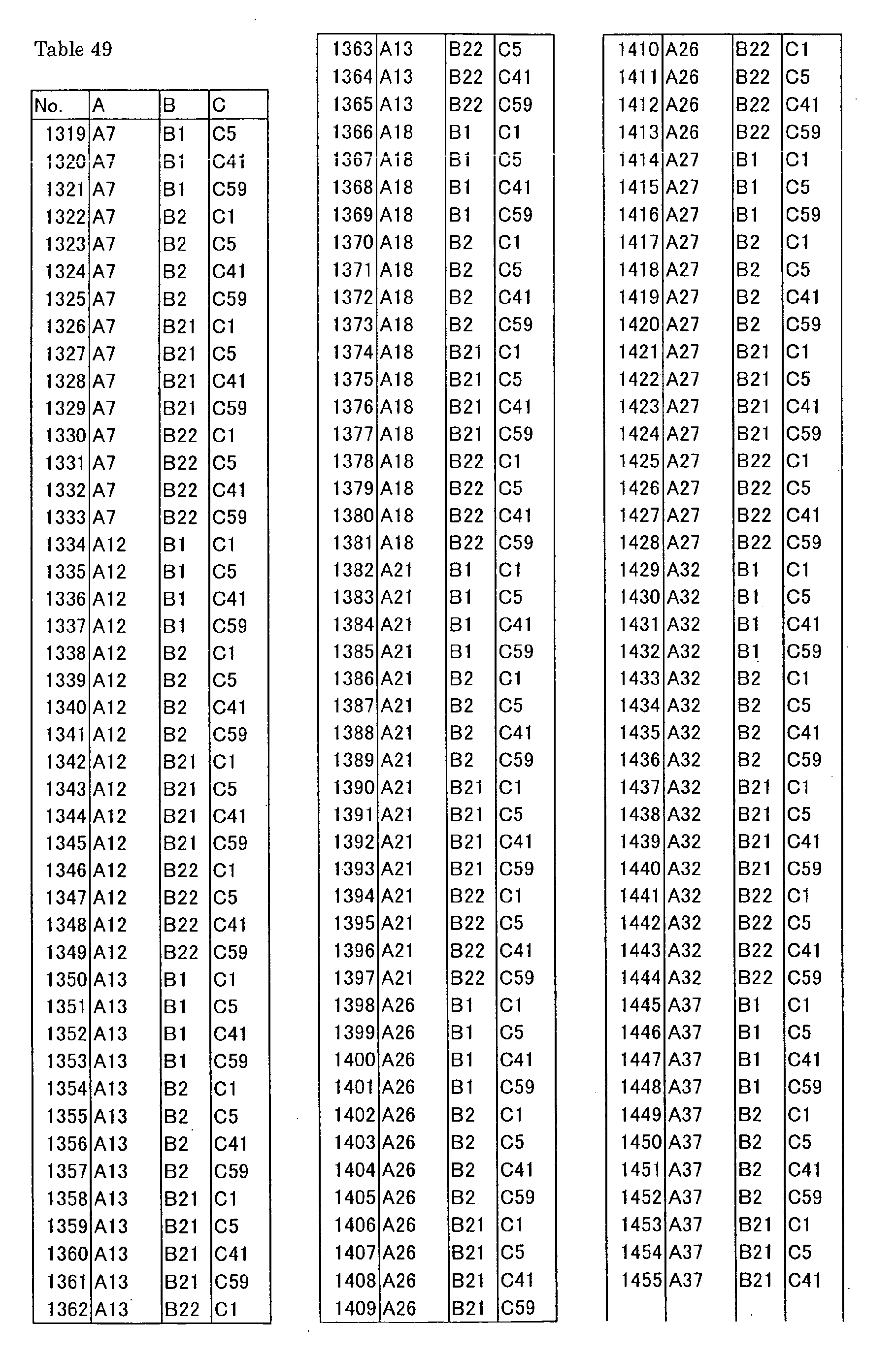

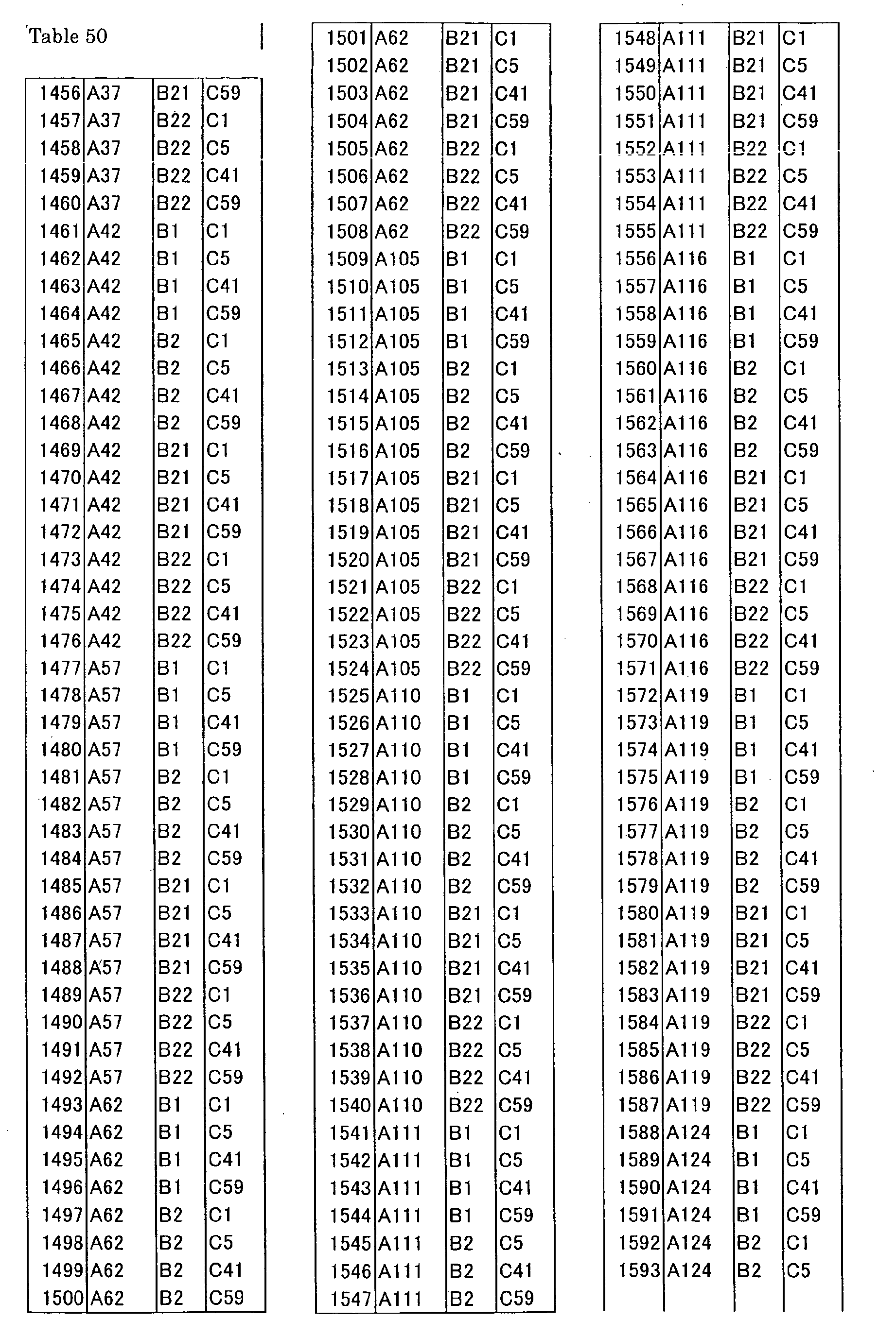

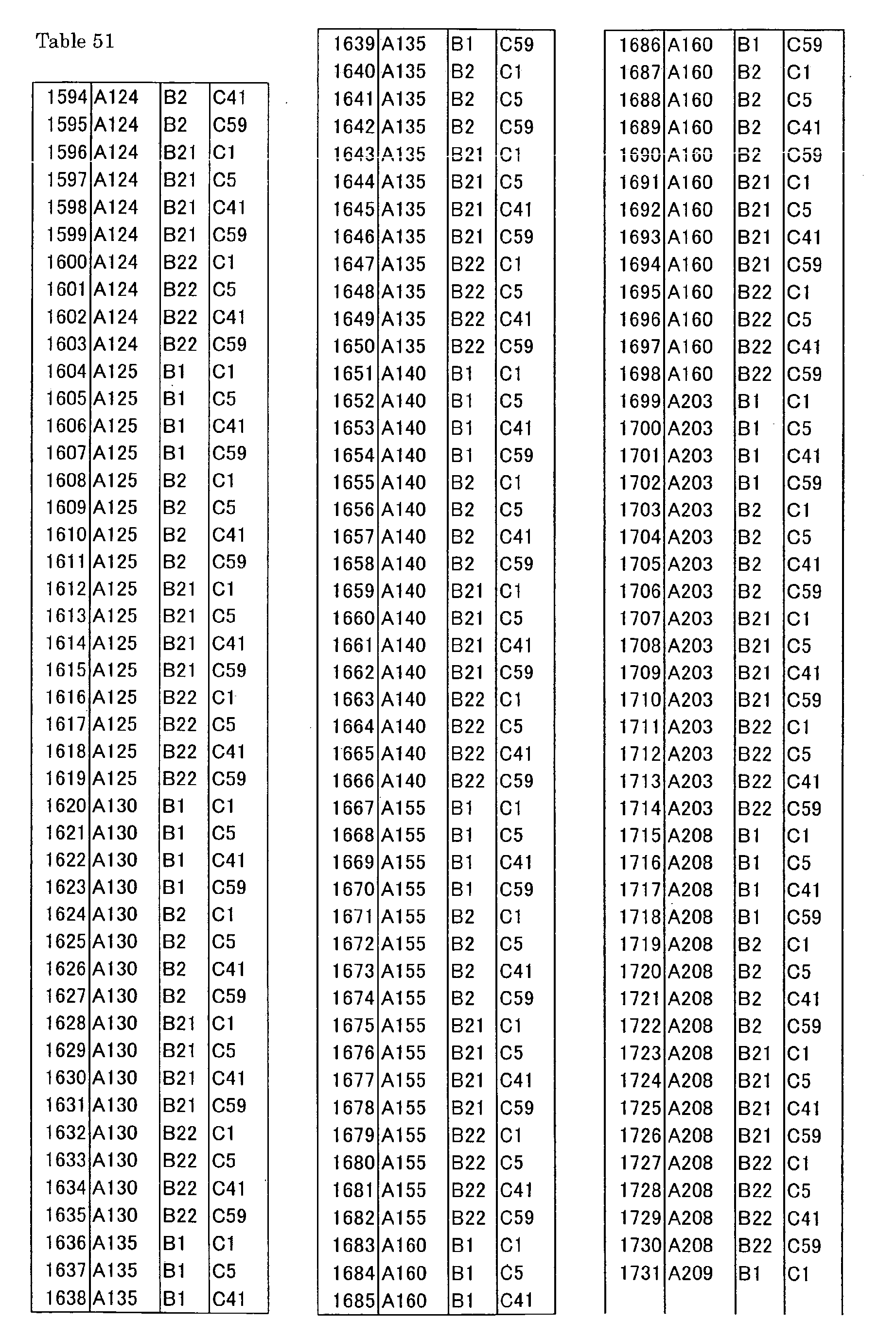

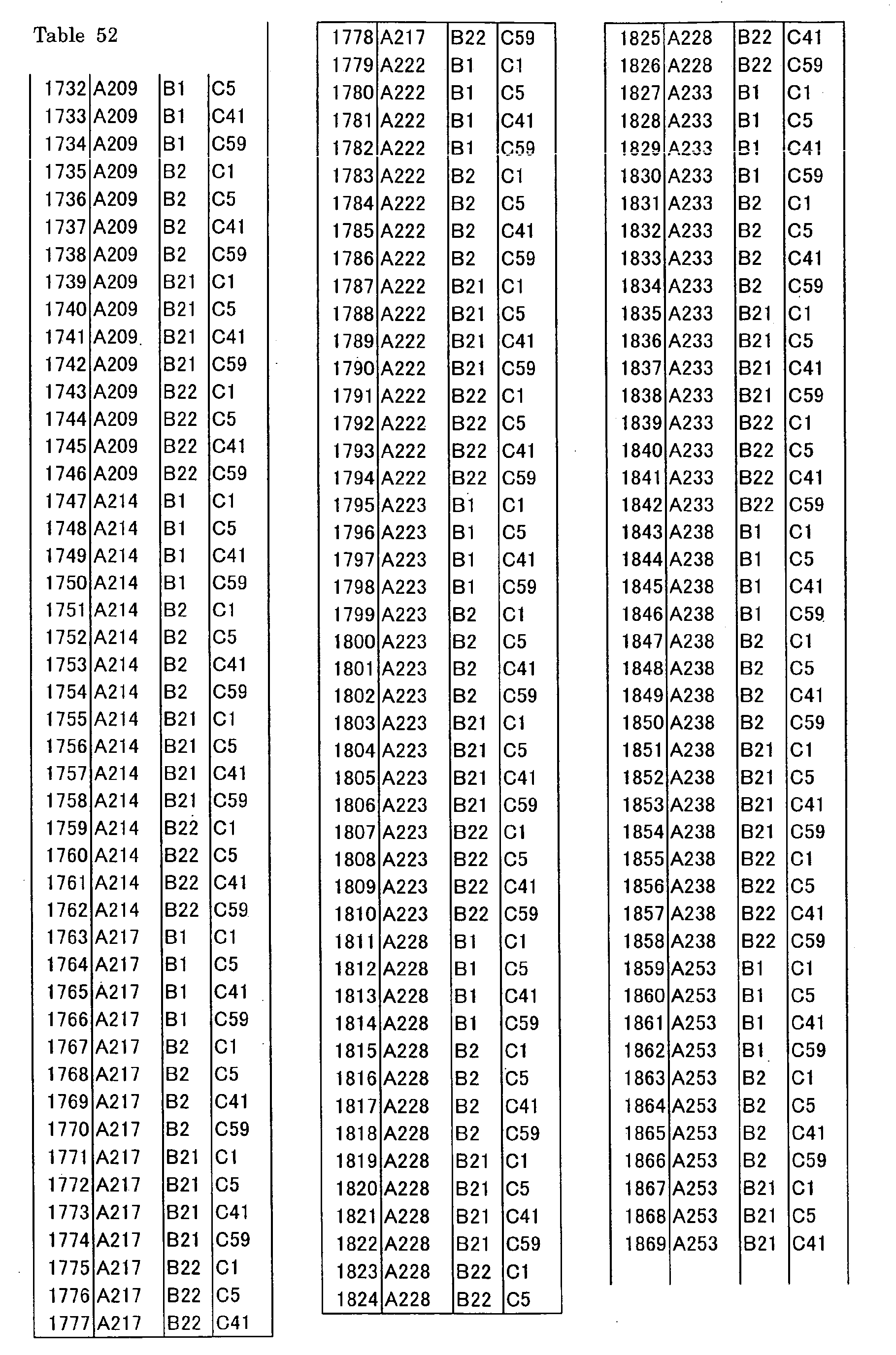

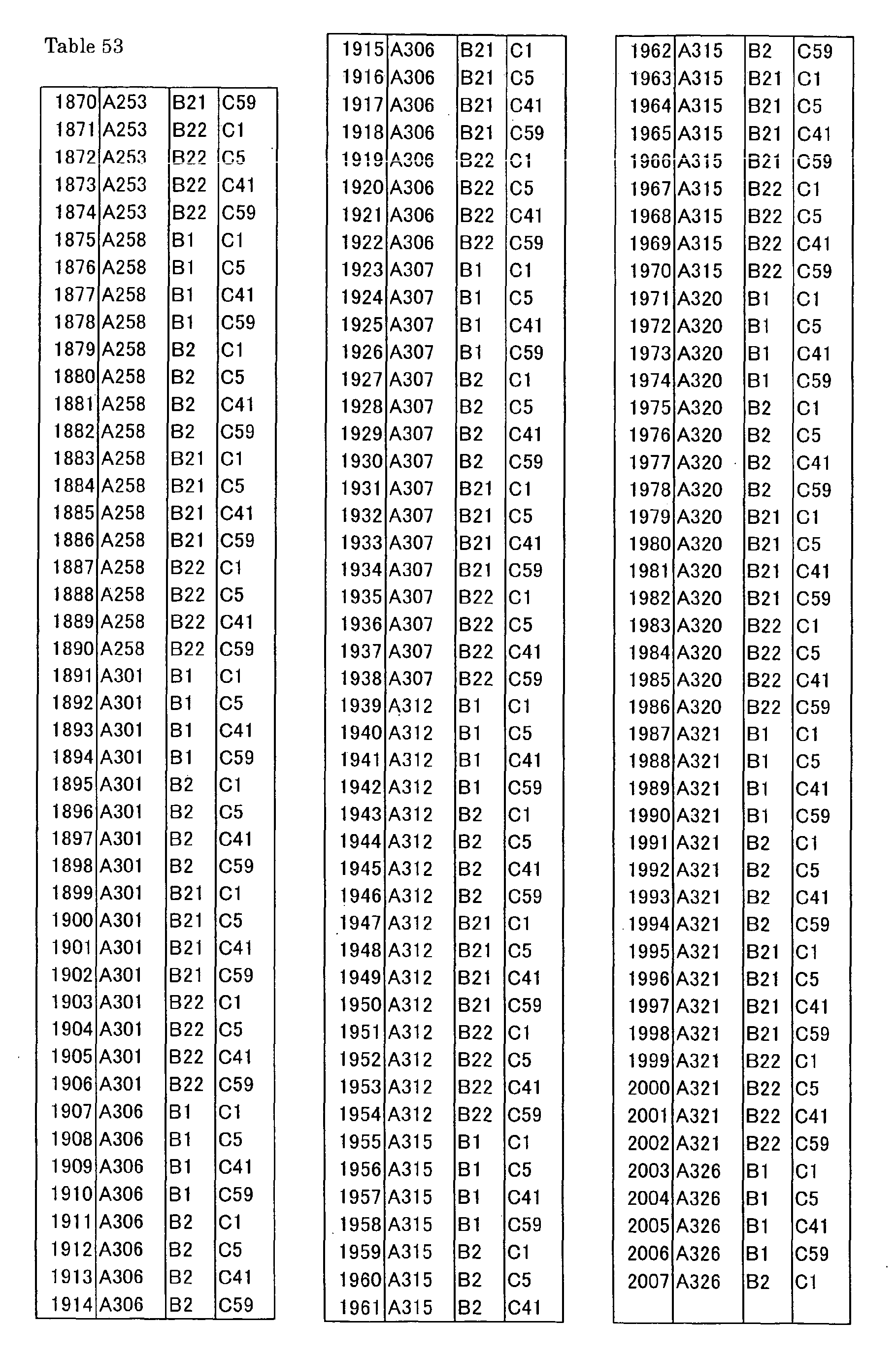

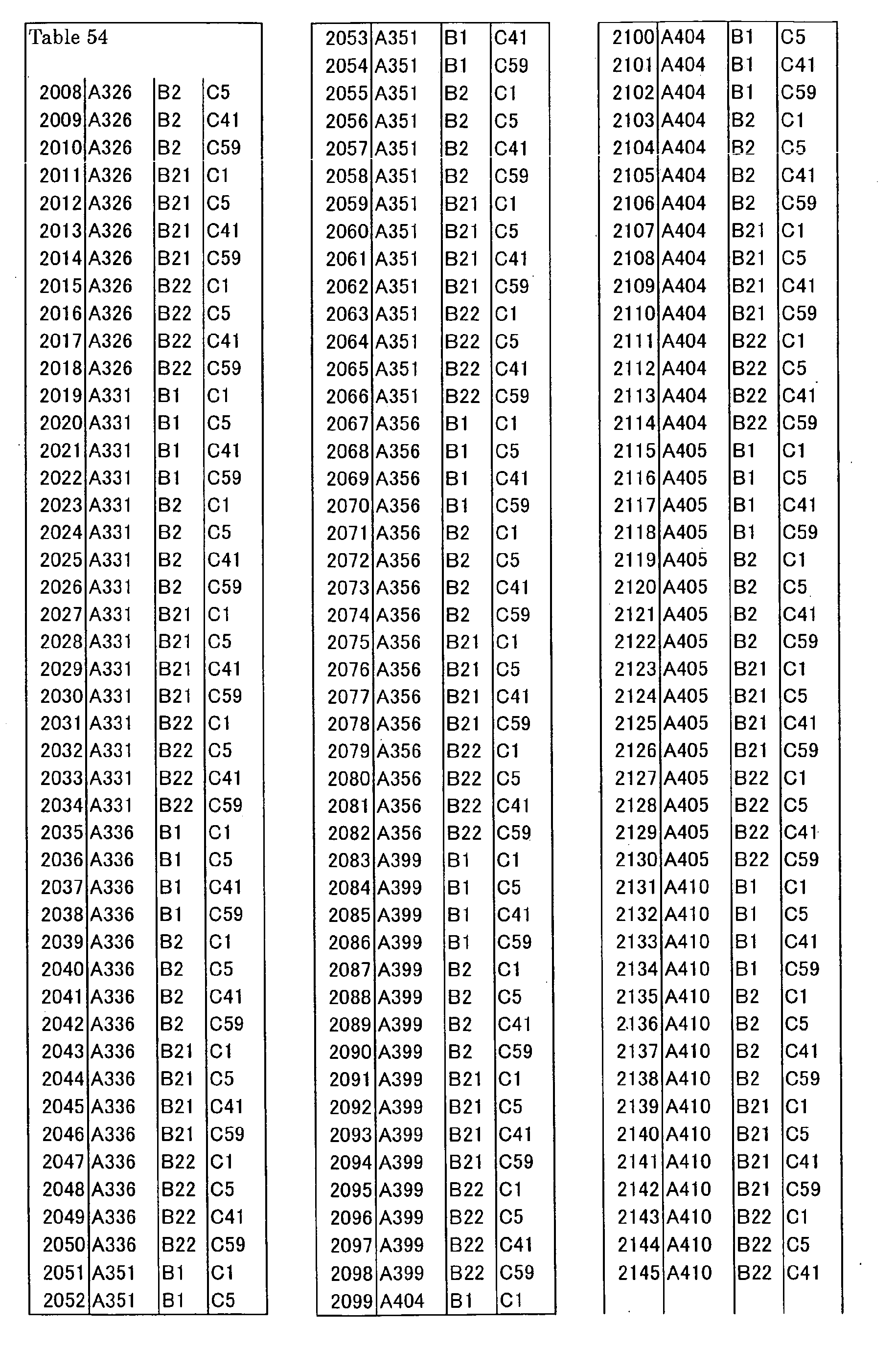

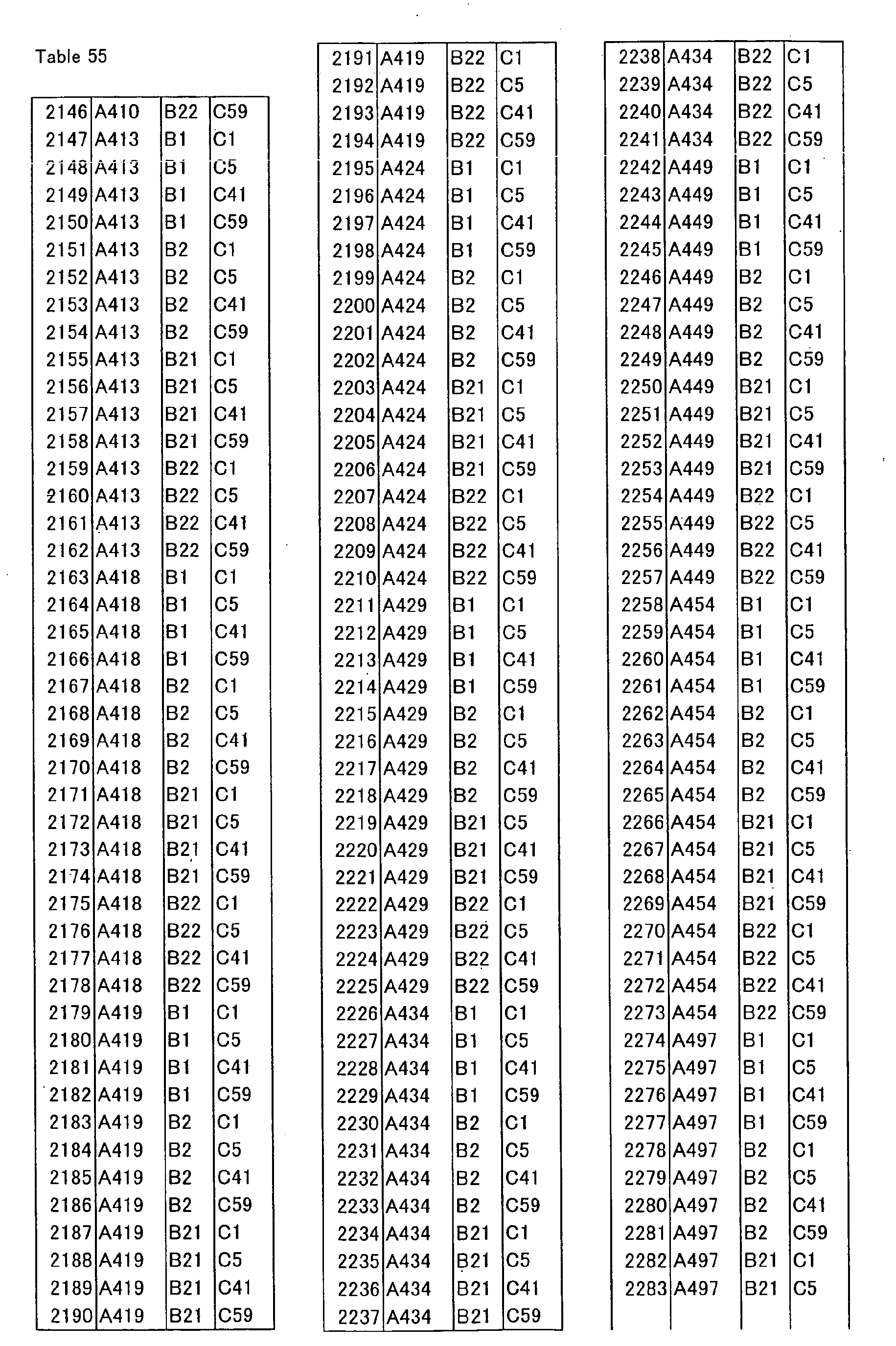

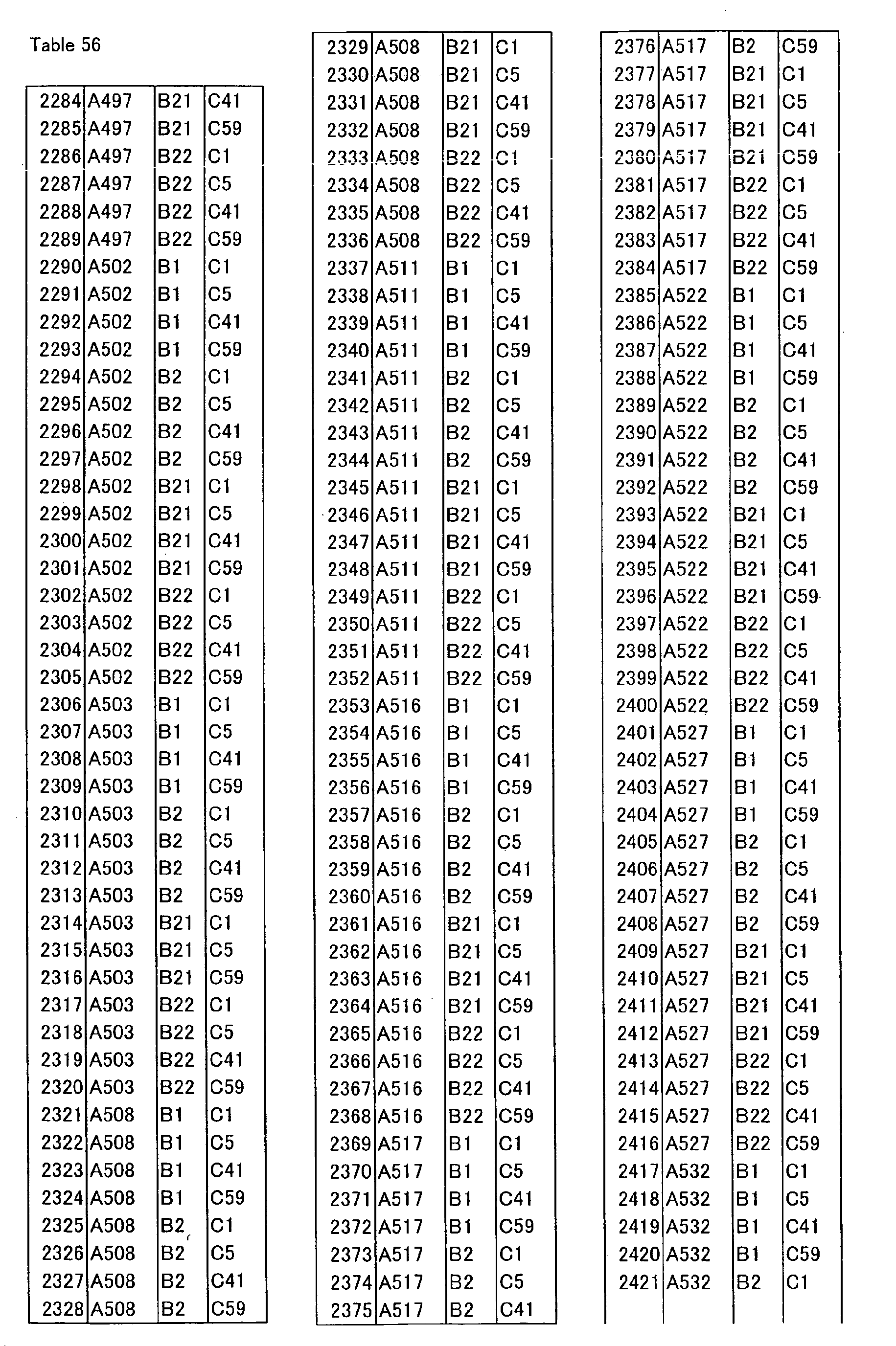

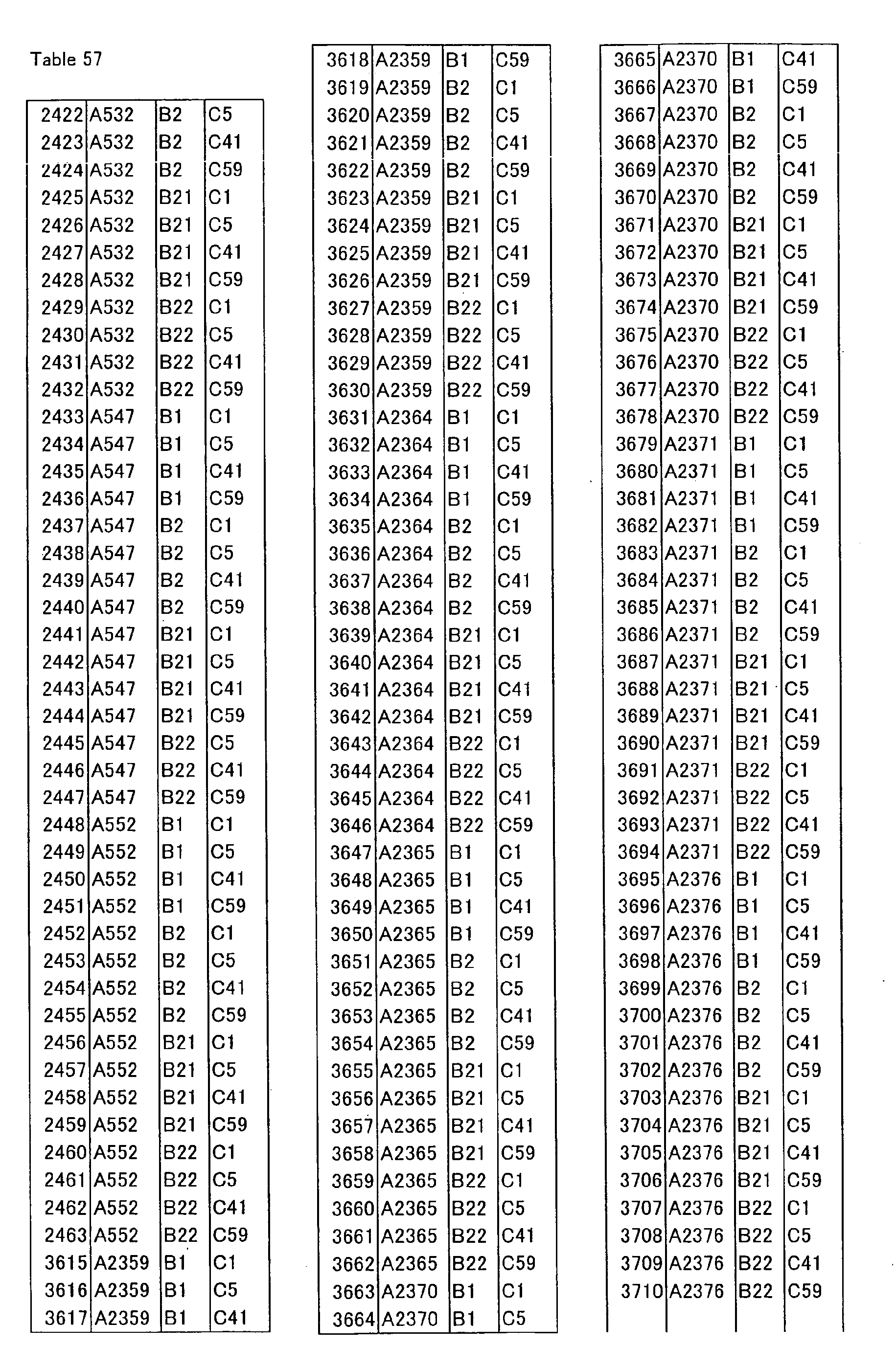

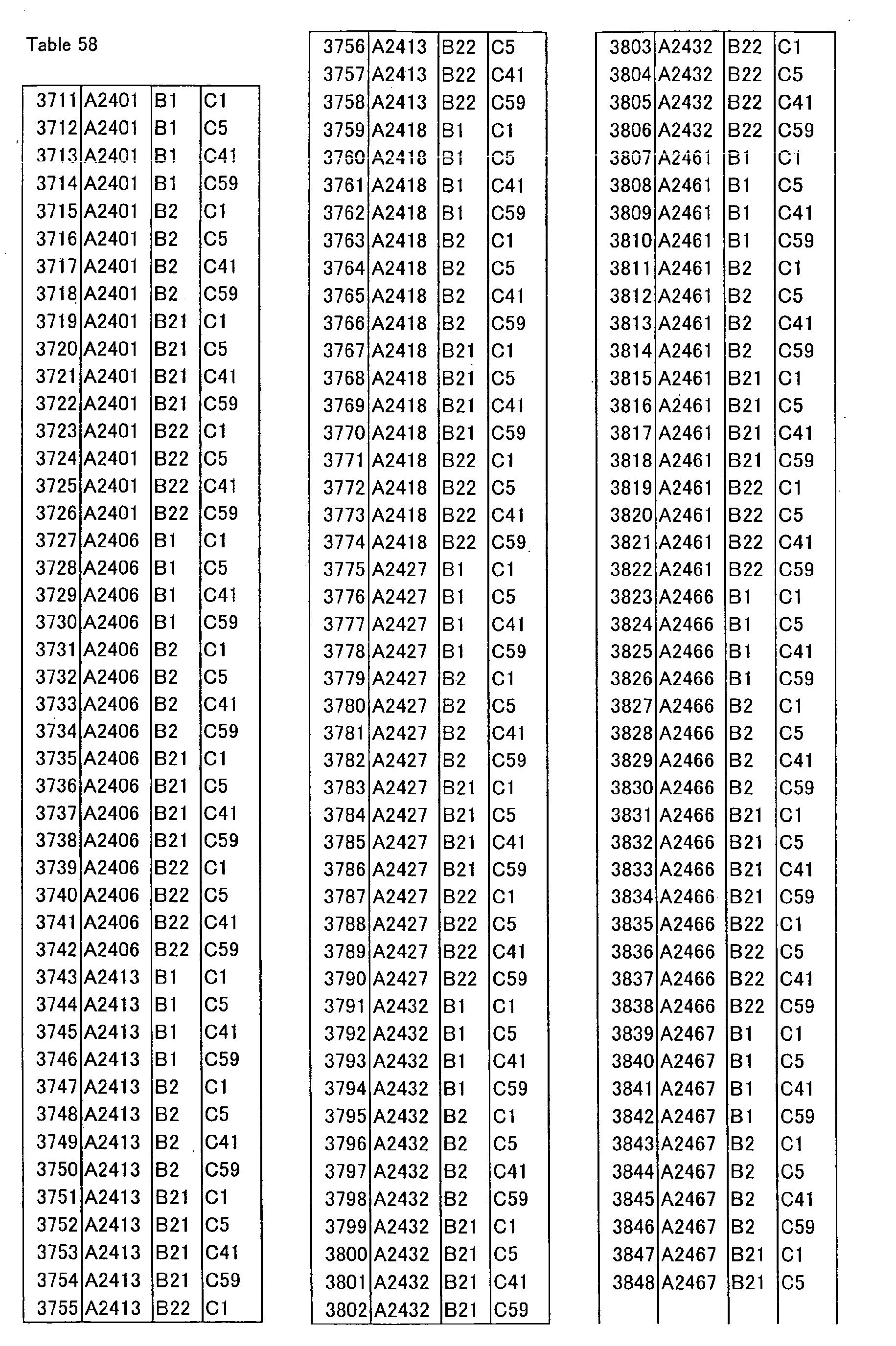

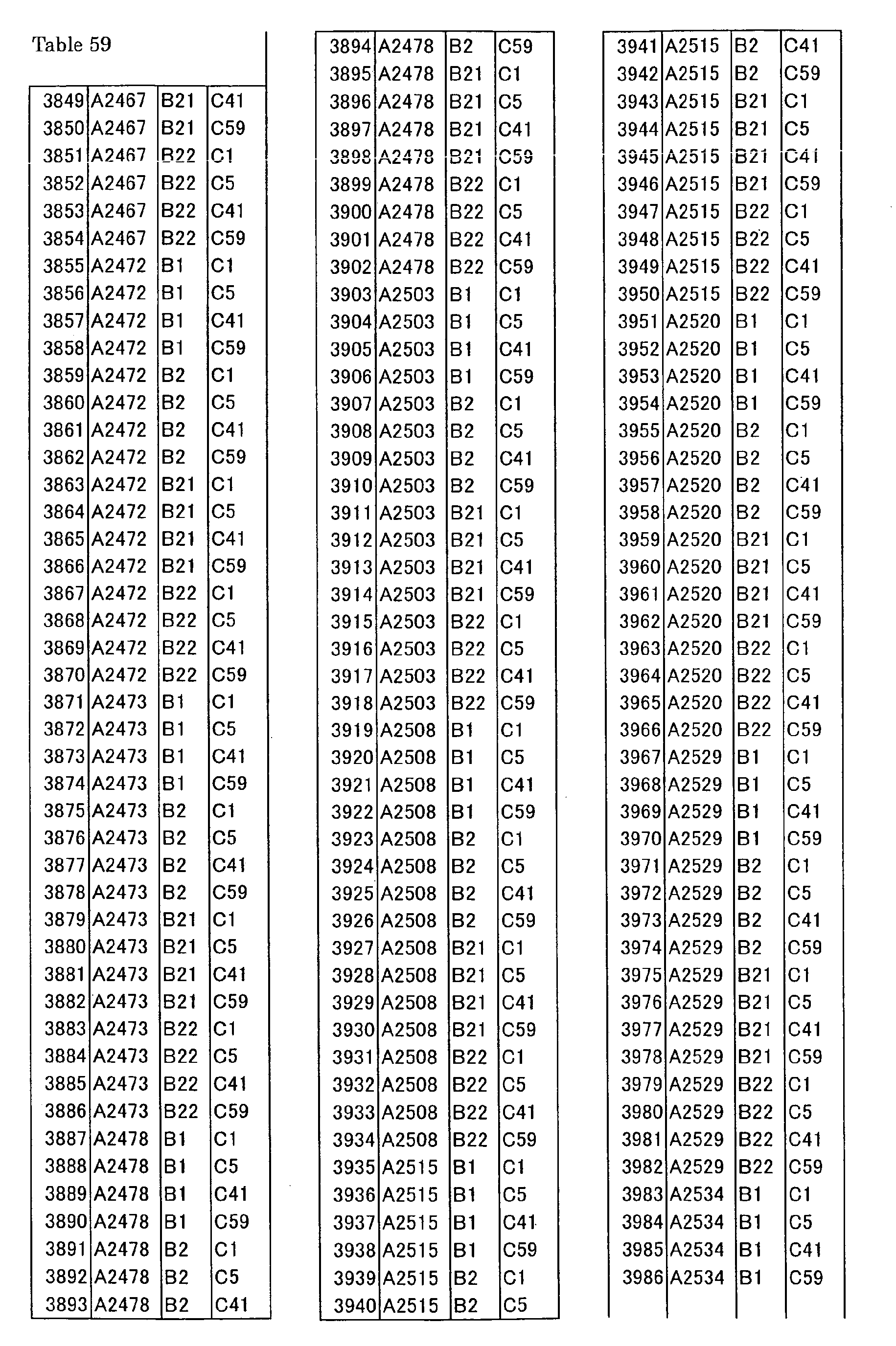

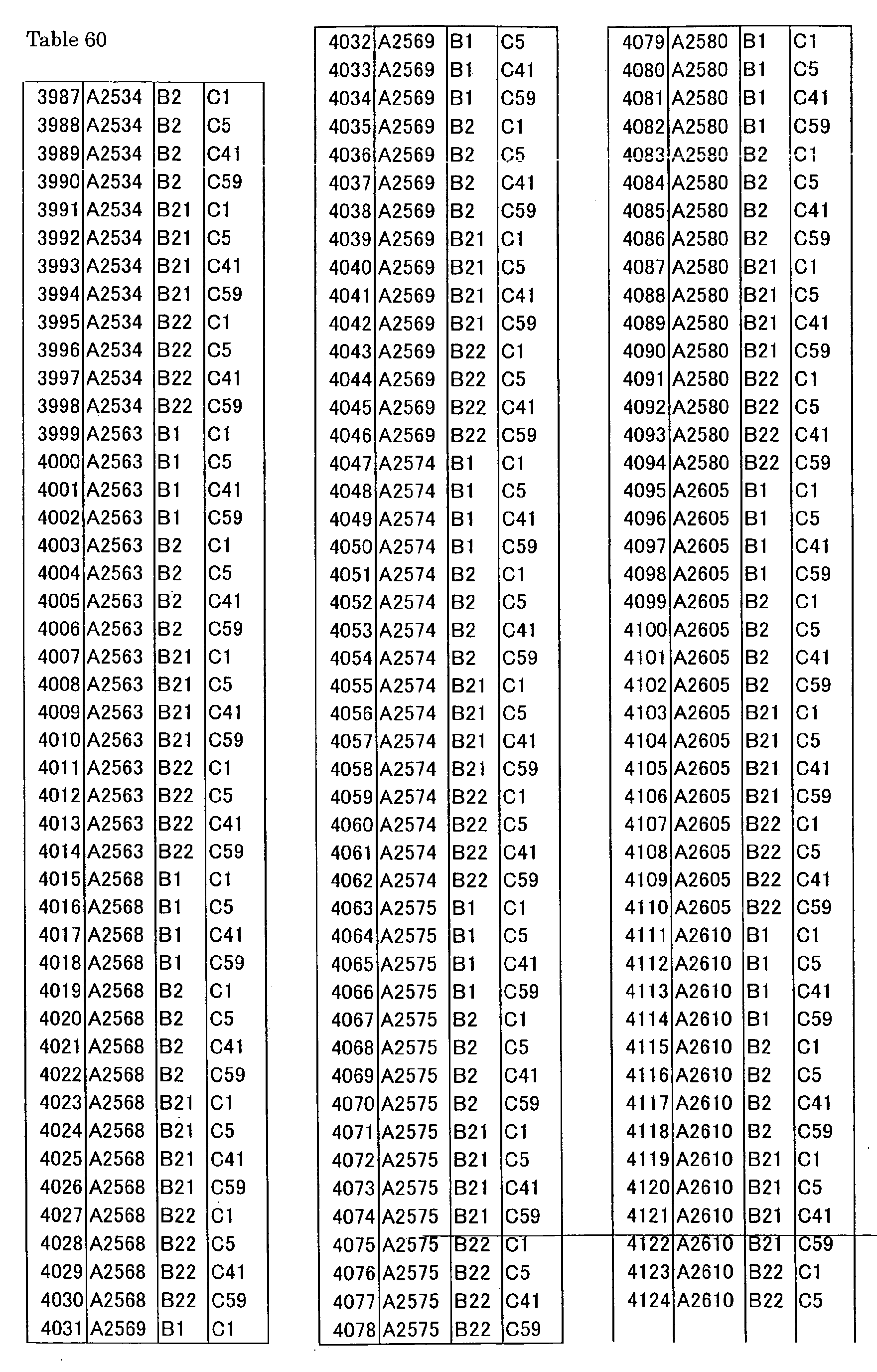

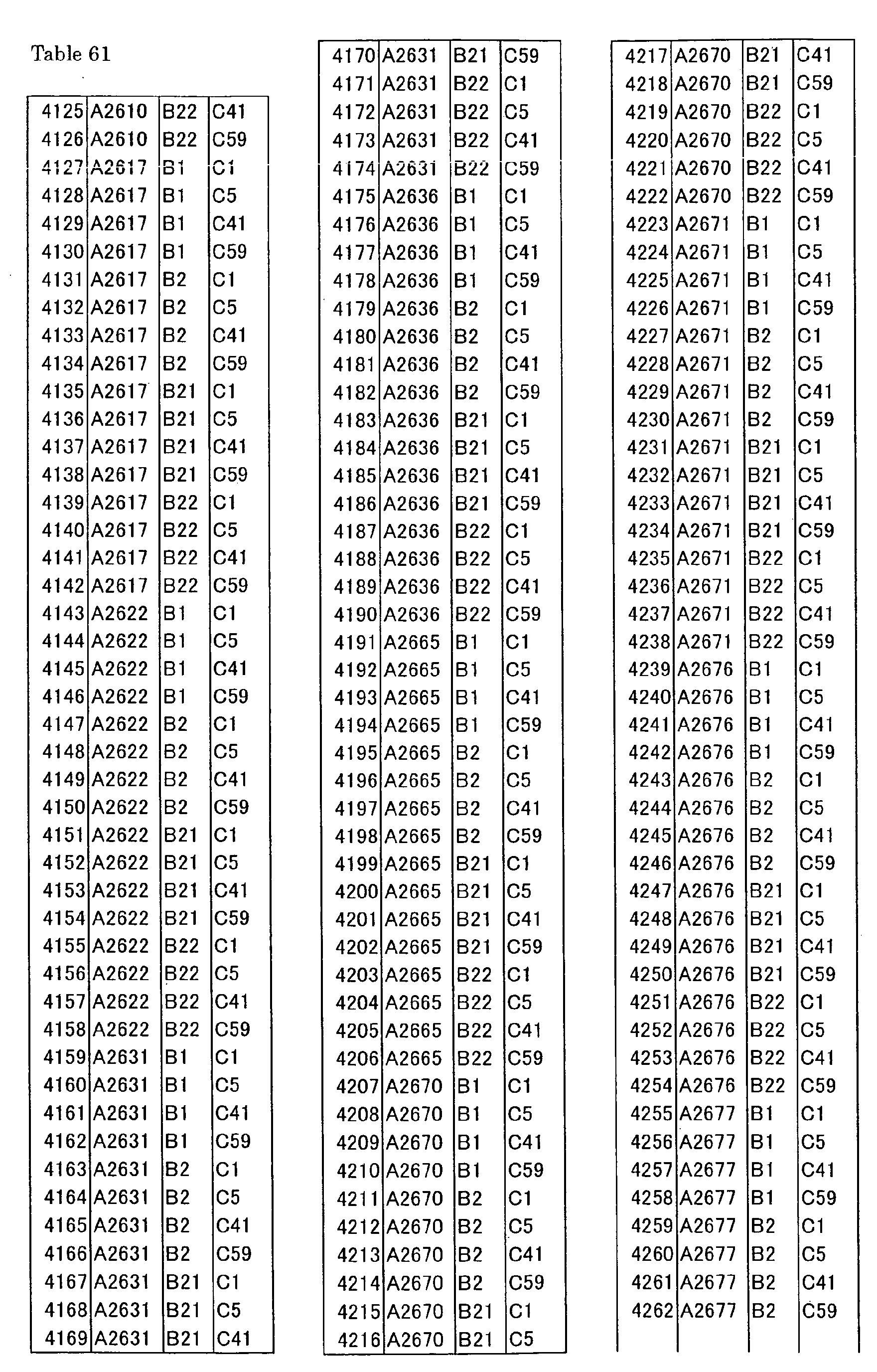

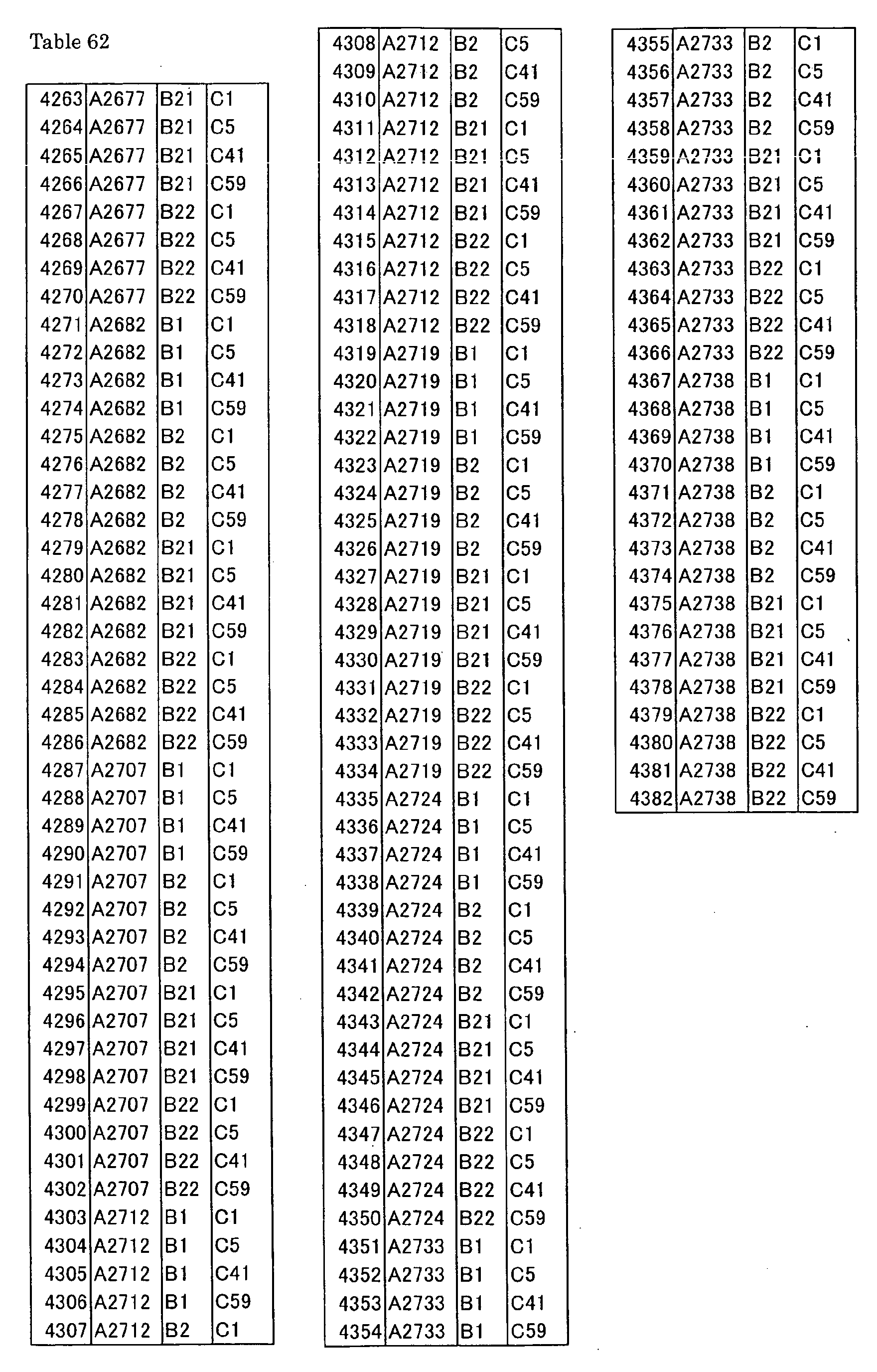

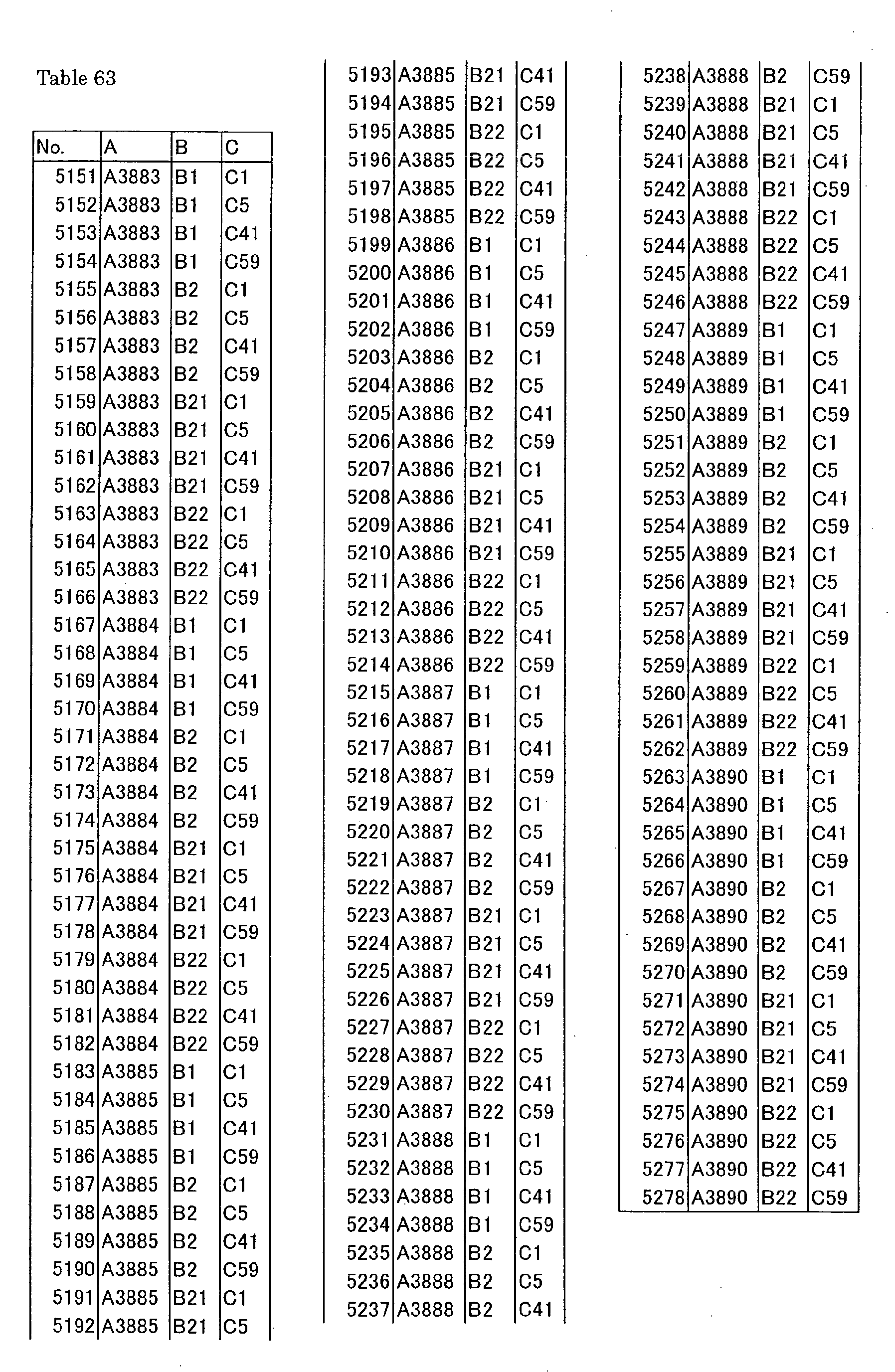

A pharmaceutical composition for PPAR agonist of this invention can be effectively acted on all diseases concerning PPAR and especially for prevention and/or treatment of hyperlipidemia, dyslipidosis, disorder of lipid metabolism, Low HDL, High LDL, High VLDL, High TG, diabetes, hyperglycosemia, insulin resistance, obesity, bulimia, arteriosclerosis, atherosclerosis, hypertension, syndrome X, ischemic disease, inflammation, allergic disease (inflammatory bowel disease, rheumatoid arthritis, chronic pancreatitis, multiple sclerosis, glomerulosclerosis, psoriasis, eczema or the like), osteoporosis, sterility, cancer (breast cancer, colonic cancer, colon cancer, ovarian cancer, lung cancer or the like), Alzheimer's disease, Parkinson syndrome or Basedow's disease. Especially, a compound having PPARδ selective agonist activity in a compound of the present invention having PPAR agonist activity can be good medicine. The reason is, for example, that it can be expected to have a high HDL increasing activity or that the side effect can be lightened.

When administering a compound of the present invention as a pharmaceutical composition for PPAR agonist, it can be administered orally or parenterally. For oral administration, the compound of the present invention can be used in any form of usual formulations, for example, tablets, granules, powders, capsules, pills, solutions, syrup, buccals, sublingual tablets or the like which are made by the usual method. For parenteral administration, the compound of the present invention can be used in any form of usual formulations, for example, injections such as intramuscular administration and intravenous administration, suppository, transdermal therapeutic agent, insufflation or the like. A compound of the present invention can be preferably used as an oral agent because it has high oral bioavailability.

The formulation according to the present invention may be manufactured by combining a curatively effective amount of a compound of the present invention with various pharmaceutically acceptable excipients such as binder, moistening agent, disintegrating agents, lubricant, diluent or the like, if necessary. When the formulation is injection, the compound of the present invention may be manufactured by sterilization treatment with an appropriate carrier.

For example, the excipient is lactose, saccharose, glucose, starch, calcium carbonate, crystalline cellulose or the like. The binder is methylcellulose, carboxy methylcellulose, hydroxy propylcellulose, gelatin, polyvinylpyrrolidone or the like. The disintegrating agent is carboxy methyl cellulose, carboxymethylcellulose sodium, starch, sodium alginate, powdered agar, sodium lauryl sulfate or the like. The lubricant is talc, magnesium stearate, macrogol or the like. As a basis for suppository, cocoa butter, macrogol, methylcellulose or the like can be used. When the present invention is manufactured as liquid medicine, emulsion injection or suspension injection, solubilizing agent, suspending agent, emulsifying agent, stabilizing agent, preservatives, isotonic agent or the like which is usually used can be appropriately added. In case of oral administration, sweetening agent, flavoring agent or the like can be added.

The dose as a pharmaceutical composition for PPAR agonist of a compound of the present invention is preferably established depending on age, body weight, kind of disease, conditions of the patient, the administration route or the like. In case of the oral administration for an adult, it is usually 0.05-100 mg/kg/day and preferably 0.1-10mg/kg/day. In case of the parenteral administration, although it is very different depending on route of administration, it is usually 0.005-10 mg/kg/day and preferably 0.01-1mg/kg/day. This can be separated and administrated at 1 time - few times a day.

The following examples are provided to explain in more detail and do not restrict the present invention.

### Example

In the examples, the meaning of each abbreviation is as below.
- Me: methyl
- Et: ethyl
- nBu: n-butyl
- tBu: tert-butyl
- nPr: n-propyl
- Ph: phenyl
- Bn: benzyl
- Ac: acetyl
- Ms: methanesulfonyl
- TMS: trimethylsilyl
- PCC: pyridinium chlorochromate
- CDI: 1,1'-carbonyldiimidazole
- DBU: 1,8- diazabicyclo [5.4.0] undec -7-ene
- DME: 1,2- dimethoxyethane
- DPM: diphenylmethyl
- TBS: 3-tert-butyldimethylsilyl
- TFMP: 4-trifluoromethylphenyl

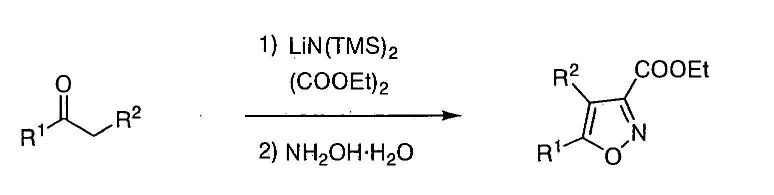

### Reference 1

### 5-(4-trifluoromethylphenyl)-isoxazole-3-carboxylic acid ethyl ester (R¹ = TFMP, R² = H, 1-1-1)

To dried ether (60 ml) was added lithium bis(trimethylsilyl) amide solution (15 ml). The mixture was cooled to -70 °C or below. 4-Trifluoromethylacetophenone (2.82 g) in ether (15 ml) was added dropwise to the mixture for 6 minutes to kept temperature at -65 °C or below. The mixture was stirred at room temperature for 17 hours. After addition of ether (100 ml), the mixture was cooled to 0°C. The resulting precipitate was filtrated to give lithium salt of pyruvate as the first crop (2.9 g). Furthermore, the filtrate was condensed, diluted with ether and cooled to 0°C. The resulting precipitate was collected by filtration to give the second crop (610 mg). To this lithium salt (3.5 g) were added ethanol (35 ml) and hydroxylamine hydrochloride (1.22 g). The mixture was refluxed for 20 hours. After the solvent was evaporated, water was added thereto and the mixture was extracted with chloroform. The organic layer was dried over magnesium sulfate anhydrous and the solvent was evaporated under reduced pressure. The obtained residue was subjected to silica gel column chromatography eluting with ethyl acetate: hexane (1: 1) to give a title compound (2.55 g) as a colorless crystal. The yield was 60 %.

(1-1-2)-(1-1-4) were synthesized as well as the above.

**Table 64**

| No | R¹ | R² | NMR |
|---|---|---|---|
| 1-1-1 | TFMP | H | 1.46(3H,t,J=6.9Hz),4.49(2H,q,J=6.9Hz),7.04(1H,s),7.77( 2H,d,J=8.7Hz),7.95(2H,d,J=8.7Hz) |
| 1-1-2 | TFMP | Me | 1.46(3H,t,J=6.9Hz),2.47(3H,s),4.49(2H,q,J=6.9Hz),7.78( 2H,d,J=8.4Hz),7.86(2H,d,J=8.4Hz) |
| 1-1-3 | p-Cl-C₆H₄- | H | 1.45(3H,t,J=7.2Hz),4.48(2H,q,J=7.2Hz),6.92(1H,s),7.47( 2H,d,J=8.4Hz),7.75(2H,d,J=8.4Hz) |
| 1-1-4 | Pyridine-4-yl | H | 1.46(3H,t,J=7.2Hz),4.50(2H,q,J=7.2Hz),7.12(1H,s),7.68( 2H,d,J=6.0Hz),8.79(2H,d,J=6.0Hz) |

### Reference 2

### 5-bromo-4-methyl-isoxazole-3-carboxylic acid ethyl ester (1-2-1)

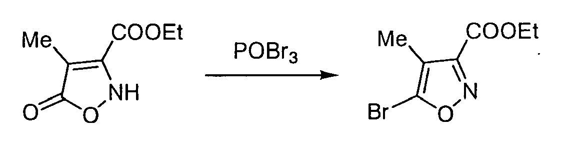

To a mixture of 4-methyl-5-oxo-2,5-dihydroisoxazole-3-carboxylic acid ethyl ester (6.45 g) and phosphorous oxybromide (54.0 g) was added triethylamine (5.3 ml), and the mixture was stirred at 80 °C for 2 hours. The reaction solution was poured to ice, extracted with ether, washed with brine and dried over magnesium sulfate anhydrous. The solvent was evaporated under reduced pressure. The obtained residue was subjected to silica gel column chromatography eluting with ethyl acetate: hexane (1: 8) to give a title compound as pale yellow oil (7.36 g). The yield was 80 %.
¹H-NMR(CDCl₃): 1.43(3H,t,J = 7.2Hz), 2.19(3H,s), 4.45(2H,q,J = 7.2Hz).

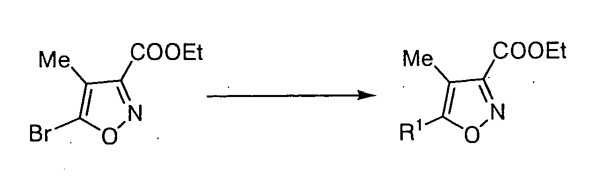

### Reference 3

### 4-Methyl-5-(4-trifluoromethyl phenyl)-isoxazole-3-carboxylic acid ethyl ester (R¹ = TFMP, 1-1-2)

To a solution of compound (1-2-1, 243 mg) in DME (6 ml) was added 4-trifluoromethyl phenylboronic acid (285 mg), potassium carbonate (420 mg) and PdCl₂ (dppf) (81 mg), and the mixture was stirred at 100 °C for 7 hours. After addition of water, the mixture was extracted with ethyl acetate and washed with brine. After drying over magnesium sulfate anhydrous, the solvent was evaporated under reduced pressure. The obtained residue was subjected to silica gel column chromatography eluting with ethyl acetate: hexane (1: 8) to give a title compound (239 mg) as a colorless crystal. The yield was 80 %.

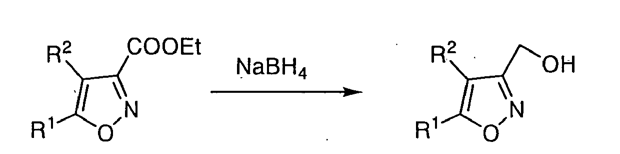

### Reference 4

### [5-(4-Trifluoromethylphenyl)-isoxazole-3-yl] methyl alcohol (R¹ = TFMP, R² = H, 2-1-1)

5-(4-trifluoromethylphenyl)-isoxazole-3-carboxylic acid ethyl ester (1-1-1, 1.0 g) was dissolved in methyl alcohol (15 ml). To this solution, sodium borohydride (358 mg) was added at 0 °C. After 5 minutes, the mixture was warmed to room temperature and stirred for more 2 hours. To the reaction solution, was added 1M hydrochloric acid at 10 °C or below to be weak acidity. The solvent was evaporated under reduced pressure and water was added to the residual solution. The mixture was extracted with chloroform, washed with brine and dried over magnesium sulfate anhydrous. The solvent was evaporated under reduced pressure. The obtained residue was subjected to silica gel column chromatography eluting with ethyl acetate: hexane (1: 8) to give a title compound (820 mg) as a crystal (The yield was 96 %). The crystal was recrystallized from ethyl acetate - hexane to give a crystal. The melting point is 111-113 °C.

(2-1-2)-(2-1-9) were synthesized as well as the above.

**Table 65**

| No | R¹ | R² | NMR(CDCl₃) |
|---|---|---|---|
| 2-1-1 | TFMP | H | 2.04(1H,t,J=6.0Hz),4.85(1H,d,J=6.0Hz),6.70(1H,s),7.74(2 H,d,J=8.4Hz), 7.91(2H,d,J=8.4Hz) |
| 2-1-2 | TFMP | Me | 1.97(1H,t,J=6.6Hz),4.80(2H,m),7.76(2H,d,J=8.4Hz),7.85( 2H,d,J=8.4Hz) |
| 2-1-3 | 4-Cl- C₆H₄- | H | 4.82(2H,s),6.58(1H,s),7.50(2H,d,J=8.7Hz),7.72(2H,d,J=8. 7Hz) |
| 2-1-4 | 4-Cl- C₆H₄- | Et | 1.25(3H,t,J=7.2Hz),2.68(2H,q,J=7.2Hz),4.80(2H,s),7.47(2 H,d,J=8.4Hz),7.63(2H,d,J=8.4Hz) |
| 2-1-5 | Me | H | 2.30(1H,s),2.42(3H,d,J=0.6Hz),4.71(2H,s),6.04(1H,q,J=0. 6Hz) |
| 2-1-6 | Et | H | 1.30(3H,t,J=7.5Hz),2.23(1H,s),2.77(2H,qd,J=7.5,0.6Hz),4 .72(2H,s),6.04(1H,t,J=0.6Hz) |
| 2-1-7 | Br | Me | 2.03(3H,s),2.06(1H,brt,J=7.5Hz),4.73(2H,d, J=5.7Hz) |
| 2-1-8 | Morpholine-4-yl | Me | 1.98(3H,s),3.35-3.38(4H,m),3.78-3.82(4H,m), 4.60(2H,s) |
| 2-1-9 | Pyridine-4-yl | H | 2.20(1H,brs),4.85(2H,s),6.81(1H,s),7.65(2H,d,J=6.0Hz),8. 75(2H,d,J=6.0Hz) |

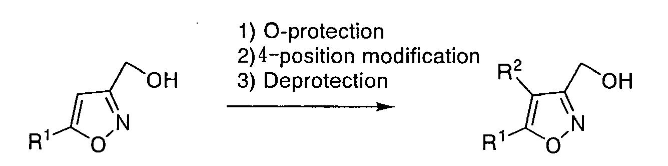

### Reference 5

### Process 1 Protection (TBS protection)

### 3-tert-butyldimethylsilyloxymethyl-5-(4-trifluoromethylphenyl) isoxazole (R¹ = TFMP, R2 = H, 2-2-1-1)

A mixture of [5-(4-trifluoromethylphenyl) isoxazole-3-yl] methyl alcohol (2-1-1, 8.31 g), t-butyldimethyl silylchloride (5.67 g), imidazole (3.49 g) and methylene chloride (160 ml) was stirred for 2 hours. To the reaction solution, was added water and the mixture was extracted twice with chloroform. The organic layer was washed successively with water and brine, and dried over magnesium sulfate. The solvent was evaporated under reduced pressure. The obtained residue was subjected to silica gel column chromatography eluting with ethyl acetate: hexane (1: 9) to give a title compound (11.5 g) as a colorless crystal. The yield was 94 %.
¹H-NMR(CDCl₃): 0.14(6H, s), 0.94(9H, s), 4.82(2H, s), 6.68(1H, s), 7.73(2H, d, J = 8.4 Hz),7.91 (2H, d, J = 8.4 Hz).

### (Methoxymethylation)

### 3-Methoxymethoxymethyl-5-(4-trifluoromethyl phenyl) isoxazole

To a mixture of [5-(4-trifluoromethyl phenyl) isoxazole-3-yl] methyl alcohol (21.9 g) and tetrahydrofuran (300 ml) was added sodium hydride (60 %, 4.14 g) at 0 °C, and the mixture was stirred at room temperature for 1 hour. To the reaction solution was added chloromethylmethylether (9.42 g), and the mixture was stirred at room temperature for 20 hours. The reaction solution was poured into ice water and the mixture was extracted with ethyl acetate. The organic layer was washed with water and brine, and dried over magnesium sulfate. The solvent was evaporated under reduced pressure. The residue was subjected to silica gel column chromatography eluting with ethyl acetate: n-hexane (1:4) to give a title compound (20.8 g).
NMR(CDCl₃) : δ 3.44(3H,s), 4.73(2H,s), 4.76(2H,s), 6.70(1H,s), 7.72(2H,d,J = 8.7Hz), 7.92(2H,d,J = 8.7Hz)

### Process 2 4-position modification

### (Rethiolation)

### TBS compound → R¹ = TFMP, R² = Br

### 4-Bromo-3-tert-butyldimethyl silyloxy methyl-5-(4-trifluoromethyl phenyl) isoxazole (2-2-2-1)

3-tert-Butyldimethyl silyloxy methyl-5-(4-trifluoromethyl phenyl) isoxazole (2-2-1-1, 9.50 g) was dissolved in tetrahydrofuran (190 ml). n-Butyllithium in hexane (1.57 M) was added dropwise to this solution at -78 °C for 15 minutes: After stirring at -78 °C for 70 minutes, bromine (9.36 g) was added dropwise for 10 minutes. After stirring at -78 °C for 2 hours, the solution was warmed to room temperature and the reaction was quenched by adding 10 % sodium sulfite solution. The mixture was extracted with ethyl acetate, washed with brine, and dried over magnesium sulfate anhydrous. Removal of solvent under reduced pressure gave a title compound (11.6 g) as yellow oil. The yield was 100 %.
¹H-NMR(CDCl₃) : 0.16(6H, s), 0.94(9H, s), 4.81(2H, s), 7.77(2H, d, J = 8.1 Hz), 8.18(2H, d, J = 8.1 Hz).

### (Cross coupling)

### TBS compound, R² = Br → R¹ = TFMP, R² = benzyl

### 4-Benzyl-3-(tert-butyldimethyl silyloxy methyl)-5-(4-trifluoromethyl phenyl) isoxazole (2-2-2-2)

To suspension of zinc (196 mg) in tetrahydrofuran 2 ml was added 1, 2-dibromoethane (28 mg), and the mixture was stirred for 5 minutes. Chlorotrimethylsilane 16 mg was added thereto and the mixture was stirred for 5 minutes. Benzylbromide 376 mg in tetrahydrofuran (4 ml) was added dropwise to the reaction solution. After refluxing for 30 minutes, the reaction solution was added dropwise to a mixture of 4-bromo-3-tert-butyldimethyl silyloxy methyl-5-(4-triffuoiomethylphenyl) isoxazole (2-2-2-1) 376 mg, palladium acetate 11 mg, tricyclohexylphosphine (14mg) and tetrahydrofuran 4 ml. The mixture was refluxed for 30 minutes followed by addition of water. The mixture was extracted with ethyl acetate, washed with water and brine, and dried over magnesium sulfate. After removal of solvent under reduced pressure, the resulting residue was subjected to silica gel column chromatography eluting with ethyl acetate: hexane (1: 50) to give a title compound (358 mg) as a yellow crystal. The yield was 80 %.
¹H-NMR(CDCl₃) 0.03(6H, s), 0.86(9H, s), 4.13(2H, s), 4.66(2H, s), 7.14-7.31(5H, m), 7.67(2H, d, J = 8.4 Hz), 7.76(2H, d, J = 8.4 Hz).

### (Formylation)

### 3-Methoxymethoxymethyl-5-(4-trifluoromethyl phenyl) isoxazole-4-carboaldehyde

To a mixture of 3-methoxymethoxymethyl-5-(4-trifluoromethyl phenyl) isoxazole (286 mg) and tetrahydrofuran (6 ml) was added n-butyl lithium (1.6 M hexane solution, 1.56 ml). After stirring at -78 °C for 0.5 hours, N,N-dimethyl formamide 257 mg was added in one portion.. The reaction solution was warmed to room temperature and ice-water was added thereto. The mixture was extracted with ethyl acetate. The organic layer was washed with water and brine, and dried over magnesium sulfate. The solvent was evaporated under reduced pressure. The residue was subjected to silica gel column chromatography eluting with ethyl acetate: n-hexane (1: 5) to give a title compound (179 mg).
NMR(CDCl₃) :δ 3.45(3H,s), 4.81(2H,s), 4.96(2H,s), 7.84(2H,d,J = 8.4Hz), 8.08(2H,d,J = 8.4Hz), 10.14(1H,s)

### (Iminoalkylate)

### 3-methoxymethoxymethyl-5-(4-trifluoromethyl phenyl) isoxazole-4-carboaldehyde ethyloxime

A mixture of 3-methoxymethoxymethyl-5-(4-trifluoromethylphenyl) isoxazole-4-carboaldehyde (12.4 g), ethoxyamine hydrochloride (4.79 g) and tetrahydrofuran (300 ml) was stirred at 60 °C for 3 hours. After the solvent was evaporated under reduced pressure, water was added to the residue and the mixture was extracted with ethyl acetate. The organic layer was washed with water and brine, and dried over magnesium sulfate. The solvent was evaporated under reduced pressure. The residue was subjected to silica gel column chromatography eluting with ethyl acetate: n-hexane (5: 95) to give a title compound (10.6 g).
NMR (CDCl₃): δ 1.33(3H,t,J = 7.2Hz), 3.46(3H,s), 4.23(2H,q,J = 7.2Hz), 4.18(2H,s), 4.89(2H,s), 7.77(2H,d,J = 8.4Hz), 7.88(2H,d,J = 8.4Hz), 8.17(1H,s).

### Process 3 Deprotection (TBS deprotection)

### 4-Benzyl-5-(4-trifluoromethyl phenyl) isoxazole-3-yl] methyl alcohol (R¹ = TFMP, R² = Bn, 2-2-3-1)

To the solution of 4-benzyl-3-(tert-butyldimethyl silyloxy methyl)-5-(4-trifluoromethyl phenyl) isoxazole (2-2-2-2, 358 mg) in tetrahydrofuran (8 ml) was added tetra-butyl ammoniumfluoride (1M tetrahydrofuran solution, 0.88 mL). The solution was stirred at room temperature for 1 hour and the reaction was quenched by adding water. The mixture was extracted with ethyl acetate, washed with water and brine and dried over magnesium sulfate. The solvent was evaporated under reduced pressure. The resiude was subjected to silica gel column chromatography eluting with ethyl acetate: hexane (1: 3) to give a title compound (207 mg) as a colorless crystal. The yield was 78 %.
¹H-NMR(CDCl₃): 4.10(2H,s), 4.62(2H,s), 7.15-7.34(5H,m), 7.70(2H,d,J = 8.7Hz),7.77(2H, d, J = 8.7Hz).

### (Demethoxymethylation)

### [4-Ethoxymethyl-5-(4-trifluothimethyl phenyl) isoxazole-3-yl] methyl alcohol

A mixture of 4-ethoxymethyl-3-methoxymethoxymethyl-5-(4-trifluoromethylphenyl) isoxazole (18.7 g), 6N hydrochloric acid (36.1 ml) and methyl alcohol (311 ml) was refluxed for 4.5 hours. After the solvent was evaporated under reduced pressure, water was added to the residue and the mixture was extracted with ethyl acetate. The organic layer was washed with water and brine and dried over magnesium sulfate. The solvent was evaporated under reduced pressure to give a title compound (15.7 g).
NMR (CDCl₃): δ 1.29(3H,t,J = 7.2Hz), 3.65(2H,q,J = 7.2Hz), 4.61(2H,s), 4.82(2H,s), 7.78-7.80(4H,m).

(2-2-3-2)-(2-2-3-6) were synthesized as well as the above.

**Table 66**

| No | R¹ | R² | Process 2 | NMR |
|---|---|---|---|---|
| 2-2-3-1 | TFMP | Bn | Cross coupling | 0.03(6H,s),0.86(9H,s),4.13(2H,s),4.66(2H,s),7.14-7.31 (5H,m),7.67(2H,d,J=8.4Hz), 7.76(2H,d,J=8.4Hz) |
| 2-2-3-2 | TFMP | Br | Rethiolation | 2.15(1H,brs),4.82(2H,s),7.49(2H,d,J=8.7Hz),7.98(2H, d,J=8.7Hz) |
| 2-2-3-3 | TFMP | CHO | Rethiolation | 3.74(1H,t,J=7.5Hz),4.89(2H,d,J=7.5Hz),7.88(2H,d,J= 8.1Hz),7.95(2H,d,J=8.1Hz),10.10(1H,s) |
| 2-2-3-4 | TFMP | SPh | Rethiolation | 0.04(6H,s),0.85(9H,s),4.74(2H,s),7.11-7.26(5H,m),7.70(2H,d,J=8.7Hz),8.22(2H,d,J=8.7Hz) |
| 2-2-3-5 | TFMP | CH2OEt | Rethiolation | 1.29(3H,t,J=7.2Hz).3.65(2H,q,J=6.9Hz),4.61(2H,s),4.8 1(2H,s),7.78-7.80(4H,m). |
| 2-2-3-6 | TFMP | CH=NOEt | Iminoalkylate | 1.36(3H,t,J=6.9Hz),4.27(2H,q,J=6.9Hz), 4.81(2H,d,J=7.5Hz), 7.79(4H,s), 8.26(1H,s). |

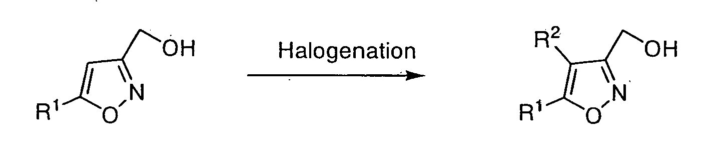

### Reference 6

### [4-Bromo-5-(4-chlorophenyl)-isoxazole-3-yl]-methyl alcohol (R¹ = 4-Cl-C₆H₄-, R² = Br, 2-3-1)

To a solution of [5-(4-chlorophenyl)-isoxazole-3-yl]-methyl alcohol (2-1-3, 2.51 g) and methylene chloride (25 ml) was added N-bromsuccinimide (2.16 g) under ice-cooling. The mixture was stirred for 30 minutes and reacted for more 16 hours at room temperature. After the reaction solution was diluted with chloroform, 1 M sodium hydroxide was added the mixture under ice-cooling. The mixture was extracted with chloroform, washed with water and dried over magnesium sulfate anhydrous. The solvent was evaporated under reduced pressure. The obtained residue was subjected to silica gel column chromatography eluting with ethyl acetate: hexane (1: 2) to give a title compound (1.41 g) as a crystal. The yield was 49 %.

(2-3-2) and (2-3-3) were synthesized with iodine monochloride as a halogen agent as well as the above.

**Table 67**

| No | R¹ | R² | NMR |
|---|---|---|---|
| 2-3-1 | 4-Cl- C₆H₄- | Br | 2.18(1H,t,J=6.6Hz),4.82(2H,d,J=6.6Hz),7.49(2H,d,J=8.7Hz),7. 98(2H,d,J=8.7Hz) |
| 2-3-2 | Me | I | 2.11(1H,t,J=6.6Hz),2.47(3H,s),4.69(2H,d,J=6.6Hz) |
| 2-3-3 | Et | I | 1.30(3H,t,J=7.5Hz),2.82(2H,q,J=7.5Hz),4.70(2H,s) |

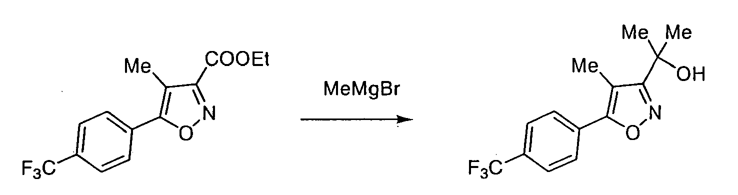

### Reference 7

### 2-[4-Methyl-5-(4-trifluoromethyl phenyl)-isoxazole-3-yl]-propane-2-ol (2-4-1)

5-(4-Trifluoromethyl phenyl)-isoxazole-3-carboxylic acid ethyl ester (1-1-2, 1.03 g) was dissolved in tetrahydrofuran anhydride (10 ml). 1M methyl magnesium bromide 7.3 ml was added thereto under ice - methyl alcohol cooling. The reaction solution was returened to room temperature and stirred for 24 hours. Saturated ammonium chloride solution was added to the reaction solution, and the mixture was extracted with ethyl acetate, washed with brine and dried over magnesium sulfate anhydrous. After removal of solvent under reduced pressure, the obtained residue was subjected to silica gel column chromatography eluting with ethyl acetate: hexane (1: 4) to give a colorless crystal. These crystals were recrystallized from ether - hexane to give a title compound (738 mg). The yield was 75 %.
Melting point: 126-127 °C
¹H-NMR(CDCl₃): 1.71(6H,s), 2.38(3H,s), 7.75(2H,d,J = 8.4Hz), 7.81(2H,d,J = 8.4Hz).

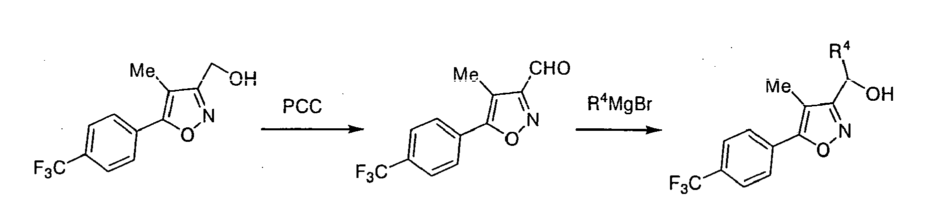

### Reference 8

### Process 1 Oxidation

### 4-Methyl-5-(4-trifluoromethylphenyl)-isoxazole-3-carbaldehyde (2-5-1-1)

Compound (2-1-2, 4.88 g) was dissolved in methylene chloride (200 ml). Pyridinium chlorochromate (8.30 g) was added thereto and the mixture was stirred at room temperature for 22 hours. The reaction solution was filtrated with silica gel and washed with chloroform. The filtration was evaporated under reduced pressure. The obtained residue was subjected to silica gel column chromatography eluting with ethyl acetate: hexane (1: 4) to give a colorless crystal. These crystals were recrystallized from hexane to give a title compound (4.14 g). The yield was 86 %.
¹H-NMR(CDCl₃): 2.49(3H,s), 7.79(2H,d,J = 8.1Hz), 7.87(2H,d,J = 8.1Hz), 10.23(1H,s).
Process 2 Alkylate
1-[4-Methyl-5-(4-triffuoromethyl phenyl)-isoxazole-3-yl]-propane-1-ol (R⁴ = Et, 2-5-2-1)

Compound (2-5-1-1, 765 mg) obtained by the first process was dissolved in tetrahydrofuran anhydride (20 ml). 1M ethyl magnesium bromide (3.2 ml) was added thereto at -70°C and the mixture was stirred for 1.5 hours. To the reaction solution was added saturated ammonium chloride solution. The mixture was extracted with ethyl acetate, washed with brine and dried over magnesium sulfate anhydrous. The solvent was evaporated under reduced pressure. The obtained residue was subjected to silica gel column chromatography eluting with ethyl acetate: hexane (1: 3) to give a title compound (345 mg) as a colorless crystal. The yield was 40%.

(2-5-2-2) was synthesized as well as the above.

**Table 68**

| No | R⁴ | NMR |
|---|---|---|
| 2-5-2-1 | Et | 1.05(3H,t,J=7.5Hz),1.92-2.04(2H,m),2.30(3H,s),4.83 (1H.t,J=6.6Hz),7.75(2H,t,J=8.4Hz), 7.83(2H,d,J=8.4Hz) |
| 2-5-2-2 | 4-F- C₆H₄- | 2.03(3H,s),6.03(1H,s),7.05-7.11(2H,m),7.42-7.47(2H,m),7.73(2H, d,J=8.4Hz),7.79(2H,d,J=8.4Hz) |

### Reference 9

### (4-Methyl-5-morpholine-4-yl-isoxazole-3-yl)-methyl alcohol (2-6-1)

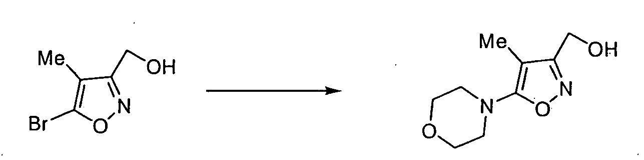

Compound (2-1-7, 1.66 g) was dissolved in morpholine (5 ml) and the solution was stirred at 140 °C for 2 hours. To the reaction solution was added water. The mixture was extracted with ethyl acetate, washed with beine and dried over magnesium sulfate anhydrous. The solvent was evaporated under reduced pressure. The obtained residue was subjected to silica gel column chromatography eluting with ethyl acetate: hexane (2: 1) to give a title compound (1.14 g) as a pale yellow crystal. The yield was 66 %.
¹H-NMR(CDCl₃): 1.98(3H,s), 3.35-3.38(4H,m), 3.78-3.82(4H,m), 4.60(2H,s).

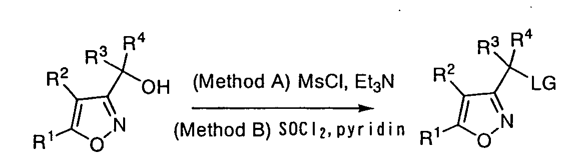

### Reference 10 Method A (LG = OMs)

### Methanesulphonate-4-formyl-5-(4-trifluoromethylphenyl)-isoxazole-3-yl methyl ester (R¹ = TFMP, R² = CHO, R³, R⁴ = H, 3-1-1-1)

Compound (2-2-4-2, 1.79 g) was mixed in methylene chloride (30 ml). Methanesulfonylchloride 0.61 ml and triethylamine 1.38 ml was added thereto under ice- cooling. After stirring 1 hour, water was added to the reaction solution. The mixture was extracted with chloroform, washed with brine and dried over magnesium sulfate anhydrous. The solvent was evaporated under reduced pressure. The obtained residue was subjected to silica gel column chromatography eluting with chloroform to give a colorless crystal. After addition of hexane, the crystal was crushed and collected to give a title compound (2.21 g) as a colorless crystal. The melting point is 129-130 °C. The yield was 96 %.

(3-1-1-2)-(3-1-1-6) were synthesized as well as the above.

**Table 69**

| No | R¹ | R² | NMR |
|---|---|---|---|
| 3-1-1-1 | TFMP | CHO | 3.21(3H,s),5.58(2H,s),7.88(2H,d,J=8.4Hz), 8.01(2H,d,J=8.4Hz),10.14(1H,s) |
| 3-1-1-2 | Morpholine-4-yl | Me | 2.01(3H,s),3.05(3H,s),3.38-3.41(2H,m),3.79-3.82(2 H,m),5.16(2H,s) |
| 3-1-1-3 | 4-Cl-C6H4- | CH2OEt | 1.28(3H,t,J=6.9Hz),3.10(3H,s),3.63(2H,q,J=6.9Hz), 4.50(2H,s),5.41(2H,s).7.50(2H,d, J=8.4Hz), 7.70(2H,d,J=8.4Hz). |
| 3-1-1-4 | TFMP | CH=NOEt | 1.34(3H,t,J=7.2Hz),3.18(3H,s),4.26(2H,q,J=7.2Hz), 5.58(2H,s),7.80-7.81(4H,m), 8.17(1H,s) |
| 3-1-1-5 | 4-Cl-C6H4- | CH=NOEt | 1.33(3H,t,J=7.2Hz),3.16(3H,s),4.25(2H,q,J=7.2Hz), 5.56(2H,s)7.51(2H,d,J=9.0Hz), 7.63(2H,q,J=9.0Hz), 8.14(1H,s) |
| 3-1-1-6 | 4-OCF3-C6H4- | CH=NOEt | 1.33(3H,t,J=7.2Hz),3.17(3H,s), 4.25(2H,q,J=7.2Hz),5.57(2H,s)7.37(2H,d,J=8.7Hz), 7.73(2H,q,J=8.7Hz),8.15(1H,s) |

### Reference 11 Method B (LG = Cl)

### 3-Chloromethyl-5-(4-chlorophenyl)-isoxazole (R¹ =4-Cl-C₆H₄, R² =H, R³ =H, R⁴ =H, 3-1-2-1)

To a solution of [5-(4-chlorophenyl)-isoxazole-3-yl]-methyl alcohol (2-1-3, 1.73 g) and chloroform (30 ml) was added thionyl chloride (2.1 g). A solution of pyridine (630 mg) in chloroform (2 ml) was added dropwise to the mixture under ice cooling for 3 minutes. The mixture was stirred at room temperature for 5 hours. After the solvent was evaporated under reduced pressure, chloroform and water were added and the mixture was extracted with chloroform. The organic layer was washed with water and brine. The solvent was evaporated under reduced pressure. The obtained residue was subjected to silica gel column chromatography eluting with ethyl acetate: hexane (1: 1) to give a title compound (1.72 g) as a crystal. The yield was 92%.

Compounds (3-1-2-2)-(3-1-2-17) were synthesized as well as the above.

**Table 70**

| No | R¹ | R² | R³,R⁴ | NMR |
|---|---|---|---|---|
| 3-1-2-1 | 4-Cl- C₆H₄- | H | H,H | 4.64(2H,s),6.63(1H,s),7.46(2H,d,J=8.4Hz),7.7 3(2H,d,J=8.4Hz) |
| 3-1-2-2 | TFMP | H | H,H | 4.66(2H,s),6.45(1H,s),7.75(2H,d,J=9.0Hz),7.9 1(2H,d,J=9.0Hz) |
| 3-1-2-3 | TFMP | Me | H,H | 2.33(3H,s),4.65(2H,s),7.76(2H,d,J=8.7Hz),7.8 5(2H,d,J=8.7Hz) |
| 3-1-2-4 | TFMP | CHO | H,H | 4.89(2H,s),7.87(2H,d,J=8.7Hz),8.03(2H,d,J=8. 7Hz),10.17(1H,s) |
| 3-1-2-5 | TFMP | Me | H,Et | 1.15(3H,t,J=7.5Hz),2.30(2H,qd,J=7.5,7.5Hz),4 .93(1H,t,J=6.6Hz),7.76(2H,t,J=8.4Hz), 7.83(2H,d,J=8.4Hz) |
| 3-1-2-6 | TFMP | Me | H,4-F-C₆H₄- | 2.14(3H,s),6.62(1H,s),7.07-7.13(2H, m),7.50-7.55(2H,m),7.75(2H,d, J=8.4Hz),7.81 (2H,d,J=8.4Hz) |
| 3-1-2-7 | TFMP | SPh | H,H | 4.55(2H,s),7.13-7.27(5H,m),7.73(2H, d,J=8.7Hz),8.25(2H,d,J=8.7Hz) |
| 3-1-2-8 | TFMP | Bn | H,H | 4.15(2H,s),4.41(2H,s),7.15-7.35(5H,m),7.71(2 H,d,J=8.7Hz),7.78(2H,d,J=8.7Hz) |
| 3-1-2-9 | 4-Cl-C₆H₄- | H | H,H | 4.64(2H,s),6.63(1H,s),7.46(2H,d,J=8.4Hz),7.7 3(2H,d,J=8.4Hz) |
| 3-1-2-10 | 4-Cl-C₆H₄- | Br | H,H | 4.46(2H,s),7.50(2H,d,J=8.7Hz),7.99(2H,d,J=8. 7Hz) |
| 3-1-2-11 | 4-Cl-C₆H₄- | Et | H,H | 1.28(3H,t,J=7.5Hz),2.72(2H,q,J=7.5Hz),4.64(2 H,s),7.47(2H,d,J=8.4Hz),7.65(2H,d,J=8.4Hz) |
| 3-1-2-12 | Br | Me | H,H | 2.06(3H,s),4.56(2H,s) |
| 3-1-2-13 | Pyridine-4-yl | H | H,H | 4.66(2H,s),6.85(1H,s),7.67(2H,d,J=6.0Hz),8.7 7(2H,d,J=6.0Hz) |
| 3-1-2-14 | Me | I | H,H | 2.49(3H,s),4.53(2H,s) |
| 3-1-2-15 | Et | I | H,H | 1.31(3H,t,J=7.5Hz),2.83(2H,q,J=7.5Hz)4.53(2 H,s) |
| 3-1-2-16 | TFMP | CH2O Et | H,H | 1.28(3H,t,J=6.9Hz), 3.64(2H,q,J=6.9 Hz),4.57(2H,s),4.73(2H,s),7.69(2H,d,J=8. 4Hz),7.90(2H,d,J=8.4Hz) |
| 3-1-2-17 | 4-OCF3-C6H4- | CH2O Et | H,H | 1.28(3H,t,J=6.9Hz), 3.69(2H,q,J=6.9 Hz),4.55(2H,s),4.72(2H,s),7.35(2H,d,J=8.7Hz), 7.82(2H,d.J=8.7Hz) |

### Reference 12

### [3-Chloromethyl-5-(4-trifluoromethyl phenyl)-isoxazole-4-yl]-methyl alcohol (3-2-1)

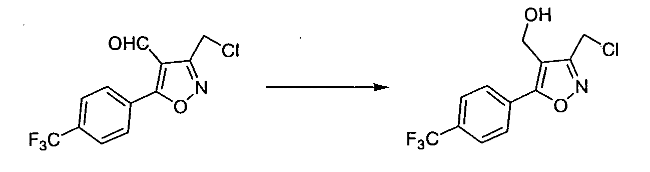

To a solution of 3-chloromethyl-5-(4-trifluoromethyl phenyl)-isoxazole-4-carbaldehyde (3-1-2-4, 203 mg) and methyl alcohol (5 ml) was added sodium borohydride (21 mg) under ice cooling. The mixture was stirred at room temperature for 2 hours. After the solvent was evaporated under reduced pressure, water was added to the residue. The mixture was extracted with chloroform, washed with brine and dried over magnesium sulfate anhydrous. The solvent was evaporated under reduced pressure. The obtained residue was subjected to silica gel column chromatography eluting with ethyl acetate: hexane (1: 3) to give a title compound (210 mg) as a crystal. The yield was 87 %.

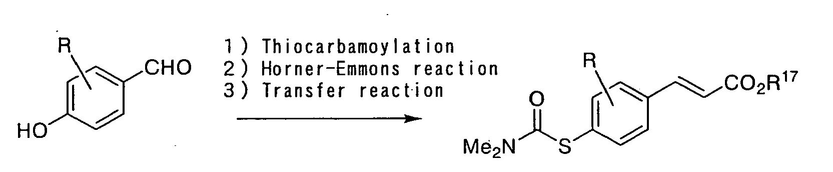

### Reference 13

### Process 1 Thiocarbamoylation

### Dimethyl thio carbamate 2-fluoro-4-formyl phenylester (R = 3-F, R¹⁷ = Me, 4-1-1)

A mixture of 3-fluoro-4-hydroxy benzaldehyde (5.00 g), N,N-dimethyl thiocarbamoyl chloride (5.29 g), triethylamine (4.33 g), N,N-dimethyl amino pyridine (436 mg) and dioxane (50 ml) was stirred for 3 hours. After addition of water, the mixture was extracted with ethyl acetate. The organic layer was washed with water and brine and dried over magnesium sulfate. The solvent was evaporated under reduced pressure. The residue was washed with isopropyl ether to give a title compound (7.05 g) as blackish brown crystal. The yield was 71 %.
¹H-NMR(CDCl₃): 3.39(3H, s), 3.47(3H, s), 7.27-7.35(1H, m), 7.67-7.74(2H, m), 9.97(1H, s).

### Process 2 Horner-Emmons reaction

### 3-(4-Dimethyl thiocarbamoyloxy-3-fluorophenyl) acrylic acid methyl ester (R = 3-F, R¹⁷ = Me, 5-1-1)

To a mixture of dimethyl thiocarbamate 2-fluoro-4-formyl phenylester (4-1-1, 7.05 g), dimethyl phosphono methyl acetate (5.89 g), lithium chloride (1.57 g) and dimethyl formamide (70 ml), was added 1,8-diazabicyclocyclo[5.4.0] undec-7-ene (5.16 g). The mixture was stirred at room temperature for 2.5 hours. After addition of water, the mixture was extracted with ethyl acetate. The organic layer was washed with water and brine and dried over magnesium sulfate. The solvent was evaporated under reduced pressure. The residue was washed with isopropyl ether to give a title compound (7.50 g) as blackish brown crystal. The yield was 86 %.
¹H-NMR(CDCl₃): 3.37(3H, s), 3.46(3H, s), 3.81(3H, s), 6.39(1H, d, J = 15.9 Hz), 7.12(1H, m), 7.30-7.35(2H, m), 7.63(1H, d, J = 15.9Hz).

### Process 3 Transfer reaction

### 3-(4-Dimethylcarbamoyl sulfanil-3-fluorophenyl) acrylic acid methyl ester (R = 3-F, R¹⁷ = Me, 6-1-1)

A mixture of 3-(4-dimethyl thiocarbamoyloxy-3-fluorophenyl) acrylic acid methyl ester (5-1-1, 7.00 g) and diphenylether was stirred at 265 °C for 30 minutes. After cooling the reaction solution to room temperature, the solution was subjected to silica gel column chromatography eluting with chloroform to give a title compound (7.00 g) as a colorless crystal. The yield was 100 %.

(6-1-2)-(6-1-17) were synthesized as well as the above.

**Table 71**

| No | R | R¹⁷ | NMR |
|---|---|---|---|
| 6-1-1 | 3-F | Me | 3.04(3H,br),3.13(3H,br),3.82(3H,s),6.45(1H,d, J=16.2Hz),7.26-7.31(2H,m),7.48-7.53(1H,m), 7.64(1H,d,J=16.2 Hz) |
| 6-1-2 | 3-OMe | Me | 2.95-3.20(6H,m),3.82(3H,s),3.90(3H,s), 6.45(1H,d,J=15.9Hz),6.95-7.18(2H,m), 7.48(1H,d,J=7.8.Hz), 7.67(1H, d, J=16.2 Hz) |
| 6-1-3 | 2-OMe | Me | 2.96-3.18(6H,m),3.80(3H,s),3.89(3H,s), 6.53(1H,d,J=16.2Hz),7.06-7.13(2H,m), 7.49(1H,d,J=8.1Hz), 7.96(1H, d, J=16.2 Hz) |
| 6-1-4 | 3-Br, 5-OMe | Me | 2.90-3.30(6H,m),3.82(3H,s),3.89(3H,s), 6.45(1H,d,J=15.9Hz),7.26(1H,brs), 7.48(1H,brs),7.59(1H,d, J=15.9 Hz) |
| 6-1-5 | 2-OMe, 6-OMe | Me | 2.90-3.20(6H,m),3.79(3H,s),3.88(6H,s), 6.73(2H,s) 6.88(1H, d, J=16.2 Hz), 8.08(1H,d, J=16.2 Hz) |
| 6-1-6 | 3-OEt | Me | 1.34(3H,t,J=6.9Hz),1.43(3H,t,J=6.6Hz),2.90-3.30 (6H,m),4.12(2H,q,J=6.9Hz),4.27(2H,q,J=7.2Hz), 6.43(1H,d,J=15.9Hz)7.04(1H,d,J=1.5Hz),7.12(1H,dd,J=7.8Hz, 1.8Hz),7.48(1H,d,J=7.8Hz) 7.64(1H,d,J=15.9 Hz) |
| 6-1-7 | 3-Br | Me | 2.95-3.23(6H,m),3.81(3H,s),6.45(1H,d,J=15.9Hz), 7.45(1H,dd,J=8.1Hz,2.1Hz),7.60(1H,d,J=16.2Hz), 7.6(1H,d,J=8.1Hz), 7.81(1H,J=2.1Hz) |
| 6-1-8 | 3,5-diBr | Me | 2.80-3.20(6H,m),3.74(3H,s),6.90(1H,d,J=15.9Hz), 7.60(1H,d,J=15.9Hz), 8.21(2H,s) |
| 6-1-9 | 3Cl,5OMe | Me | 2.90-3.30(6H,m),3.82(3H,s),3.90(3H,s),6.45(1H,d, J=16.2Hz),6.96(1H,d,J=1.5Hz),7.31(1H,d,J=1.5Hz), 7.60(1H, d,J=16.2Hz) |
| 6-1-10 | 3-OMe, 5-OMe | Me | 2.85-3.35(6H,m),3.82(3H,s),3.89(6H,s),6.46(1H,d, J=15.9Hz)6.76(2H,s),7.66(1H,d,J=15.9Hz) |
| 6-1-11 | 2-Cl | Me | 2.90-3.20(6H,m),3.82(3H,s),6.44(1H,d,J=15.9Hz), 7.36-7.60(2H,m),7.60(1H,d,J=8.1Hz), 8.06(1H,J=16.2 Hz) |
| 6-1-12 | 3-Br, 5-OEt | Me | 1.42(3H,t,J=7.2Hz),2.85-3.35(6H,m),3.01(3H,s), 4.10(2H,q,J=7.2Hz),6.43(1H,d,J=15.9Hz),6.97 (1H,brs),7.46(1H,brs), 7.57 (1H, d, J=15.9 Hz) |
| 6-1-13 | 2-F | Me | 2.95-3.15(6H,m),3.82(3H,s),6.55(1H,d,J=16.5Hz), 7.26-7.33(2H,m),7.52(1H,d,J=7.8Hz), 7.79(1H,J=16.2 Hz) |
| 6-1-14 | 2-Me | Me | 2.43(3H,s),3.04(3H,br),3.09(3H,br),3.81(3H,s),6.37(1H,d,J=15 .9Hz),7.33-7.35(2H,m), 7.54(1H,d,J=8.7Hz),7.94(1Hm,d,J=15.9Hz) |
| 6-1-15 | H | Me | 3.06(6H,br),3.81(3H,s),6.45(1H,d,J=15.9Hz),7.51(4H,brs),7.6 8(1H,d,J=15.9Hz) |
| 6-1-16 | 2-Me, 3-OMe | Me | 3.02(3H,Br),3.12(3H,Br),3.82(3H,s),3.88(3H,s),6.37(1H,d,J=1 5.9Hz),7.07(1H,s),7.32(1H,s),7.92(1H,d,J=15.9Hz) |
| 6-1-17 | 3-Cl | Me | 3.05(3H,br),3.13(3H,br),3.81 (3H,s),6.45(1H,d,J=15.9Hz),7.40( 1H,dd,J=1.8Hz,8.1Hz),7.58-7.63(3H,m) |

### Reference 14

### (5-Hydroxyindole-1-yl) acetic acid methyl ester

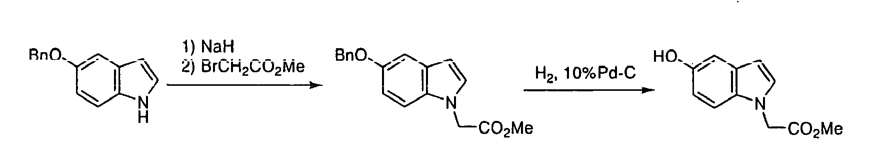

### Process 1

### (5-Henzyloxyindole-1-yl) acetic acid methyl ester

To 5-benzyloxy indole 446 mg in dimethyl formamide (5 ml) was added sodium hydride (88 mg) under ice cooling. The mixture was stirred at room temperature for 3 hours. The reaction solution was cooled with ice. Bromomethyl acetate (228 ml) was added thereto and the mixture was stirred for 1 hour 30 minutes. To the reaction solution, were added 2N hydrochloric acid and water. The mixture was extracted with ethyl acetate. The organic layer was washed successively with water and brine and dried over magnesium sulfate. The solvent was evaporated under reduced pressure. The obtained residue was purified with silica gel column chromatography eluted with ethyl acetate: hexane (1: 4) to give a title compound (400 mg). The yield was 68 %.
¹H-NMR, (CDCl₃) δ: 3.74(3H,s), 4.82(2H,s), 5.10(2H,s), 6.47(1H,dd,J = 0.6,3.3Hz), 6.94-7.50 (10H,m).

### Process 2

### (5-Hydroxyindole-1-yl) acetic acid methyl ester

To (5-Benzyloxyindole-1-yl) acetic acid methyl ester (400 mg) in tetrahydrofuran (5 ml) - methyl alcohol (5 ml) was added 10 % palladiumcarbon (120 mg). The mixture was stirred in hydrogen atmosphere at room temperature for 3 hours. The reaction solution was filtrated and the solvent was evaporated under reduced pressure. The obtained residue was purified with silica gel column chromatography eluting with ethyl acetate: hexane (2: 3) to give a title compound (256 mg). The yield was 92 %.
¹H-NMR (CDCl₃) δ: 3.74(3H,s), 4.49(1H,s), 4.82(2H,s), 6.44(1H,d,J = 3.0Hz), 6.79(1H,dd,J = 2.7,9.0Hz), 7.04(1H,d,J = 2.7Hz), 7.06(1H,d,J = 3.0Hz), 7.10(1H,d,J = 9.0Hz).

### Reference 15

### (5-Dimethyl carbamoyl sulfanilindole-1-yl) acetic acid methyl ester

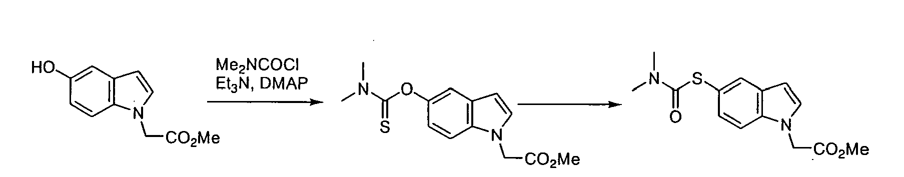

### Process 1

### (5-Dimethyl thiocarbamoyloxy indole-1-yl) acetic acid methyl ester

A mixture of (5-hydroxyindole-1-yl) acetic acid methyl ester (724 mg), N,N-dimethyl thiocarbamoyl chloride (523 mg), triethylamine (0.59 ml), N,N-dimethyl amino pyridine (43 mg) and dioxane (7 ml) was stirred for 3 hours 30 minutes. After addition of water, the mixture was extracted with ethyl acetate. The organic layer was washed with water and brine and dried over magnesium sulfate. The solvent was evaporated under reduced pressure. The residue was washed with isopropyl ether - methyl alcohol to give a title compound (443 mg) as a blackish brown crystal. The yield was 43 %.
¹H-NMR (CDCl₃) δ: 3.37(3H,s), 3.48(3H,s), 3.75(3H,s), 4.84(2H,s), 6.55(1H,d,J = 3.3Hz), 6.95(1H,dd,J = 2.4,9.0Hz), 7.12(1H,d,J = 3.3Hz), 7.23(1H,d,J = 9.0Hz), 7.29(1H,d,J = 2.4Hz).

### Process 2

### (5-Dimethylcarbamoyl sulfanilindole-1-yl) acetic acid methyl ester

A mixture of (5-dimethyl thiocarbamoyloxyindole-1-yl) acetic acid methyl ester (214 mg) and diphenylether (3 ml) was stirred at 270 °C for 5 hours. The reaction solution was cooled to room temperature and subjected to silica gel column chromatography eluting with ethyl acetate: hexane (1: 3) to give a title compound (139 mg). The yield was 65 %.
¹H-NMR (CDCl₃) δ: 3.07(6H,s), 3.73(3H,s), 4.85(2H,s), 6.55(1H,d,J = 3.3Hz), 7.10(1H,d,J = 3.3Hz), 7.08-7.35 (2H,m), 7.78(1H,d,J = 1.5Hz).

### Reference 16

### 2-(4-Dimethyl carbamoyl sulfanilphenyl) thiophene-3-carboxylate methyl ester

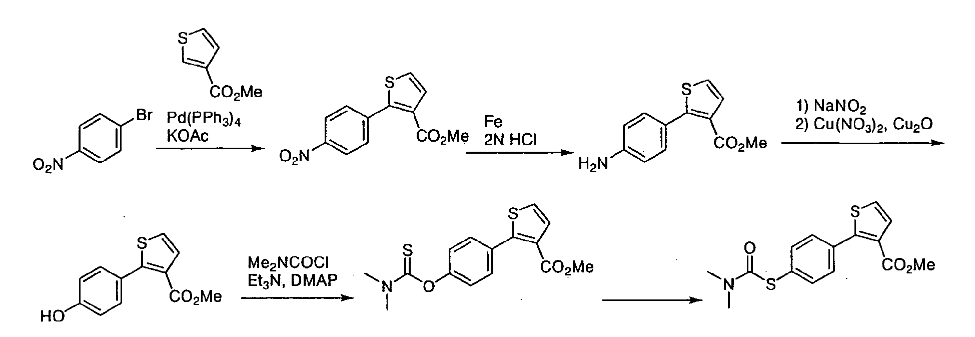

### Process 1

### 2-(4-Nitrophenyl) thiophene-3-carboxylate methyl ester

A mixture of 4-bromonitro benzene (3.49 g), thiophene-3-carboxylate methyl ester (3.44 g), tetrakis triphenylphosphine palladium (1.0g), potassium acetate (2.54 g) and toluene (35 ml) was refluxed under heating for 60 hours. To the reaction solution was added water and the mixture was extracted with ethyl acetate. The organic layer was washed with brine and dried over magnesium sulfate. The solvent was evaporated under reduced pressure. The obtained residue was subjected to silica gel column chromatography eluting with ethyl acetate: hexane (1: 6) to give a title compound (2.78 g). The yield was 61 %.
¹H-NMR, (CDCl₃) δ: 3.77(3H,s), 7.37(1H,d,J = 5.4Hz), 7.56(1H,d,J = 5.4Hz), 7.67(2H,d,J = 9.0Hz), 8.26(2H,d,J = 9.0Hz).

### Process 2

### 2-(4-Aminophenyl) thiophene-3-carboxylate methyl ester

A mixture of iron (318 mg), 2N hydrochloric acid (95 ml), 2-(4-nitrophenyl) thiophene-3-carboxylate methyl ester (250 mg) and ethanol (4.8 ml) - water (1.2 ml) was refluxed for 15 minutes. After cooling, the reaction solution was filtrated and concentrated under reduced pressure. The obtained residue was subjected to silica gel column chromatography eluting with ethyl acetate: hexane (1: 2) to give a title compound (213 mg). The yield was 96 %.
¹H-NMR (CDCl₃) δ: 3.75(3H,s), 4.23(2H,brs), 6.73(2H,d,J = 8.7Hz), 7.15(1H,d,J = 5.4Hz), 7.33(2H,d,J = 8.7Hz), 7.46(1H,d,J = 5.4Hz).

### Process 3

### 2-(4-Hydroxy phenyl) thiophene-3-carboxylate methyl ester

A suspension of 2-(4-amino phenyl) thiophene-3-carboxylate methyl ester (790 mg) in water (90 ml) - concentrated sulfuric acid (5.3 ml) was cooled to - 4 °C. A solution of sodium nitrite (237 mg) in (2.5 ml) was added dropwise to the mixture for 5 minutes. The mixture was stirred at -4 °C for 40 minutes and a solution of copper nitrate (II) (3.77 g) in water (15 ml) and copper oxide (I) (822 mg) were added thereto. The mixture was stirred at the same temperature for 20 minutes and at room temperature for 45 minutes. To the reaction solution was added water and the mixture was extracted with ethyl acetate. The organic layer was washed with brine and dried over magnesium sulfate. The solvent was evaporated under reduced pressure. The obtained residue was subjected to silica gel column chromatography eluting with ethyl acetate: hexane (1: 3) to give a title compound (363 mg). The yield was 46 %.
¹H-NMR (CDCl₃) δ: 3.76(3H,s), 4.49(1H,brs), 6.84(2H,d,J = 8.4Hz), 7.19(1H,d,J = 5.7Hz), 7.39(2H,d,J = 8.4Hz), 7.48(1H,d,J = 5.7Hz).

### Process 4

### 2-(4-Dimethyl thiocarbamoyl oxy phenyl) thiophene-3-carboxylate methyl ester

A mixture of 2-(4-hydroxy phenyl) thiophene-3-carboxylate methyl ester (530 mg), N,N-dimethyl thiocarbamoyl chloride (336 mg), triethylamine (0.38 ml), N,N-dimethyl amino pyridine (28 mg) and dioxane (6 ml) was stirred for 5 hours. After addition of water, the mixture was extracted with ethyl acetate. The organic layer was washed with water and brine and dried over magnesium sulfate. The solvent was evaporated under reduced pressure. The residue was washed with isopropyl ether - methyl alcohol to give a title compound (632 mg) as a blackish brown crystal. The yield was 87 %.
¹H·NMR (CDCl₃) δ: 3.36(3H,s), 3.48(3H,s), 3.74(3H,s), 7.11(2H,d,J = 8.7Hz), 7.24(1H,d,J = 5.4Hz), 7.50(1H,d,J = 5.4Hz), 7.51(2H,d,J = 8.7Hz).

### Process 5

### 2-(4-Dimethyl carbamoyl sulfanilphenyl) thiophene-3-carboxylate methyl ester

A mixture 2-(4-dimethyl thiocarbamoyloxy phenyl) thiophene-3-carboxylate methyl ester (660 mg) and diphenylether (6 ml) was stirred at 270 °C for 1 hour 30 minutes. The reaction solution was cooled to room temperature and subjected to silica gel column chromatography eluting with ethyl acetate: hexane (1: 4) to give a title compound (601 mg). The yield was 91 %.
¹H-NMR (CDCl₃) δ: 3.06(6H,brs), 3.74(3H,s), 7.25-7.55(6H,m).

### Reference 17

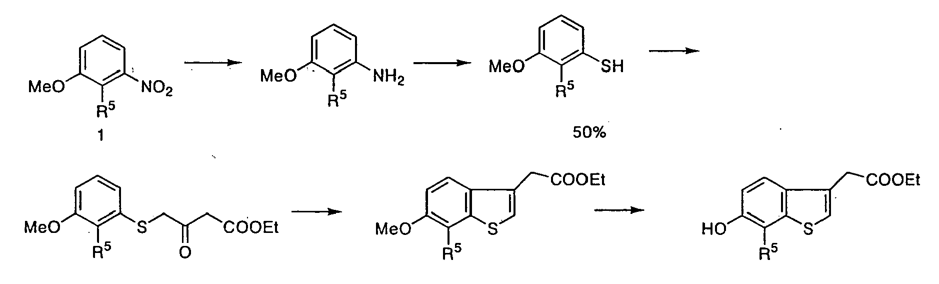

### Process 1

### 3-Methoxy-2-methyl phenylamine (R⁵ = Me)

A mixture of 2-methyl-3-nitroanisole (16.7 g), 10 % Pd-C (1.6 g) and ethanol (330 ml) was stirred in hydrogen atmosphere for 6 hours. The insoluble residue was filtrated and the filtrate was concentrated under reduced pressure to give a title compound (12.5 g).
NMR (CDCl₃): δ 2.04(3H,s), 3.71(3H,s), 6.33-6.36(2H,m), 6.94-7.00(1H,m).

### Process 2

### 3-Methoxy-2-methyl benzenethiol (R⁵ = Me)

A solution of sodium nitrite (5.92 g) in water (12 ml) was added to a mixture of 3-methoxy-2-methyl phenylamine (10.7 g), water (30 ml) and 35 % hydrochloric acid (15 ml) under ice cooling. This mixture was added to a mixture of potassium xanthate (12.5 g) and water (13 ml) at 40 °C. The mixture was stirred at 50 °C for 2 hours and ice water (50 ml) was added. The mixture was extracted with ethyl acetate. The organic layer was washed with water and brine and dried over magnesium sulfate. The solvent was evaporated under reduced pressure to give a title compound (6.12 g). The yield was 61%.
NMR (CDCl₃): δ 2.17(3H,s),3.31(1H,s),3.80(3H,s),6.65(1H,d,J = 8.4Hz), 6.87(1H,dd,J = 7.5Hz),6.97-7.03(1H,m).

### Process 3

### 4-(3-Methoxy-2-methyl phenylsulfanil)-3-oxo butanoic acid ethyl ester (R⁵ = Me)

A mixture of 3-methoxy-2-methyl benzenethiol (6.1 g), ethylmalonylchloride (6.25 g), cesium carbonates (27.9 g) and acetonitrile (160 ml) was stirred at room temperature for 23 hours. The insoluble residue was filtrated and the filtrate was evaporated under reduced pressure. After addition of water, the mixture was extracted with ethyl acetate. The organic layer was washed with water and brine and dried over magnesium sulfate. The solvent was evaporated under reduced pressure. The obtained residue was subjected to silica gel column chromatography eluting with ethyl acetate: n-hexane (1: 2) to give a title compound (4.05 g).
NMR (CDCl3) δ: 1.26 (3H, t, J = 7.2 Hz), 2.31 (3H, s), 3.60 (2H, s), 3.77 (2H,s), 3.81 (3H, s), 4.17 (2H, q, J = 7.2Hz), 6.75 (1H, d, J = 8.1 Hz), 6.89 (1H, dd, J = 8.1 Hz, 0.6 Hz), 7.08-7.14 (1H, m).

### Process 4

### (6-Methoxy-7-methyl benzo [b] thiophene-3-yl) ethyl acetate ester (R⁵ = Me)

To methanesulfonic acid (27 ml) was added 4-(3-methoxy-2-methyl phenylsulfanil)-3-oxo butanoic acid ethyl ester 4.50 g under ice cooling. The mixture was stirred at room temperature for 1.5 hours. To the reaction solution, was added ice water 100 ml and the mixture was extracted with ethyl acetate. The organic layer was washed with water and saturated saline solution and dried over magnesium sulfate. The solvent was evaporated under reduced pressure. The obtained residue was subjected to silica gel column chromatography and eluted with ethyl acetate: n-hexane (1: 4) to give a title compound 1.5 g.
NMR (CDC13) δ: 1.17 (3H, t, J = 7.2 Hz), 2.31 (3H, s), 3.84 (3H, s), 3.86 (2H, d, J = 0.9 Hz), 4.07 (2H, q, J = 7.2 Hz), 7.15 (1H, d, J = 8.7 Hz), 7.34 (1H, s), 7.56 (1H, d, J = 8.7 Hz)

### Process 5

### (6-hydroxy-7-methyl benzo [b] thiophene-3-yl) ethyl acetate ester (R⁵ = Me)

To a mixture of (6-methoxy-7-methyl benzo [b] thiophene-3-yl) ethyl acetate ester (4.6 g) and methylene chloride (120 ml) was added boron tribromide in methylene chloride (1M solution) at -40 °C. The reaction solution was warmed to room temperature and stirred for 0.5 hours. The reaction solution was poured into ice water (200 ml) and the organic layer was separated. The organic layer was washed with water and brine and dried over magnesium sulfate. The solvent was evaporated under reduced pressure. The obtained residue was subjected to silica gel column chromatography eluting with ethyl acetate: n-hexane (1: 3) to give a title compound (2.1 g).
NMR (CDCl₃): δ 1.78(3H,t,J = 6.9Hz), 2.28(3H,s), 3.83(2H,s), 4.08(2H,q,J = 6.9Hz), 6.95(1H,d,J = 8.4Hz), 7.28(1H,s), 7.40(1H,d,J = 8.4Hz), 9.47(1H,br).

### Reference 18

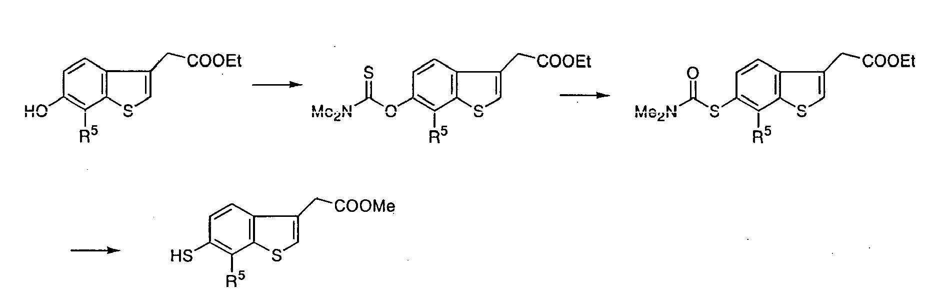

### Process 1

### (6-Dimethyl thiocarbamoyl oxy-7-methyl benzo [b] thiophene-3-yl) ethyl acetate ester (R⁵=Me)

A mixture of (6-hydroxy-7-methyl benzo [b] thiophene-3-yl) ethyl acetate ester (2.70 g), N,N-dimethyl thiocarbamoyl chloride (1.65 g), triethylamine (1.32 g), N,N-dimethyl amino pyridine (264 mg) and acetonitrile (40 ml) was refluxed for 4 hours. The reaction solution was poured into ice water and the mixture was extracted with ethyl acetate. The organic layer was washed with water and brine and dried over magnesium sulfate. The solvent was evaporated under reduced pressure. The obtained residue was subjected to silica gel column chromatography eluting with ethyl acetate: n-hexane (1: 2) to give a title compound (2.95 g).
NMR (CDCl₃): δ 1.26(3H,s), 2.39(3H,s), 3.41(3H,s), 3.49(3H,s), 3.82(2H,s), 4.17(2H,q), 7.09(1H,d,J = 8.7Hz), 7.34(1H,s), 7.61(1H,d,J = 8.7Hz).

### Process 2

### (6-Dimethyl carbamoyl sulfanil-7-methyl benzo [b] thiophene-3-yl) ethyl acetate ester (R⁵ = Me)

A mixture of (6-dimethyl thiocarbamoyl oxy-7-methyl benzo [b] thiophene-3-yl) ethyl acetate ester (2.90 g) and phenylxylylethane (29 ml) was stirred at 265 °C for 8 hours. The reaction solution was subjected to silica gel column chromatography eluting with n-hexane and ethyl acetate: n-hexane (1 : 2) to give a title compound (2.34 g).
NMR (CDCl₃): δ 1.25(3H,t,J = 7.2Hz), 2.66(3H,s), 3.04-3.14(6H,br), 3.82(2H,d,J = 0.9Hz), 4.16(2H,q,J = 7.2Hz), 7.41(1H,d,J = 0.9Hz), 7.51(1H,d,J = 8.1Hz), 7.60(1H,d,J = 8.1Hz)

### Process 3

### (6-Mercapto-7-methyl benzo [b] thiophene-3-yl) acetic acid methyl ester (R⁵ = Me)

A mixture of (6-dimethyl carbamoyl sulfanil-7-methyl benzo [b] thiophene-3-yl) ethyl acetate ester (2.34 g) and 1M sodium methoxide solution (methyl alcohol solution, 14.9 ml) was refluxed for 2.5 hours. The reaction solution was neutralized with 2N hydrochloric acid. The solution was extracted with ethyl acetate. The organic layer was washed with brine and dried over magnesium sulfate. The solvent was evaporated under reduced pressure to give a title compound (1.65 g).
NMR (CDCl₃): δ 2.57(3H,s), 3.30(1H,s), 3.69(3H,s), 3.82(2H,s), 7.28(1H,s), 7.34(1H,d,J = 8.4Hz), 7.46(1H,d,J = 8.4Hz).

### Reference 19

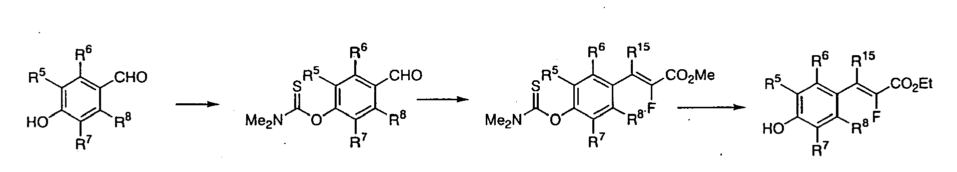

### Process 1

### 4-Dimethyl thiocarbamoyloxy-3-fluoro benzaldehyde (R⁵ = F, R⁶ = R⁷ = R⁸ = R¹⁵ = H)

A mixture of 3-fluoro-4-hydroxy acetophenone (7.5 g), N,N-dimethylthiocarbamoyl chloride (7.84 g), triethylamine (6.50 g), N,N-dimethyl amino pyridine (0.65 g) and 1,4-dioxane (80 ml) was stirred at 110 °C for 4 hours. After cooling to room temperature, 2N hydrochloric acid was added. The mixture was extracted with ethyl acetate. The organic layer was washed with water and brine and dried over magnesium sulfate. The solvent was evaporated under reduced pressure. The resulting residue was washed with a mixed solvent of isopropyl ether and n-hexane to give a title compound (11.6 g).
NMR (CDCl₃): δ 3.39(3H,s), 3.47(3H,s), 7.30-7.35(1H,m), 7.67-7.73(2H,m), 9:96(1H, s).

### Process 2

### 3-(4-Dimethyl thiocarbamoyloxy-3-fluoro phenyl)-2-fluoro acrylic acid ethyl ester (R⁵ = F, R⁶ = R⁷ = R⁸ = R¹⁵ = H)

A mixture of 4-dimethyl carbamoyloxy-3-fluoro benzaldehyde (1.5 g), triethyl-2-fluoro-2-phosphonoacetate 1.68 g, lithium chloride (0.34 mg), 1,8-diazabicyclo[5.4.0]undec-7-ene (1.11 g) and N,N-dimethyl formamide (15 ml) was stirred at room temperature under ice cooling for 19 hours. To the reaction solution was added water and the mixture was extracted with ethyl acetate. The organic layer was washed with water and brine and dried over magnesium sulfate. The solvent was evaporated under reduced pressure. The residue was subjected to silica gel column chromatography eluting with ethyl acetate: n-hexane (1: 3) to give a title compound (1.84 g).
NMR (CDCl₃): δ 1.28(3H,t,J = 7.2Hz), 3.37(3H,s), 3.46(3H,s),4.27(2H, d,J = 7.2Hz), 6.85(1H,d,J = 7.2Hz), 6.85(1H,d,J = 21.6Hz), 7.07-7.13(1H,m), 7.21-7.24(1H,m), 7.42(1H,dd,J = 2.1Hz,11.4Hz).

### Process 3

### (Z)-3-(3-Fluoro-4-hydroxy phenyl)-2-fluoro acrylic acid ethyl ester (R⁵ = F, R⁶ = R⁷ = R⁸ = R¹⁵ = H)

A mixture of 3-(4-dimethyl thiocarbamoyl oxy-3-fluoro phenyl) acrylic acid ethyl ester (1.0 g) and 1M sodium methoxide solution (methyl alcohol solution, 6.5 ml) was stirred at 100 °C for 4.5 hours. After addition of 2N hydrochloric acid, the mixture was extracted with ethyl acetate. The organic layer was washed with water and brine and dried over magnesium sulfate. The solvent was evaporated under reduced pressure. The residue was subjected to silica gel column chromatography eluting with ethyl acetate: n-hexane (1: 1) to give a title compound (1.18 g).

### Reference 20

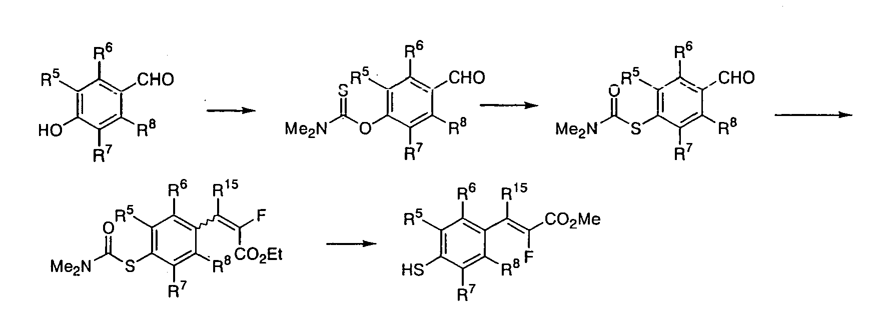

### Process 1

### 4-Dimethyl thiocarbamoyloxy benzaldehyde (R⁵ = R⁶ = R⁷ = R⁸ = R¹⁵ = H)

A mixture of 4-hydroxy benzaldehyde (25 g), N,N-dimethyl thiocarbamoyl chloride (30 g), triethylamine (24.9 g), N,N-dimethyl amino pyridine (4.5 g) and 1,4-dioxane (300 ml) was stirred at 110 °C for 3 hours. The mixture was cooled to room temperature and 2N hydrochloric acid and water were added thereto. The mixture was extracted with ethyl acetate. The organic layer was washed with water and brine and dried over magnesium sulfate. After removal of the solvent under reduced pressure, the resulting residue was washed with a mixed solvent of isopropyl ether and ethyl acetate to give a title compound (35.2 g).
NMR (CDCl₃): δ 3.37(3H,s), 3.47(3H,s), 7.24(2H,d,J = 8.7Hz), 7.93(2H,d,J = 8.7Hz), 10.00(1H,s).

### Process 2

### 4-dimethyl carbamoyl sulfanilbenzaldehyde (R⁵ = R⁶ = R⁷ = R⁸ = R¹⁵ = H)

A mixture of 4-dimethyl thiocarbamoyl oxy benzaldehyde (35.2 g) and biphenyl ether (350 ml) was stirred at 270 °C for 45 minutes. The reaction solution was subjected to silica gel column chromatography eluting with n-hexane and ethyl acetate: n-hexane (1 1) to give a title compound (32.9 g).
NMR (CDCl₃): δ 3.07(6H,br), 7.67(2H,d,J = 8.1Hz), 7.87(2H,d,J = 8.1Hz),10.03(1H,s).

### Process 3

### (E)-3-(4-Dimethyl carbamoyl sulfanilphenyl)-2-fluoro acrylic acid ethyl ester (R⁵ = R⁶ = R⁷=R⁸=R¹⁵=H)

A mixture of 4-dimethyl carbamoyl sulfanilbenzaldehyde (209 mg), triethyl 2-ffuoro-2-phosphonoacetate (254 mg), lithium chloride (51 mg), 1,8-diazabicyclo[5.4.0]undec-7-ene (167 mg) and N,N-dimethyl formamide (2 ml) was stirred under ice cooling for 1.5 hours. After addition of water, the mixture was extracted with diethyl ether. The organic layer was washed with water and brine and dried over magnesium sulfate. The solvent was evaporated under reduced pressure to give a title compound (297 mg).
NMR (CDCl₃): δ 1.25(3H,t,J = 7.2Hz), 3.04(6H,br), 4.25(2H,q,J = 7.2Hz), 6.89(1H,d,J = 21.6Hz), 7.47(4H,s).

### Process 4

### (Z)-2-Fluoro-3-(4-mercapto phenyl) acrylic acid methyl ester (R⁵ = R⁶ = R⁷ = R⁸ = R¹⁵ = H)

A mixture of (E)-3-(4-dimethoxycarbamoyl sulfanilphenyl)-2-fluoro acrylic acid ethyl ester (297 mg) and 1M sodium methoxide solution (methyl alcohol solution, (2.1 ml) was stirred for 5.5 hours. The mixture was poured to ice water and extracted with ethyl acetate. The organic layer was washed with water and brine and dried over magnesium sulfate. The solvent was evaporated under reduced pressure to give a title compound (212 mg).
NMR (CDCl₃): δ 3.89(3H,s), 3.76(1H,s), 6.86(1H,d,J = 34.8Hz), 7.27(2H,d,J = 8.4Hz), 7.50(2H,d,J = 8.4Hz).

### Reference 21

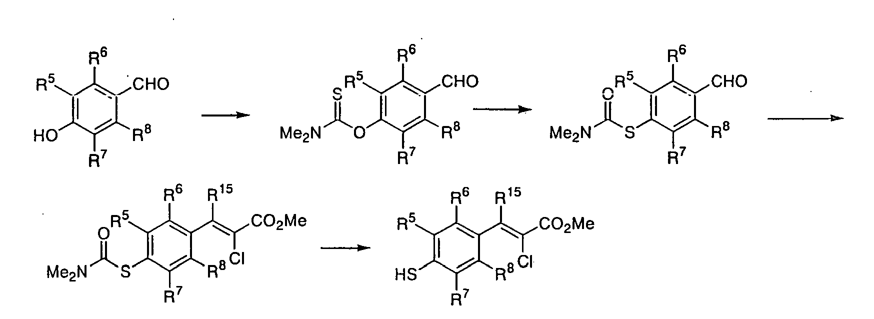

### Process 1

### 4-Dimethyl thiocarbamoyloxy-3-methoxybenzaldehyde (R⁵ = OMe, R⁶ = R⁷ = R⁸ = R¹⁵ = H)

A mixture of vanillin (50.0 g), N,N-dimethyl thiocarbamoyl chloride (48.7 g), triethylamine (39.9 mg), N,N-dimethyl amino pyridine (4.0 g) and 1,4-dioxane (250 ml) was stirred for 3 hours. After addition of water, the mixture was extracted with ethyl acetate. The organic layer was washed with water and brine and dried over magnesium sulfate. The solvent was evaporated under reduced pressure. The obtained residue was washed with isopropyl ether to give a title compound (68.0 g).
NMR (CDCl₃): δ 3.38(3H,s), 3.47(3H,s), 3.90(3H,s); 7.21-7.26(1H,m), 7.48-7.52(2H,m), 9.95(1H,s).

### Process 2

### 4-Dimethyl carbamoyl sulfanil-3-methoxybenzaldehyde (R⁵ = OMe, R⁶ = R⁷ = R⁸ = R¹⁵ = H)

A mixture of 4-dimethyl thiocarbamoyloxy-3-methoxybenzaldehyde (61.6 g) and biphenylether (300 ml) was stirred at 270 °C for 1 hour. The mixture was cooled to room temperature and a resulting crystal was filtrated to obtain a title compound 46.2 g.
NMR (CDCl₃): δ 3.09(6H,br), 3.95(3H,s), 7.44(1H,s), 7.47(1H,d,J = 1.8Hz), 7.69(1H,d,J =7.8Hz), 9.99(1H,s).

### Process 3

### (Z)-2-Chloro-3-(4-dimethyl carbamoyl sulfanil-3-methoxyphenyl) acrylic acid methyl ester (R⁵ = OMe, R⁶ = R⁷ = R⁸ = R¹⁵ = H)

To a mixture of chromium dichloride (5.00 g) and tetrahydrofuran (70 ml), was added a mixture of 4-dimethyl carbamoyl sulfanil-3-methoxybenzaldehyde (2.16 g), trichloro methyl acetate (1.61 g) and tetrahydrofuran (35 ml) at room temperature. The mixture was stirred at room temperature for 25 minutes. After addition of ice-water, the mixture was extracted with ethyl acetate. The organic layer was washed with water and brine and dried over magnesium sulfate. The solvent was evaporated under reduced pressure. The residue was subjected to silica gel column chromatography eluting with toluene: ethyl acetate (4: 1). The obtained crude product was recrystallized from a mixed solvent of ethyl acetate - n-hexane to give a title compound (2.36 g).
NMR (CDCl₃): δ 3.08(6H,br), 3.91(6H,s), 7.37-7.41(1H,m), 7.49(1H,d,J = 1.5Hz), 7.53(1H,d,J = 8.1Hz),7.90(1H,s).

### Process 4

### (Z)-2-Chloro-3-(4-mercapto-3-methoxyphenyl) acrylic acid methyl ester (R⁵ = OMe, R⁶ = R⁷ = R⁸ = R¹⁵ = H)

A mixture of (Z)-2-chloro-3-(4-dimethyl carbamoyl sulfanil-3-methoxyphenyl) acrylic acid methyl ester (2.21 g) and 1 M sodium methoxide (13.4 ml) was refluxed for 6 hours. After ice cooling, 2N hydrochloric acid was added to the reaction solution. The mixture was extracted with ethyl acetate. The organic layer was washed with water and brine and dried over magnesium sulfate. The solvent was evaporated under reduced pressure to give a title compound (1.09 g).
NMR (CDCl₃): δ 3.90(3H,s), 7.29(1H,s), 7.30(1H,d,J = 1.5Hz), 7.45(1H,d,J = 1.5Hz), 7.85(1H,s).

### Reference 22

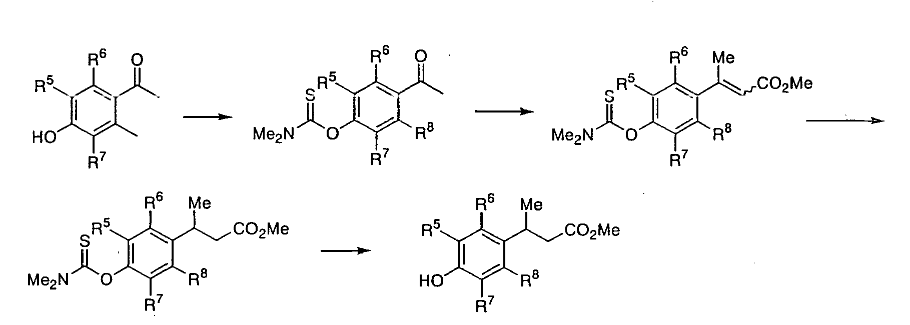

### Process 1

### 4-Dimethyl thiocarbamoyloxy-3-methoxyacetophenone (R⁵ = OMe, R⁶ = R⁷ = R⁸ = H)

A mixture of acetovanillone (15.11 g), N,N-dimethyl thiocarbamoyl chloride (12.8 g), N,N-dimethyl amino pyridine (1.1 g), triethylamine (13 ml) and 1,4-dioxane (100 ml) was refluxed for 1.5 hours. To the reaction solution was added water and the mixture was extracted with ethyl acetate. The organic layer was washed with water and brine and dried over magnesium sulfate. The solvent was evaporated under reduced pressure. The obtained residue was recrystallized from a mixed solvent of ethyl acetate - n-hexane to give a title compound (20.2 g).
NMR (CDCl₃): δ 2.61(3H,s), 3.37(3H,s), 3.47(3H,s), 3.89(3H,s), 7.13(1H,d,J = 8.1Hz), 7.57-7.61(2H,m).

### Process 2

### 3-(4-Dimethyl thiocarbamoyl oxy-3-methoxyphenyl) crotonic acid methyl ester (R⁵ = OMe, R⁶ = R⁷ = R⁸ = H)

To a mixture of dimethyl phosphonomethyl acetate (17.4 g) and tetrahydrofuran (100 ml), was added potassium t-butoxide (11.3 g) at -78 °C. The mixture was stirred at room temperature for 40 minutes and 4-dimethyl thiocarbamoyl oxy-3-methoxyacetophenone (20.2 g) was added thereto. The mixture was stirred at room temperature for 16 hours. To the reaction solution was added ethyl acetate 500 ml. The mixture was washed successively with 1N hydrochloric acid, water and brine and dried over magnesium sulfate. The solvent was evaporated under reduced pressure and the obtained residue was washed with isopropyl ether to give a title compound (16.6 g).

### Process 3

### 3-(4-Dimethyl thiocarbamoyl oxy-3-methoxyphenyl) butyric acid methyl ester (R⁵ = OMe, R⁶ = R⁷ = R⁸ = H)

To a mixture of 3-(4-dimethyl thiocarbamoyl oxy-3-methoxyphenyl) crotonic acid methyl ester (16.6 g) and methyl alcohol (100 ml) was added magnesium (5.23 g). The mixture was stirred at room temperature for 1.5 hours. The reaction solution was poured to a mixture of ethyl acetate (400 ml) and 1N hydrochloric acid (400 ml) and the organic layer was separated. The organic layer was washed with water and brine and dried over magnesium sulfate. The solvent was evaporated under reduced pressure. The obtained residue was subjected to silica gel column chromatography eluting with ethyl acetate: n-hexane (1: 1) to give a title compound (11.6 g).
NMR (CDCl₃): δ 1.32(3H,d,J = 6.9Hz), 2.49(2H,m), 3.22-3.34(1H,m), 3:34(3H,s), 3.45(3H,s), 3.64(3H,s), 3.82(3H,s), 6.81(2H,m), 6.96(1H,d,J = 8.7Hz).

### Process 4

### 3-(4-Dydroxy-3-methoxyphenyl) butyric acid methyl ester (R⁵ = OMe, R⁶ = R⁷ = R⁸ = H)

A mixture of 3-(4-dimethyl thiocarbamoyloxy-3-methoxyphenyl) butyric acid methyl ester (3.1 g) and 1M sodium methoxide solution (methyl alcohol solution, 23 ml) was refluxed for 2.5 hours. The reaction solution was poured into a mixture of ethyl acetate 100 ml and 2N hydrochloric acid and the organic layer was separated. The organic layer was washed with water and brine and dried over magnesium sulfate. The solvent was evaporated under reduced pressure to give a title compound (2.10 g).
NMR (CDCl₃): δ 1.27(3H,d,J = 6.9Hz), 2.47-2.63(2H,m), 3.18-3.27(1H,m), 3.63(3H,s), 3.88(3H,s), 6.69-6.73(2H,m), 6.84(1H,d,J = 8.7Hz).

### Reference 23

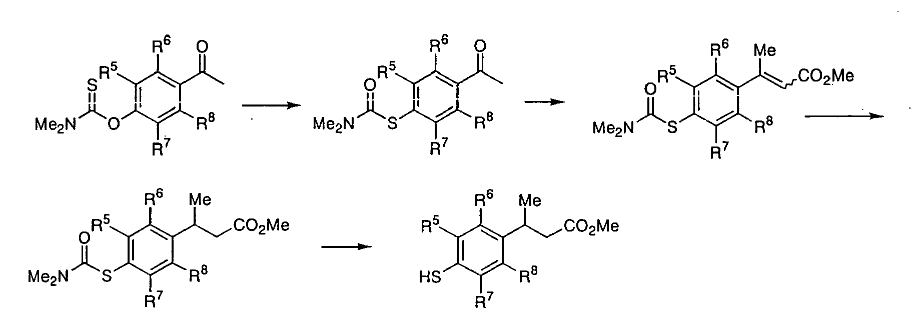

### Process 1

### 4-Dimethyl carbamoyl sulfanil-3-methoxyacetophenone (R⁵ = OMe, R⁶ = R⁷ = R⁸ = H)

A mixture of 4-dimethyl thiocarbamoyloxy-3-methoxyacetophenone (21.7 g) and biphenylether (100 ml) was stirred at 270 °C for 1 hour. The mixture was cooled to room temperature. To the reaction solution, was added n-hexane. A crystal deposited was filtrated to obtain a title compound (18.9 g).
NMR (CDCl₃): δ 2.61(3H,s), 3.08(6H,br), 3.94(3H,s), 7.51-7.61(3H,m).

### Process 2

### 3-(4-Dimethyl carbamoyl sulfanil-3-methoxyphenyl) crotonic acid methyl ester (R⁵ = OMe, R⁶ =R⁷ =R⁸ =H)

To a mixture of dimethyl phosphonomethyl acetate (16.3 g) and tetrahydrofuran (200 ml), was added potassium t-butoxide (10.6 g) at -78 °C. The mixture was stirred at room temperature for 30 minutes and 4-dimethyl thiocarbamoyl oxy-3-methoxyacetophenone (18.9 g) was added thereto. The mixture was stirred at room temperature for 2 hours. To the reaction solution were added saturated ammonium acetate water solution and water. The mixture was extracted with ethyl acetate. The organic layer was washed with water and brine and dried over magnesium sulfate. The solvent was evaporated under reduced pressure. The obtained residue was recrystallized from a mixed solvent of ethyl acetate - n-hexane to give a title compound (15.6 g).

### Process 3

### 3-(4-Dimethyl carbamoyl sulfanil-3-methoxyphenyl) butyric acid methyl ester (R⁵ = OMe, R⁶ = R⁷ = R⁸ = H)

To a mixture of 3-(4-dimethyl carbamoyl sulfanil-3-methoxyphenyl) crotonic acid methyl ester (22.3 g) and methyl alcohol (200 ml) was added magnesium (4.56 g). The mixture was stirred at room temperature for 2 hours. The reaction solution was poured into a mixture of water 200 ml and 2N hydrochloric acid 250 ml, and the mixture was extracted with ethyl acetate. The organic layer was washed with water and brine and dried over magnesium sulfate. The solvent was evaporated under reduced pressure. The obtained residue was recrystallized from a mixed solvent of n-hexane - isopropyl ether to give a title compound (15.0) g.
NMR (CDCl₃): δ 1.30(3H,d,J = 6.9Hz), 2.50-2.68(2H,m), 3.06(6H,br), 3.24-3.33(1H,m), 3.65(3H,s), 3.87(3H,s), 6.81-6.85(2H,m), 7.38(1H,d,J = 7.8Hz).

### Process 4

### 3-(4-Mercapto-3-methoxyphenyl) butyric acid methyl ester (R⁵ = OMe, R⁶ = R⁷ = R⁸ = H)

A mixture of 3-(4-dimethyl thiocarbamoyloxy-3-methoxyphenyl) butyric acid methyl ester (5.0 g), 1M sodium methoxide (34 ml) was refluxed for 2 hours. The reaction solution was poured into a mixture of 2N hydrochloric acid (100 ml) and water (100 ml) and the mixture was extracted with ether. The organic layer was washed with water and brine and dried over magnesium sulfate. The solvent was evaporated under reduced pressure to give a title compound (3.65 g).
NMR (CDCl₃): δ 1.28(3H,s), 2.28-2.64(2H,m), 3.20-3.27(1H,m), 3.63(3H,s), 3.89(3H,s), 6.71-6.74(2H,m), 7.18(1H,d,J = 8.4Hz).

3-(2-Dluoro-4-mercapto phenyl) butyric acid methyl ester (R⁶ = F, R⁵ = R⁷ = R⁸ = H) and 3-(2-Methyl-4-mercapto phenyl) butyric acid methyl ester (R⁶ = Me, R⁵ = R⁷ = R⁸ = H) were obtained as well as the above.
3-(2-Fluoro-4-mercapto phenyl) butyric acid methyl ester
NMR (CDCl3): δ 1.28(3H,d,J = 7.2Hz), 2.52-2.69(2H,m), 3.47(1H,s), 3.43-3.55(1H,m), 3.63(3H,s), 6.94-7.10(3H,m).
3-(2-Methyl-4-mercapto phenyl) butyric acid methyl ester
NMR (CDCl3): δ 1.22(3H,d,J = 6.9Hz), 2.32(3H,s), 2.46-2.61(2H,m), 3.35(1H,s), 3.41-3.53(1H,s), 3.62(3H,s), 7.02-7.11(3H,m)

### Reference 24

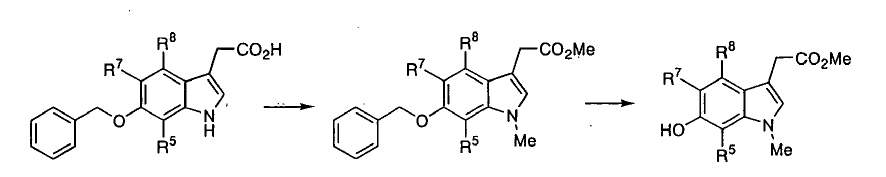

### Process 1

### [6-Benzyloxy-1-methyl-1H-indole-3-yl] acetic acid methyl ester (R⁵ = R⁷ = R⁸ = H)

To a mixture of [6-benzyloxy-1H-indole-3-yl] acetic acid (4.00 g) and N,N-dimethyl formamide (60 ml), was added sodium hydride (60 %) 1.71 g at 0 °C. The mixture was stirred at the same tempareture for 30 minutes. Methyl iodide (6.05 g) was added thereto and the mixture was stirred at 60 °C for 3 hours. To the reaction solution were added ice water and aqueous ammonium acetate. The mixture was extracted with ethyl acetate. The organic layer was washed with water and brine and dried over magnesium sulfate. The solvent was evaporated under reduced pressure. The residue was subjected to silica gel column chromatography eluting with ethyl acetate: n-hexane (1: 6) to give a title compound (1.65 g).
NMR (CDCl₃): δ 3.68(3H,s), 3.69(3H,s), 3.73(2H,s), 5.13(2H,s), 6.83-6.92(3H,m), 7.32-7.49(6H,m).

### Process 2

### [6-Hydroxy-1-methyl-1H-indole-3-yl] acetic acid methyl ester (R⁵ = R⁷ = R⁸ = H)

A mixture of 6-benzyloxy-1-methyl-1H-indole-3-yl] acetic acid methyl ester (1.65 g), 10 % Pd-C (330 mg) and tetrahydrofuran (41 ml) was stirred in hydrogen atmosphere for 1 hour. The insoluble residue was filtrated and the filtrate was concentrated under reduced pressure. The residue was subjected to silica gel column chromatography eluting with ethyl acetate: n-hexane (1: 2) to give a title compound (615 mg).
NMR (CDCl₃): δ 3.61(3H,s), 3.70(3H,s), 3.72(2H,s), 6.66-6.71(2H,m), 6.88(1H,s), 7.19(1H,d,J = 8.4Hz).

### Reference 25

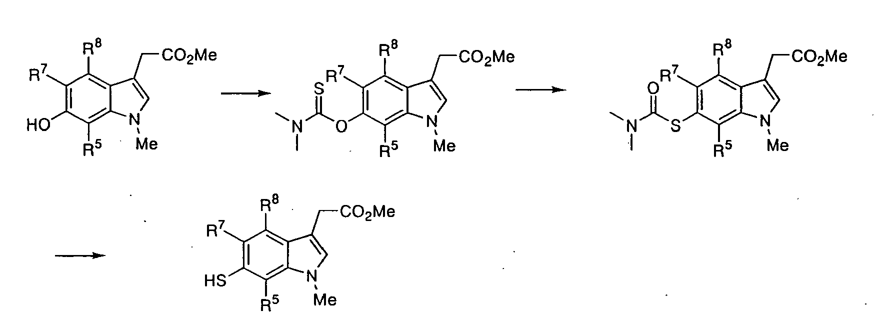

### Process 1

### (6-Dimethyl thiocarbamoyl oxy-1-methyl-1H-indole-3-yl) acetic acid methyl ester (R⁵ = R⁷ = R⁸ = H)

A mixture of (6-hydroxy-1-methyl-1H-indole-3-yl) acetic acid methyl ester (600 mg), N,N-dimethyl thiocarbamoyl chloride (372 mg), N,N-dimethyl amino pyridine (33 mg), triethylamine (763 mg) and dioxane (6 ml) was refluxed for 6 hours. To the reaction solution was added ice-water and the mixture was extracted with ethyl acetate. The organic layer was washed with water and brine and dried over magnesium sulfate. The solvent was evaporated under reduced pressure. The residue was subjected to silica gel column chromatography eluting with ethyl acetate: n-hexane (1: 2) to give a title compound (724 mg).
NMR (CDCl₃): δ 3.38(3H,s), 3.48(3H,s), 3.69(3H,s), 3.72(3H,s), 3.74(2H,s), 6.83(1H,dd,J = 1.5,8.4Hz), 7.00(1H,d,J =1.5Hz), 7.04(1H,s), 7.56(1H,s,J = 8.4Hz).

### Process 2

### (6-Dimethyl carbamoyl sulfanil-1-methyl-1H-indole-3-yl) acetic acid methyl ester (R⁵ = R⁷ = R⁸ = H)

A mixture of (6-dimethyl thiocarbamoyloxy-1-methyl-1H-indole-3-yl) acetic acid methyl ester (724 mg) and biphenylether (3.6 ml) was stirred at 270 °C for 7 hours. The reaction solution was cooled to room temperature and subjected to silica gel column chromatography eluting with ethyl acetate: hexane (1: 3) to give a compound (493 mg).
NMR (CDCl₃): δ 3.07(6H,br), 3.68(3H,s), 3.74(3H,s), 3.75(2H,s), 7.08(1H,s), 7.21(1H,dd,J = 1,5Hz,8.1Hz), 7.47-7.48(1H,m), 7.58(1H,d,J = 8.4Hz).

### Process 3

### (6-Mercapto-1-methyl-1H-indole-3-yl) acetic acid methyl ester (R⁵ = R⁷ = R⁸ = H)

A mixture of (6-dimethyl carbamoyl sulfanil-1-methyl-1H-indole-3-yl) acetic acid methyl ester (493 mg), 1M sodium methoxide (3.4 ml) and methyl alcohol (5 ml) was refluxed for 4 hours. To the reaction solution was added 2N hydrochloric acid and the mixture was extracted with ethyl acetate. The organic layer was washed with water and brine and dried over magnesium sulfate. The solvent was evaporated under reduced pressure to give a title compound (383 mg).

### Reference 26

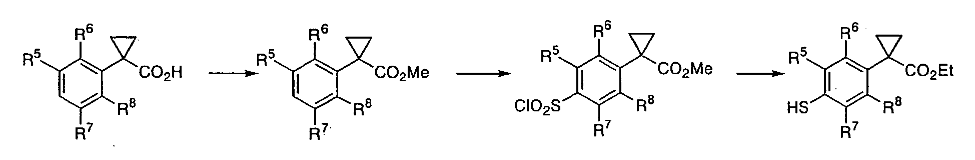

### Process 1

### 1-Phenyl-1-cyclopropanecarboxylate methyl ester (R⁵ = R⁶ = R⁷= R⁸ = H)

A mixture of 1-phenyl-l-cyclopropane carboxylic acid (8.55 g), methyl alcohol (160 ml) and strong sulfuric acid (4 ml) was refluxed for 2 hours. The reaction solution was concentrated under reduced pressure and water (100 ml) was added thereto. The mixture was extracted with ethyl acetate. The organic layer was washed with water and brine and dried over magnesium sulfate. The solvent was evaporated under reduced pressure to give a title compound (9.16 g).
NMR (CDCl₃): δ 1.16-1.20(2H,m), 1.58-1.61(2H,m), 3.60(3H,s), 7.22-7.35(5H,m).

### Process 2

### 1-(4-Chlorosulfonylphenyl)-1-cyclopropanecarboxylate methyl ester (R⁵ = R⁶ = R⁷ = R⁸ = H)

1-phenyl-1-cyclopropanecarboxylate methyl ester (2.00 g) was added to chlorosulfuric acid (3.0 ml) under ice cooling. The mixture was stirred at room temperature for 3 hours and the reaction solution was poured into ice-water. The resulting crystal was filtrated to give a title compound (631 mg).
NMR (CDCl₃): δ 1.16-1.21(2H,m), 1.45-1.50(2H,m), 3.54(3H,s), 7.25-7.28(2H,m), 7.50-7.53(2H,m).

### Process 3

### 1-(4-Mercapto phenyl)-1-cyclopropanecarboxylate methyl ester (R⁵ = R⁶ = R⁷ = R⁸ = H)

A mixture of 1-(4-chlorosulfonylphenyl)-1-cyclopropanecarboxylate methyl ester (300 mg), tin (powder, 683 mg), 4N hydrochloric acid (1,4-dioxane solution, 1.43 ml) and methyl alcohol (1.5 ml) was refluxed for 1.5 hours. The insoluble residue was filtrated, and water was added to the filtrate. The mixture was extracted with ethyl acetate. The organic layer washed with aqueous sodium hydrogen carbonate and brine and dried over magnesium sulfate. The solvent was evaporated under reduced pressure to give a title compound (219 mg).
NMR (CDCl₃): δ 1.11-1.19(2H,m), 1.56-1.60(2H,m), 3.61(3H,s), 4.10(2H,q,J = 6.9Hz), 7.20(4H,s).

### Example 1

### (Method α-1)

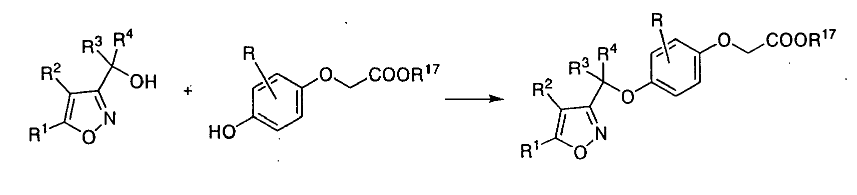

### {2-Methyl-4-[5-(4-trifluoromethylphenyl)-isoxazole-3-ylmethoxy]-phenoxy}-acetic acid methyl ester (R¹ = TFMP, R² = R³ = R⁴ = H, R = 2-Me, R¹⁷ = Me, α-1-1)

To the mixture of [5-(4-trifluoromethylphenyl)-isoxazole-3-yl] methanol (2-1-1,243 mg), triphenylphosphine (266 mg), 4-(chlorosulfonyl-phenoxy)-acetic acid methyl ester (176 mg) and tetrahydrofuran (8 ml) was added 1,1'-(azodicarbonyl) dipiperidine (252 mg) under ice cooling and the mixture was stirred at room temperature for 20 hours. Chloroform and water were added to the reaction solution, and the organic layer was separated. After dried over anhydrous magnesium sulphate, the solvent was evaporated under reduced pressure. The obtained residue was subjected to silica gel column chromatography eluting with ethyl acetate: hexane (1: 2) to give a title compound (270 mg, the yield was 64 %.) as a colorless crystal.

This was recrystallized from a mixed solvent of ethyl acetate: hexane to give a crystal whose melting point is 107-109 °C.

### Example 2

### (Method α-2)

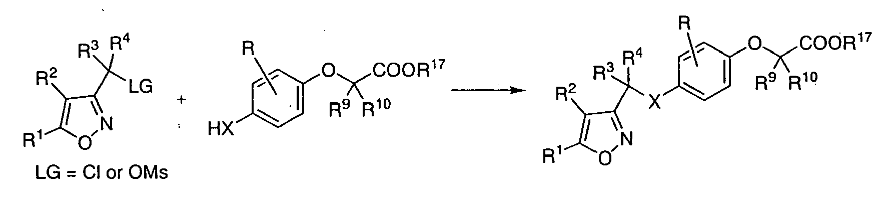

### {2-Methyl-4-[5-(4-trifluoromethylphenyl)-isoxazole-3-yl methylsulfanil]-phenoxy}-acetic acid ethyl ester (R¹ = TFMP, R² = R³ = R⁴ = H, R = 2-Me, R⁹ = R¹⁰ = H, R¹⁷ = Et, α-2-1)

3-chloromethyl-5-(4-trifluoromethylphenyl)-isoxazole (3-1-2-1, 277) mg and (4-mercapto-2-methyl-phenoxy)-acetic acid ethyl ester (255 mg) were dissolved in acetonitrile (5 ml). To the solution was added cesium carbonate (740 mg) and the mixture was stirred at 80 °C for 2 hours. After removing acetonitrile, water was added thereto. The mixture was extracted with chloroform, washed with brine and dried over magnesium sulfate anhydrous. The solvent was evaporated under reduced pressure. The obtained residue was subjected to silica gel column chromatography eluting with ethyl acetate: hexane (1: 6) to give a colorless crystal. This recrystallized from ether - petroleum ether to give a title compound (358 mg) as a colorless crystal. The melting point was 63-64 °C. The yield was 75 %.

### Example 3

### (Method α-3)

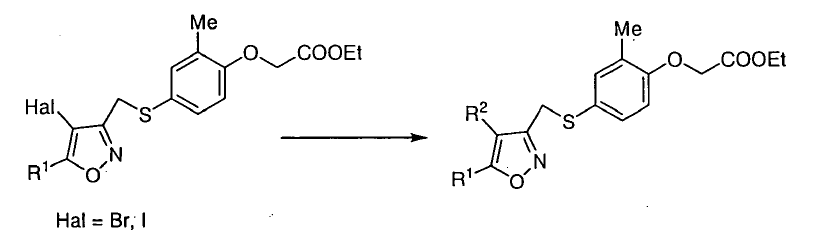

### [2-Methyl-4-[4-(4-trifluoromethylbenzil)-5-(4-trifluoromethylphenyl) isoxazole-3-yl methyl sulfanil] phenoxy] acetic acid ethyl ester (Hal = Br, R¹ = TFMP, R² = 4-trifluoromethylbenzil, α-3-8)

Zinc (111 mg) was suspended in tetrahydrofuran (2 ml). 1,2-Dibromoethane (16 mg) was added and the mixture was stirred for 5 minutes. Chlorotrimethylsilane (9 mg) was added and the mixture was stirred for 5 minutes. To the reaction solution was added p-trifluoromethylbenzilbromide (297 mg) and the mixture was refluxed for 30 minutes. After cooling to room temperature, [4- [4-bromo-5-(4-trifluoromethylphenyl) isoxazole-3-yl methylsulfanil]-2-methylphenoxy] acetic acid ethyl ester (α-2-22, 300 mg), palladium acetate (6 mg) and tricyclohexylphosphine (16 mg) were added thereto and the mixture was refluxed for 45 minutes. After adding water, the mixture was extracted with ethyl acetate, washed with water and brine and dried over magnesium sulfate. The solvent was evaporated under reduced pressure. The residue was subjected to silica gel column chromatography eluting with ethyl acetate: hexane (1: 9) to give a title compound 239 mg as a colorless crystal. The yield was 68 %.

### Example 4

### (Method α-4)

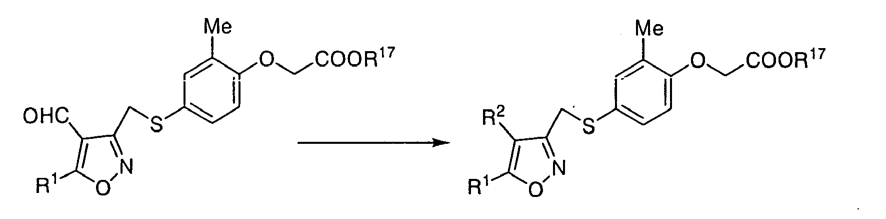

### {4-[4-Butylaminomethyl-5-(4-trifluoromethylphenyl)-isoxazole-3-yl methyl sulfanil]-2-methyl- phenoxy}-acetic acid tert-butyl ester (R¹ = TFMP, R² = CH₂NHnBu, R¹⁷ = tBu, α-4-1)

Compound (α-2-16, 238 mg) and n-butylamine (43 mg) were dissolved in methanol (6 ml) and the solution was stirred at room temperature for 26 hours. Sodium borohydride (36 mg) was added, and the mixture was stirred for 1 hour. After addition of water, the mixture was extracted with chloroform, washed with brine and dried over magnesium sulfate anhydrous. The solvent was evaporated under reduced pressure. The resulting residue was subjected to alumina chromatography eluting with ethyl acetate: hexane (1: 6) to give a title compound (225 mg) as colorless oil. The yield was 85 %.

### {2-Methyl-4-[4-morpholine-4-ylmethyl-5-(4-trifluoromethylphenyl)-isoxazole-3-yl methylsulfanil]-phenoxy}-acetic acid ethyl ester (α-4-2) was obtained as well as the above.

### Example 5

### (Method α-5)

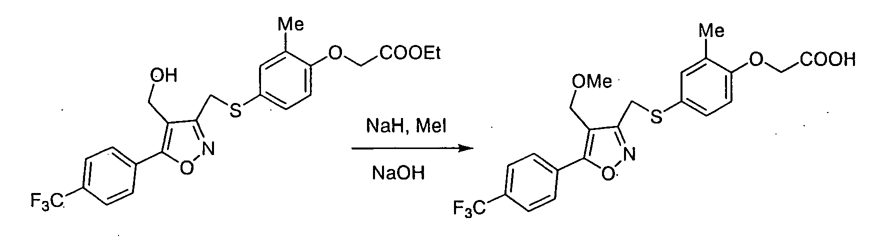

### {4-[4-Methoxymethyl-5-(4-triffuoromethylphenyl)-isoxazole-3-ylmethoxy]-2-methyl-ph enoxy} -acetic acid (α-5-1)

To {4-[4-hydroxymethyl-5-(4-trifluoromethyl phenyl)-isoxazole-3-ylmethoxy] -2-methyl-phenoxy}-acetic acid ethyl ester (α-2-11, 210 mg) in tetrahydrofuran (3 ml) was added sodium hydride (19 mg). The mixture was stirred at room temperature for 30 minutes. To the reaction solution was added a solution of methyl iodide (90 mg) in tetrahydrofuran (0.5 ml). The mixture was stirred for 16 hours. Under ice-cooling, 1M sodium hydroxide solution (1.5 ml) was added, and the mixture was stirred at room temperature for 5 hours. To the reaction solution were added ice and dilute hydrochloric acid to neutralize. The mixture was extracted with ethyl acetate. The organic layer was washed with brine and dried over magnesium sulfate anhydrous. The solvent was evaporated under reduced pressure. The residue was subjected to silica gel column chromatography eluting with ethyl acetate: hexane (2: 1) to give a title compound (175 mg) as a colorless crystal. The yield was 86 %. These crystals were recrystallized from a mixed solvent of ethyl acetate - isopropyl ether to give a crystal.

### Example 6

### (Method α-6)

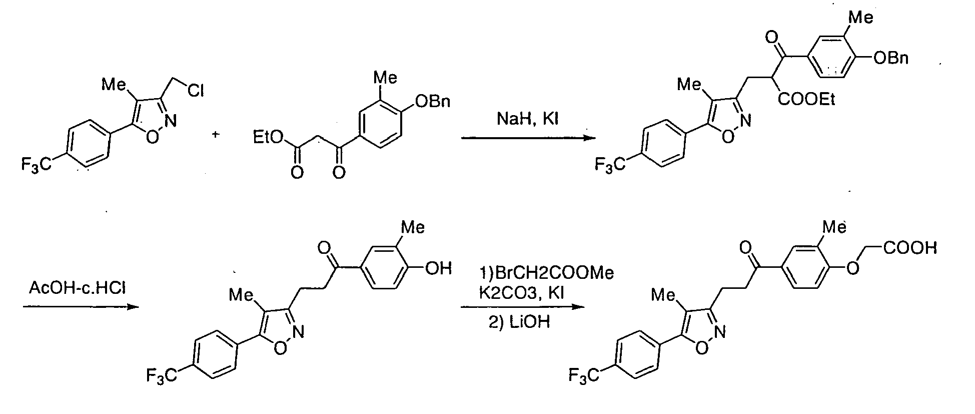

### Process 1 Alkylate

### (3-(4-Benziloxy-3-methyl-phenyl)-2-[4-methyl-5-(4-trifluoromethylphenyl)-isoxazole-3-ylmethyl]-3-oxo-propionic acid ethyl ester (α-6-1-1)

Under ice cooling, to tetrahydrofuran (7ml) was added sodium hydride (48 mg) and added dropwise 3-(4-benziloxy-3-methyl-phenyl)-3-oxo-propionic acid ethyl ester (375 mg) in tetrahydrofuran solution (6 ml) for 15 minutes. After returning to room temperature, 3-chloromethyl-3-methyl-5-(4-trifluoromethylphenyl)-isoxazole (3-1-2-2, 276 mg) and potassium iodide (187 mg) were added, and the mixture was refluxed under heating for 17 hours. After cooling, the mixture was extracted with ethyl acetate and dried over magnesium sulfate anhydrous. The solvent was evaporated under reduced pressure. The residue was subjected to silica gel column chromatography and eluted with ethyl acetate: hexane (1: 2) to give a title compound (530 mg) as colorless oil. The yield was 96 %.

### Process 2, Decarboxylation

### 1-(4-Hydroxy-3-methyl-phenyl)-3-[4-methyl-5-(4-trifluoromethylphenyl)-isoxazole-3-yl] -propane 1-on (α-6-2-1)

To ester (α-6-1-1, 530 mg) obtained above were added acetic acid (4 ml) and concentrated hydrochloric acid (1.2 ml). The mixture was refluxed under heating for 6 hours. After cooling, the mixture was poured into ice-cooling water, neutralized with ammonia water and extracted with ethyl acetate. The organic layer was washed with brine and dried over magnesium sulfate anhydrous. The solvent was evaporated under reduced pressure. The residue was subjected to silica gel column chromatography eluting with ethyl acetate: hexane (1: 2) to give a title compound (210 mg) as a colorless crystal. The yield was 58 %. This was recrystallized with a mixed solvent of ethyl acetate - hexane to give a crystal.
¹HNMR(CDCl₃): 2.26 (3H,s), 2.27(3H,s), 3.07(2H,t, J = 7.8Hz), 3.48(2H,t, J = 7.8Hz), 6.81(1H,d, J = 8.4Hz), 7.74-7.85(6H,m).

### Process 3 Alkylate

### (2-Methyl-4-{3-[4-methyl-5-(4-trifluoromethylphenyl)-isoxazole-3-yl]-propionyl}-pheno xy)-acetic acid methyl ester (α-6-3-1)

To a solution of phenolic compound (α-6-2-1, 130 mg) obtained above and dimethyl formamide (3 ml), were added bromo acetic acid methyl ester (55 mg), potassium carbonate (50 mg) and potassium iodide (9 mg). The mixture was stirred at room temperature for 7 hours, poured to ice-cooling water and extracted with chloroform. The organic layer was washed with brine and dried over magnesium sulfate anhydrous. The solvent was evaporated under reduced pressure. The residue was subjected to silica gel column chromatography eluting with ethyl acetate: hexane (1: 2) to give a title compound (140 mg) as a crystal. The yield was 93 %. This was recrystallized with a mixed solvent of ethyl acetate - isopropyl ether to give a crystal.

### Process 4 Hydrolysis

### (2-Methyl-4-{3-[4-methyl-5-(4-trifluoromethylphenyl)-isoxazole-3-yl]-propionyl}-pheno xy)-acetic acid (α-6-4-1)

The above ester (α-6-3-1, 130 mg) was dissolved in tetrahydrofuran (4.5 ml). 1M lithium hydroxide water solution (0.57 ml) was added, and the mixture was stirred at room temperature for 1 hour. Under ice-cooling, the mixture was neutralized with 1M hydrochloric acid. The solvent was concentrated under reduced pressure and the residual solution was diluted with water. A crystal, which was precipitated under ice cooling, was filtrated to give a title compound (110 mg). The yield was 87 %. This was recrystallized with a mixed solvent of ethyl acetate - isopropyl ether to give a crystal.

### Example 7

### (Method α-7)

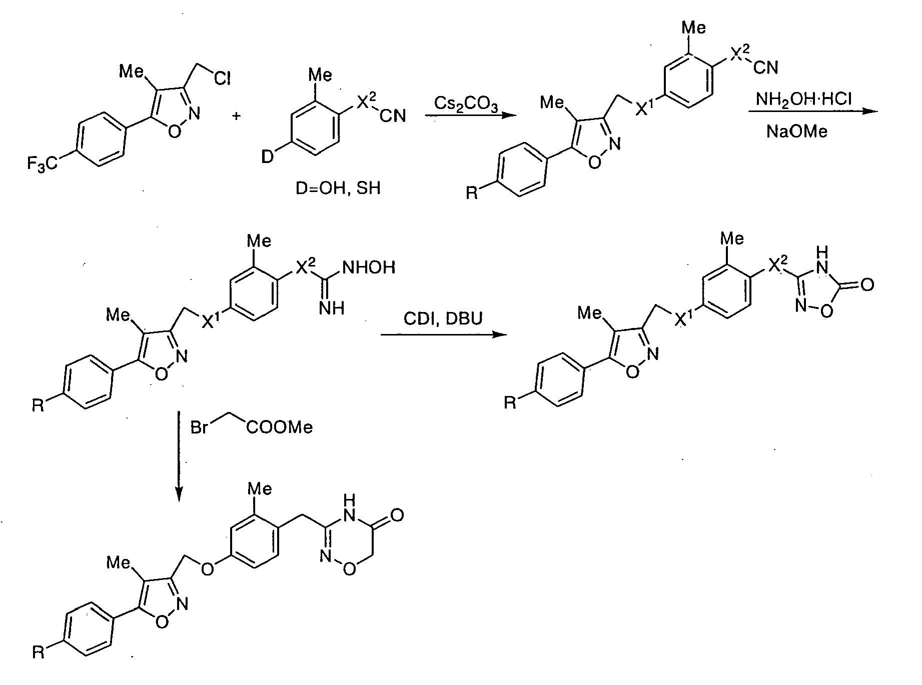

### Process 1

### [2-Methyl-4-[4-methyl-5-(4-trifluoromethylphenyl) isoxazole-3-yl methylsulfanil] phenyl] acetonitrile (R = CF₃, X¹ = S, X² = CH₂, α-7-1-1)

A mixture of 3-chloromethyl-4-methyl-5-(4-triffuoromethylphenyl) isoxazole (3-1-2-3, 225 mg), (4-mercapto-2-methylphenyl) acetonitrile (140 mg), cesium carbonate (585 mg) and acetonitrile (5 ml) was stirred at room temperature for 20 hours. To the reaction solution was added water. The mixture was extracted with ethyl acetate and washed with water and brine. After drying over magnesium sulfate, the solvent was evaporated under reduced pressure. The residue was subjected to silica gel column chromatography eluting with toluene: ethyl acetate (95: 5) to give a title compound (300 mg) as a yellow crystal. The yield was 92 %.
¹H-NMR(CDCl₃): 2.29(3H, s), 2.31(3H, s), 3.63(2H, s), 4.14(2H, s), 7.26-7.28(3H, m), 7.74(2H, d, J = 8.4 Hz), 7.82(2H, d, J = 8.4 Hz)

[2-Methyl-4-[4-methyl-5-(4-trifluoromethylphenyl) isoxazole-3-ylmethoxy] phenyl] acetonitrile (α-7-1-2, X¹ = O) was obtained by the same method. The yield was 88%, Rf = 0.25 (Merck silica gel plate, Developing with ethyl acetate: hexane = 1: 3).

### Process 2

### N-Hydroxy-2-[2-methyl-4-[4-methyl-5-(4-trifluoromethylphenyl) isoxazole-3-yl methylsulfanil] phenyl] acetamidine (α-7-2-1)

A mixture of [2-methyl-4-[4-methyl-5-(4-trifluoromethylphenyl) isoxazole-3-yl methylsulfanil] phenyl] acetonitrile (α-7-1-1, 300 mg), hydroxylamine hydrochloride (259 mg), 28 % sodium methoxide (0.76 ml) and methanol (10 ml) was refluxed for 20 hours. The solvent was evaporated under reduced pressure. Water was added to the residue. The mixture was extracted with ethyl acetate, washed with water and brine and dried over magnesium sulfate. The solvent was evaporated under reduced pressure to give a title compound (299 mg) as a colorless crystal. The yield was 92 %.

N-Hydroxy-2-[2-methyl-4-[4-methyl-5-(4-trifluoromethylphenyl) isoxazole-3-ylmethoxy] phenyl] acetamidine (α-7-2-2, X¹ = O) was obtained by the same method. The yield was 57 %.

### Process 3

### 3-[2-methyl-4-[4-methyl-5-(4-trifluoromethylphenyl) isoxazole-3-yl methylsulfanil] benzil]-4H-[1,2,4] oxadiazole-5-on (α-7-3-1)

A mixture of N-hydroxy-2-[2-methyl-4-[4-methyl-5-(4-trifluoromethylphenyl) isoxazole-3-yl methylsulfanil] phenyl] acetamidine (α-7-2-1, 299 mg), 1,1'-carbonyldiimidazole 123 mg, 1,8-diazabicyclo [5,4,0] undec-7-ene (419 mg) and tetrahydrofuran (10 ml) was stirred at room temperature for 1 hour. To the reaction solution was added water. The mixture was neutralized with 1M hydrochloric acid. The mixture was extracted with ethyl acetate, washed with water and brine and dried over magnesium sulfate. The solvent was evaporated under reduced pressure. The residue was subjected to silica gel column chromatography eluting with toluene: ethyl acetate (95: 5). The obtained crude product was recrystallized from acetone to give a title compound (133 mg) as a colorless crystal. The yield was 42 %.

### Example 8

### (Method α-7)

### 3-{2-Methyl-4-[4-methyl-5-(4-trifluoromethylphenyl)-isoxazole-3-ylmethoxy]-benzil}-4 H-[1,2,4]oxadiazin-5-on(α-7-4-1)

A mixture of N-hydroxy-2-[2-methyl-4-[4-methyl-5-(4-trifluoromethylphenyl) isoxazole-3-yl methanol] phenyl] acetamidine (α-7-2-2, 100 mg), methyl bromoacetate (55 mg), cesium carbonate (155 mg) and dimethyl formamide (3 ml) was stirred at room temperature for 20 hours and at 100 °C for 1 hour. After addition of water, the mixture was extracted with ether, washed with water and brine and dried over magnesium sulfate. The solvent was evaporated under reduced pressure. The residue was subjected to silica gel column chromatography eluting with chloroform: acetonitrile (95: 5) to give a title compound (40 mg) as a yellow crystal. The yield was 37%.

### Example 9

### (Method α-8)

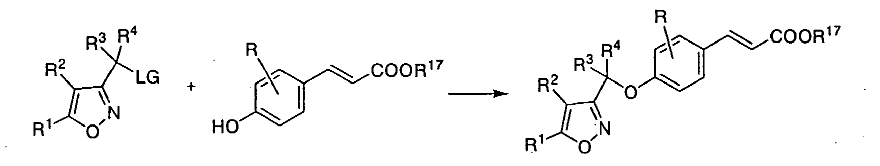

### 3-{2-Methyl-4-[4-methyl-5-(4-trifluoromethylphenyl) isoxazole-3-yl methoxy] phenyl} acryl acid methylester (R¹ = TFMP, R² = Me, R³ = R⁴ = H, R = 2-Me, R¹⁷ = Me, α-8-10)

To the solution of 3-chloromethyl-4-methyl-5-(4-trifluoromethylphenyl)-isoxazole (3-1-2-3, 223 mg) and 3-(4-hydroxy-2-methylphenyl) acryl acid methylester (200 mg) in acetonitrile (8 ml), was added cesium carbonate (316 mg). The mixture was stirred at room temperature for 24 hours and at 60 °C for 3 hours. The reaction solution was filtrated and the filtrate was evaporated under reduced pressure. The obtained residue was subjected to silica gel column chromatography eluting with ethyl acetate: hexane (1: 4) and recrystallized with a mixed solvent of ethyl acetate - hexane to give a title compound (268 mg) as a colorless crystal. The yield was 74 %.

### Example 10

### (Method α-9)

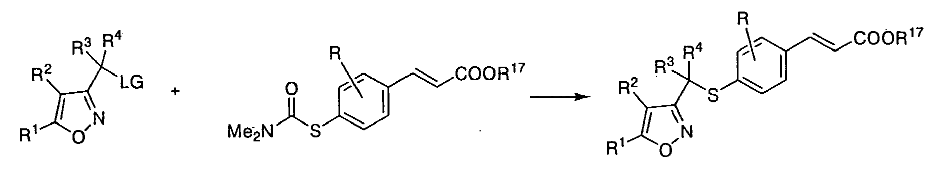

### 3-{3-Methoxy-4-[4-methyl-5-(4-trifluoromethylphenyl) isoxazole-3-yl methylsulfanil] phenyl} acryl acid methylester (R¹ = TFMP, R² = Me, R³ = R⁴ = H, R = 3-OMe, R¹⁷ = Me, α-9-8)

A mixture of 3-(4-dimethylcarbamoyl sulfanil-3-methoxyphenyl) acryl acid methylester (6-1-2, 224 mg) and 1 mol/L sodium methoxide in methanol (1.3 ml) was refluexed for 2 hours and neutralized with 1M hydrochloric acid under ice cooling. The solution was extracted with ethyl acetate. The organic layer was washed with brine and dried over magnesium sulfate anhydrous. The solvent was evaporated under reduced pressure. The obtained residue was dissolved in acetonitrile (4 mL). 3-chloromethyl-4-methyl-5-(4-trifluoromethyl phenyl) isoxazole (3-1-2-3, 209 mg) and cesium carbonate (296 mg) were added thereto and stirred at room temperature for 2 hours. To the reaction solution was added water. The mixture was extracted with ethyl acetate, washed with water and brine and dried over magnesium sulfate. The solvent was evaporated under reduced pressure. The residue was subjected to silica gel column chromatography eluting with chloroform to give a title compound (227 mg) as a colorless crystal. The yield was 65 %.

### Example 11

### (Method α-10)

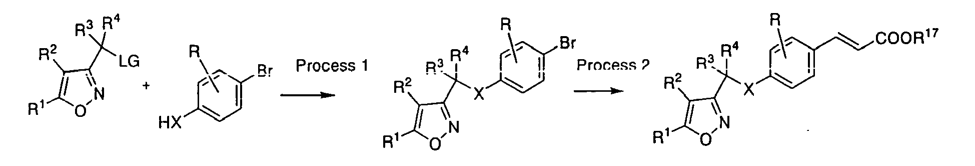

### Process 1 Alkylating

### 3-(4-Bromo-2-fluorophenoxymethyl)-4-methyl-5-(4-trifluoromethylphenyl) isoxazole (R¹ = TFMP, R² = Me, R³ = R⁴ = H, R = 2-F, X = O, α-10-1-1)

A mixture of 3-chloromethyl-4-methyl-5-(trifluoromethylphenyl) isoxazole (3-1-2-3, 1.5 g), 4-bromo-2-fluorophenol (1.25 g), cesium carbonate (2.13 g) and acetonitrile (20 ml) was stirred at 75 °C for 11 hours. To the reaction solution was added water, and the mixture was extracted with ethyl acetate. The organic layer was washed with water and brine and dried over magnesium sulfate. The solvent was evaporated under reduced pressure. The residue was washed with n-hexane to give a title compound (1.82 g) as a crystal. The yield was 78 %.

(α-10-1-2)-(α-10-1-5) were synthesized as well as the above.

**Table 72**

| No. | R | X | NMR |
|---|---|---|---|
| α-10-1-1 | 2-F | O | 2.35(3H,s),5.25(2H,s),7.00-7.30(3H,m),7.76(2H,d,J=8.1Hz), 7.84(2H,d,J=8.1Hz) |
| α-10-1-2 | H | O | 2.28(3H,s),4.12(2H,s),7.25-7.45(4H,m), 7.74(2H,d,J=8.4Hz),7.82(2H,d,J=8.4Hz) |
| α-10-1-3 | 3,5-diF | O | 2.40(3H,s),5.25(2H,s),7.06-7.16(2H,m), 7.76(2H,d,J=8.4Hz),7.86(2H,d,J=8.4Hz) |
| α-10-1-4 | 3-CF₃ | S | 2.29(3H,s),4.17(2H,s),7.51(2H,d,J=8.4Hz). 7.62(1H,dd,J=8.4Hz,2.1Hz),7.74(2H,d,J=8.4Hz), 7.77(1H,d,J=2.1Hz),7.81(2H,d,J=8.4Hz) |
| α-10-1-5 | 2-CF₃ | S | 2.29(3H,s),4.16(2H,s),7.43(1H,dd,J=8.4Hz,2.4Hz), 7.62(1H,d,J=8.4Hz),7.65(1H,d,J=2.4Hz), 7.74(2H,d,J=8.7Hz),7.81(2H,d,J=8.7Hz) |

### Process 2 Heck reaction

### 3-{3-Fluoro-4-[4-methyl-5-(4-trifluoromethylphenyl) isoxazole-3-ylmethoxy] phenyl} acryl acid methylester (R¹ = TFMP, R² = Me, R³ = R⁴ = H, R = 3-F, X = O, R¹⁷ = Me, α-10-2-1)

A mixture of 3-(4-bromo-2-fluorophenoxymethyl)-4-methyl-5-(4-trifluoromethylphenyl) isoxazole (α-10-1-1, 0.35 g), methyl acrylate (1.06 g), palladium acetate (II) (37 mg), triethylamine (0.16 g), triphenylphosphine (86 mg) and dimethyl formamide (2 ml) was stirred in a stream of argon at 100 °C for 11 hours. To the reaction solution was added water and the mixture was extracted with ethyl acetate. The organic layer was washed with water and brine and dried over magnesium sulfate. The solvent was evaporated under reduced pressure. The residue was purified with silica gel column chromatography (n-hexane /ethyl acetate) to give a title compound (0.33 mg) as a crystal. The yield was 92 %.

### Example 12

### (Method α-11)

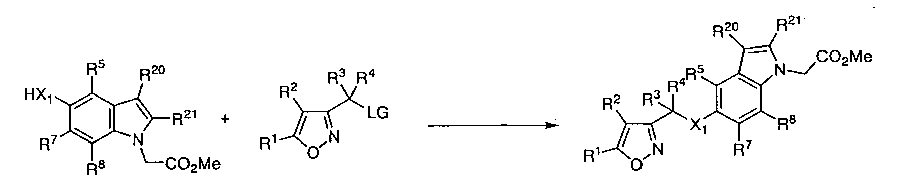

### {5-[4-Methyl-5-(4-trifluoromethylphenyl) isoxazole-3-ylmethoxy] indole-1-yl} acetic acid methyl ester (R¹ = TFMP, R² = Me, R³ = R⁴ = R⁵ = R⁷ = R⁸ = R²⁰ = R²¹ = H, X¹ = O, α-11-1)

To a solution of (5-hydroxyindole-1-yl) acetic acid methyl ester (200 mg) in acetonitrile (5 ml) were added 3-chloromethyl-4-methyl-5-(4-trifluoromethylphenyl)-isoxazole (224 mg) and cesium carbonate (318 mg). The mixture was stirred at room temperature for 15 hours and at 60 °C for 1 hour 30 minutes. The reaction solution was filtrated and the filtrate was evaporated under reduced pressure. The resulting residue was subjected to silica gel column chromatography eluting with ethyl acetate: hexane (1: 4) to give a title compound (243 mg). The yield was 67 %.

### Example 13

### (Method α-12)

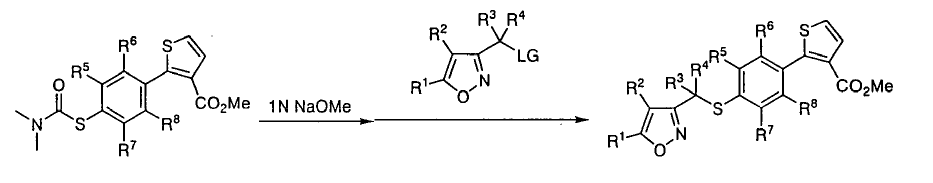

### 2-{4-[4-Methyl-5-(4-trifluoromethylphenyl) isoxazole-3-yl methylsulfanil] phenyl} thiophene-3-carboxylic acid methyl ester (R¹ = TFMP, R² = Me, R³ = R⁴ = R⁵ = R⁶ = R⁷ = R⁸ = H, α-12-1)

To 2-(4-dimethyl carbamoyl sulfanilphenyl) thiophene-3-carboxylic acid methyl ester (321 mg) in methanol (7 ml) was added IN sodium methoxide solution (methanol solution, 1.5 ml) and the mixture was refluxed under heating for 3 hours. After cooling the reaction solution, 2N hydrochloric acid and ice water were added thereto. The mixture was extracted with ethyl acetate. The organic layer was washed with brine and dried over magnesium sulfate. The solvent was evaporated under reduced pressure. To the obtained residue (249 mg) in acetonitrile (5 ml) were added 3-chloromethyl-4-methyl-5-(4-trifluoromethyl phenyl)-isoxazole (228 mg)and cesium carbonate (323 mg), and the mixture was stirred at room temperature for 3 hours. The reaction solution was filtrated and the filtrate was evaporated under reduced pressure. The resulting residue was recrystallized from a mixed solvent of ethyl acetate - hexane to give a title compound (349 mg). The yield was 72 %.

### Example 14

### (Method α-13)

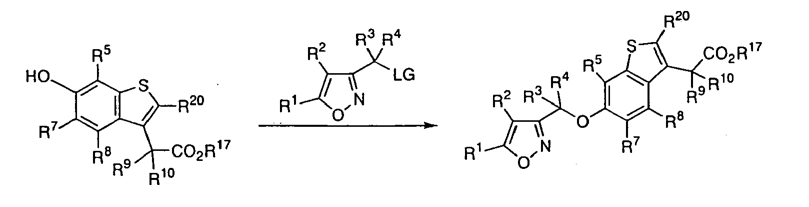

### [6-[4-(Ethoxyiminomethyl)-5-(4-trifluoromethyl phenyl) isoxazole-3-yl methoxy]-7-methyl benzo [b] thiophene-3-yl] acetic acid ethyl ester (R¹ = TFMP, R² = CH=NOEt, R³ = R⁴ = R⁷ = R⁸ = R⁹ = R¹⁰ = R²⁰ = H, R⁵ = Me, R¹⁷ = Et)

A mixture of (6-hydroxy-7-methyl benzo [b] thiophene-3-yl) acetic acid ethyl ester (201 mg), methanesulfonic acid 4-(ethoxyiminomethyl)-5-(4-trifluoromethylphenyl) isoxazole-3-yl methyl ester (314 mg), cesium carbonate (573 mg) and acetonitrile (9 ml) was stirred at room temperature for 10 minutes. The solvent was evaporated under reduced pressure. After addition of water, the mixture was extracted with ethyl acetate. The organic layer was washed with water and brine and dried over magnesium sulfate. The solvent was evaporated under reduced pressure. The obtained residue was subjected to silica gel column chromatography eluting with ethyl acetate: n-hexane (1: 3) to give a title compound (397 mg) . The yield was 91 %.

### Example 15

### (Method α-14)

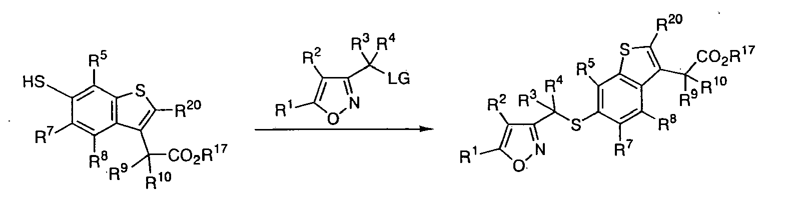

### [6-[4-Ethoxymethyl-5-(4-triffuoromethylphenyl) isoxazole-3-yl methyl sulfamoyl]-7-methyl benzo [b] thiophene-3-yl] acetic acid methyl ester (R¹ = TFMP, R² = CH2OEt, R³ = R⁴ = R⁷ = R⁸ = R⁹ = R¹⁰ = R²⁰ = H, R⁵ = Me, R¹⁷ = Me)

A mixture of 6-mercapto-7-methylbenzo [b] thiophene-3-yl) acetic acid methyl ester (242 mg) 3-chloromethyl-4-ethoxymethyl-5-(4-trifluoromethylphenyl) isoxazole (256 mg), cesium carbonate (573 mg) and acetonitrile (8 ml) was stirred at room temperature for 18 hours. The solvent was evaporated under reduced pressure. To the residue, was added water. The mixture was extracted with ethyl acetate. The organic layer was washed with water and saturated saline solution and dried over magnesium sulfate. The solvent was evaporated under reduced pressure. The obtained residue was subjected to silica gel column chromatography and eluted with ethyl acetate: n-hexane (1: 3) to give a title compound 352 mg.

### Example 16

### (Method α-15)

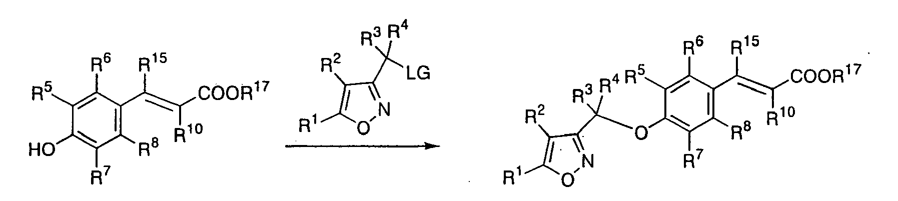

### (Z)-3-[4-[4-ethoxymethyl-5-(4-trifluoromethoxyphenyl) isoxazole-3-yl methoxy]-3-fluoro phenyl]-2-fluoro acryl acid methylester (R¹ = TFMP, R² = CH2OEt, R³ = R⁴ = R⁶ = R⁷= R⁸ = R¹⁵ = H, R⁵ = R¹⁰ = F, R¹⁷ = Me)

A mixture of (Z)-2-fluoro-3-(3-fluoro-4-hydroxyphenyl) acryl acid methylester (300 mg), 3-chloromethyl-4-ethoxymethyl-5-(4-trifluoromethylphenyl) isoxazole (450 mg), cesium carbonate (910 mg) and acetonitrile (20 ml) was stirred at 60°C for 17 hours. After cooling to room temperature, 2N hydrochloric acid was added thereto. The mixture was extracted with ethyl acetate. The organic layer was washed with water and brine and dried over magnesium sulfate. The solvent was evaporated under reduced pressure. The obtained residue was subjected to silica gel column chromatography and eluted with ethyl acetate: n-hexane (1: 5) to give a title compound (240 mg).

### Example 17

### (α-16)

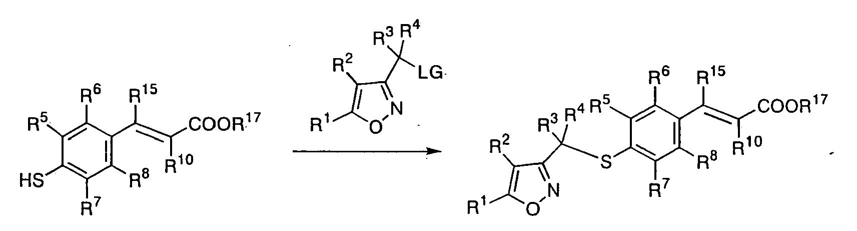

### (Z)-3-[4-[4-Ethoxymethyl-5-(4-trifluoromethylphenyl) isoxazole-3-yl methylsulfanil] phenyl]-2-fluoro acryl acid methylester (R¹ = TFMP, R2 = CH2OEt, R³ = R⁴ = R⁵ = R⁶ = R⁷ = R⁸ = R¹⁵ = H, R¹⁰ = F, R¹⁷ = Me)

A mixture of 3-chloromethyl-4-ethoxymethyl-5-(4-trifluoromethyl phenyl) isoxazole (320 mg), (Z)-2-fluoro-3-(4-mercaptophenyl) acryl acid methylester (212 mg), cesium carbonate (391 mg) and acetonitrile (6 ml) was stirred at room temperature for 2 hours. The insoluble residue was filtrated and the filtrate was concentrated under reduced pressure. To the obtained residue was added water. The mixture was extracted with ethyl acetate. The organic layer was washed with water and saturated saline solution and dried over magnesium sulfate. The solvent was evaporated under reduced pressure. The residue was subjected to silica gel column chromatography eluting with ethyl acetate: n-hexane (1: 6) to give a title compound (216 mg). The yield was 44%.

### Example 18

### (α-17)

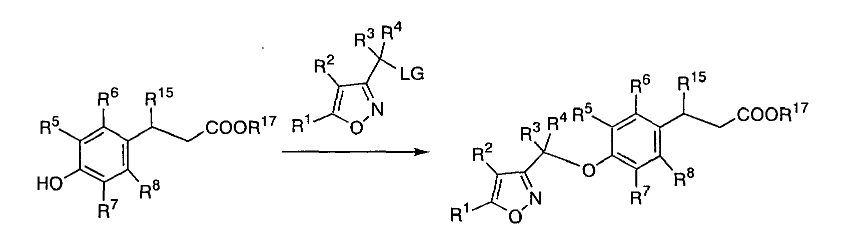

### 3-[4-[4-Ethoxymethyl-5-(4-trifluoromethylphenyl) isoxazole-3-ylmethoxy]-3-methoxyphenyl] butyric acid methyl ester (R¹ = TFMP, R² = CH2OEt, R³ = R⁴ = R⁶ = R⁷ = R⁸ = H, R⁵ = OMe, R¹⁵ = Me, R¹⁷ = Me)

A mixture of 3-(4-hydroxy-3-methoxyphenyl) butyric acid methyl ester (420 mg), 3-chloromethyl-4-ethoxymethyl-5-(4-trifluoromethylphenyl) isoxazole (450 mg), cesium carbonate (1.5 g) and acetonitrile (7 ml) was stirred at 60°C for 3 hours. The reaction solution was added to a mixture of ethyl acetate (100 ml), 2N hydrochloric acid (10 ml) and water (50 ml). The organic layer was separated, washed with water and brine and dried over magnesium sulfate. The solvent was evaporated under reduced pressure. The obtained residue was subjected to silica gel column chromatography eluting with ethyl acetate : n-hexane (1 : 5) to give a title compound 739 mg.

### Example 19

### (α-18)

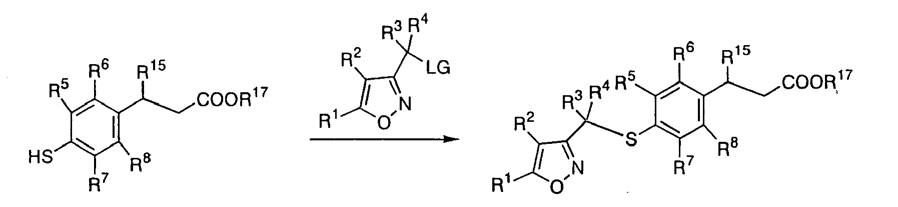

### 3-[4-[4-Ethoxymethyl-5-(4-trifluoromethylphenyl) isoxazole-3-ylsulfanil]-3-methoxyphenyl] butyric acid methyl ester (R¹ = TFMP, R² = CH2OEt, R³ = R⁴ = R⁶ = R⁷ = R⁸ = H, R⁵ = OMe, R¹⁵ = Me, R¹⁷ = Me)

A mixture of 3-(4-mercapto-3-methoxyphenyl) butyric acid methyl ester (300 mg), 3-chloromethyl-4-ethoxymethyl-5-(4-trifluoromethylphenyl) isoxazole (382 mg), cesium carbonate (930 mg) and acetonitrile (6 ml) was stirred at room temperature for 2 hours. The reaction solution was poured to 0.5N hydrochloric acid (60 ml) and water (50 ml) and extracted with ethyl acetate. The organic layer was washed with water and brine and dried over magnesium sulfate. The solvent was evaporated under reduced pressure. The residue was subjected to silica gel column chromatography eluting with ethyl acetate: n-hexane (1: 4) to give a title compound (550 mg).

### Example 20

### (α-19)

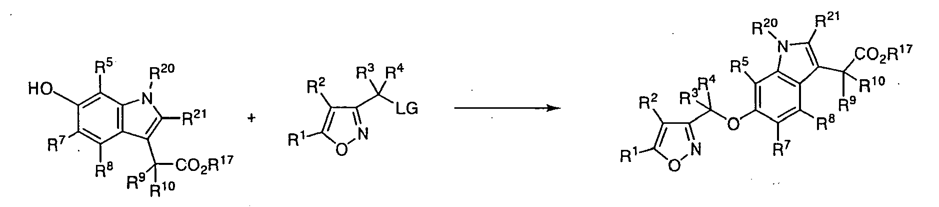

### [6-[4-Ethoxymethyl-5-(4-trifluoromethylphenyl) isoxazole-3-yl methyloxy]-1-methyl-1H-indole -3-yl] acetic acid methyl ester (R¹ = TFMP, R² = CH2OEt, R³ = R⁴ = R⁵ = R⁷ = R⁸ = R⁹ = R¹⁰ = R²¹ = H, R²⁰ = Me, R¹⁷ = Me)

A mixture of [6-hydroxy-1-methyl-1H-indole-3-yl] acetic acid methyl ester (250 mg), 3-chloromethyl-4-ethoxymethyl-5-(4-trifluoromethylphenyl) isoxazole (401 mg), cesium carbonate (742 mg) and acetonitrile (5 ml) was stirred at 60 °C for 5 hours. To the reaction solution was added aqueous ammonium chloride. The mixture was extracted with ethyl acetate. The organic layer was washed with water and brine and dried over magnesium sulfate. The solvent was evaporated under reduced pressure. The residue was subjected to silica gel column chromatography and eluted with ethyl acetate: n-hexane (1: 4) to give a title compound (306 mg).

### Example 21

### (α-20)

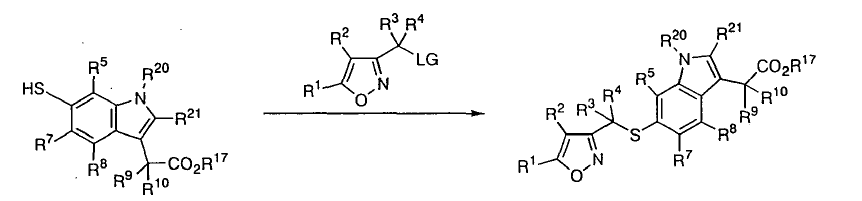

### [6- [4-Ethoxymethyl-5-(4-trifhioromethylphenyl) isoxazole-3-yl methylsulfanil] -1-methyl-1H-indole-3-yl] acetic acid methyl ester (R¹ = TFMP, R² = CH2OEt, R³ = R⁴ = R⁵ = R⁷ = R⁸ = R⁹ = R¹⁰ = R²¹ = H, R²⁰ = Me, R¹⁷ = Me)

A mixture of 6-mercapto-1-methyl-1H-indole-3-yl) acetic acid methyl ester (190 mg), 3-chloromethyl-4-ethoxymethyl-5-(4-trifluoromethylphenyl) isoxazole (284 mg), cesium carbonate (526 mg) and acetonitrile (5 ml) was stirred at room temperature for 26 hours. To the reaction solution was added 2N hydrochloric acid and the mixture was extracted with ethyl acetate. The organic layer was washed with water and brine and dried over magnesium sulfate. The solvent was evaporated under reduced pressure to give a title compound (418 mg).

### Example 22

### (α-21)

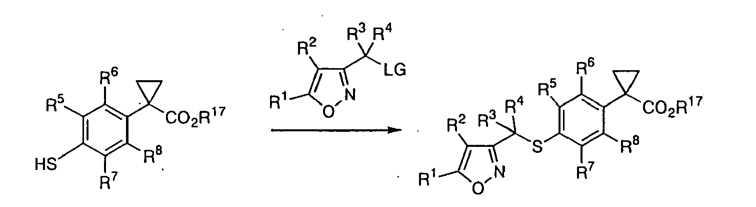

### 1- [4- [4-Ethoxymethyl-5-(4-trifluoromethylphenyl) isoxazole-3-yl methylsulfanil] phenyl] cyclo propane carboxylic acid methyl ester (R¹ = TFMP, R² = CH2OEt, R³ = R⁴ = R⁵ = R⁶ = R⁷ = R⁸ = H, R¹⁷ = Me)

A mixture of 1-(4-mercaptophenyl)-1-cyclo propane carboxylic acid methyl ester (219 mg), 3-chloromethyl-4-ethoxymethyl-5-(4-trifluoromethylphenyl) isoxazole (300 mg), cesium carbonate (716 mg) and acetonitrile (5 ml) was stirred at room temperature for 16 hours. The insoluble residue was filtrated and the filtrate was concentrated under reduced pressure. The residue was subjected to silica gel column chromatography eluting with ethyl acetate: n-hexane (1: 10) to give a title compound (363 mg).

### Example 23

### (Method β-1)

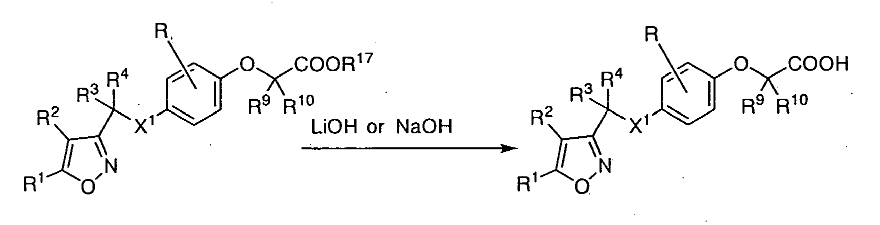

### {2-Methyl-4- [5-(4-trifluoromethylphenyl)-isoxazole-3-yl methylsulfanil]-phenoxy}-acetic acid (R¹ = TFMP, R² = R³ = R⁴ = R⁹= R¹⁰ = H, R = 2-Me, X¹ = S, β-1-2)

{2-Methyl-4-[5-(4-trifluoromethyl phenyl)-isoxazole-3-yl methyl sulfanil]-phenoxy}-acetic acid ethyl ester (α-2-1, 226 mg) was dissolved in tetrahydrofuran (5 ml). 1M lithium hydroxide (1 ml) was added thereto and the mixture was stirred at room temperature for 17 hours. Under ice cooling, 1M hydrochloric acid (1ml) was added. The solution was extracted with ethyl acetate, washed with brine and dried over magnesium sulfate anhydrous. The solvent was evaporated under reduced pressure to give a colorless solid. This was recrystallized from methanol - water to give a title compound (206 mg). The yield was 97 %.

### Example 24

### (Method β-2)

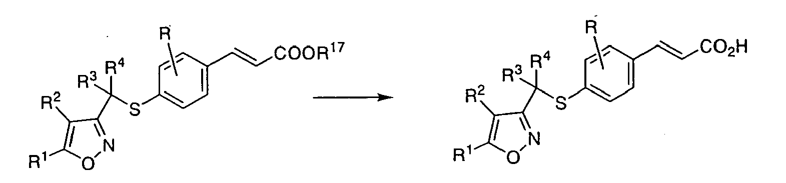

### 3-{3-Fluoro-4-[4-methyl-5-(4-trifluoromethylphenyl) isoxazole-3-ylmethoxy] phenyl} acrylic acid (10) (R¹ = TFMP, R² = Me, R³ = R⁴ = H, R = 3-F, X¹ = O, R¹⁷ = Me, β-2-15)

A mixture of 3-{3-fluoro-4-[4-methyl-5-(4-trifluoromethylphenyl) isoxazole-3-ylmethoxy] phenyl} acryl acid methylester (α-10-2-1, 0.79 g), 4N-LiOH (1.5 ml), water (3 ml) and THF (20 ml) was stirred at 55°C for 4.5 hours. The solvent was evaporated under reduced pressure and acidified with 2N-HCl. Precipitated crystals was washed with water and recrystallized from acetone to give a title compound (0.7 g). The yield was 91%

### (Method β-3)

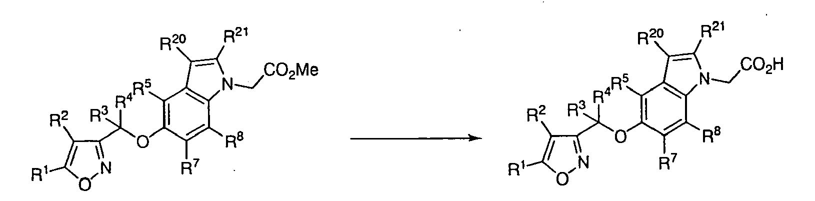

### {5-[4-Methyl-5-(4-trifluoromethylphenyl) isoxazole-3-ylmethoxy] indole-1-yl} acetic acid (R¹ = TFMP, R² = Me, R³ = R⁴ = R⁵ = R⁷ = R⁸ = R²⁰ = R²¹ = H, β-3-1)

To {5-[4-methyl-5-(4-trifluoromethylphenyl) isoxazole-3-ylmethoxy] indole-1-yl} acetic acid methyl ester (242 mg) in tetrahydrofuran (2.5 ml) - methanol (2.5 ml), was added 2N sodium hydroxide solution (0.41 ml) and the mixture was stirred at room temperature for 2 hours. To the reaction solution were added 2N hydrochloric acid (0.5 ml) and water. The mixture was extracted with ethyl acetate. The organic layer was washed with brine and dried over magnesium sulfate. The solvent was evaporated under reduced pressure. The obtained residue was recrystallized by a mixed solvent of acetone - hexane to give a title compound (203 mg). The yield was 87%.

### (Method β-4)

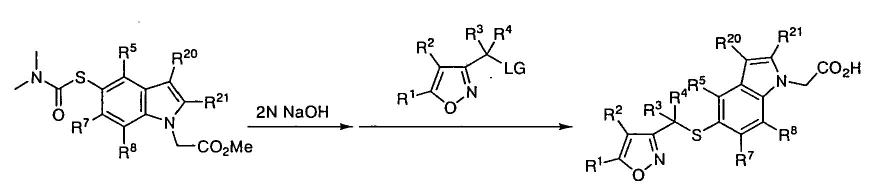

### {5-[4-Methyl-5-(4-trifluoromethylphenyl) isoxazole-3-yl methylsulfanil] indole-1-yl} acetic acid (R¹ = TFMP, R² = Me, R³ = R⁴ = R⁵ = R⁷ = R⁸ = R²⁰ = R²¹ = H, β-4-1)

(5-Dimethyl carbamoyl sulfanilindole-1-yl) acetic acid methyl ester (220 mg) in methanol (5 ml) was added 2N sodium hydroxide solution (3 ml) and the mixture was refluxed under heating for 8 hours. To the reaction solution were added 2N hydrochloric acid and water. The mixture was extracted with ethyl acetate. The organic layer was washed with brine and dried over magnesium sulfate. The solvent was evaporated under reduced pressure. To the resulting residue (177 mg) in acetonitrile (5 ml) were added 3-chloromethyl-4-methyl-5-(4-trifluoromethylphenyl)-isoxazole (207mg) and cesium carbonate (290 mg). The mixture was stirred at 60 °C for 1 hour 30 minutes. To the reaction solution were added 2N hydrochloric acid and water. The mixture was extracted with ethyl acetate. The organic layer was washed with brine and dried over magnesium sulfate. The solvent was evaporated under reduced pressure. The obtained residue was subjected to silica gel column chromatography eluting with chloroform: methanol (20: 1) and recrystallized from a mixed solvent of acetone - hexane to give a title compound (50 mg). The yield was 15 %.

### (Method β-5)

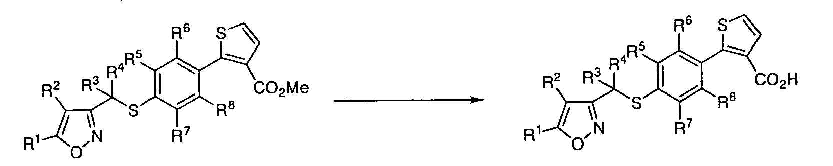

### 2-{4-[4-Methyl-5-(4-trifluoromethylphenyl) isoxazole-3-yl methylsulfanil] phenyl} thiophene-3-carboxylic acid (R¹ = TFMP, R² = Me, R³ = R⁴ = R⁵ = R⁶ = R⁷ = R⁸ = H, β-5-1)

2-{4-[4-Methyl-5-(4-trifluoromethylphenyl) isoxazole-3-yl methylsulfanil] phenyl} thiophene-3-carboxylic acid methyl ester (347 mg) in tetrahydrofuran (7 ml) - methanol (3.5 ml) was added 2N sodium hydroxide solution (0.43 ml) at room temperature and the mixture was stirred for 2 hours. To the reaction solution was added 2N sodium hydroxide solution (0.1 ml) and the mixture was stirred at 60 °C for 1 hour 30 minutes. After cooling, 2N hydrochloric acid (1.5 ml) and water (20 ml) were added to the reaction mixture. Precipitated crystals were filtrated, washed with water and dried. The obtained crude crystals were recrystallized from a mixed solvent of acetone - hexane to give a title compound (289 mg). The yield was 86 %.

### Example 25

### (Method β-6)

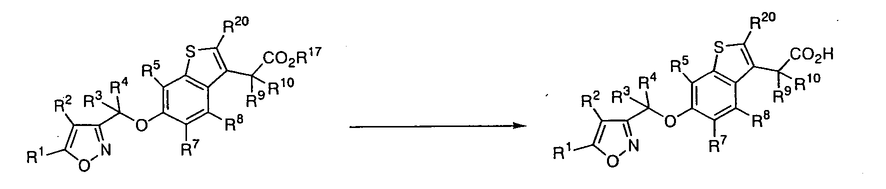

### [6-[4-(Ethoxyiminomethyl)-5-(4-trifluoromethylphenyl) isoxazole-3-ylmethoxy]-7-methylbenzo [b] thiophene-3-yl] acetic acid (R¹ = TFMP, R² = CH = NOEt, R³ = R⁴ = R⁷ = R⁸ = R⁹ = R¹⁰ = R²⁰ = H, R⁵ = Me)

A mixture of [6-[4-(ethoxyiminomethyl)-5-(4-trifluoromethylphenyl) isoxazole-3-yl methoxy]-7-methylbenzo [b] thiophene-3-yl] acetic acid ethyl ester (R¹⁷ = Et, 393 mg), 4N lithium hydroxide (0.4 ml), water (1.2 ml), methanol (4 ml) and tetrahydrofuran (4 ml) was stirred at room temperature for 8 hours. The solvent was evaporated under reduced pressure. To the residue was added 1N hydrochloric acid. After filtrating precipitated crystals, the residue was subjected to silica gel column chromatography eluting with ethyl acetate : n-hexane (3 : 1) to give a title compound (355 mg). The yield was 95 %.

### Example 26

### (β-7)

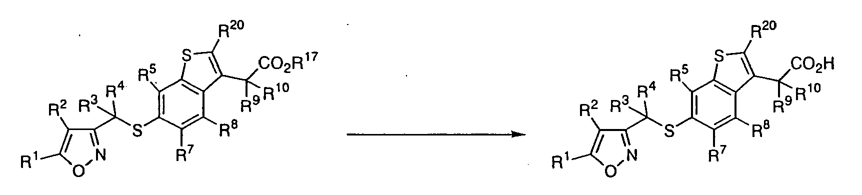

### [6-[4-eEthoxymethyl-5-(4-trifluoromethylphenyl) isoxazole-3-yl methylsulfamoyl]-7-methylbenzo [b] thiophene-3-yl] acetic acid (R¹ = TFMP, R² = CH2OEt, R³ = R⁴ = R⁷ = R⁸ = R⁹ = R¹⁰ = R²⁰ = H, R⁵ = Me)

A mixture of [6-[4-ethoxymethyl-5-(4-trifluoromethylphenyl) isoxazole-3-yl methylsulfamoyl]-7-methylbenzo [b] thiophene-3-yl] acetic acid methyl ester (R¹⁷ = Me, 350 mg), 4N lithium hydroxide (0.33 ml), water (1 ml), methanol (4 ml) and tetrahydrofuran (4 ml) was stirred at room temperature for 1.5 hours. Under ice cooling, 1N hydrochloric acid was added thereto. Precipitated crystals were filtrated. The obtained crystal was recrystallized from a mixed solvent of ethyl acetate and n-hexane to give a title compound (310 mg).

### Example 27

### (Method β-8)

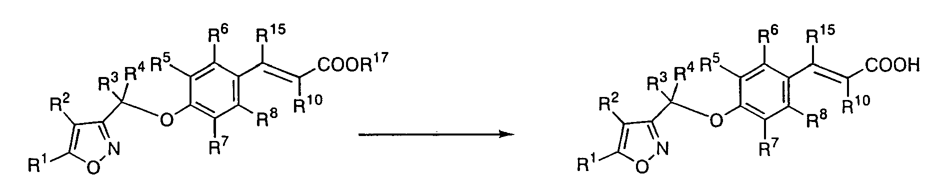

### (Z)-3-[4-[4-Ethoxymethyl-5-(4-trifluoromethoxyphenyl) isoxazole-3-yl methoxy]-3-fluoro phenyl]-2-fluoro acrylic acid (R¹ = TFMP, R² = CH2OEt, R³ = R⁴ = R⁶ = R⁷ = R⁸ = R¹⁵ = H, R⁵ = R¹⁰ = F)

A mixture of (Z)-3-[4-[4-ethoxymethyl-5-(4-trifluoromethoxyphenyl) isoxazole-3-yl methoxy]-3-fluorophenyl]-2-fluoro acryl acid methylester (R¹⁷ = Me, 240 mg), 4N lithium hydroxide (1.4 ml), methanol (2 ml) and tetrahydrofuran 2 ml was stirred at room temperature for 1.5 hours. 2N hydrochloric acid was added thereto and the mixture was extracted with ethyl acetate. The organic layer was washed with water and saturated saline solution and dried over magnesium sulfate. The solvent was evaporated under reduced pressure. The obtained residue was recrystallized from a mixed solvent of ethyl acetate: n-hexane to give a title compound (210 mg).

### Example 28

### (β-9)

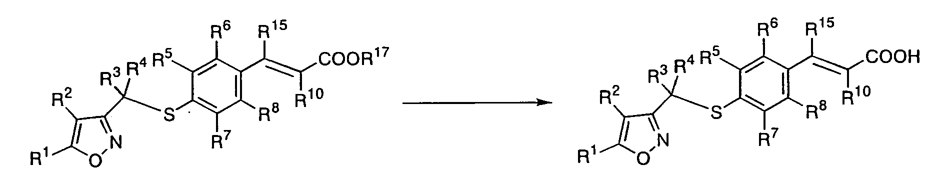

### (Z)-3-[4-[4-Ethoxymethyl-5-(4-trifluoromethylphenyl) isoxazole-3-yl methylsulfanil] phenyl] -2-fluoro acrylic acid (R¹ = TFMP, R² = CH2OEt, R³ = R⁴ = R⁵ = R⁶ = R⁷ = R⁸ = R¹⁵ = H, Rio = F)

A mixture of (Z)-3-[4-[4-ethoxymethyl-5-(4-trifluoromethyl phenyl) isoxazole-3-yl methyl sulfanil] phenyl]-2-fluoro acryl acid methylester (R¹⁷ = Me, 200 mg), 4N lithium hydroxide (0.11 ml), water (0.33 ml), methanol (2 ml) and tetrahydrofuran (3 ml) was stirred at room temperature for 30 minutes. After removal pf the solvent under reduced pressure, water and 1N hydrochloric acid were successively added to the residue. The mixture was extracted with ethyl acetate. The organic layer was washed with water and brine and dried over magnesium sulfate. The solvent was evaporated under reduced pressure. The obtained residue was recrystallized from a mixed solvent of acetone - isopropyl ether to give a title compound (150 mg). The yield was 77 %.

### Example 29

### (β-10).

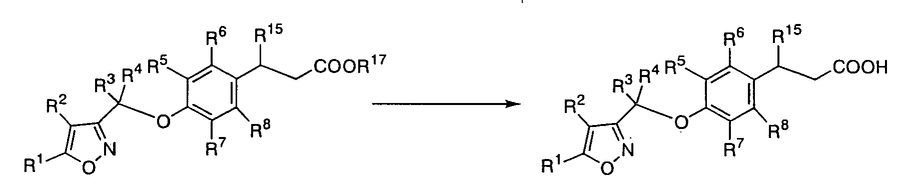

### 3-[4-[4-Ethoxymethyl-5-(4-trifluoromethylphenyl) isoxazole-3-ylmethoxy]-3-methoxy phenyl] butyric acid (R¹ = TFMP, R² = CH2OEt, R³ = R⁴ = R⁶ = R⁷ = R⁸ = H, R⁵ = OMe, R¹⁵ = Me)

A mixture of 3-[4-[4-ethoxymethyl-5-(4-trifluoromethylphenyl) isoxazole-3-yl methoxy] -3-methoxy phenyl] butyric acid methyl ester (R¹⁷ = Me, 739 mg), 4N lithium hydroxide (1ml), tetrahydrofuran (10 ml) and water (5 ml) was stirred at room temperature for 16 hours. To the reaction solution were added water (50 ml) and 2N hydrochloric acid (20 ml). The mixture was extracted with ethyl acetate. The organic layer was washed with water and brine and dried over magnesium sulfate. The solvent was evaporated under reduced pressure. The obtained residue was subjected to silica gel column chromatography eluting with chloroform: methanol (30 : 1) to give a title compound (363 mg).

### Example 30

### (β-11)

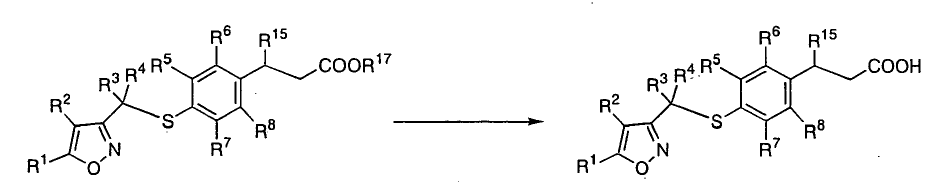

### 3-[4-[4-Ethoxymethyl-5-(4-trifluoromethylphenyl) isoxazole-3-ylsulfanil]-3-methoxy phenyl] butyric acid (R¹ = TFMP, R² = CH2OEt, R³ = R⁴ = R⁶ = R⁷ = R⁸ = H, R⁵ = OMe, R¹⁵=Me)

A mixture of 3-[4-[4-ethoxymethyl-5-(4-trifluoromethylphenyl) isoxazole-3-yl sulfanil]-3-methoxy phenyl] butyric acid methyl ester (R¹⁷ = Me, 550 mg), 4N lithium hydroxide (2.3 ml), tetrahydrofuran (4 ml) and methanol (6 ml) was stirred at room temperature for 3 hours. To the reaction solution were added water (30 ml) and 2N hydrochloric acid (6 ml). The mixture was extracted with ether. The organic layer was washed with water and brine and dried over magnesium sulfate. After removal of the solvent under reduced pressure, the residue was subjected to silica gel column chromatography eluting with ethyl acetate: n-hexane (1: 1). The obtained crude product was recrystallized from a mixed solvent of ethyl acetate - n-hexane to give a title compound (130 mg).

### Example 31

### (β-12)

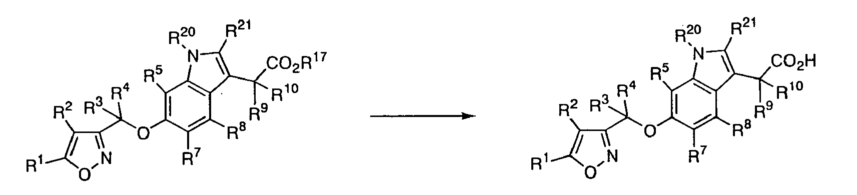

### [6-[4-Ethoxymethyl-5-(4-trifluoromethyl phenyl) isoxazole-3-yl methyl oxy]-1-methyl-1H-indole-3-yl] acetic acid (R¹ = TFMP, R² = CH2OEt, R³ = R⁴ = R⁵ = R⁷ = R⁸ = R⁹ = R¹⁰ = R²¹ = H, R²⁰ = Me)

A mixture of [6-[4-ethoxymethyl-5-(4-trifluoromethyl phenyl) isoxazole-3-yl methyl oxy]-1-methyl-1H-indole-3-yl] acetic acid methyl ester (R¹⁷ = Me, 300 mg), 4N lithium hydroxide (0.3 ml), tetrahydrofuran (6 ml) and methanol (3 ml) was stirred at room temperature for 16 hours. After addition of 2N hydrochloric acid, the mixture was extracted with ethyl acetate. The organic layer was washed with water and brine and dried over magnesium sulfate. The solvent was evaporated under reduced pressure. The residue was subjected to silica gel column chromatography eluting with chloroform: methanol (25: 1). The obtained crude product was recrystallized from ethyl acetate - n-hexane to give a title compound (169 mg).

### Example 32

### (β-13)

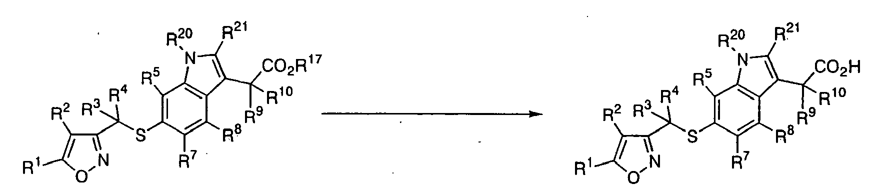

### [6-[4-Ethoxymethyl-5-(4-trifluoromethyl phenyl) isoxazole-3-yl methyl sulfanil]-1-methyl-1H-indole-3-yl] acetic acid (R¹ = TFMP, R² = CH2OEt, R³ = R⁴ = R⁵ = R⁷ = R⁸ = R⁹ = R¹⁰ = R²¹ = H, R²⁰ = Me)

A mixture of [6-[4-ethoxymethyl-5-(4-trifluoromethyl phenyl) isoxazole-3-yl methyl sulfanil]-1-methyl-1H-indole-3-yl] acetic acid methyl ester (R¹⁷ = Me, 437 mg), 4N lithium hydroxide, tetrahydrofuran (9.6 ml) and methanol (4.8 ml) was stirred for 4.5 hours. To the reaction solution was added 2N hydrochloric acid. The mixture was extracted with ethyl acetate. The organic layer was washed with water and brine and dried over magnesium sulfate. The solvent was evaporated under reduced pressure. The residue was subjected to silica gel column chromatography eluting with ethyl acetate: n-hexane (2: 1). The obtained crude product was recrystallized from a mixed solvent of ethyl acetate - n-hexane to give a title compound (217 mg).

### Example 33

### (β-14)

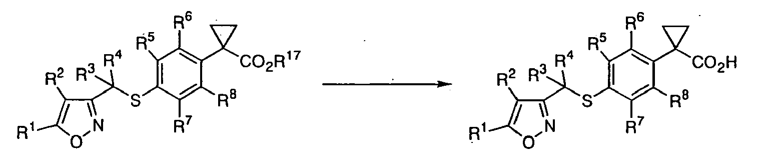

### 1-[4-[4-Ethoxymethyl-5-(4-trifluoromethyl phenyl) isoxazole-3-yl methyl sulfanil] phenyl] cyclo propane carboxylic acid (R¹ = TFMP, R² = CH2OEt, R³ = R⁴ = R⁵ = R⁶ = R⁷ = R⁸ = H).

A mixture of 1-[4-[4-ethoxymethyl-5-(4-trifluoromethyl phenyl) isoxazole-3-yl methyl sulfanil] phenyl] cyclo propane carboxylic acid methyl ester (R¹⁷ = Me, 363 mg), 4N lithium hydroxide water solution (0.42 ml), tetrahydrofuran (5 ml) and methanol (10 ml) was stirred at room temperature for 16 hours. After addition of 2N hydrochloric acid, the mixture was extracted with ethyl acetate. The organic layer was washed with aqueous sodium hydrogencarbonate solution and brine and dried over magnesium sulfate. The solvent was evaporated under reduced pressure to give a title compound (200 mg).

The following compounds synthesized as well as the above were included in the present invention. Additionally, Table 74 continued to Table 75. Table 79 continued to Table 80 - 81. Table 83 continued to Table 84 - 87. Table 88 continued to Table 89 - 93. Table 94 continued to Table 95 - 98. Table 99 continued to Table 100 and 101. Table 102 continued to Table 103 - 105. Table 106 continued to Table 107 and 108. Table 109 continued to Table 110. Table 111 continued to Table 112 - 114. Table 115 continued to Table 116. Table 117 continued to Table 118 - 120. Table 122 continued to Table 123. Table 125 continued to Table 126. Table 127 continued to Table 128 - 131. Table 132 continued to Table 133 - 136. Table 137 continued to Table 138 - 144. Table 145 continued to Table 146 - 152. Table 153 continued to Table 154. Table 155 continued to Table 156. Table 160 continued to Table 161. Table 162 continued to Table 163.

**Table 73**

| | | | | | | | | |
|---|---|---|---|---|---|---|---|---|
| 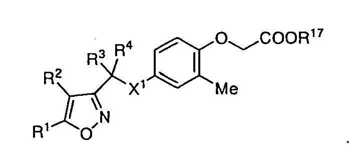 | | | | | | | | |

| No | Synthetic method | R1 | R2 | X1 | R3,R4 | R17 | mp | NMR(CDCl3 or DMSO-d6) |
|---|---|---|---|---|---|---|---|---|
| α-1-2 | α-1 | 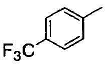 | Me | O | H,H | Me | oil | 2.29(3H,s),2.32(3H,s),3.80(3H,s),4.61(2H,s)5.13(2H,s ),6.67(1H,d,J=9.0Hz),6.79(1H,dd,J=9.0,2.7Hz),6.86(1H,d,J=2.7Hz),7.75(2H,d,J=8.1Hz),7.84(2H,J=8.1Hz) |
| α-1-3 | α-1 | 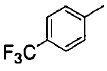 | Me | O | Me,Me | Me | oil | 1.76(6H,s),2.20(3H,s),2.37(3H,s),3.78(3H,s),4.56(2H, s),6.49-6.50(2H,m), 6.67(1H,m),7.75(2H,dJ=8.1Hz),7.84(2H,d,J=8.1Hz) |

**Table 74**

| | | | | | | | | |
|---|---|---|---|---|---|---|---|---|
| 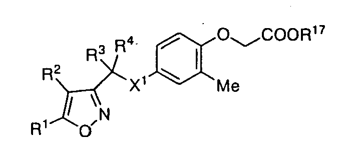 | | | | | | | | |

| No | Synthetic method | R1 | R2 | X1 | R3,R4 | R17 | mp | NMR(CDCl3 or DMSO-d6) |
|---|---|---|---|---|---|---|---|---|
| α-2-2 | α-2 | 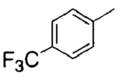 | Me | S | H,H | Et | 63-64 | 1.29(3H,t,J=7.2Hz),2.23(3H,s),2.24(3H,s),4.03( 2H,s),4.25(2H,q,J=7.2Hz),4.61(2H,s)6.61(1H,d, J=8.4Hz),7.18(1H,dd,J=8.4,2.1Hz),7.23(1H,J=2 .1Hz),7.74(2H,d,J=8.1Hz),7.82(2H,d,J=8.1Hz) |
| α-2-4 | α-2 | 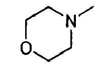 | Me | S | H,H | Et | 58-59 | 1.30(3H,t,J=7.2Hz),1.91(3H,s)2.25(3H,s),3.34( 4H,t,J=4.8Hz),3.79(4H,t,J=4.8Hz),3.87(2H,s),4. 26(2H,q,J=7.2Hz),4.61(2H,s),6.62(1H,d,J=8.4H z),7.71-7.22(2H,m) |

**Table 75**

| No | Synthetic method | R1 | R2 | X1 | R3,R4 | R17 | mp | NMR(CDCl3 or DMSO-d6) |
|---|---|---|---|---|---|---|---|---|
| α-2 -5 | α-2 | 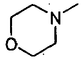 | Me | O | H,H | Me | 112-113 | 1.99(3H,s)2.27(3H,s),3.37(4H,t,J=4.8Hz),3.78-3. 81(4H,m),4.60(2H,s),4.93(2H,s), 6.65(1H,d,J=8.7Hz),6.76(1H,dd,J=8.7,3.0Hz),6.83 (1H,dJ=3.0Hz) |
| α-2 -6 | α-2 | 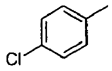 | Me | S | H,H | Et | oil | 1.28(3H,t,J=7.2Hz),2.19(3H,s),2.24(3H,s),4.01(2 H,s),4.25(2H,q,J=7.2Hz),4.61(2H,s)6.61(1H,d,J=8 .7Hz),7.18(1H,dd,J=8.4,2.4Hz),7.22(1H,J=2.4Hz), 7.46(2H,d,J=8.4Hz),7.63(2H,d,J=8.4Hz) |
| α-2 -7 | α-2 | 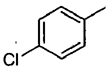 | 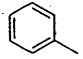 | S | H,H | Et | oil | 1.29(3H,t,J=7.2Hz),2.22(3H,s),3.93(3H,s),4.25(2 H,q,J=7.2Hz),4.61(2H,s)6.58(1H,d,J=9.0Hz),7.12 -7.14(2H,m),7.26-7.32 (5H,m),7.42-7.45(4H,m) |
| α-2 -8 | α-2 | 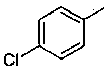 | 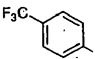 | S | H,H | Et | oil | 129(3H,t,J=7.2Hz),2.21(3H,s),3.93(3H,s),4.25(2 H,q,J=7.2Hz),4.61(2H,s)6.57(1H,d,J=8.1Hz),7.07 -7.12(2H,m),729-7.46(6H,m),7.70(2H,d,J=8.1Hz ) |
| α-2 -9 | α-2 | 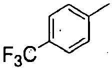 | Me | S | H,Et | Et | oil | 1.07(3H,t,J=7.5Hz),1.28(3H,t,J=7.2Hz), 1.98-217(2H,m),2.21(3H,s),2.26(3H,s), 4.03(1H,dd,J=8.4,7.5Hz),4.24(2H,q,J=7.2Hz),4.60 (2H,s),6.57(1H,d,J=8.1Hz),7.09-7.14(2H,m),7.74( 2H,dJ=8.4Hz),7.81(2H,d,J=8.4Hz) |
| α-2 -10 | α-2 | 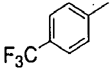 | Me | S | H, 4-F-C6H4 | Et | oil | 1.28(3H,t,J=7.2Hz),2.09(3H,s),2.20(3H,s),4.22(2 H,q,J=7.2Hz),4.60(2H,s),5.28(1H,s),6.55(1H,d,J= 8.4Hz),6.95-7.03(2H,m), 7.06-7.14(2H,m),7.32-7.38(2H,m),7.73 (2H,dJ=8.4Hz),7.80(2H,d,J=8.4Hz) |
| α-2 -11 | α-2 | 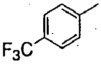 | 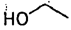 | S | H,H | Et | oil | 1.28(3H,t,J=7.2Hz),2.23(3H,s),4.11(2H,s),4.24(2 H,q,J=7.2Hz),4.61(2H,s),4.66(2H,s),6.60(1H,d,J= 8.4Hz),7.15(1H,dd,J=8.4,2.4Hz),7.22(1H,d,J=2.4 Hz),7.77(2H,d,J=8.1Hz),796(2H,d,J=8.1Hz) |
| α-2 -12 | α-2 | 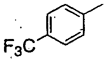 | 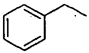 | S | H,H | Et | oil | 1.29(3H,t,J=6.9Hz),2.23(3H,s),3.82(2H,s),4.10(2 H,s),4.25(2H,q,J=6.9Hz),4.61(2H,s),6.60(1H,d,J= 8.4Hz),7.11-7.73(7H,m), 7.68(2H,d,J=8.1Hz),7.76(2H,d,J=8.1Hz) |
| α-2 -13 | α-2 | 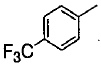 | 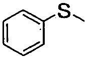 | S | H,H | Et | oil | 1.29(3H,t,J=7.2Hz),223(3H,s),3.96(2H,s),4.25(2 H,q,J=7.2Hz),4.60(2H,s),6.59(1H,d,J=8.1Hz),7.07 -7.28(7H,m),7.70(2H,d, J=9.0Hz),8.22(2H,d,J=9.0Hz) |
| α-2 -14 | α-2 | Me | I | S | H,H | Et | 53-54 | 1.29(3H,t,J=7.2Hz),2.24(3H,s),2.44(3H,s),3.92(2 H,s),4.26(2H,q,J=7.2Hz),4.61 (2H,s),6.61 (1H,d,J= 8.4Hz),7.17(1H,dd,J=8.4,2.4Hz),7.19(1H,d,J=2.4 Hz) |
| α-2 -15 | α-2 | 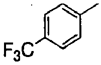 | 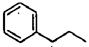 | S | H,H | Et | oil | 1.29(3H,t,J=7.2Hz),2.25(3H,s),2.92-2.99 (4H,m),3.79(2H,s),4.26(2H,q,J=7.2Hz),4.61(2H,s), 6.61(1H,d,J=8.4Hz),7.09-7.26 (7H,m),7.70(4H,s) |
| α-2 -16 | α-3 | 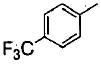 | OHC- | S | H,H | tBu | oil | 1.47(9H,s),2.24(3H,s),4.28(2H,s),4.51(2H,s),6.60(1H,d,J=8.4Hz),7.18-7.24(2H,m), 7.84(2H,d,J=8.7Hz),8.03(2H,d,J=8.7Hz),10.10(1H.d.J=0.6Hz) |

**Table 76**

| | | | | | | | | |
|---|---|---|---|---|---|---|---|---|
| 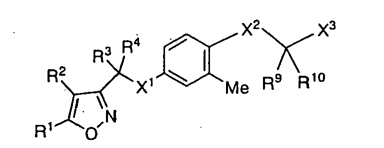 | | | | | | | | |

| No | Synthetic method | R1 | R2 | X1 | R3,R4 | 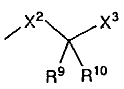 | mp | NMR(CDCl3 or DMSO-d6) |
|---|---|---|---|---|---|---|---|---|
| α-2-17 | α-2 | 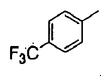 | Me | S | H,H | 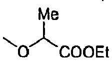 | oil | 1.23(3H,t,J=7.2Hz),1.66(3H,d,J=6.9Hz),2.22(3 H,s),4.02(2H,s),4.20(2H,q,J=7.7Hz),4.71 (1H,q, J=6.9Hz),6.79(2H,d,J=9.0Hz),7.33(2H,d,J=9.0 Hz),7.74(2H,d,J=8.1Hz),7.82(2H,d,J=8.1Hz) |
| α-2-18 | α-2 | 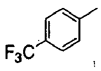 | Me | S | H,H | 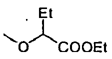 | oil | 1.06(3H,t,J=7.2Hz),1.23(3H,t,J=7.2Hz),1.93-2.02(2H,m),2.22(3H,s),4:03(2H,s),4.16-4.23(2H,m),4.51 (1H,t,J=6.3Hz),6.80(2H,d,J=9. 0Hz),7.32(2H,d,J=9.0Hz),8.13(2H,d,J=8.4Hz),7 .82(2H,d,J=8.4Hz) |
| α-2-19 | α-2 | 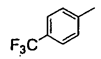 | Me | S | H,H | 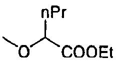 | oil | 0.97(3H,t,J=7.2Hz),1.23(3H,t,J=7.2Hz), 1.48-1.57(2H,m),1.86-1.96(2H,m),2.22(3H,s),4.02(2H,s),4.19(2H,q,J= 7.2Hz),4.54-4.58(1H,m),6.79(2H,d,J=9.0Hz),7.32(2H,d,J=9. 0Hz),7.74(2H,d,J=8.1Hz),7.81(2H,d,J=8.1Hz) |
| α-2-20 | α-2 | 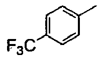 | Me | S | H,nPr | 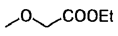 | oil | 0.90(3H,t,J=7.2Hz),1.27(3H,t,J=7.2Hz),1.55-1.62(2H,m),2.22(3H,s),2.59(2H,t,J=7.5Hz),4.02 (2H,s),4.24(2H,q,J=7.2Hz),4.61(2H,s),6.62(1H, d,J=8.1Hz),7.17-7.22(2H,m),7.74(2H,d,J=8.3Hz),7.81(2H,d,J=8. |
| α-2-21 | α-2 | 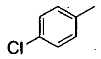 | Br | S | H,H | 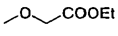 | 55-57 | 1.29(3H,t,J=7.2Hz),2.24(3H,s),4.02(2H,s),4.25( 2H,q,J=7.2Hz),4.61(2H,s),6.61(1H,d,J=8.4Hz), 7.19-7.26(2H,m),7.48(2H,d,J=9.0Hz),7.98(2H,d,J=9. |
| α-2-22 | α-2 | 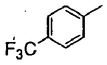 | Br | S | H,H | 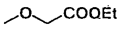 | | 1.30(3H,t,J=7.2Hz),2.25(3H,s),4.04(2H,s),4.25( 2H,q,J=7.2Hz),4.61(2H,s),6.62(1H,d,J=8.4Hz), 7.19-7.23(2H,m),7.77(2H,d,J=9.0Hz),8.16(2H,d,J=9.0Hz) |

**Table 77**

| | | | | | | | | |
|---|---|---|---|---|---|---|---|---|
| 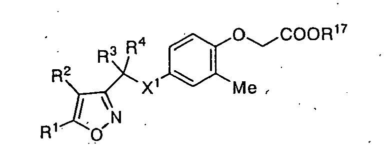 | | | | | | | | |

| No | Synthetic method | R1 | R2 | X1 | R3,R4 | R17 | mp | NMR(CDCl3 or DMSO-d6) |
|---|---|---|---|---|---|---|---|---|
| α-3-1 | α-3 | Me | 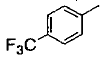 | S | H,H | Et | oil | 1.30(3H,t,J=7.2Hz),2.21(3H,s),2.40(3H,s ),3.98(2H,s),4.26(2H,q,J=7.2Hz),4.61(2H ,s),6.56(1H,d,J=8.4Hz),7.06-7.12(2H,m),7.41(2H,d,J=8.1Hz),7.68(2H, d,J=8.1Hz) |
| α-3-2 | α-3 | Me | 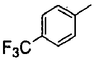 | O | H,H | Me | 105-107 | 2.25(3H,s),2.48(3H,s),3.78(3H,s),4.59(2 H,s),5.01(2H,s),6.61-6.72(3H,m),7.50(2H,d,J=8.4Hz),7.68(2H, d,J=8.4Hz) |
| α-3-3 | α-3 | 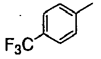 | 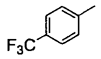 | S | H,H | Et | oil | 1.28(3H,t,J=7.2Hz),2.21(3H,s),3.94(2H,s ),4.25(2H,q,J=7.2Hz),4.61(2H,s),6.57(1H ,d,J=8.4Hz),6.90(1H,d,J=9.0Hz),7.07-7.12(2H,m),7.43(3H,m),7.56(2H,s),7.72(2 H,d,J=8.4Hz) |
| α-3-4 | α-3 | 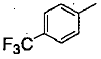 | 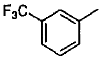 | S | H,H | Et | oil | 1.29(3H,t,J=7.2Hz),2.21(3H,s),3.95(2H,s ),4.25(2H,q,J=7.2Hz),4.61(2H,s),6.58(1H ,d,J=9.0Hz),7.09(2H,m),7.51-7.74(8H,m) |
| α-3-5 | α-3 | 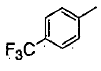 | 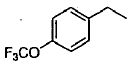 | S | H,H | Et | oil | 1.29(3H,t,J=7.2Hz),2.23(3H,s),3.83(2H,s ),4.12(2H,s),4.25(2H,q),4.61(2H,s),6.59(1H,d,J=8.4Hz),7.09-7.14(6H,m),7.71-7.72(4H,m) |
| α-3-6 | α-3 | 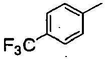 | 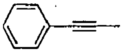 | S | H,H | Et | oil | 1.28(3H,t,J=7.2Hz),2.19(3H,s),4.13(2H,s ),4.24(2H,q,J=7.2Hz).4.56(2H,s),6.58(1H ,d,J=8.4Hz),7.23(3H,m),7.41-7.42(2H,m),7.52-7.55(2H,m),7.77(2H,d,J=9.0Hz), 8.30(2H,d,J=9.0Hz) |
| α-3-7 | α-3 | 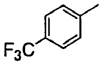 | 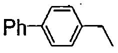 | S | H,H | Et | | Rf=0.34 (EtOAc:Hexane=1:3 Merck silica gel) |
| α-3-8 | α-3 | 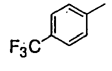 | 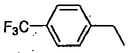 | S | H,H | Et | oil | 1.29(3H, t, J=7.2 Hz), 2.22(3H, s), 3.83(2H, s), 4:15(2H, s), 4.25(2H, q, J=7.2 Hz), 4.61(2H, s), 6.59(1H, d, J=7.8Hz), 7.09-7.12(2H, m), 7.23(2H, d, J=8.1Hz), 7.55(2H, d, J=8.1Hz), 7.71(4H, s) |
| α-3-9 | α-3 | 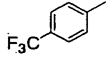 | 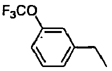 | S | H,H | Et | oil | 1.29(3H,t,J=6.9Hz),2.23(3H,s),3.84(2H,s ),4.15(2H,s),4.25(2H,q,J=7.2Hz),4.61(2H ,s),6.60(1H,d,J=8.1Hz),6.99-7.14(5H,m),7.29-7.35(1H,m),7.70-7.71(4H,m) |
| α-3-10 | α-3 | 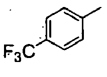 | 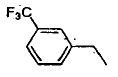 | S | H,H | Et | oil | 1.29(3H,t,J=7.2Hz),2.23(3H,s),3.83(2H,s ),4.14(2H,s),4.25(2H,q,J=7.2Hz),4.61(2H .s),6.60(1H,d,J=8.4Hz),7.09-7.13(2H,m),7.29-7.53(4H,m),7.71(4H,s) |

**Table 78**

| | | | | | | |
|---|---|---|---|---|---|---|
| 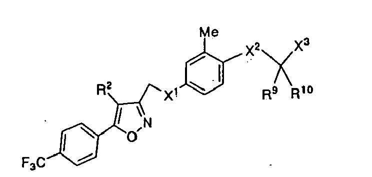 | | | | | | |

| No | Syhthetic method | R2 | X1 | 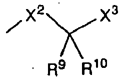 | mp | NMR(CDCl3 or DMSO-d6) |
|---|---|---|---|---|---|---|
| α-4-1 | α-4 | nBuNHCH2- | S | OCH2COOtBu | | 0.93(3h,t,J=7.5Hz),1.33-1.60(13H.m).2.24(3H.s).2.69 (2H,t,J=6.9Hz), 3.73(2H,s),4.12(2H,s),4.50(2H,s), 6.59 (1H,d,J=8.4Hz),7.15(1H,dd,J=8.4,2,1H z), 7.21(1H, d, |
| α-4-2 | α-4 | 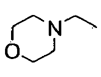 | S | OCH2COOEt | | 1.29(3H,t,J=7.2Hz),2.25(3H,s),2.44(4 H,m),3.54(2H,s),3.68(4H,m), 4.19(2H,q,J=7.2Hz),4.19(2H,s),4.25(2 H, q, J=7.2 Hz),4.61(2H,s),6.61 (1H,d,J=8.4Hz), 7.18(1H, dd, J=8.4,2.1Hz),7.22(1H,m), 7.75(2H,d, |
| α-5-1 | α-5 | -CH2OMe | S | OCH2COOH | 105-107 | 2.24(3H,s), 3.43(3H,s),4.12(2H,s), 4.46(2H,s),4.66 (2H,s), 6.65(1H,d, J=8.5Hz),7.18-7.24(2H,m),7.76(2H, d,J=8.7Hz),7.88(2H,d,J=8.7Hz) |
| α-6-3-1 | α-6 | Me | CH2CO | OCH2COOMe | 133-134 | 2.26(3H,s),2.33(3H,s),3.08(2H,t,J=7.5 Hz),3.50(2H,t,J=7.5Hz),6.72(1H,d,J=9 .0Hz)),7.72-7.87(6H,m). |
| α-6-4-1 | α-6 | Me | CH2CO | OCH2COOH | 191-194 | 2.27(3H,s),2.34(3H,s),3.08(2H,t,J=7.2 Hz),3.50(2H,t,J=7.2Hz),4.72(2H,s),6.7 7(1H,d,J=9.0Hz),7.73-7.88(6H,m). |
| α-7-2-1 | α-7 | Me | S | CH2C(=NH)NHOH | | MS *m*/*e* 452 (MH+) |
| α-7-2-2 | α-7 | Me | O | CH2C(=NH)NHOH | 152-154 | 2.32(6H,s),3.42(2H,s),5.17(2H,s),6.8-6.90(2H,m),7.14(1H,d,J=7.8Hz),7.75(2 H,d,J=8.1Hz),7.84(2H,d,J=8.1Hz) MS m/e 420 (MH+) |
| α-7-3-1 | α-7 | Me | S | 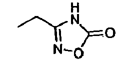 | 203-204.5 | 2.29(3H,s),2.31 (3H,s), 3.83(2H,s),4.06(2H,s),7.11-7.22(3H,m), 7.76(2H,d,J=8.6Hz),7.82 |
| α-7-3-2 | α-7 | Me | O | 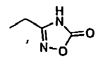 | 190-192 | 2.33(6H,s),3.80(2H,s),5.18(2H,s),6.86( 2H,m),7.15(1H,d,J=8.1Hz),7.77(2H,d,J =8.7Hz),7.87(2H,d,J=8.7Hz) |
| α-7-3-3 | α-7 | Me | S | 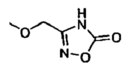 | 156.5-158.5 | 2.18(3H,s),2.28(3H,s),4.01(2H,s),4.97( 2H,s),6.75(1H,d.J=8.4Hz),7.19-7.21(2H,m),7.74(2H,d,J=8.4Hz),7.80(2 H,d,J=8.4Hz),9.93(1H,br) |
| α-7-3-4 | α-7 | Me | O | 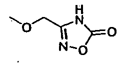 | 163-165 | 2.24(3H,s),2.32(3H,s),4.96(2H,s),5.14( 2H,s),6.80-6.88(3H,m),7.75(2H,d,J=8.6Hz),7.84(2 H,d,J=8.6Hz) |
| α-7-4-1 | α-7 | Me | O | 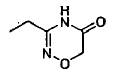 | 166.5-168.5 | 2.32(3H,s), 2.34(3H,s), 3.68(2H,s),4.18(2H,s),5.19(2H,s),6.87 -6.90(2H, m),7.12(1H,d, J=8.1Hz), 7.24 (1H,br),7.75(2H,d,J=8.4Hz), 7.85(2H, d, J=8.4Hz) |

**Table 79**

| | | | | | | | |
|---|---|---|---|---|---|---|---|
| 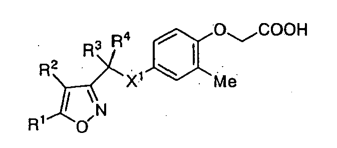 | | | | | | | |

| No | Synthetic method | R1 | R2 | X1 | R3,R4 | mp | NMR(CDCl3 or DMSO-d6) |
|---|---|---|---|---|---|---|---|
| β-1-3 | β-1 | 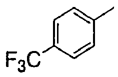 | Me | S | H,H | 129-131 | 2.24(3H,s),2.25(3H,s),4.04(2H,s),4.67(2H ,s),6.65(1H,d,J=8.1Hz),7.18-7.23(2H,m), 7.74(2H,d,J=8.1Hz),7.82(2H,d,J=8.1Hz) |
| β-1-4 | β-1 | 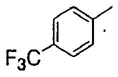 | Me | O | H,H | 136-138 | 2.28(3H,s),2.31(3H,s)4.62(2H,s),5.13(2H, s),6.71(1H,d,J=9.0),6.80(1H,dd,J=9.0,2.7 Hz),6.87(1H,d,J=2.7Hz),7.75(2H,d,J=8.1Hz),7.84(2H,d,J=8.1Hz) |
| β-1-6 | β-1 | 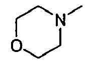 | Me | S | H,H | 134-136 | 1.88(3H,s)2.15(3H,s),3.24-3.27(4H,m),3. 67(4H,t,J=4.8Hz),3.94(2H,s),4.69(2H,s), 6.77(1H,d,J=8.4Hz)7.15-7.21(2H,m),13. 00(1H,brs) |
| β-1-7 | β-1 | 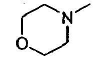 | Me | O | H,H | 126-127 | 1.94(3H,s)2.17(3H,s),3.28-3.32(4H,m),3. 67-3.70(4H,m),4.61 (2H,s),4.90(2H,s),6.7 2-6.86(3H,m)12.89(1H,brs) |
| β-1-8 | β-1 | 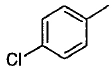 | Me | S | H,H | 157-159 | 2.21(3H,s),2.24(3H,s),4.02(2H,s),4.66(2H ,s),6.65(1H,d,J=8.4Hz),7,20(1H,dd,J=8.4 2.4Hz),7.22(1H,m),746(2H,d,J=9.0Hz),7.63(2H,d,J=9.0Hz) |
| β-1-9 | β-1 | 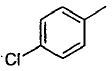 | 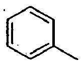 | S | H,H | 131-132 | 2.22(3H,s),3.93(3H,s),4.66(2H,s)6.62(1H, d,J=9.0Hz),7.14-7.16(2H,m),7.27-7.33(5 H,m),7.42-7.45(4H,m) |
| β -1-10 | β-1 | 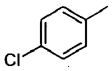 | 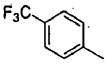 | S | H,H | 131-133 | 2.22(3H,s),3.93(3H,s),4.67(2H,s)6.62(1H, d,J=8.1Hz),7.10-7.14(2H,m),7.30-7.47(6 H,m),7.70(2H,d,J=8.1Hz) |
| β -1-11 | β-1 | 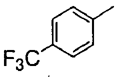 | Me | O | Me,Me | 115-116 | 1.76(6H,s),2.20(3H,s),2.37(3H,s),3.78(3H ,S),4.56(2H,s),6.49-6.50(2H,m), 6.67(1H,m),7.75(2H,dJ=8.1Fiz),7.84(2H,d ,J=8.1Hz) |

**Table 80**

| No | Synthetic method | R1 | R2 | X1 | R3,R4 | mp | NMR(CDCl3 or DMSO-d6) |
|---|---|---|---|---|---|---|---|
| β-1-12 | β-1 | 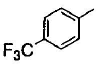 | Me | S | H,Et. | 115-117 | 1.07(3H,t,J=7.5Hz),1.98-2.16(2H,m), 2.20(3H,s),2.29(3H,s),4.04(1H,t,J=7.5Hz), 4.65(2H,s),6.61(1H,d,J=8.1Hz), 7.10-7.14(2H,m), 7.74(2H,dJ=8.4Hz),7.81(2H,d,J=8.4Hz) |
| β-1-13 | β-1 | 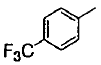 | Me | S | H, 4-F-C6H4 | 110-112 | 2.29(3H,s),2.20(3H,s),4.67(2H,s),5.29(1H, s),6.59(1H,d,J=8.4Hz), 6.96-7.15(4H,m).7.32-7.37(2H,m),7.73(2H,dJ=8.4Hz).7.79(2H,d, J=8.4Hz) |
| β-1-14 | β-1 | 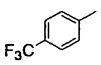 | 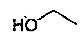 | S | H,H | 138-139 | 2.23(3H,s),4.11 (2H,s),4.66(2H.d,J=3.6),3. 34(1H,br,s),6.64(1H,d,J=8.4Hz),7.16-7.29(2H,m),7.77(2H,d,J=8.4Hz),7.95(2H,d ,J=8.4Hz) |
| β-1-15 | β-1 | 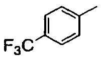 | 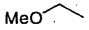 | S | H,H | 105-107 | 2.24(3H,s),3.43(3H,s),4.12(2H,s),4.46(2H, s),4.66(2H,s),6.65(1H,d,J=8.5Hz),7.18-7.24(2H,m),7.76(2H,d,J=8.7Hz),7.88(2H,d ,J=8.7Hz) |
| β-1-16 | β-1 | 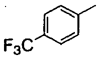 | 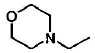 | S | H,H | oil 183-186 (as HClsalt) | 2.23(3H,s),2.49(4H,m),3.62(2H,s),3.69(4H ,m),4.18(2H,s),4.64(2H,s),6.65(1H,d,J=9.0 Hz),7.18-7.21(2H,m),7.74(2H,d,J=7.8Hz),790(2H,d, J=7.8Hz) |
| β-1-17 | β-1 | 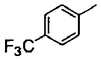 | 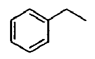 | S | H,H | 138-139 | 2.23(3H,s),3.83(2H,s),4.12(2H,s),4.66(2H, s),6.64(1H,d,J=9.0Hz),7.11-7.16(2H,m),7.24-7.31(m,5H),7.08(2H,d,J=8.4Hz),7.76(2H,d ,J=8.4Hz) |
| β-1-18 | β-1 | 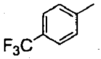 | 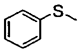 | S | H,H | 123-124 | 2.23(3H,s),3.97(2H,s),4.67(2H,s),6.63(1H. d,J=8.1Hz),7.08-7.26(7H,m), 7.70(2H,d,J=8.4Hz),8.22(2H,d,J=8.4Hz) |
| β-1-19 | β-1 | Me | I | S | H,H | 126-127 | 2.24(3H,s),2.44(3H,s),3.92(2H,s),4.66(2H, s),6.64(1H,d,J=8.1Hz),7.18(2H,dd,J=8.1,1 .8Hz),7.22(2H,d,J=1.8Hz) |
| β-1-20 | β-1 | Me | 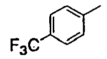 | S | H,H | oil | 2.21(3H,s),2.40(3H,s),3.98(2H,s),4.66(2H, s),6.60(1H,d,J=8.1Hz),7.08-7.12(2H,m),7.42(2H,d,J=8.1Hz),7.68(2H,d ,J=8.1Hz) |
| β-1-21 | β-1 | Me | 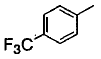 | O | H,H | 153-154 | 2.25(3H,s),2.49(3H,s),4.62(2H,s),5.02(2H, s),6.65-6.73(3H,m),7.50(2H,d,J=8.4Hz),7.68(2H,d ,J=8.4Hz). |
| β-1-22 | β-1 | 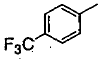 | 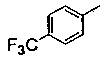 | S | H,H | 136.5-137.5 | 2.22(3H,s),3.95(2H,s),4.67(2H,S),6.62(1H ,d,J=8.1Hz),7.11-7,14(2H,m),7.47(2H,d,J=8.4Hz),7.60(4H,s ),7.72(2H,d,J=8.4Hz) |
| β-1-23 | β-1 | 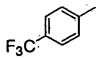 | 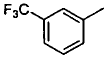 | S | H,H | 128-129.5 | 2.22(3H,s),3.95(2H,s),4.67(2H,s),6.62(1H, d,J=9.0Hz),7.13-7.15(2H,m),7.50-7.74(8H,m) |

**Table 81**

| No | Synthetic method | R1 | R2 | X1 | R3,R4 | mp | NMR(CDCl3 or DMSO-d6) |
|---|---|---|---|---|---|---|---|
| β-1-24 | β-1 | 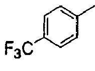 | 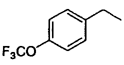 | S | H,H | 135-136 | 2.23(3H,s),3.84(2H,s),4.12(2H,s),4.67(2H, s),6.64(1H,d,J=9.0Hz),7.11-7,14(6H,m),7.71-7.72(4H,m) |
| β-1-25 | β-1 | 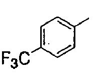 | 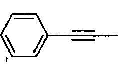 | S | H,H | 196-197.5 | 2.19(3H,s),4.13(2H,s),4.55(2H,s),6.63(1H, d,J=8.4Hz),7.28(2H,m), 7.41-7.43(3H,s),7.53(2H,s),7.79(2H,d,J=8.4Hz) ,8.31(2H,d,J=8.4Hz) |
| β-1-26 | β-1 | 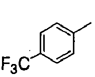 | 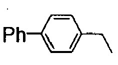 | S | H,H | 137-138 | 2.22(3H,s),3.87(2H,s),4.16(2H,s),4.65(2H, s),6.63(1H,d,J=9.0Hz),7.14-7.21(4H,m),7.34-7.56(7H,m),7.70(2H,d,J=8.1Hz),7.78(2H,d .J=8,1Hz) |
| β-1-27 | β-1 | 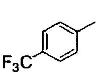 | BuNHCH2- | S | H,H | 177-178 | 0.84(3h,t,J=7.2Hz),1.22-1.45(4H,m),2.14(3H,s), 2.56 (2H,t,J=7.2Hz), 3.72(2H,s),4.27(2H,s),4.63(2H,s), 6.76(1H,d,J=8.4Hz),7.15-7.23(2H,m), 7.91(2H,d,J=8.4Hz), 8.08(2H,d,J=8.4Hz) |
| β-1-28 | β-1 | 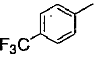 | 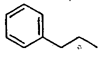 | S | H,H | 150-152 | 2.24(3H,s),2.93-2.30(4H,m),3.79(2H,s),4.67(2H,s),6.65(1H ,d,J=8.1Hz),7.09-7.29(7H,m),7.70(4H,s) |
| β-1-29 | β-1 | 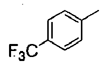 | 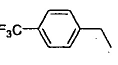 | S | H,H | 141.5-142.5 | 2.23(3H,s),3.84(2H,s),4.12(2H,s),4,67(2H, s),6.64(1H,d,J=9.0Hz),7.11-7.13(2H,m),7.24(2H,d,J=8.7Hz),7.56(2H,d ,J=8.7Hz),7.71(4H,s) |
| β-1-30 | β-1 | 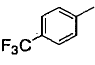 | 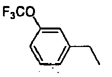 | S | H,H | 130-132 | 2.23(3H,s),3.85(2H,s),4.13(2H,s),4.67(2H, s),6.64(1H,d,J=9.6Hz),6.99-7.15(5H,m),7.30-7.35(1H,m),7.71(4H,s) |
| β-1-31 | β-1 | 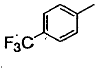 | 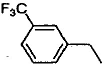 | S | H,H | 127-128.5 | 2.23(3H,s),3.84(2H,s),3.84(2H,s),4.67(2H, s),6.63(1H,d.J=8.4Hz),7.11-7.14(2H,m),7.27-7.53(4H,m),7.71(4H,s) |

**Table 82**

| | | | | | | | | |
|---|---|---|---|---|---|---|---|---|
| 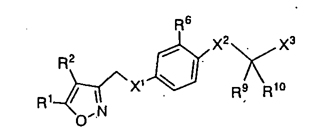 | | | | | | | | |

| No | Synthetic method | R1 | R2 | X1 | R6 | 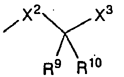 | mp | NMR(CDCl3 or DMSO-d6) |
|---|---|---|---|---|---|---|---|---|
| β-1-32 | β-1 | 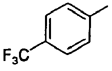 | Me | S | H | 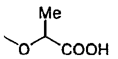 | 121-122 | 1.65(3H,d,J=6.9Hz),2.24(3H,s),4.03(2H, s),4.77(1H,q,J=6,9Hz),6.82(2H,d,J=9.0H z),7.34(2H,d,J=9.0Hz),7.74(2H,d,J=8.4H z),7.81(2H,d,J=8.4Hz) |
| β-1-33 | β-1 | 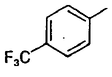 | Me | S | H | 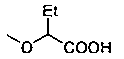 | 116-118 | 1.09(3H,t,J=7.5Hz),1.99-2.04(2H,m),2.24(3H,s),4.03(2H,s),4.56-4.60(1H,m),6.82(2H,d,J=8.7Hz),7.33(2H, d,J=8.7Hz),7.73(2H,d,J=8.5Hz),7.81(2H, d,J=8.5Hz) |
| β-1-34 | β-1 | 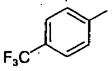 | Me | S | H | 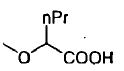 | 75.5-77.5 | 0.97(3H,t,J=7.2Hz),1.50-1.60(2H,m),1.91-2.00(2H,m),2.24(3H,s),4.03(2H,s),4.61-4.65(1H,m),6.82(2H,d,J=8.7Hz),7.35(2H, d,J=8.7Hz),7.73(2H,d,J=8.7Hz),7.81(2H, |
| β-1-35 | β-1 | 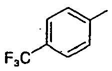 | Me | S | nPr | 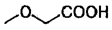 | 85-87 | 0.89(3H,t,J=7.2Hz),1.51-1.63(2H,m),2.24(3H,s),2.58(2H,t,J=7.2H z),4.03(2H,s),4.66(2H,m),6.70(1H,d,J=8. 4Hz),7.17-7.24(2H,m),7.74(2H,d,J=8.6Hz),7.81(2H, d,J=8.6Hz) |
| β-1-36 | β-1 | 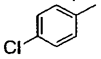 | Br | S | H | 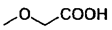 | 150-151 | 2.24(3H,s),4.03(2H,s),4.66(2H,s),6.65(1H,d,J=8.4Hz),7.21-7.26 (2H,m), 7.47 (2H,d,J=8.7Hz), 7.97(2H,d,J=8.7Hz) |

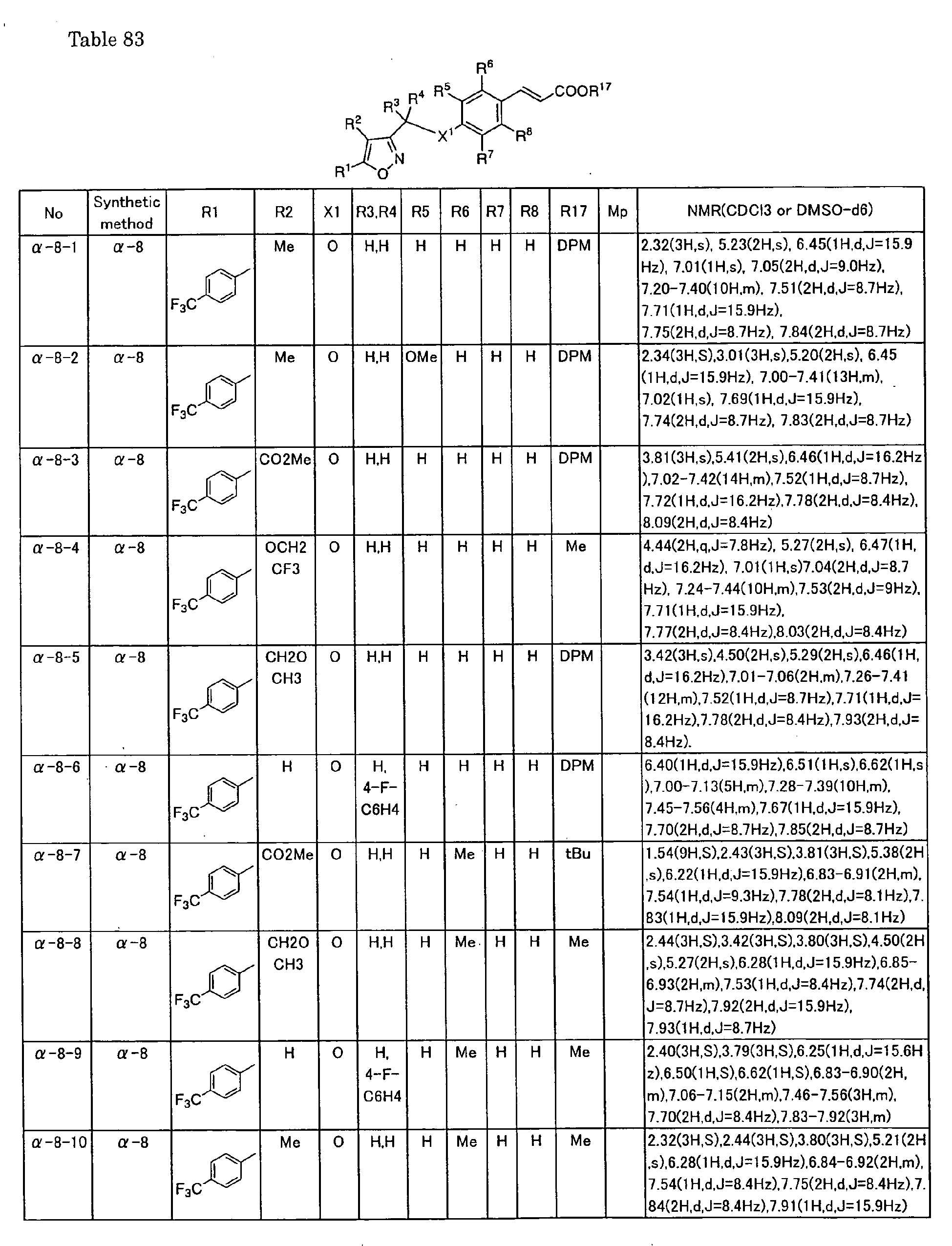

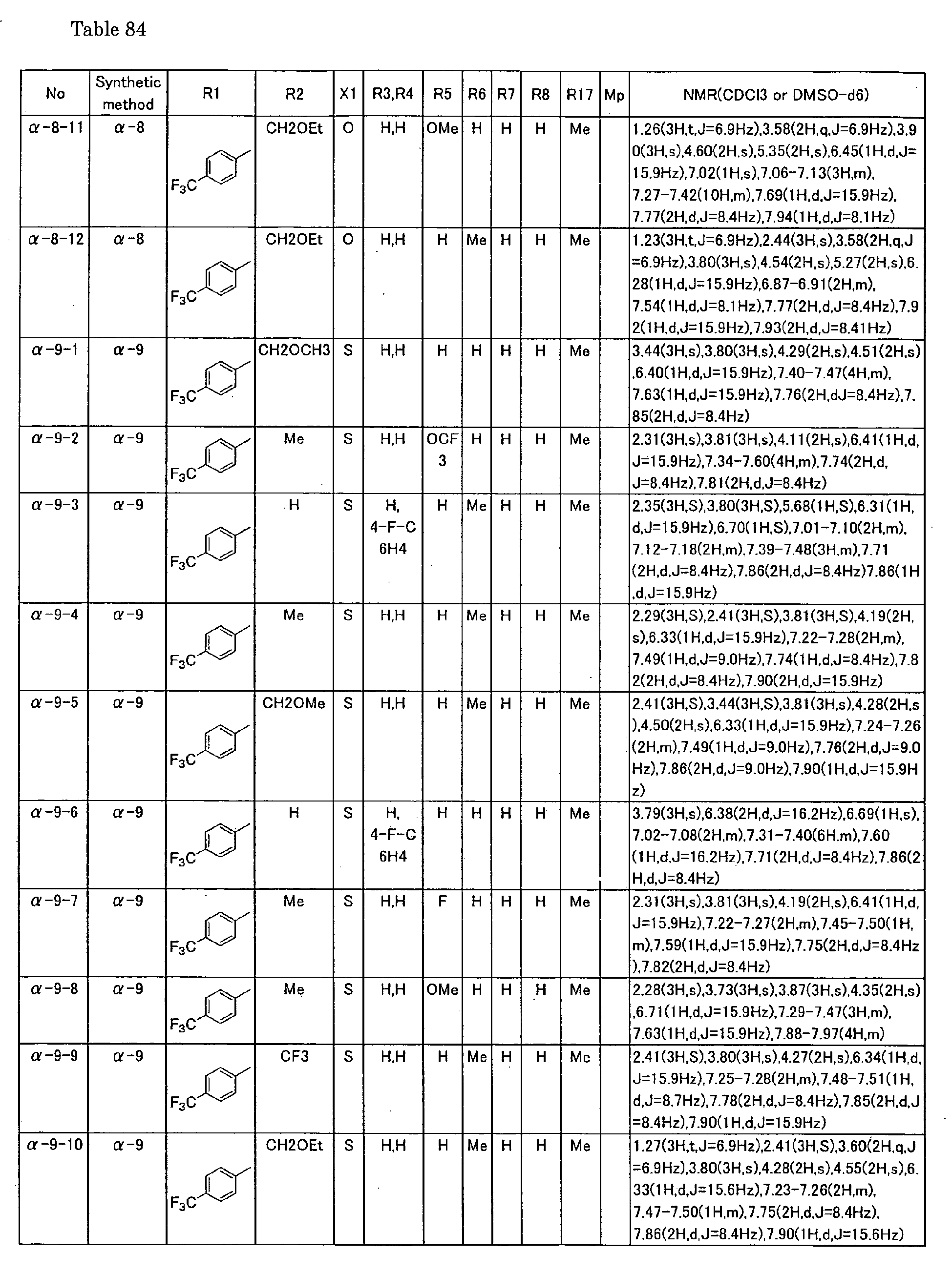

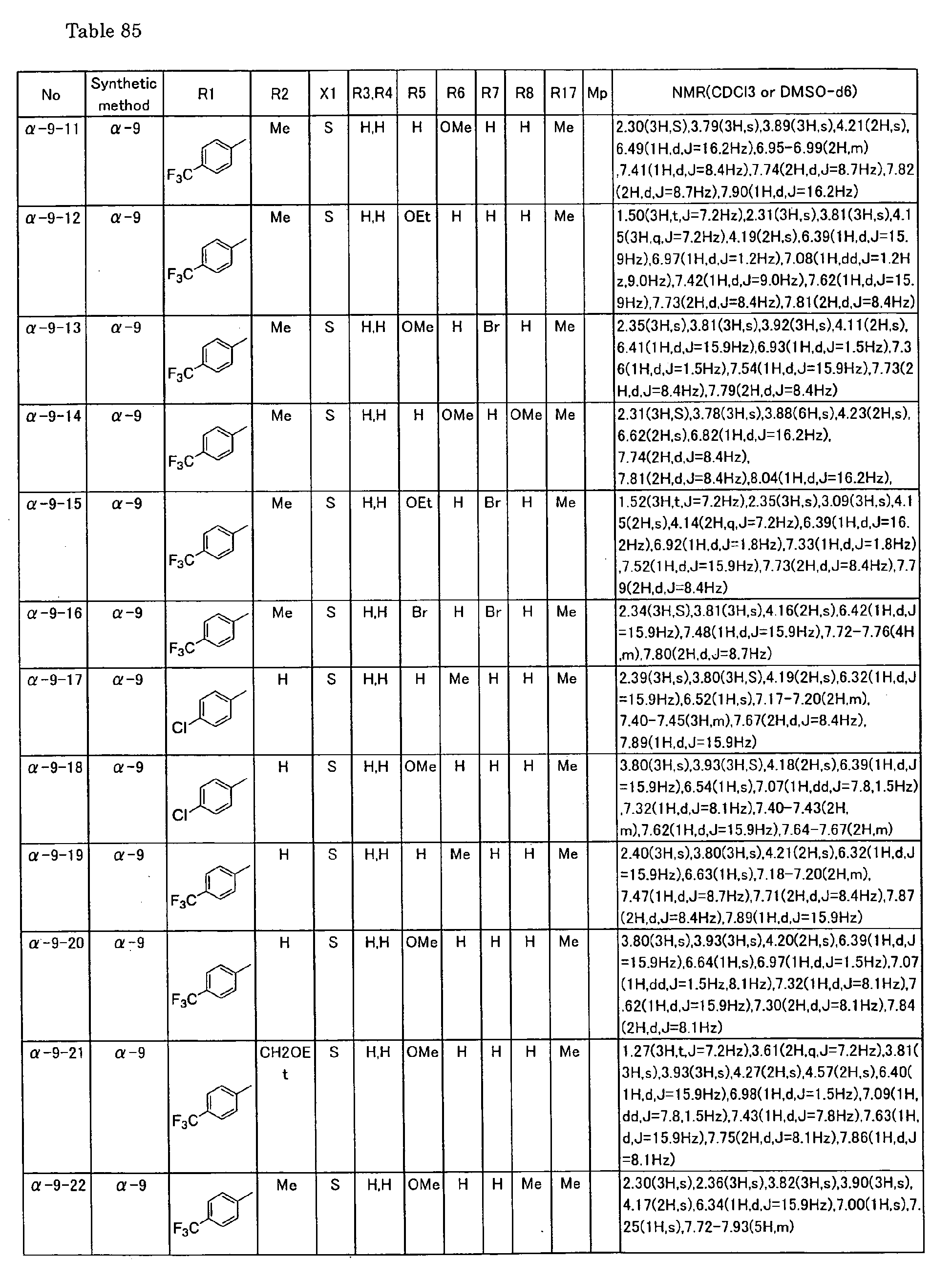

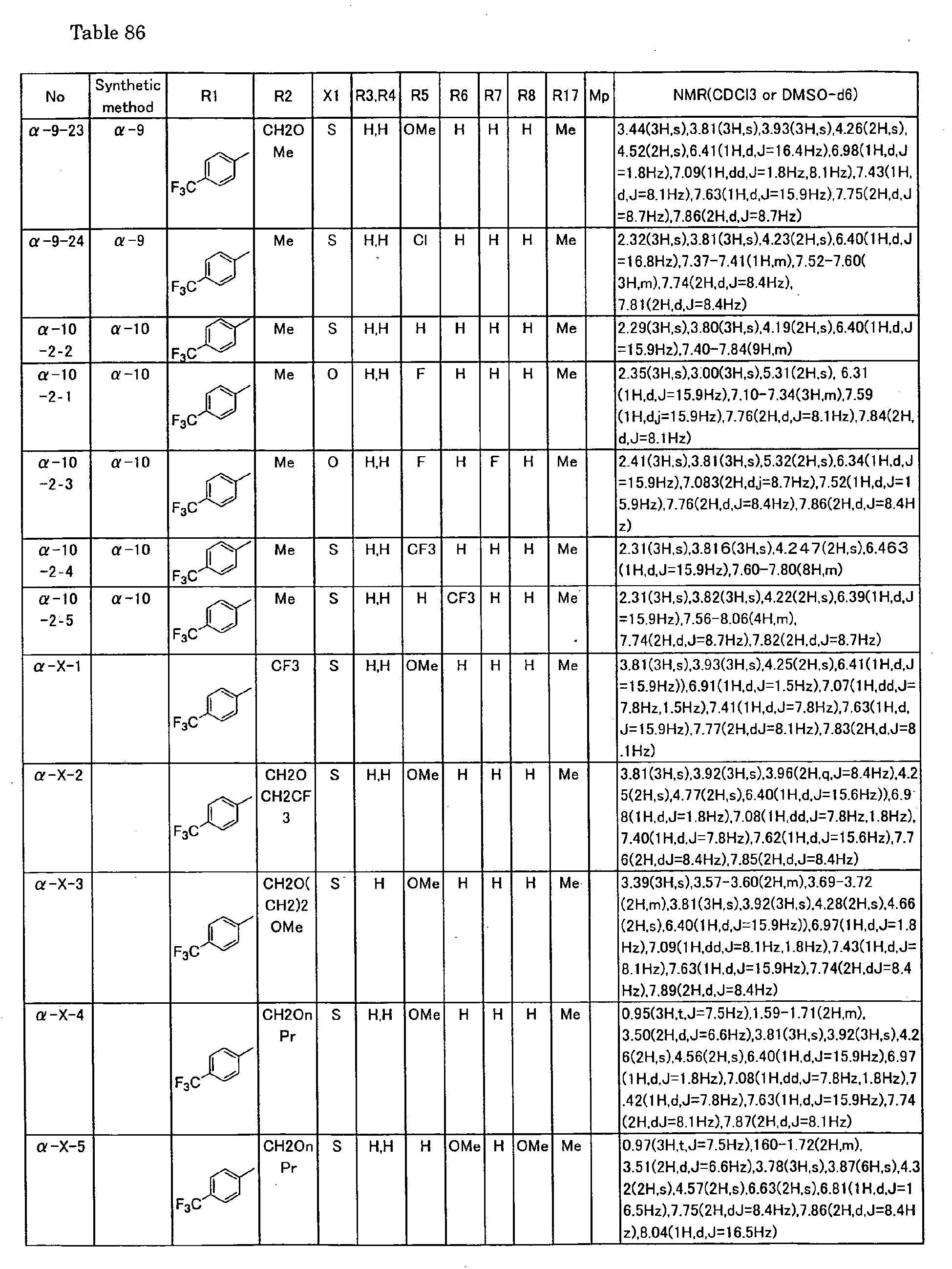

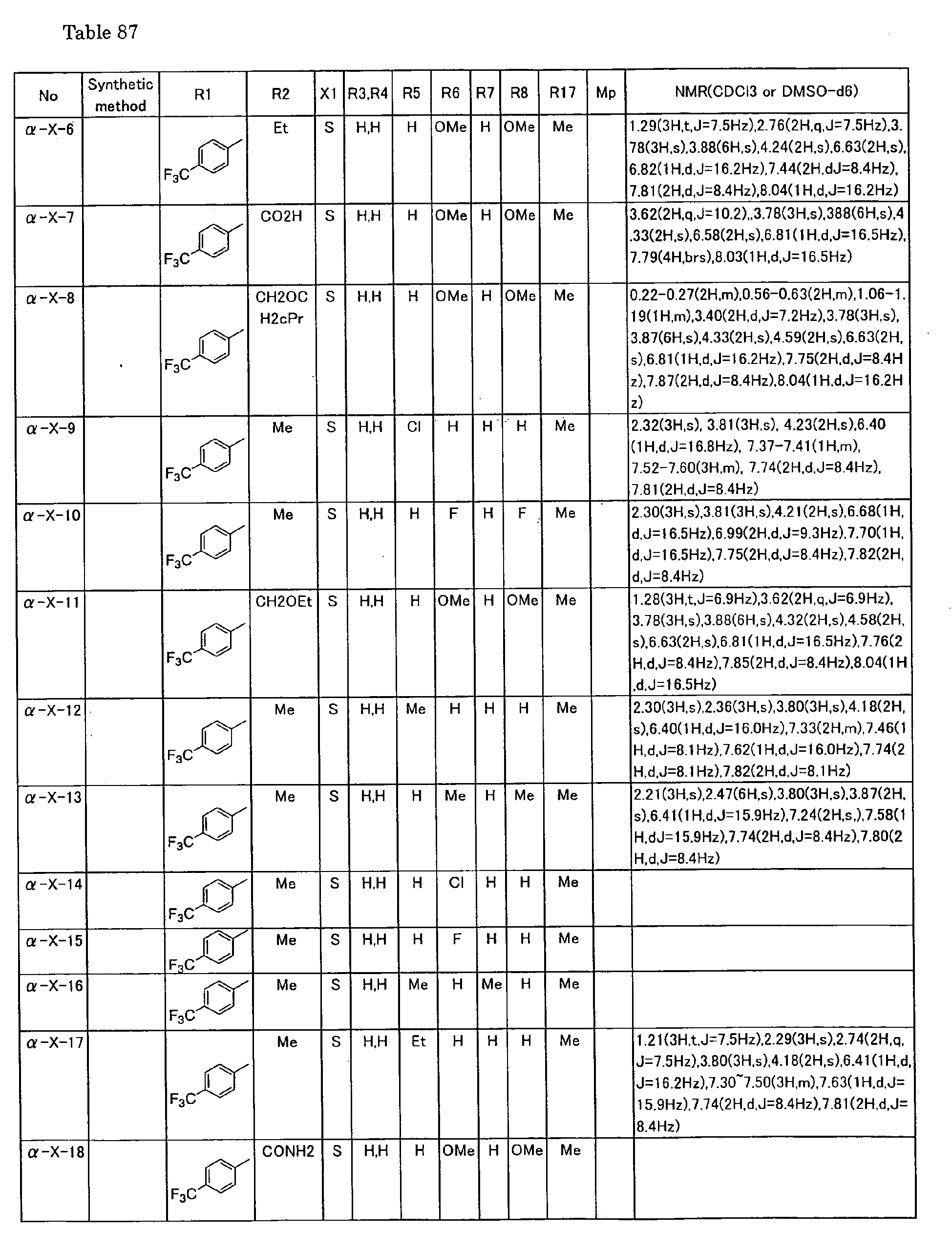

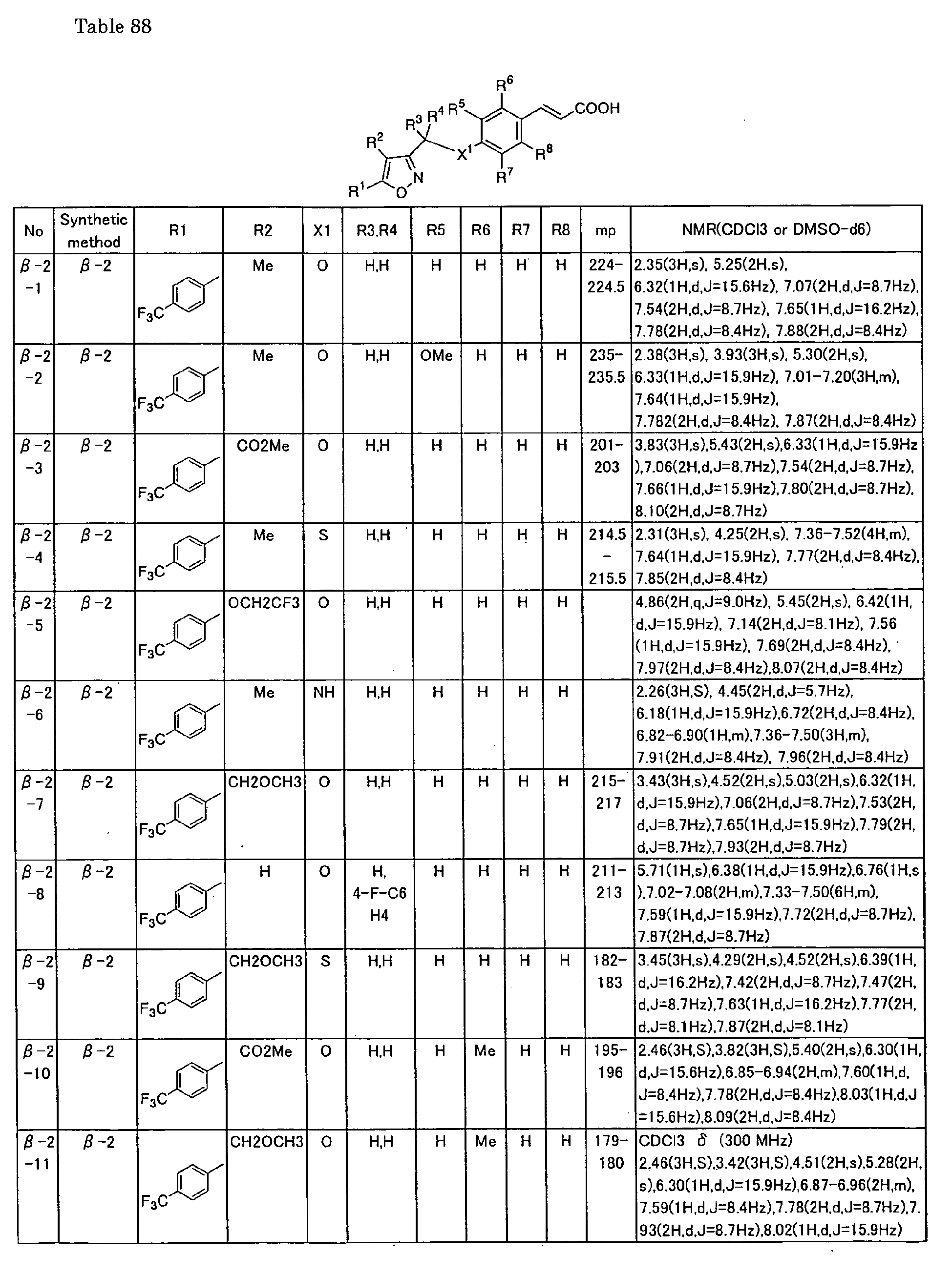

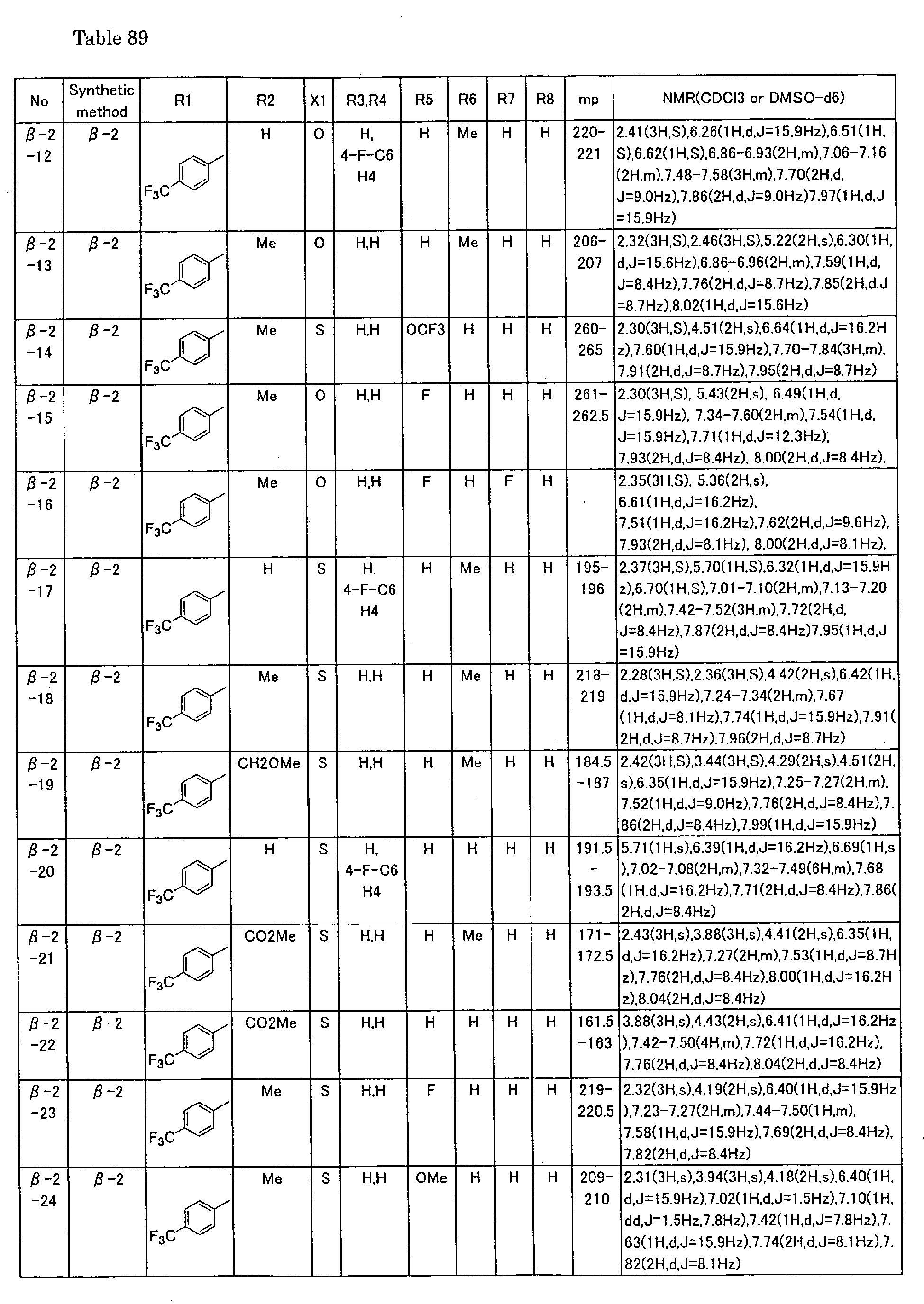

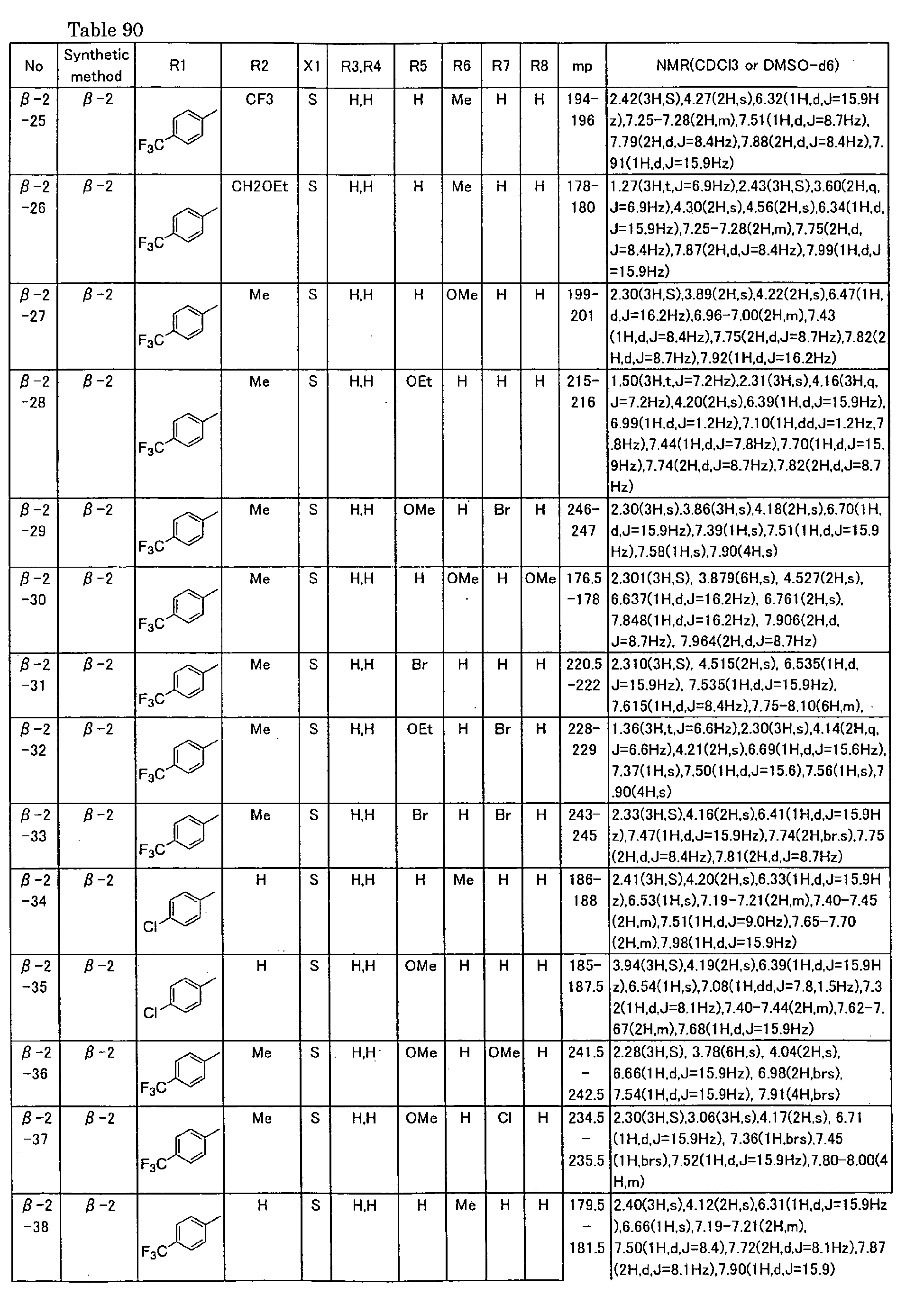

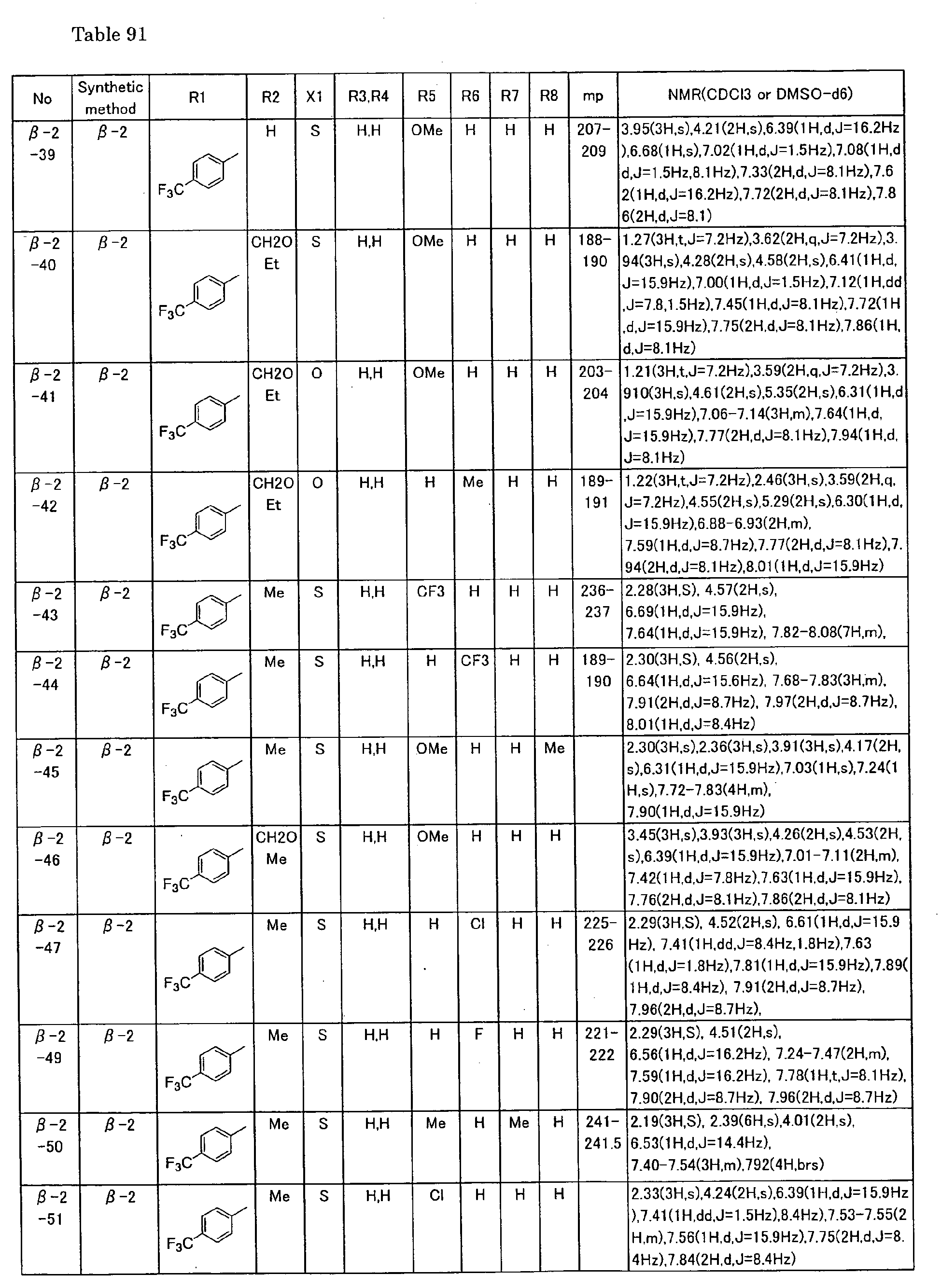

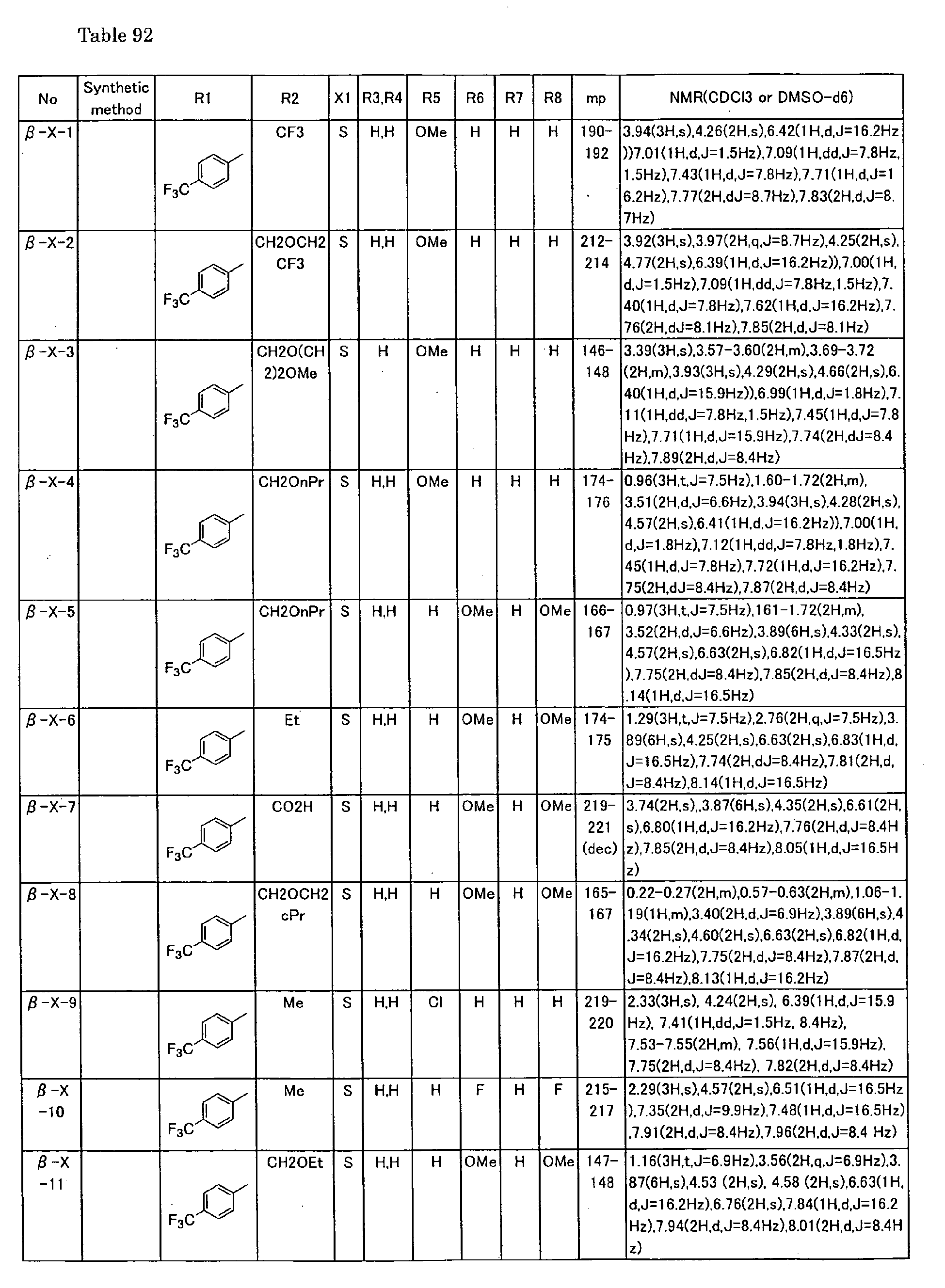

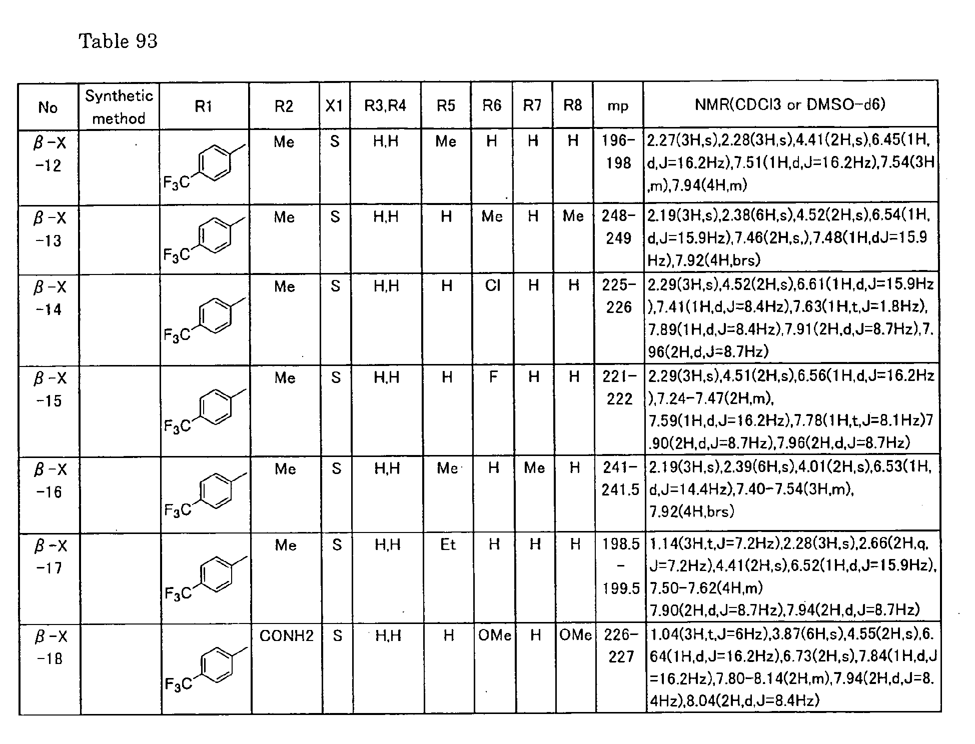

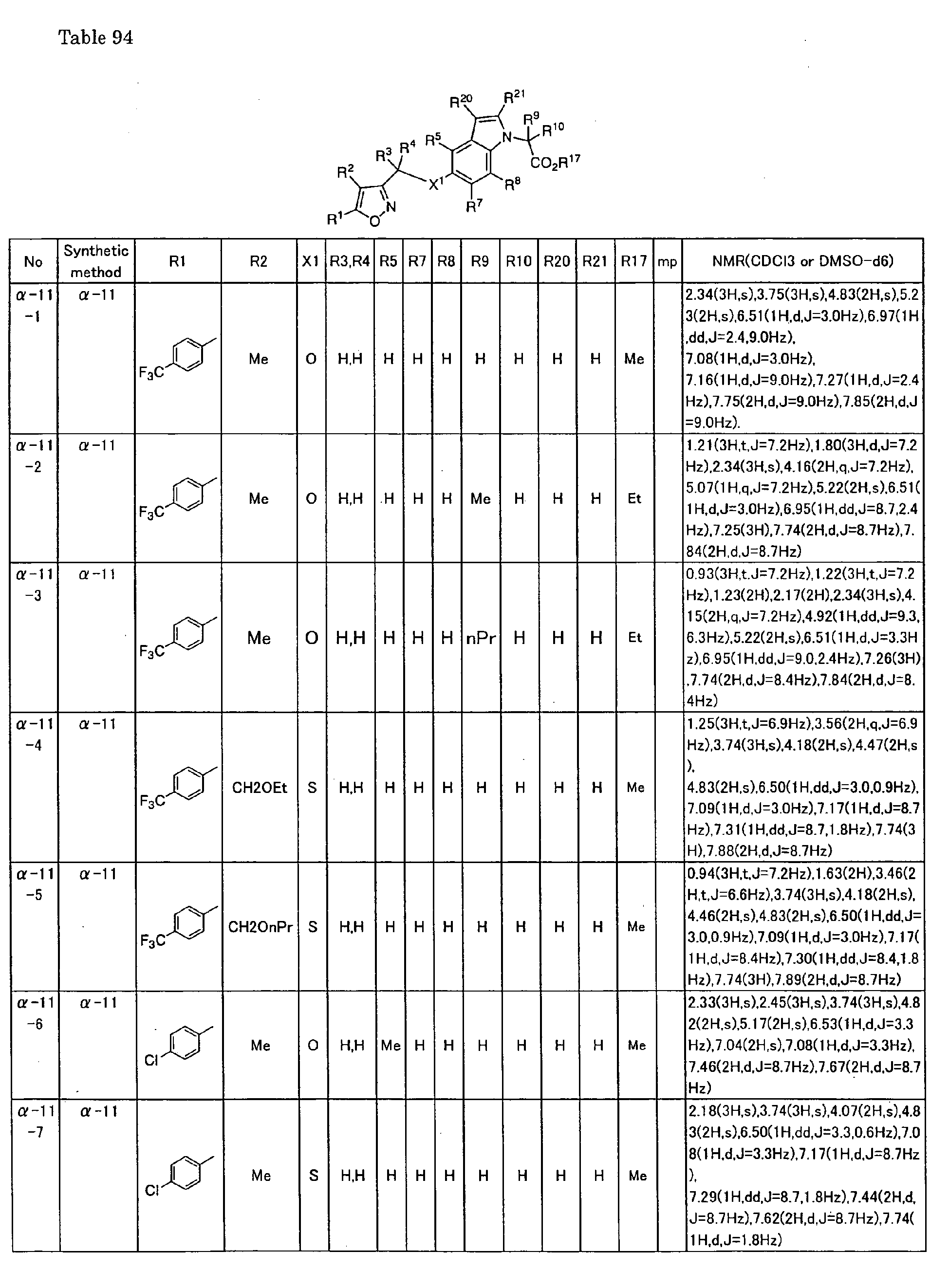

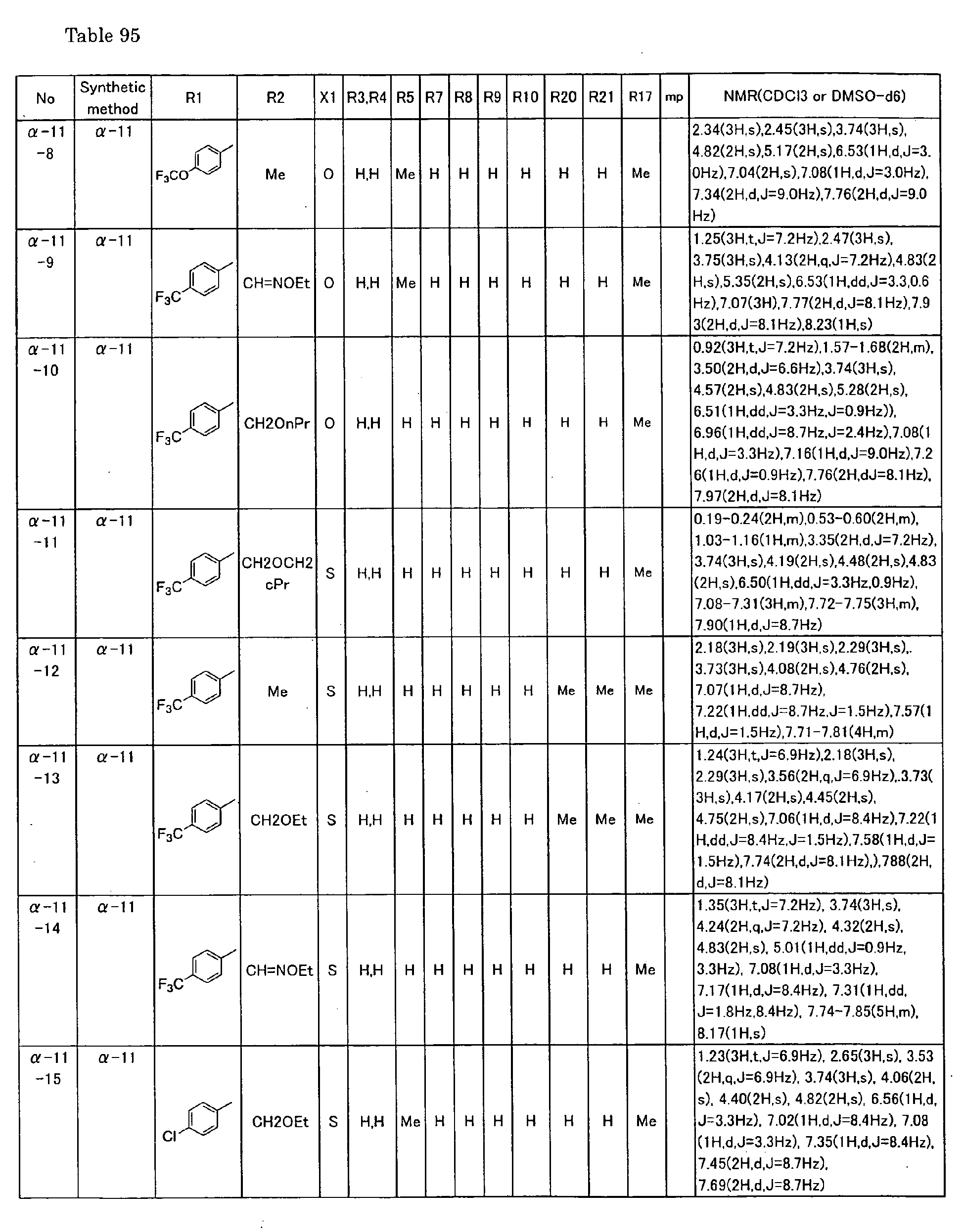

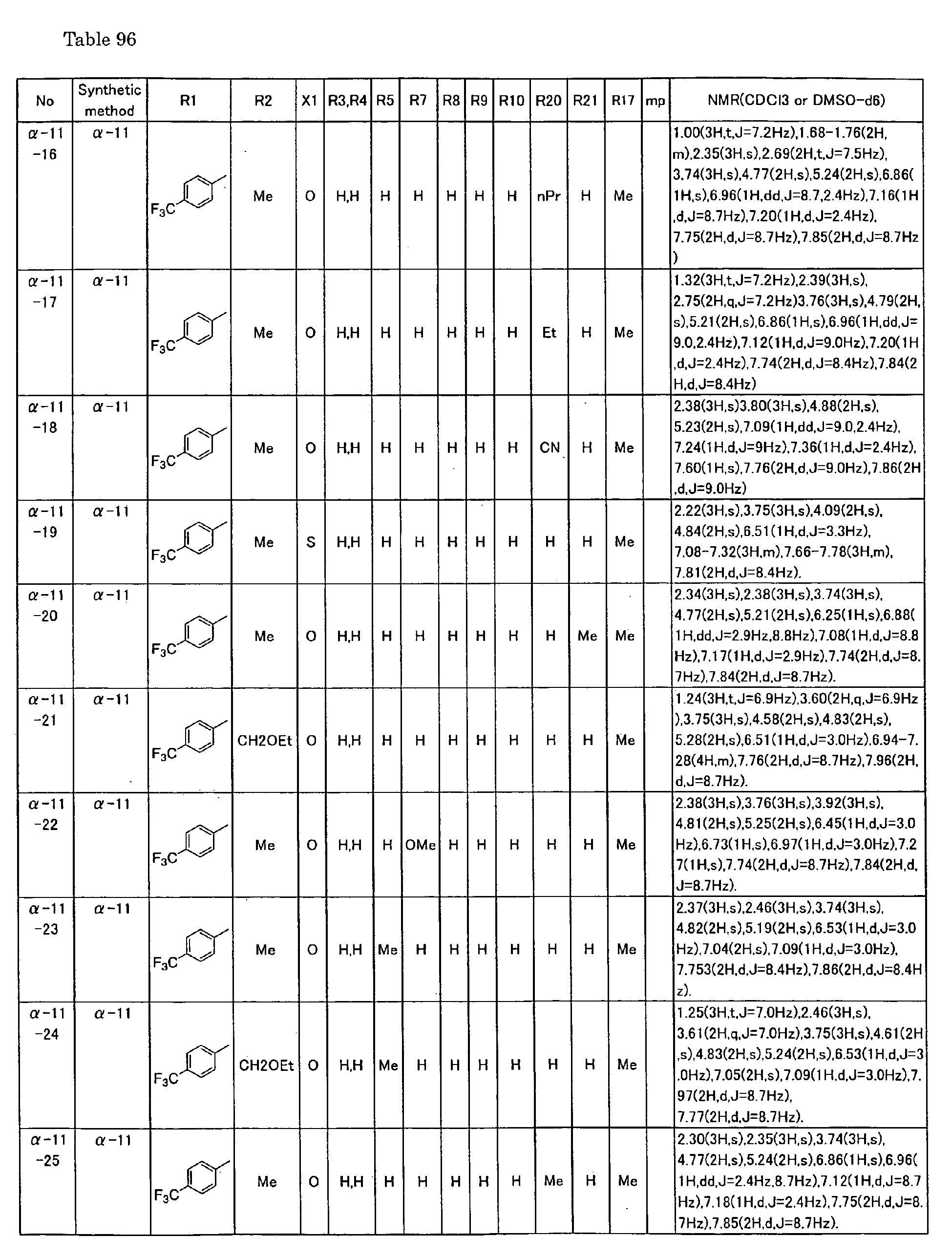

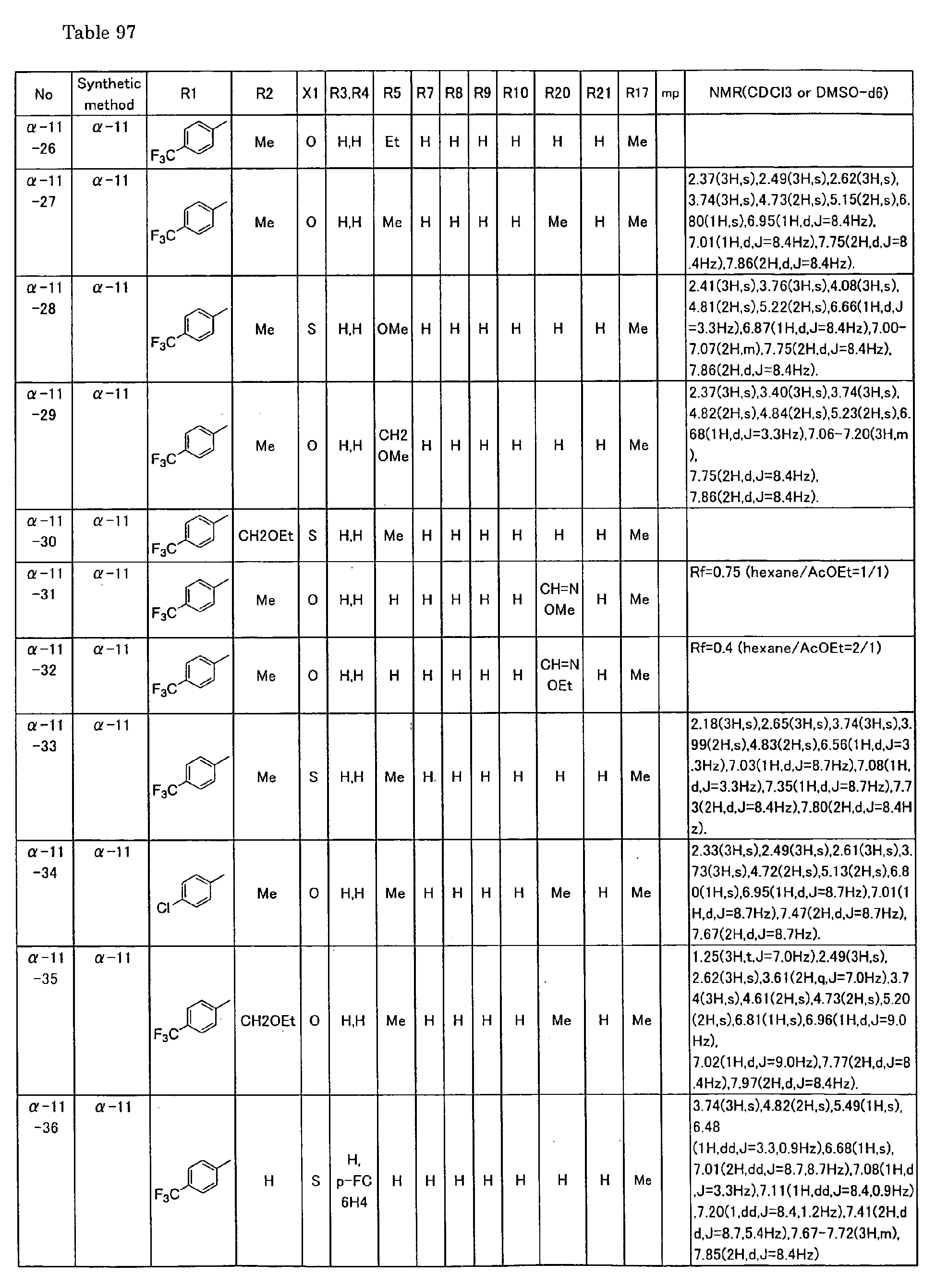

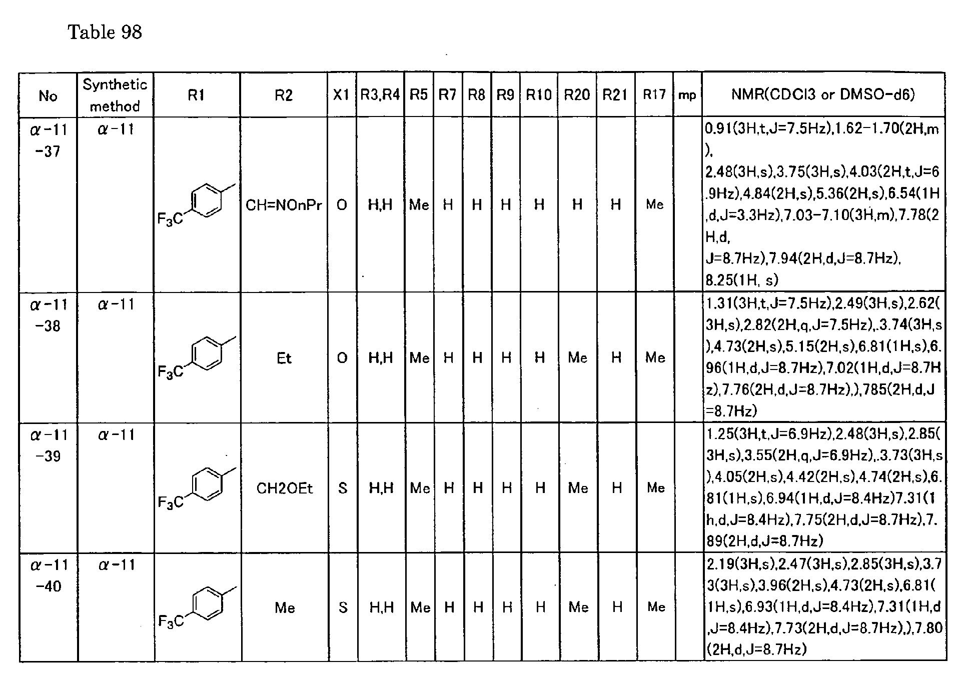

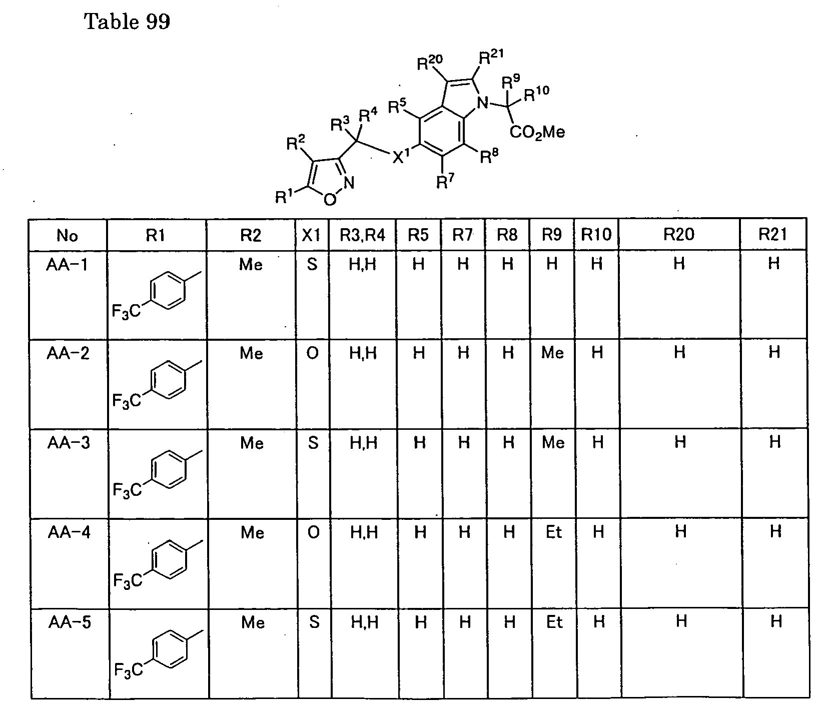

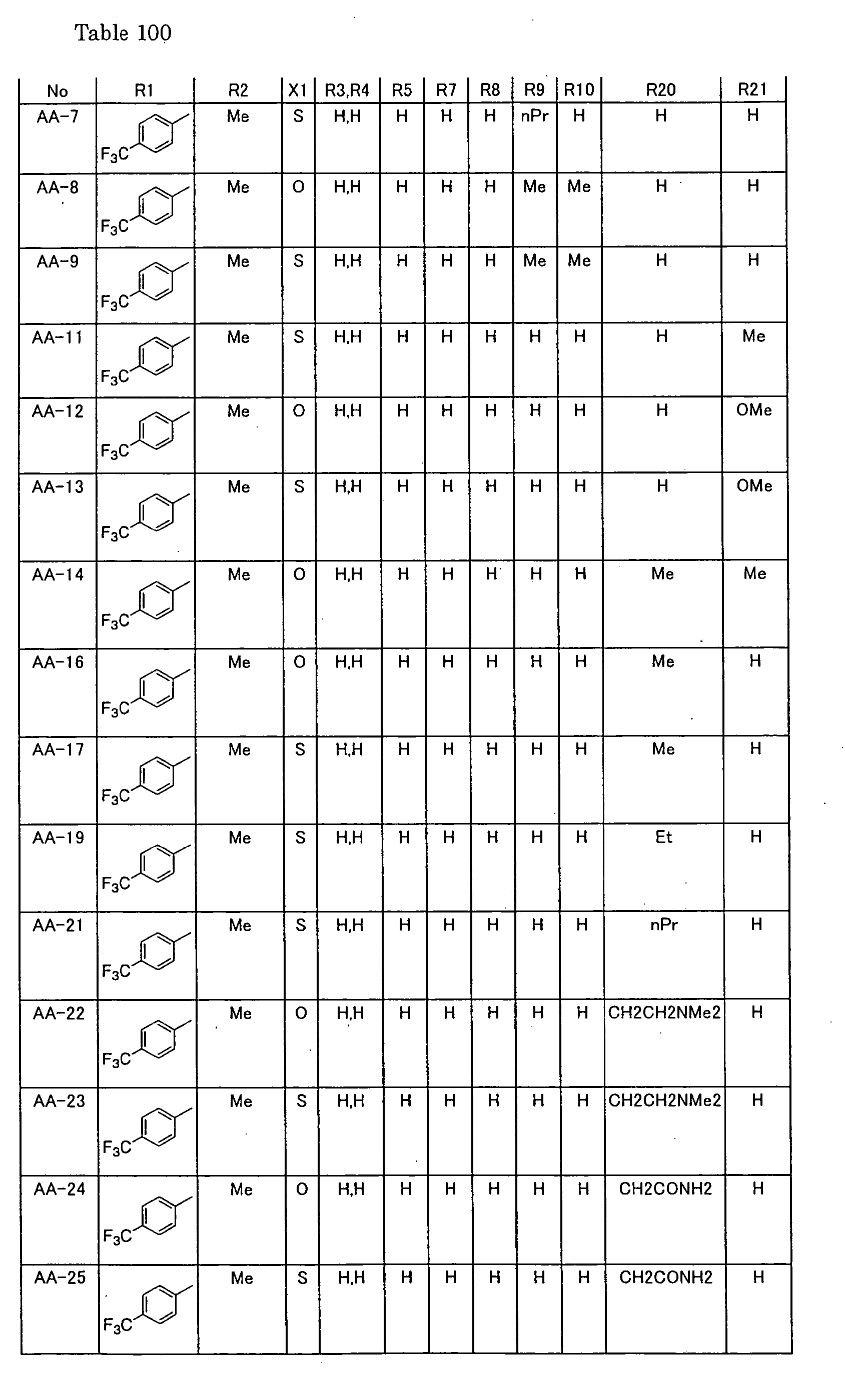

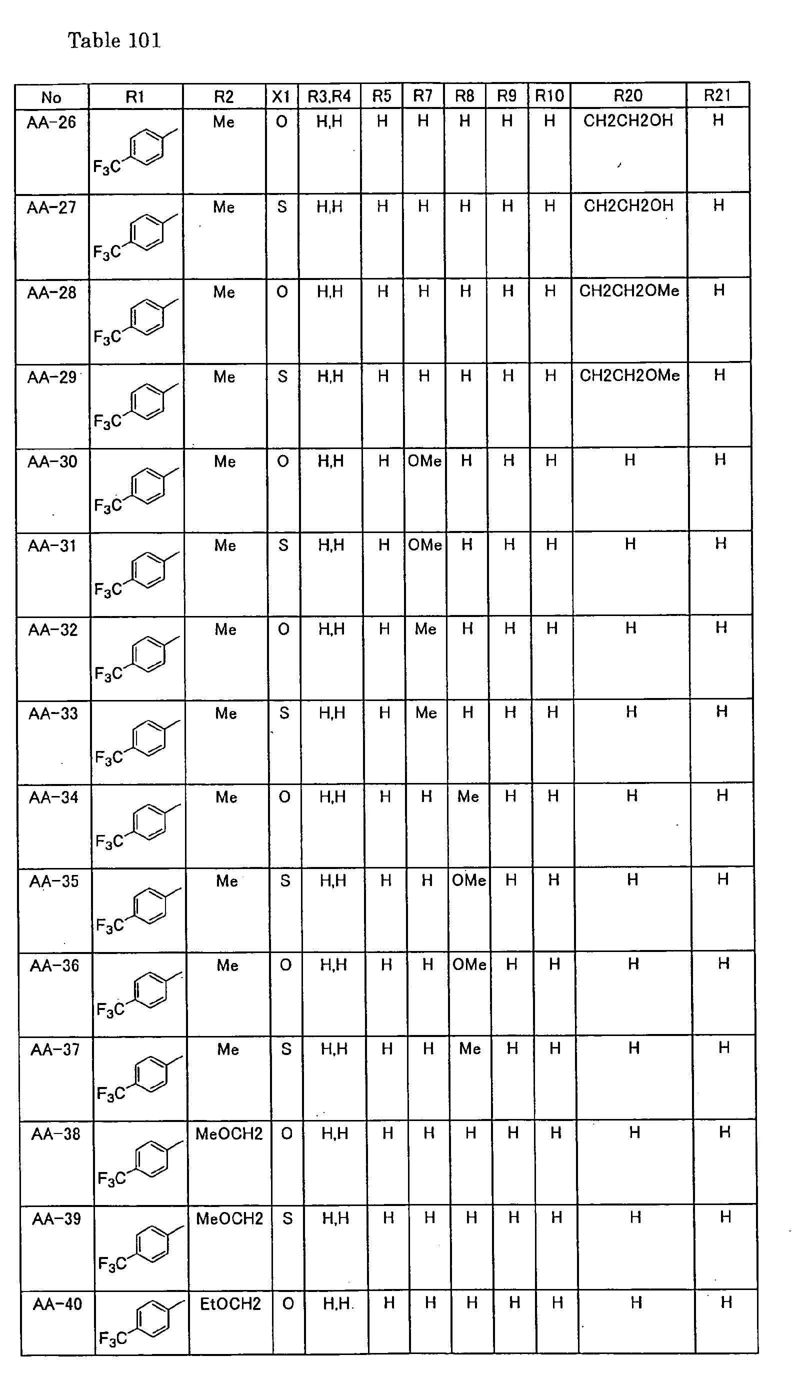

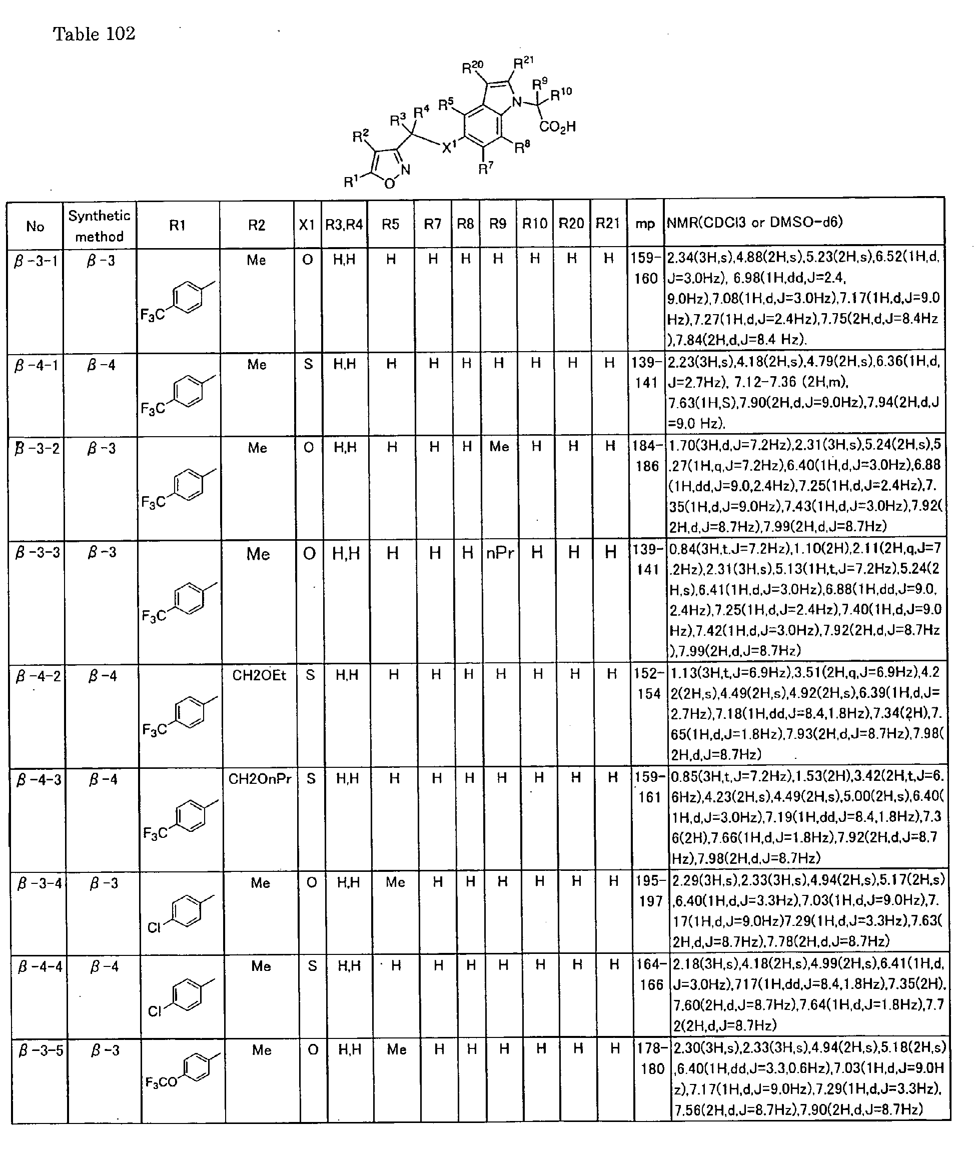

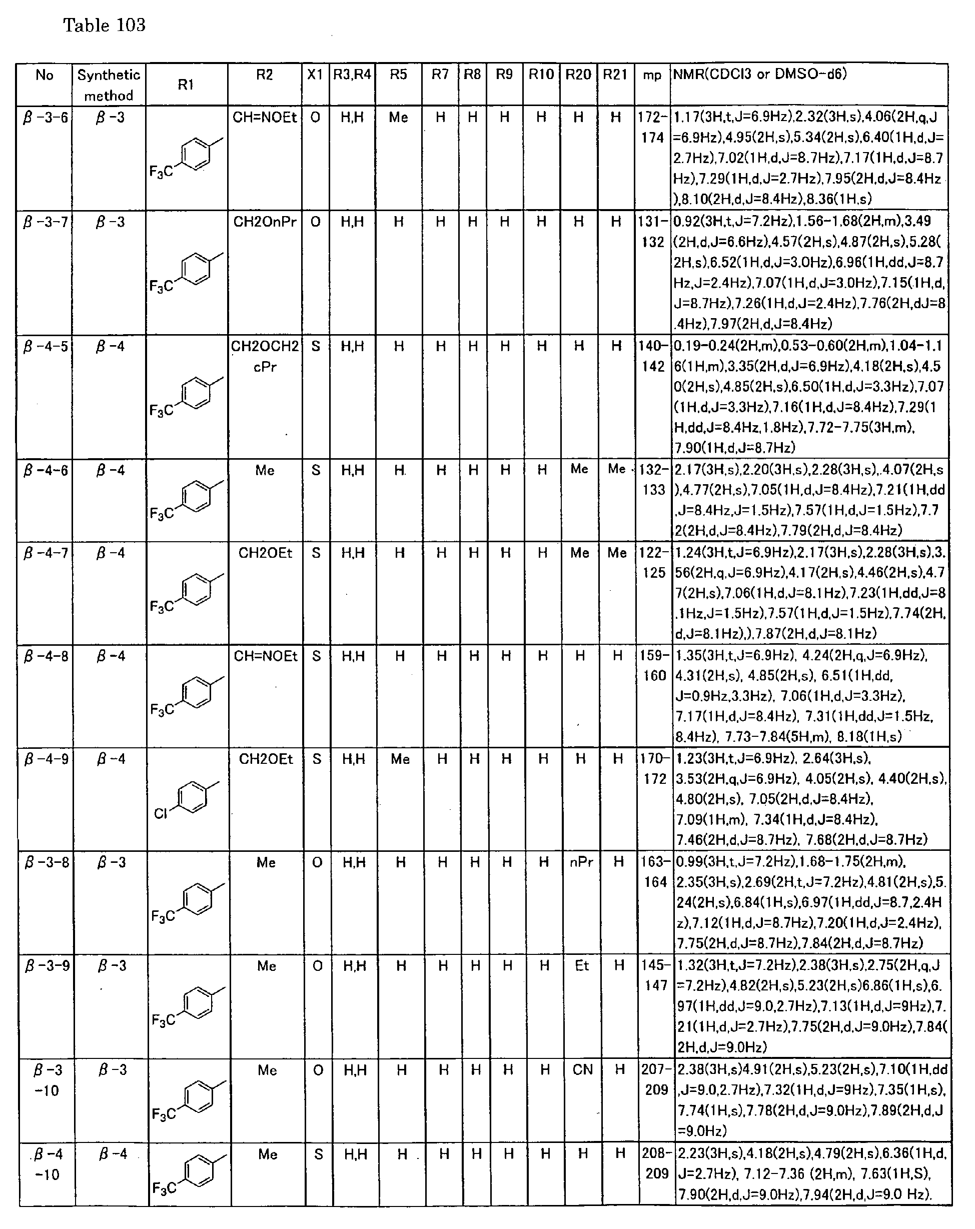

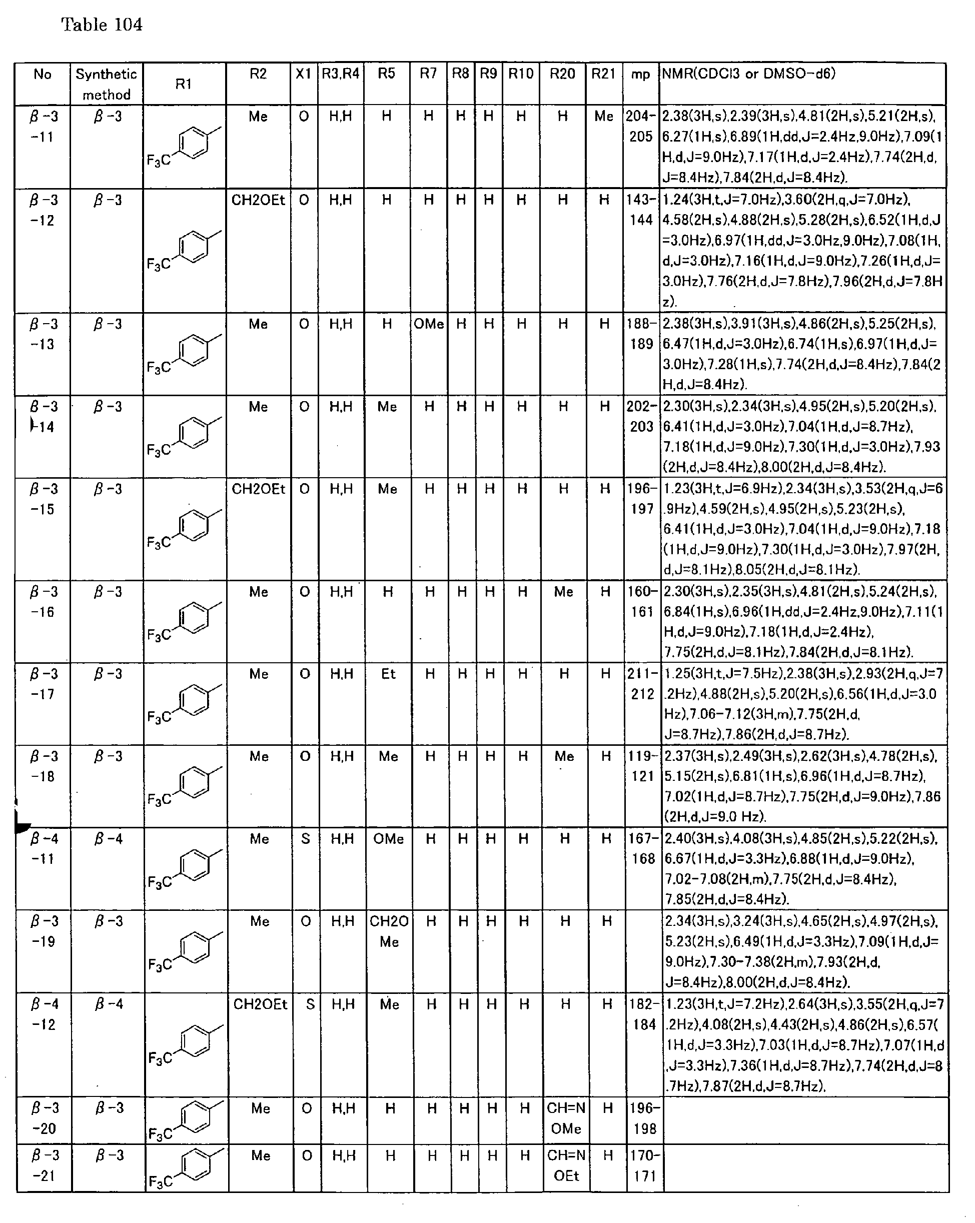

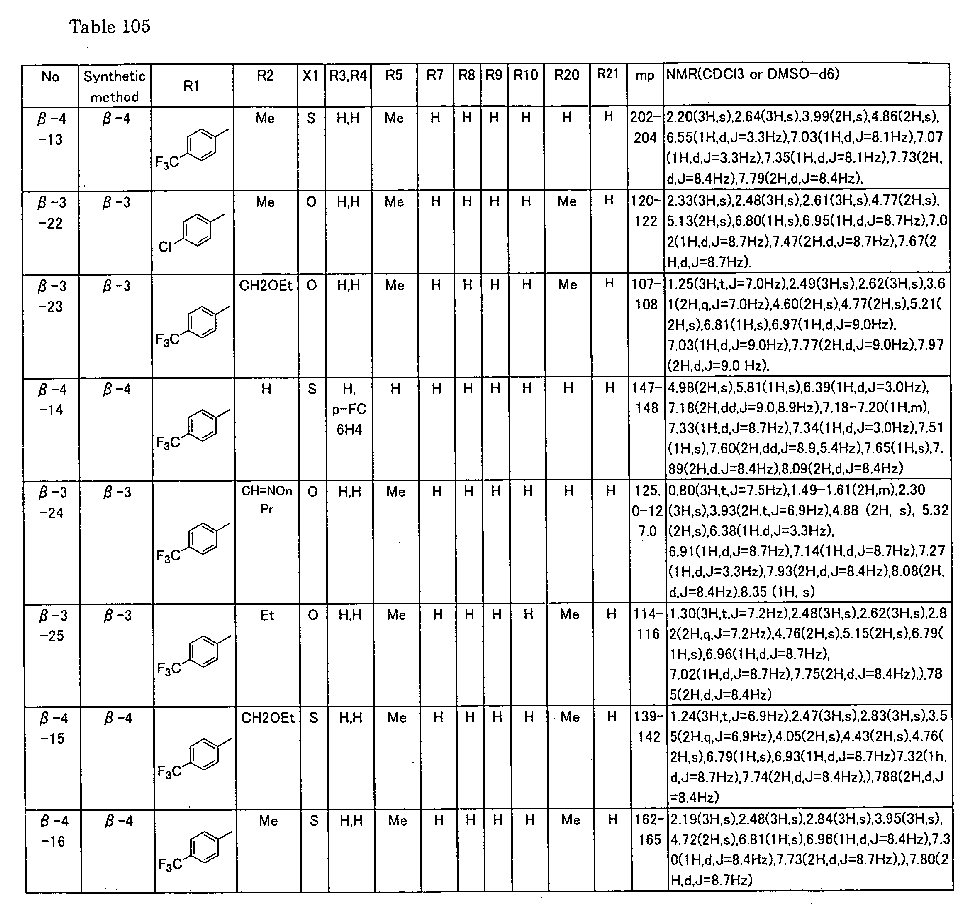

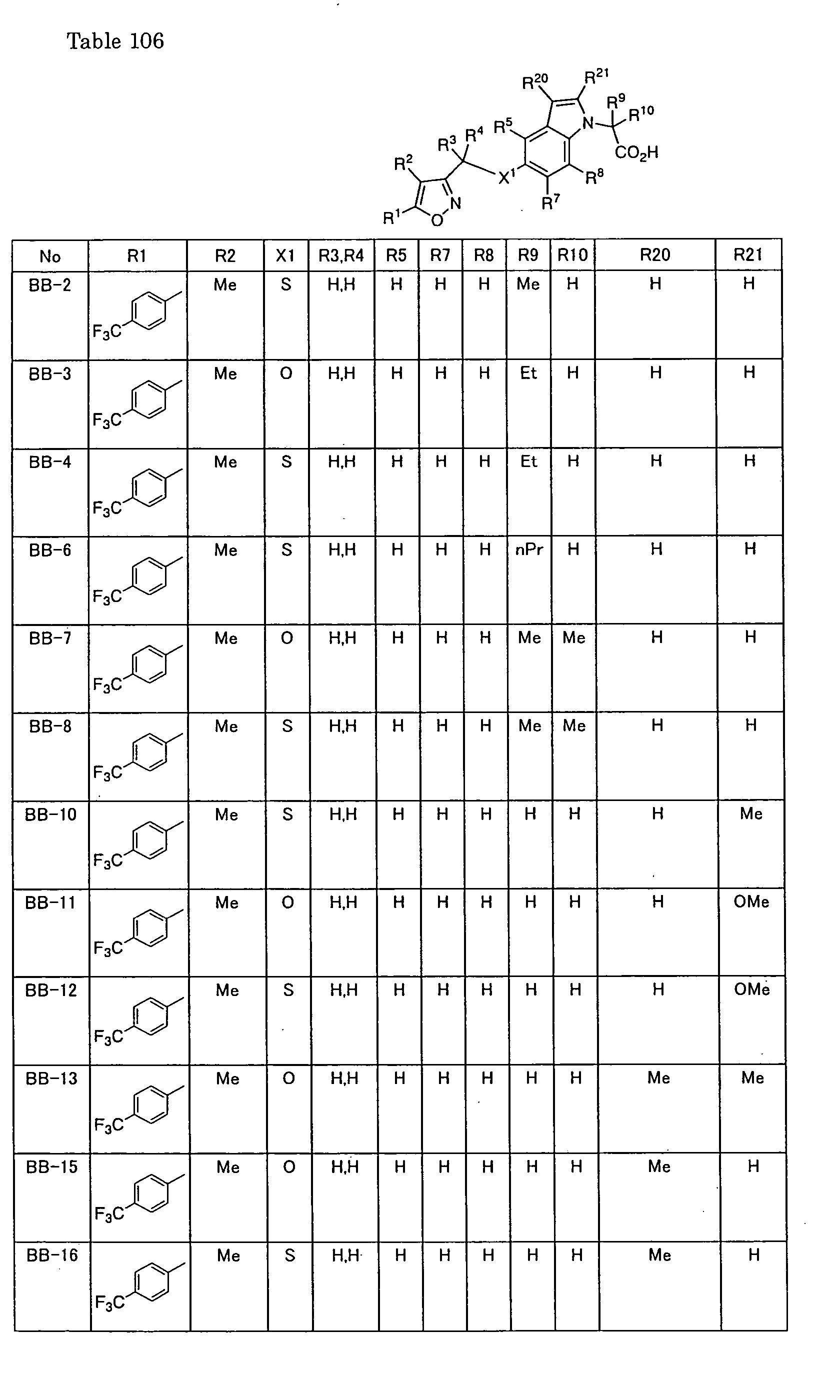

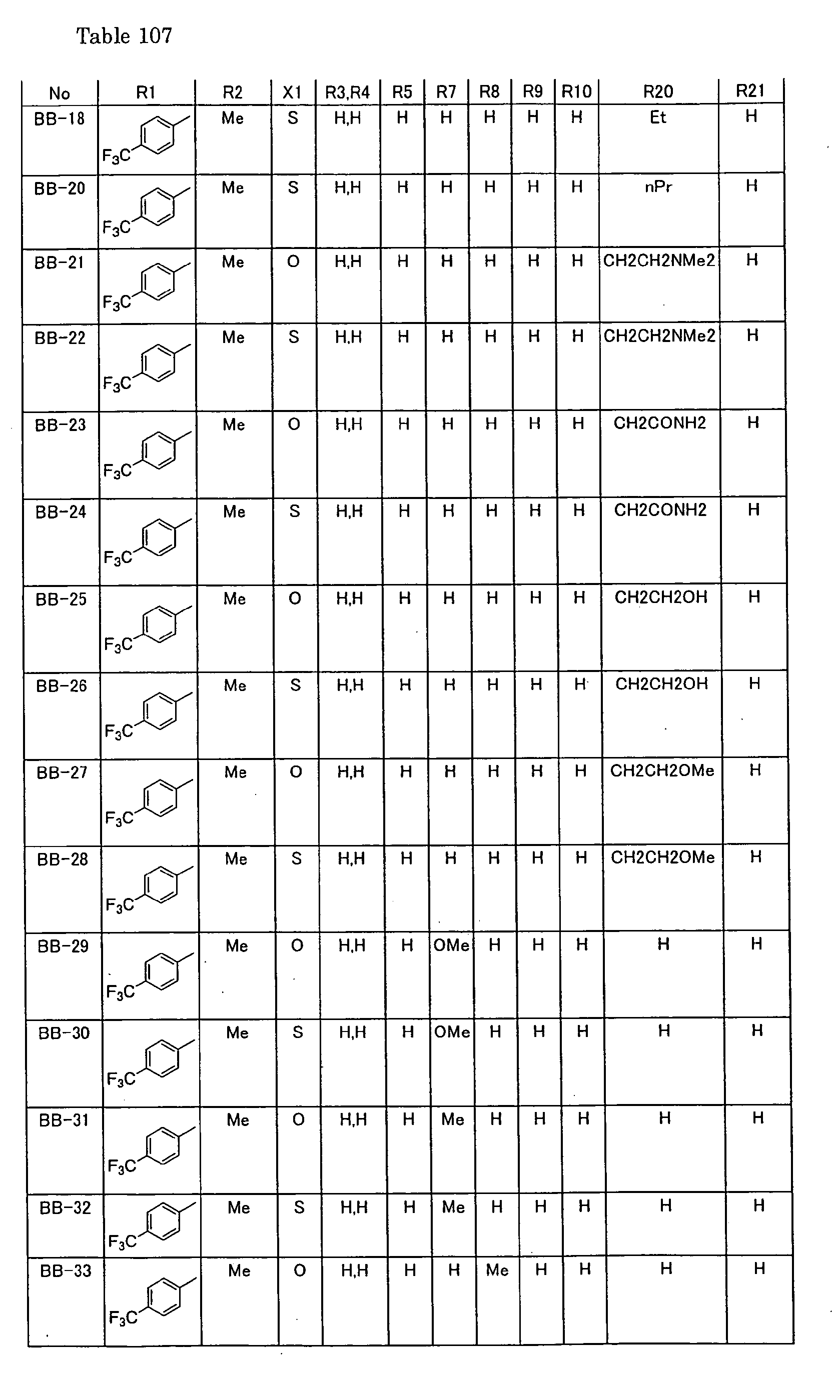

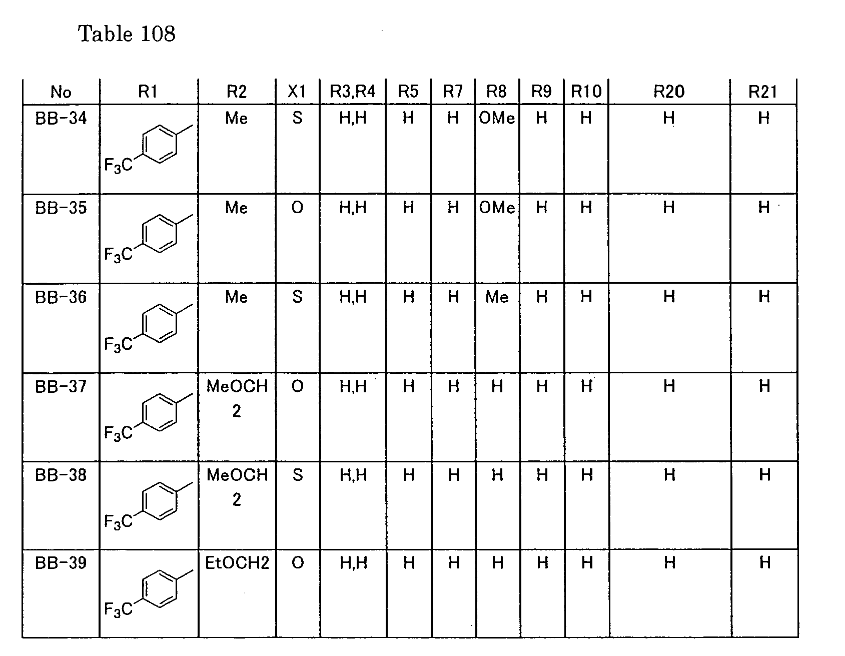

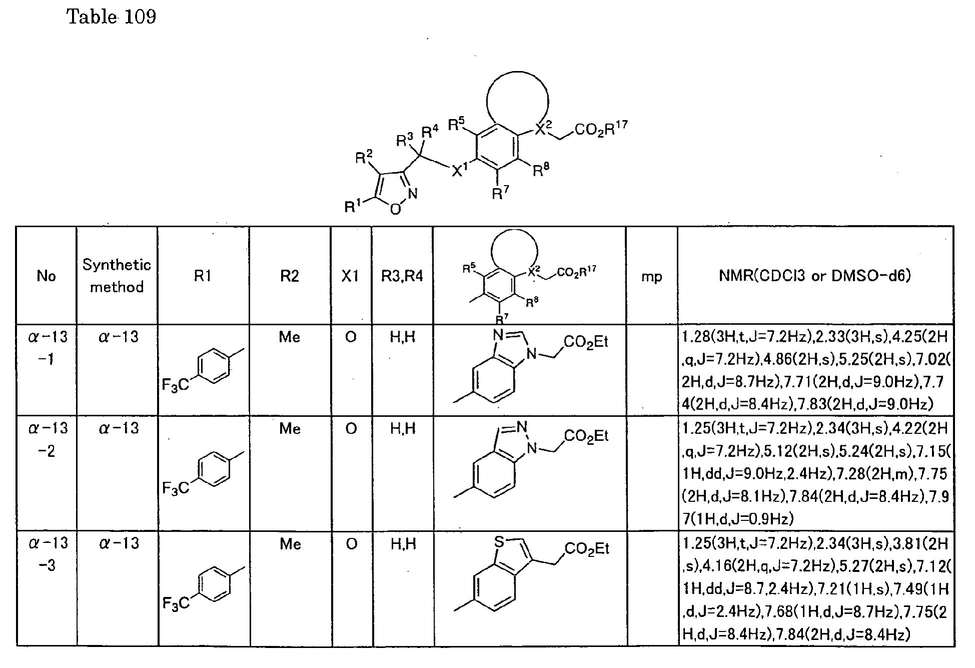

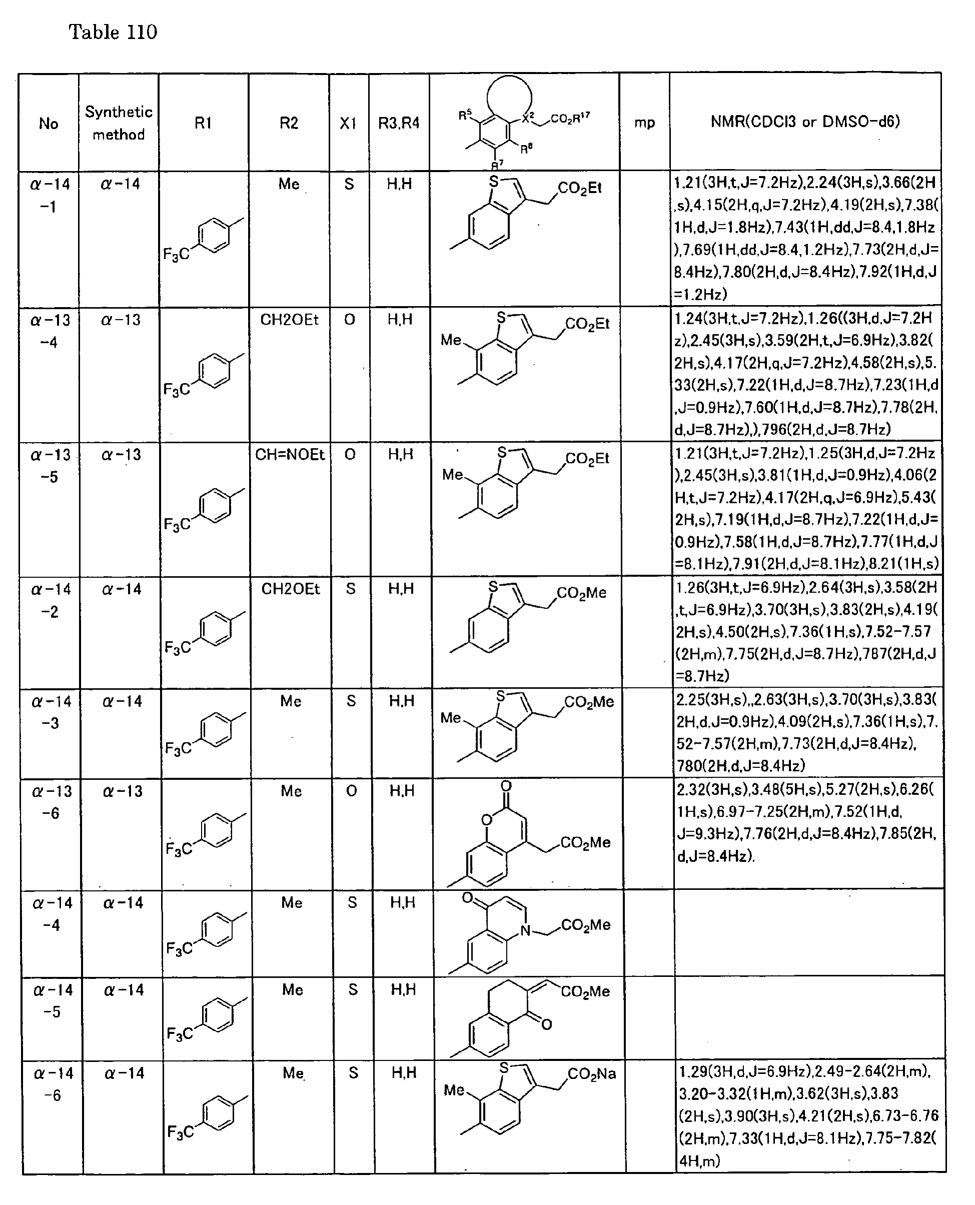

**Table 111**

| | | | | | |
|---|---|---|---|---|---|
| 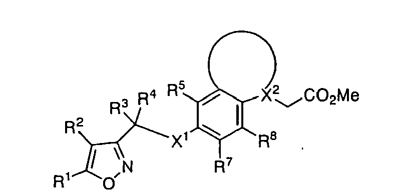 | | | | | |

| No | R1 | R2 | X1 | R3,R4 | 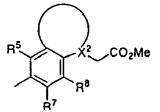 |
|---|---|---|---|---|---|
| AAA-1 | 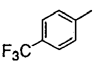 | Me | O | H,H | 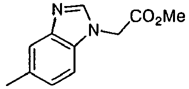 |
| AAA-2 | 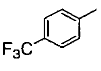 | Me | S | H,H | 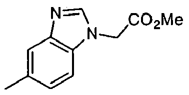 |
| AAA-3 | 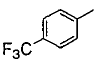 | Me | O | H,H | 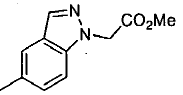 |
| AAA-4 | 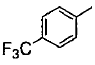 | Me | S | H,H | 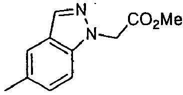 |
| AAA-5 | 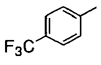 | Me | O | H,H | 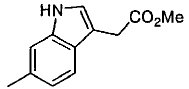 |
| AAA-6 | 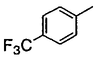 | Me | S | H,H | 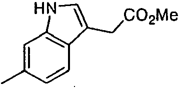 |
| AAA-7 | 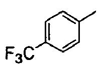 | Me | O | H,H | 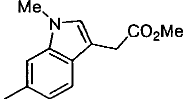 |
| AAA-8 | 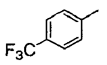 | Me | S | H,H | 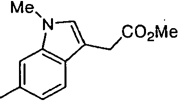 |
| AAA-9 | 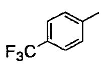 | Me | O | H,H | 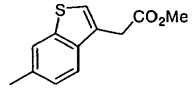 |
| AAA-11 | 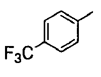 | Me | O | H,H | 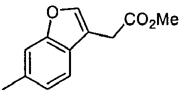 |
| AAA-12 | 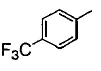 | Me | S | H,H | 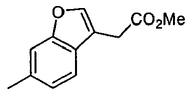 |

**Table 112**

| No | R1 | R2 | X1 | R3,R4 | 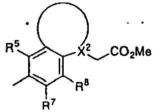 |
|---|---|---|---|---|---|
| AAA-13 | 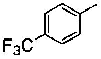 | Me | O | H,H | 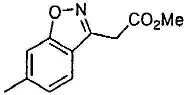 |
| AAA-14 | 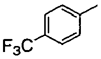 | Me | S | H,H | 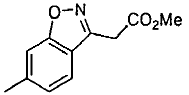 |
| AAA-15 | 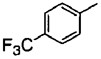 | Me | O | H,H | 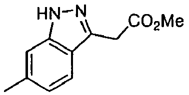 |
| AAA-16 | 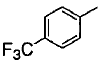 | Me | S | H,H | 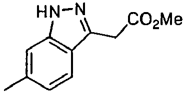 |
| AAA-17 | 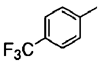 | Me | O | H,H | 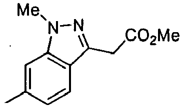 |
| AAA-18 | 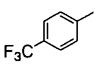 | Me | S | H,H | 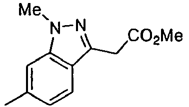 |
| AAA-19 | 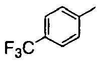 | Me | O | H,H | 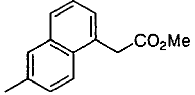 |
| AAA-20 | 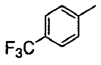 | Me | S | H,H | 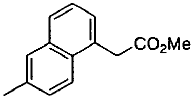 |
| AAA-21 | 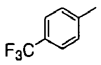 | Me | O | H,H | 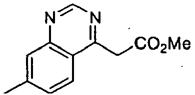 |
| AAA-22 | 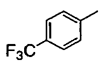 | Me | S | H,H | 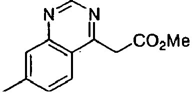 |
| AAA-23 | 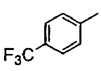 | Me | O | H,H | 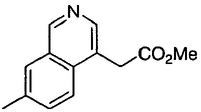 |
| AAA-24 | 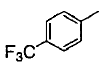 | Me | S | H,H | 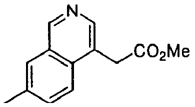 |
| AAA-25 | 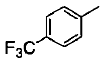 | Me | O | H,H | 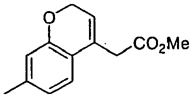 |

**Table 113**

| No | R1 | R2 | X1 | R3,R4 | 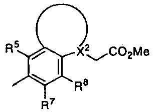 |
|---|---|---|---|---|---|
| AAA-26 | 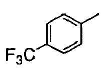 | Me | S | H,H | 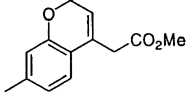 |
| AAA-27 | 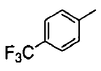 | Me | O | H,H | 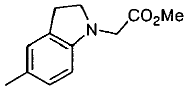 |
| AAA-28 | 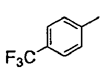 | Me | S | H,H | 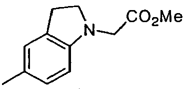 |
| AAA-29 | 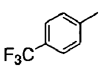 | Me | O | H,H | 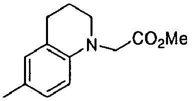 |
| AAA-30 | 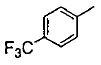 | Me | S | H,H | 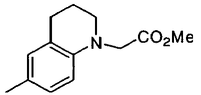 |
| AAA-31 | 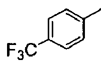 | Me | O | H,H | 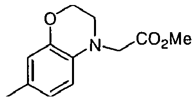 |
| AAA-32 | 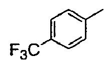 | Me | S | H,H | 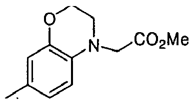 |
| AAA-35 | 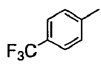 | Me | O | H,H | 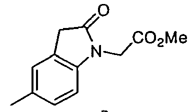 |
| AAA-36 | 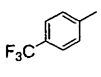 | Me | S | H,H | 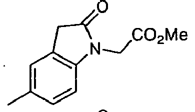 |
| AAA-37 | 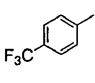 | Me | O | H,H | 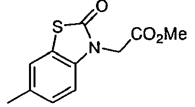 |
| AAA-38 | 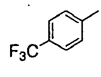 | Me | S | H,H | 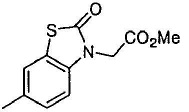 |
| AAA-39 | 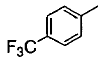 | Me | O | H,H | 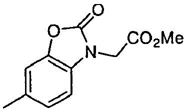 |
| AAA-40 | 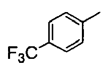 | Me | S | H,H | 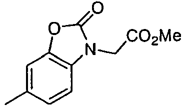 |

**Table 114**

| No | R1 | R2 | X1 | R3,R4 | 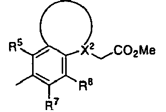 |
|---|---|---|---|---|---|
| AAA-42 | 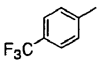 | Me | S | H,H | 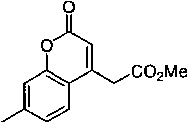 |
| AAA-43 | 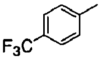 | Me | O | H,H | 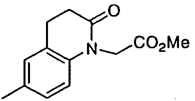 |
| AAA-44 | 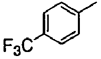 | Me | S | H,H | 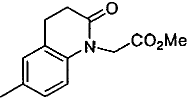 |
| AAA-45 | 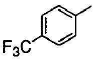 | Me | O | H,H | 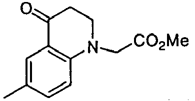 |
| AAA-46 | 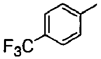 | Me | S | H,H | 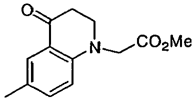 |
| AAA-47 | 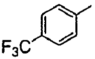 | Me | O | H,H | 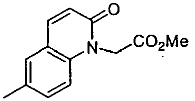 |
| AAA-48 | 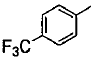 | Me | S | H,H | 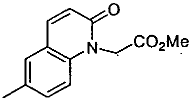 |
| AAA-49 | 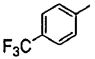 | Me | O | H,H | 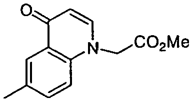 |
| AAA-50 | 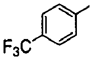 | Me | S | H,H | 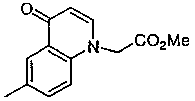 |

**Table 115**

| | | | | | | | | |
|---|---|---|---|---|---|---|---|---|
| 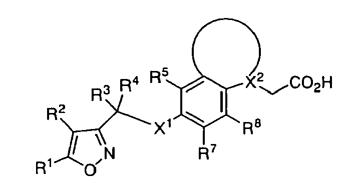 | | | | | | | | |

| No | Synthetic method | R1 | R2 | X1 | R3,R4 | 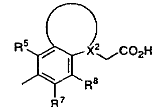 | mp | NMR(CDCl3 or DMSO-d6) |
|---|---|---|---|---|---|---|---|---|
| β-6-1 | β-6 | 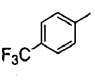 | Me | O | H,H | 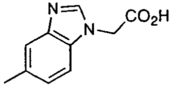 | 221-222 | 2.37(3H,s),4.95(2H,s),5.27(2H,s),7.09(2 H,m),7.66(1H,d,J=8.7Hz),7.78(2H,d,J=8. 4Hz),7.88(2H,d,J=8.1Hz),8.11(1H,s) |
| β-6-2 | β-6 | 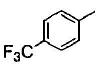 | Me | O | H,H | 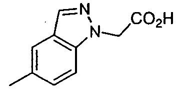 | 237-238.5 | 2.35(3H,s),5.12(2H,s),5.25(2H,s),7.18(1H,m),7.33(1H,m),7.75-7.98(4H,m),7.98(1H,s) |
| β-6-3 | β-6 | 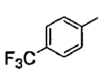 | Me | O | H,H | 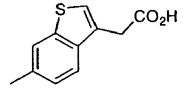 | 163-164 | 2.33(3H,s),3.87(2H,s),5.27(2H,s),7.16(1H,dd,J=8.7,2.4Hz),7.21(1H,s),7.51(1H,d, J=2.4Hz),7.68(1H,d,J=8.7Hz).7.76(2H,d, J=8.4Hz),7.85(2H,d,J=8.4Hz) |
| β-7-1 | β-7 | 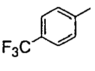 | Me | S | H,H | 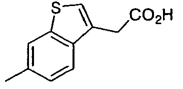 | 143 | 2.27(3H,s),3.87(2H,s),4.18(2H,s),7.38(1H,d,J=1.8Hz),7.43(1H,dd,J=8.4,1.8Hz),7.67(1H,d,J=8.4Hz),7.73(2H,d,J=8.4Hz).7.80(2H,d,J=8.4Hz),7.92(1H,d,J=1.2Hz) |
| β-6-4 | β-6 | 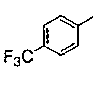 | CH2OEt | O | H,H | 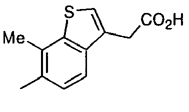 | 181-182 | 1.33(3H,t,J=7.2Hz),2.45(3H,s),3.59(2H,t, J=7.2Hz),3.86(2H,d,J=0.9Hz).4.58(2H,s) ,5.32(2H,s),7.23(1H,d,J=8.7Hz),7.24(1H, d,J=0.9Hz)),7.58(1H,d,J=8.7Hz),7.77(2H ,d,J=8.7Hz),),795(2H,d,J=8.7Hz) |
| β-6-5 | β-6 | 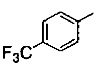 | CH=NOEt | O | H,H | 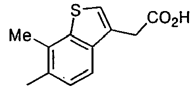 | 160-162 | 1.20(3H,t,J=6.9Hz),2.45(3H,s),3.86(1H,d ,J=0.9Hz),4.05(2H,t,J=6.9Hz),5.43(2H,s ),7.19(1H,d,J=8.1Hz),7.24(1H,d,J=0.9Hz ),7.56(1H.d,J=8.1Hz),7.77(2H,d,J=8.1Hz ),7.90(2H,d,J=8.1Hz),),8.21(1H,s) |
| β-7-2 | β-7 | 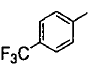 | CH2OEt | S | H,H | 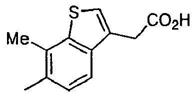 | 163-164 | 1.25(3H,t,J=6.9Hz),2.64(3H,s),3.57(2H,q ,J=6.9Hz),3.86(2H,s),4.19(2H,s),4.50(2H .s).7.38(1H,s),7.52-7.57(2H,m),7.74 (2H,d,J=8.4Hz),7.86(2H,d,J=8.4Hz) |
| β-7-3 | β-7 | 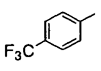 | Me | S | H,H | 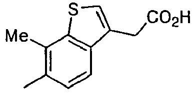 | 190-191 | 2.25(3H,s),2.63(3H,s),3.82(2H,s),4.09(2 H,s),7.39(1H,s),7.51-7.60(2H,m),7.74 (2H,d,J=8.7Hz),),7.80(2H,d,J=8.7Hz) |
| β-6-6 | β-6 | 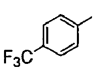 | Me | O | H,H | 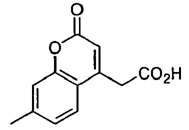 | 176-177 | 2.32(3H,s),3.78(2H,s),5.27(2H,s),6.30(1H,s),6.98-7.04(2H,m),7.52(1H,d, J=9.6Hz),7.76(2H,d,J=8.4Hz),7.85(2H,d, J=8.4Hz). |
| β-7-4 | β-7 | 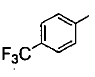 | Me | S | H,H | 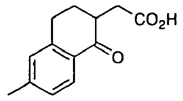 | | 1.97(1H,m),2.24(1H,m),2.30(3H,s),2.48(1H,m),2.98(2H,m),3.06(2H,m),4.25(2H,s) ,7.27(2H,m),7.72~7.83(4H,m),7.94(1H,d, J=8.1Hz) |

**Table 116**

| No | Synthetic method | R1 | R2 | X1 | R3,R4 | 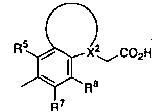 | mp | NMR(CDCl3 or DMSO-d6) |
|---|---|---|---|---|---|---|---|---|
| β-7-5 | β-7 | 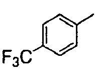 | Me | s | H,H | 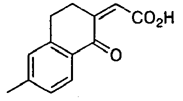 | | 2.30(3H,s),3.00(2H,t,J=6.9Hz),3.42(2H,t d,J=6.3Hz,1.8Hz),4.27(2H,s),6.89(2H,t,J =1.8Hz),7.33(1H,m),7.74(1H,d,J=8.4Hz), 7.81(1H,d,J=8.7Hz) |

**Table 117**

| | | | | | |
|---|---|---|---|---|---|
| 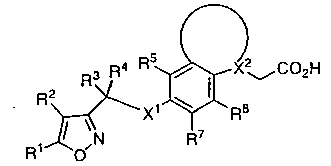 | | | | | |

| No | R1 | R2 | X1 | R3,R4 | 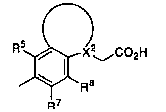 |
|---|---|---|---|---|---|
| BBB-2 | 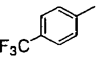 | Me | S | H,H | 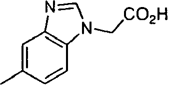 |
| BBB-3 | 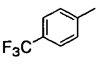 | Me | O | H,H | 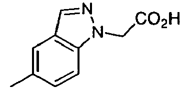 |
| BBB-4 | 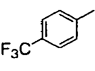 | Me | S | H,H | 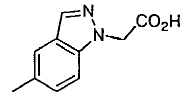 |
| BBB-5 | 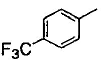 | Me | O | H,H | 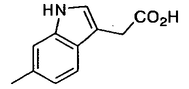 |
| BBB-6 | 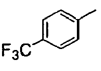 | Me | S | H,H | 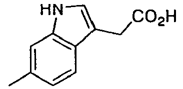 |
| BBB-7 | 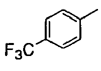 | Me | O | H,H | 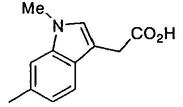 |
| BBB-8 | 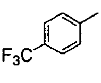 | Me | S | H,H | 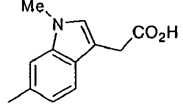 |
| BBB-9 | 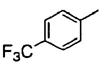 | Me | O | H,H | 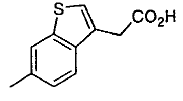 |

**Table 118**

| No | R1 | R2 | X1 | R3,R4 | 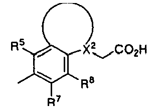 |
|---|---|---|---|---|---|
| BBB-11 | 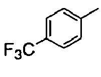 | Me | O | H,H | 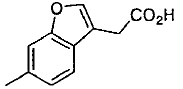 |
| BBB-12 | 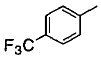 | Me | S | H,H | 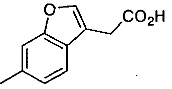 |
| BBB-13 | 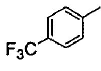 | Me | O | H,H | 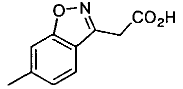 |
| BBB-14 | 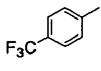 | Me | S | H,H | 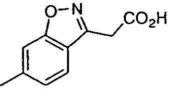 |
| BBB-15 | 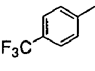 | Me | O | H,H | 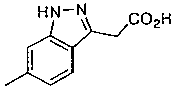 |
| BBB-16 | 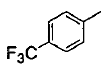 | Me | S | H,H | 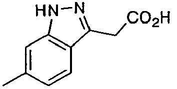 |
| BBB-17 | 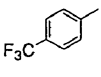 | Me | O | H,H | 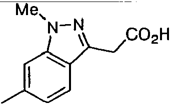 |
| BBB-18 | 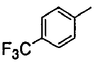 | Me | S | H,H | 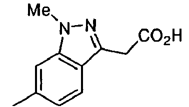 |
| BBB-19 | 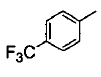 | Me | O | H,H | 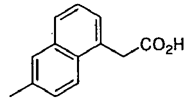 |
| BBB-20 | 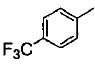 | Me | S | H,H | 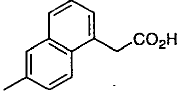 |
| BBB-21 | 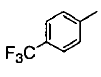 | Me | O | H,H | 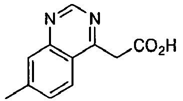 |
| BBB-22 | 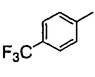 | Me | S | H,H | 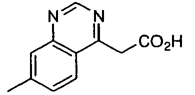 |
| BBB-23 | 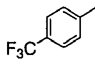 | Me | O | H,H | 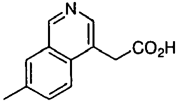 |

**Table 119**

| No | R1 | R2 | X1 | R3,R4 | 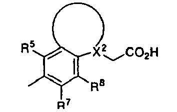 |
|---|---|---|---|---|---|
| BBB-24 | 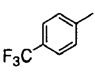 | Me | S | H,H | 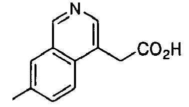 |
| BBB-25 | 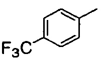 | Me | O | H,H | 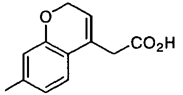 |
| BBB-26 | 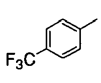 | Me | S | H,H | 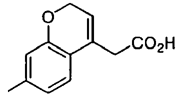 |
| BBB-27 | 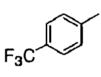 | Me | O | H,H | 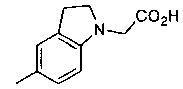 |
| BBB-28 | 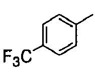 | Me | S | H,H | 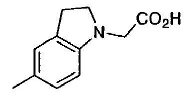 |
| BBB-29 | 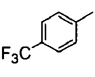 | Me | O | H,H | 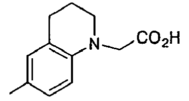 |
| BBB-30 | 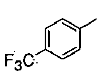 | Me | S | H,H | 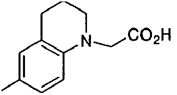 |
| BBB-31 | 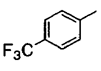 | Me | O | H,H | 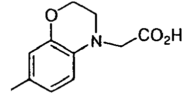 |
| BBB-32 | 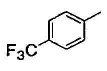 | Me | S | H,H | 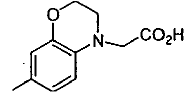 |
| BBB-35 | 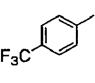 | Me | O | H,H | 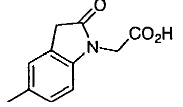 |
| BBB-36 | 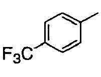 | Me | S | H.H | 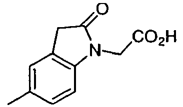 |
| BBB-37 | 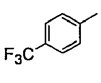 | Me | O | H,H | 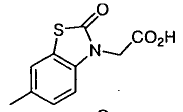 |
| BBB-38 | 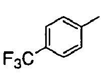 | Me | S | H,H | 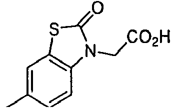 |

**Table 120**

| No. | R1 | R2 | X1 | R3,R4 | 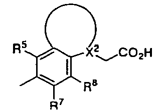 |
|---|---|---|---|---|---|
| BBB-39 | 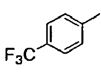 | Me | O | H,H | 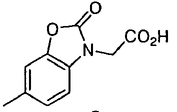 |
| BBB-40 | 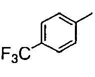 | Me | S | H,H | 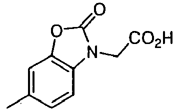 |
| BBB-42 | 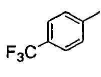 | Me | S | H,H | 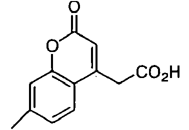 |
| BBB-43 | 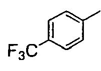 | Me | O | H,H | 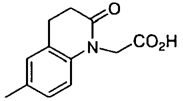 |
| BBB-44 | 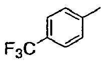 | Me | S | H,H | 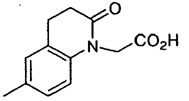 |
| BBB-45 | 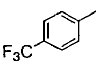 | Me | O | H,H | 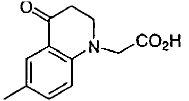 |
| BBB-46 | 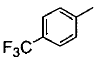 | Me | S | H,H | 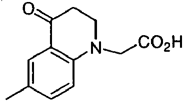 |
| BBB-47 | 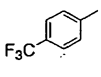 | Me | O | H,H | 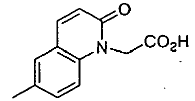 |
| BBB-48 | 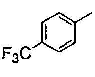 | Me | S | H,H | 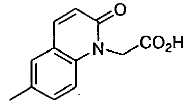 |
| BBB-49 | 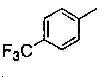 | Me | O | H,H | 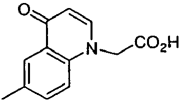 |
| BBB-50 | 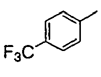 | Me | S | H,H | 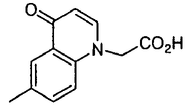 |

**Table 121**

| | | | | | | | | |
|---|---|---|---|---|---|---|---|---|
| 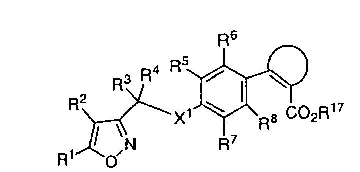 | | | | | | | | |

| No | Sythetic method | R1 | R2 | X1 | R3,R4 | 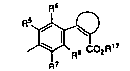 | mp | NMR(CDCl3 or DMSO-d6) |
|---|---|---|---|---|---|---|---|---|
| α-12 -1 | α-12 | 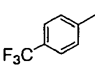 | Me | S | H,H | 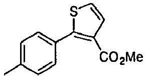 | | 2.29(3H,s),3.74(3H,s),4.21(2H,s),7.23-7.5 2(6H,m),7.74(2H,d,J=8.7Hz), 7.83(2H,d,J=8.7Hz). |
| α-12 -2 | α-12 | 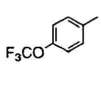 | CH2OEt | S | H,H | 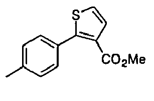 | | 1.27(3H,t,J=6.9Hz), 3.60 (2H,q,J=6.9Hz), 3.74(3H,s), 4.29(2H,s), 4.53(2H,s), 7.24(2H,d,J=5.4Hz), 7.33(2H,d,J=9.0Hz), 7.43(2H,s), 7.49(2H,d,J=5.4Hz), 7.79(2H,d,J=9.0Hz) |
| α-12 -3 | α-12 | 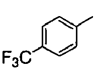 | CH2OEt | S | H,H | 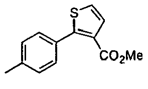 | | 1.29(3H,t,J=6.93Hz),3.61 (3H,t,J=6.9Hz),3 .74(3H,s),4.30(2H,s),4.55(2H,s),7.24(1H,d, J=5.4Hz),7.44(4H,s),7.50(1H,d,J=5.4Hz),7 .76(2H,d,J=8.4Hz),7.88(2H,d,J=8.4Hz). |
| α-12 -4 | α-12 | 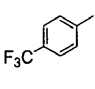 | CH2OnPr | S | H,H | 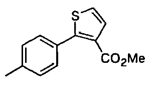 | | 0.97(3H,t,J=7.4Hz),1.57-1.73(2H,m), 3.51(3H,t,J=6.6Hz),3.74(3H,s),4.30(2H,s), 4.55(2H,s),7.24(1H,d,J=5.4Hz),7.44(4H,s), 7.50(1H,d,J=5.4Hz),7.75(2H,d,J=8.4Hz),7. 89(2H,d,J=8.4Hz). |
| α -XXX-1 | | 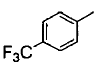 | Me | O | H,H | 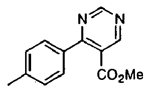 | | 1.21(3H,t,J=7.2Hz),2.33(3H,s),4.29(2H,q, J=7.2Hz),5.27(2H,s),7.13(2H,d,J=8.7Hz),7 .65(2H,d,J=8.7Hz),7.76(2H,d,J=8.7Hz),7.8 5(2H,d,J=8.7Hz),9.03(1H,s),9.35(1H,s) |
| α -XXX-2 | | 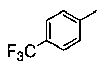 | Me | O | H,H | 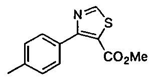 | | 2.34(3H,s),3.85(3H,s),5.26(2H,s),7.11(2H, d,J=8.7Hz):7.76(2H,d,J=8.4Hz),7.81 (2H,d, J=8.4Hz),7.85(2H,d,J=8.7Hz)8.88(1H,s) |
| α -XXX-3 | | 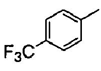 | Me | O | H,H | 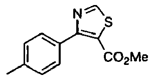 | | 2.33(3H,s),2.74(3H,s),3.81 (3H,m),5.25(2H ,s),7.09(2H,d,J=9.0Hz),7.76(4H,d,J=8.7Hz ),7.85(2H,d,J=8.1Hz) |
| α -XXX-4 | | 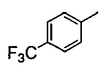 | Me | S | H,H | 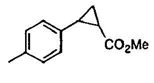 | | 1.28(1H,m),1.60(1H,m),1.87(1H,m),2.27(3 H,s),2.48(1H,m),3.71(3H,s),4.10(2H,s),7.0 2(2H,d,J=8.4Hz),7.32(2H.d,J=8.4Hz),7.74 (2H,d,J=8.1Hz),7.81(2H,d,J=8.1Hz) |

**Table 122**

| | | | | | |
|---|---|---|---|---|---|
| 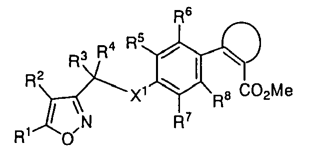 | | | | | |

| No | R1 | R2 | X1 | R3,R4 | 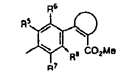 |
|---|---|---|---|---|---|
| AAAA-1 | 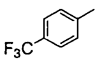 | Me | O | H,H | 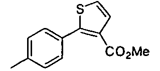 |
| AAAA-2 | 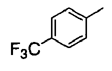 | MeOCH2 | O | H,H | 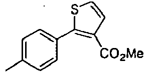 |
| AAAA-3 | 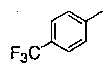 | MeOCH2 | S | H,H | 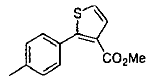 |
| AAAA-4 | 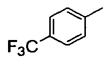 | EtOCH2 | O | H,H | 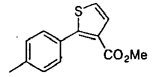 |
| AAAA-5 | 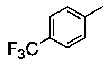 | EtOCH2 | S | H,H | 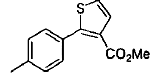 |
| AAAA-7 | 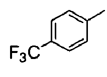 | Me | S | H,H | 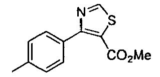 |
| AAAA-8 | 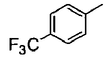 | Me | O | H,H | 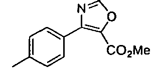 |
| AAAA-9 | 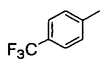 | Me | S | H,H | 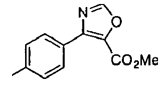 |
| AAAA-10 | 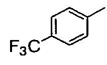 | Me | O | H,H | 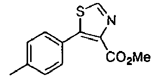 |
| AAAA-11 | 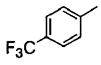 | Me | S | H,H | 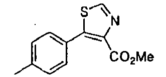 |
| AAAA-12 | 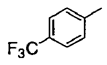 | Me | O | H,H | 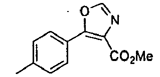 |
| AAAA-13 | 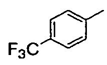 | Me | S | H,H | 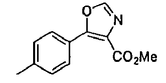 |
| AAAA-14 | 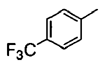 | Me | O | H,H | 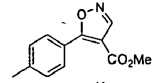 |
| AAAA-15 | 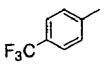 | Me | S | H,H | 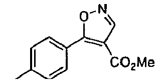 |

**Table 123**

| No | R1 | R2 | X1 | R3,R4 | 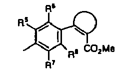 |
|---|---|---|---|---|---|
| AAAA-16 | 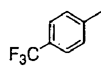 | Me | O | H,H | 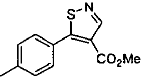 |
| AAAA-17 | 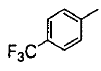 | Me | S | H,H | 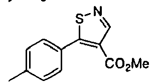 |
| AAAA-18 | 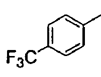 | Me | O | H,H | 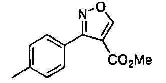 |
| AAAA-19 | 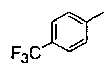 | Me | S | H,H | 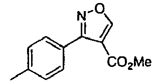 |
| AAAA-20 | 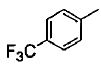 | Me | O | H,H | 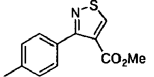 |
| AAAA-21 | 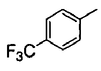 | Me | S | H,H | 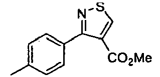 |
| AAAA-22 | 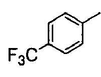 | Me | O | H,H | 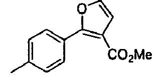 |
| AAAA-23 | 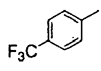 | Me | S | H,H | 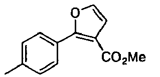 |
| AAAA-25 | 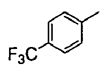 | Me | S | H,H | 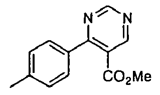 |
| AAAA-26 | 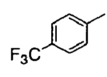 | Me | O | H,H | 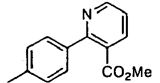 |
| AAAA-27 | 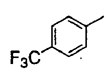 | Me | S | H,H | 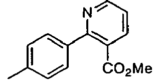 |
| AAAA-28 | 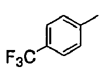 | Me | O | H,H | 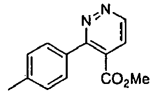 |
| AAAA-29 | 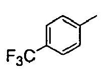 | Me | S | H,H | 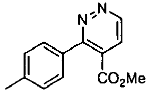 |
| AAAA-30 | 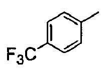 | Me | O | H,H | 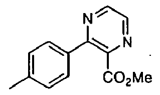 |
| AAAA-31 | 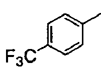 | Me | S | H,H | 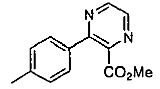 |

**Table 124**

| | | | | | | | | |
|---|---|---|---|---|---|---|---|---|
| 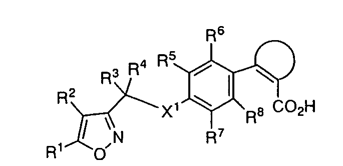 | | | | | | | | |

| No | Synthetic method | R1 | R2 | X1 | R3,R4 | 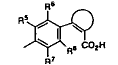 | mp | NMR(CDCl3 or DMSO-d6) |
|---|---|---|---|---|---|---|---|---|
| β-5-1 | β-5 | 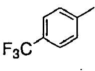 | Me | S | H,H | 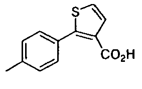 | 139-141 | 2.52(3H,s),4.20(2H,s),7.26(1H,d,J=5.4H z),7.41(2H,d,J=8.7Hz),7.45(2H,d,J=8.7H z),7.54(1H,d,J=5.4Hz),7.72(2H,d,J=8.4H z),7.81(2H,d,J=8.4Hz). |
| β-5-2 | β-5 | 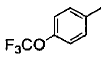 | CH2OEt | S | H,H | 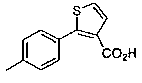 | 106-107 | 1.26(3H,t,J=6.9Hz), 3.59(2H,q,J=6.9Hz), 4.29(2H,s), 4.52(2H,s), 7.24-7.54(8H,m), 7.79(2H,d,J=9.0Hz) |
| β-5-3 | β-5 | 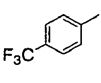 | CH2OEt | S | H,H | 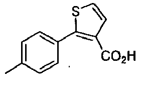 | 127-128 | 1.27(3H,t,J=6.9Hz),3.60(3H,t,J=6.9Hz), 4.31(2H,s),4.54(2H,s),7.24-7.29(1H,m), 7.40-7.56(5H,m),7.75(2H,d,J=8.4Hz), 7.87(2H,d,J=8.4Hz). |
| β-5-4 | β-5 | 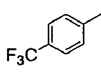 | CH2OnPr | S | H,H | 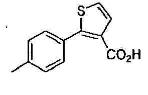 | 132-133 | 0.96(3H,t,J=7.3Hz),1.57-1.74(2H,m), 3.50(3H,t,J=7.3Hz),4.30(2H,s), 4.54(2H,s),7.25(1H,d,J=5.4Hz),7.42(2H, d,J=8.7Hz),7.46(2H,d,J=8.7Hz),7.53(1H, d,J=5.4Hz),7.74(2H,d,J=8.1Hz),7.88(2H, d,J=8.1Hz). |
| β -XXX-1 | | 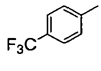 | Me | O | H,H | 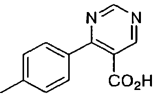 | 182 | 2.33(3H,s), 5.27(2H,s), 7.14(2H,d, J=6.9Hz),7.71-7.77(4H,m), 7.83(2H,d, J=8.4Hz), 9.18(1H,s), 9.37(1H,s) |
| β -XXX-2 | | 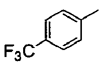 | Me | O | H,H | 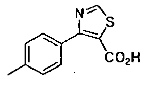 | 258-259 | 2.36(3H,s),5.27(2H,s),7.1 1(2H,m),7.80(4 H,m),7.86(2H,m),8.92(1H,s) |
| β -XXX-3 | | 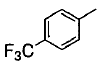 | Me | O | H,H | 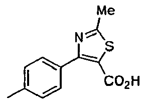 | 233234 | 2.31(3H,s),2.68(3H,s),5.34(2H,s),7.12(2 H,d,J=8.7Hz),7.74(2H,d,J=8.7Hz),7.93(2 H,d,J=8.4Hz),8.00(2H.d,J=8.4Hz) |
| β -5-5 | β-5 | 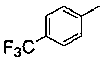 | Me | S | H,H | 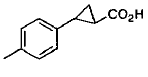 | 153-155 | 1.37(1H,m),1.63(1H,m),1.88(1H,m),2.27( 3H,s),2.51(1H,m),4.10(2H,s),7.04(2H,d,J =8.4Hz),7.33(2H,d,J=8.4Hz),7.74(2H,d,J =8.4Hz),7.82(2H,d,J=8.4Hz) |

**Table 125**

| | | | | | |
|---|---|---|---|---|---|
| 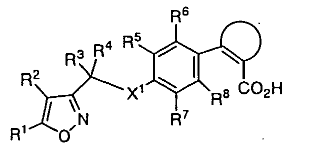 | | | | | |

| No | R1 | R2 | X1 | R3,R4 | 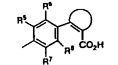 |
|---|---|---|---|---|---|
| BBBB-1 | 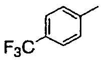 | Me | O | H,H | 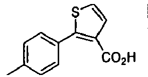 |
| BBBB-2 | 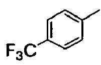 | MeOCH2 | O | H,H | 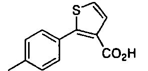 |
| BBBB-3 | 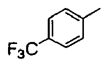 | MeOCH2 | S | H,H | 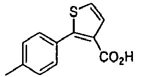 |
| BBBB-4 | 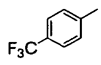 | EtOCH2 | O | H,H | 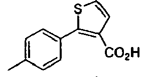 |
| BBBB-5 | 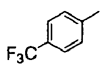 | EtOCH2 | S | H,H | 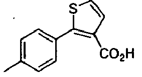 |
| BBBB-7 | 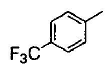 | Me | S | H.H | 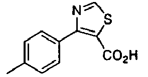 |
| BBBB-8 | 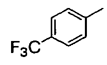 | Me | O | H,H | 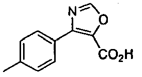 |
| BBBB-9 | 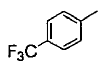 | Me | S | H,H | 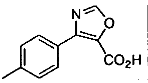 |
| BBBB-10 | 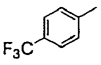 | Me | O | H,H | 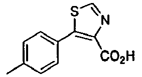 |
| BBBB-11 | 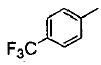 | Me | S | H,H | 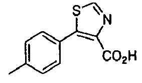 |
| BBBB-12 | 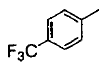 | Me | O | H,H | 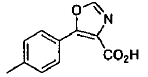 |
| BBBB-13 | 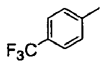 | Me | S | H,H | 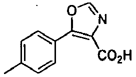 |
| BBBB-14 | 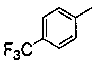 | Me | O | H,H | 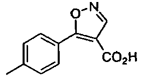 |
| BBBB-15 | 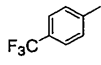 | Me | S | H,H | 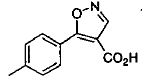 |

**Table 126**

| No | R1 | R2 | X1 | R3,R4 | 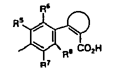 |
|---|---|---|---|---|---|
| BBBB-16 | 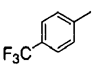 | Me | O | H,H | 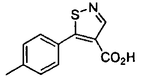 |
| BBBB-17 | 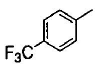 | Me | S | H,H | 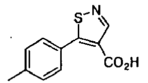 |
| BBBB-18 | 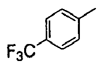 | Me | O | H,H | 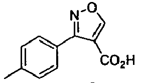 |
| BBBB-19 | 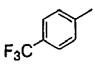 | Me | S | H,H | 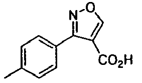 |
| BBBB-20 | 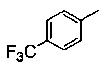 | Me | O | H,H | 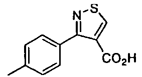 |
| BBBB-21 | 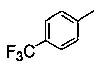 | Me | S | H,H | 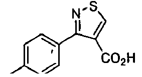 |
| BBBB-22 | 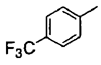 | Me | O | H,H | 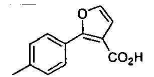 |
| BBBB-23 | 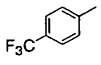 | Me | S | H,H | 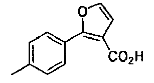 |
| BBBB-25 | 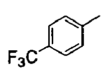 | Me | S | H,H | 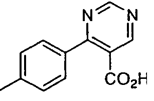 |
| BBBB-26 | 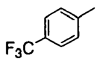 | Me | O | H,H | 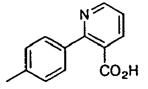 |
| BBBB-27 | 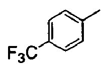 | Me | S | H,H | 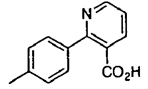 |
| BBBB-28 | 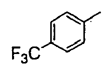 | Me | O | H,H | 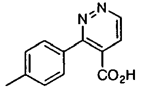 |
| BBBB-29 | 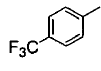 | Me | S | H,H | 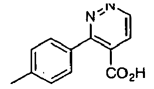 |
| BBBB-30 | 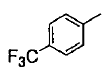 | Me | O | H,H | 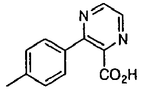 |
| BBBB-31 | 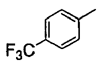 | Me | S | H,H | 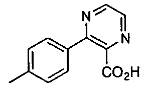 |

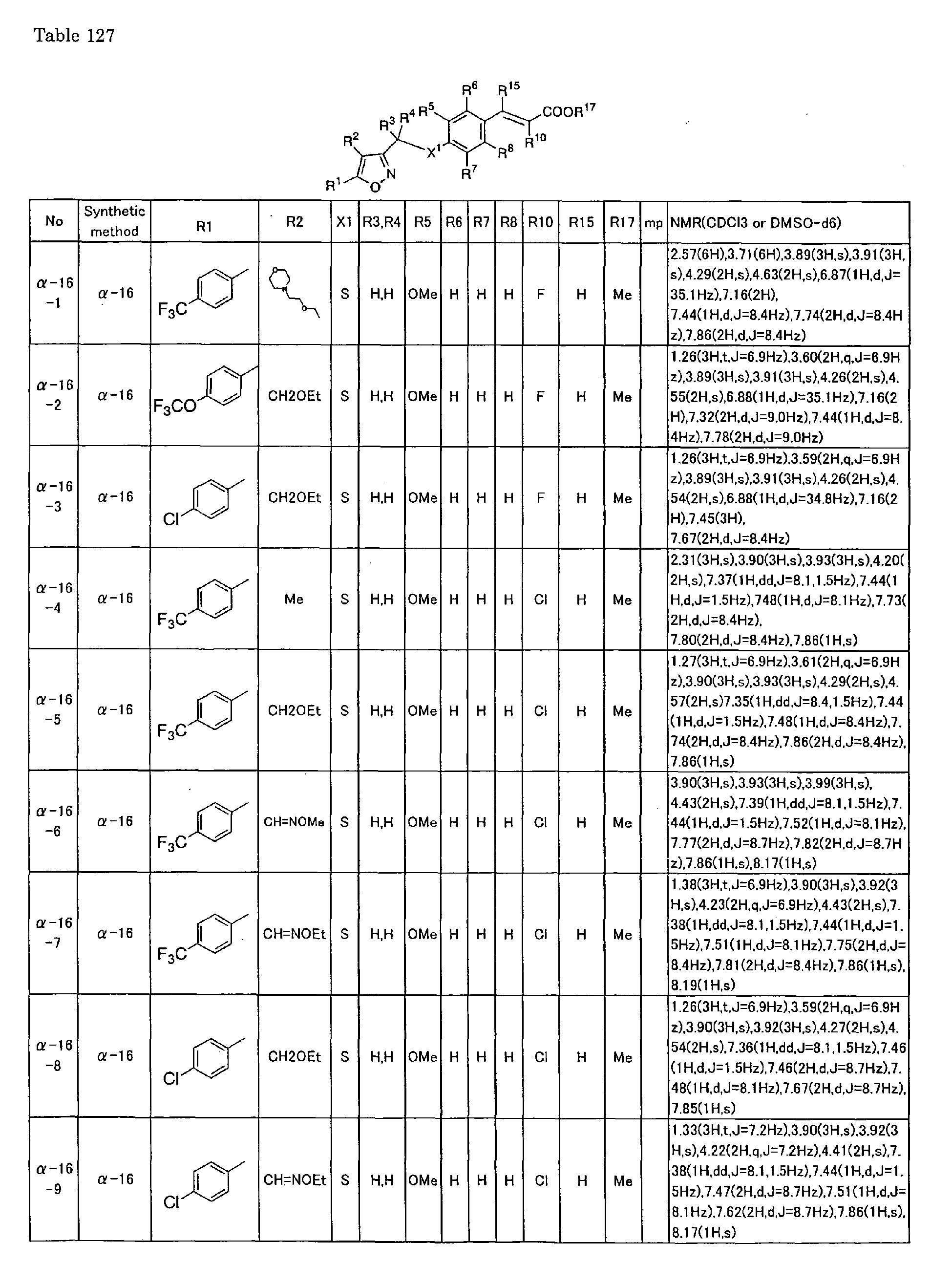

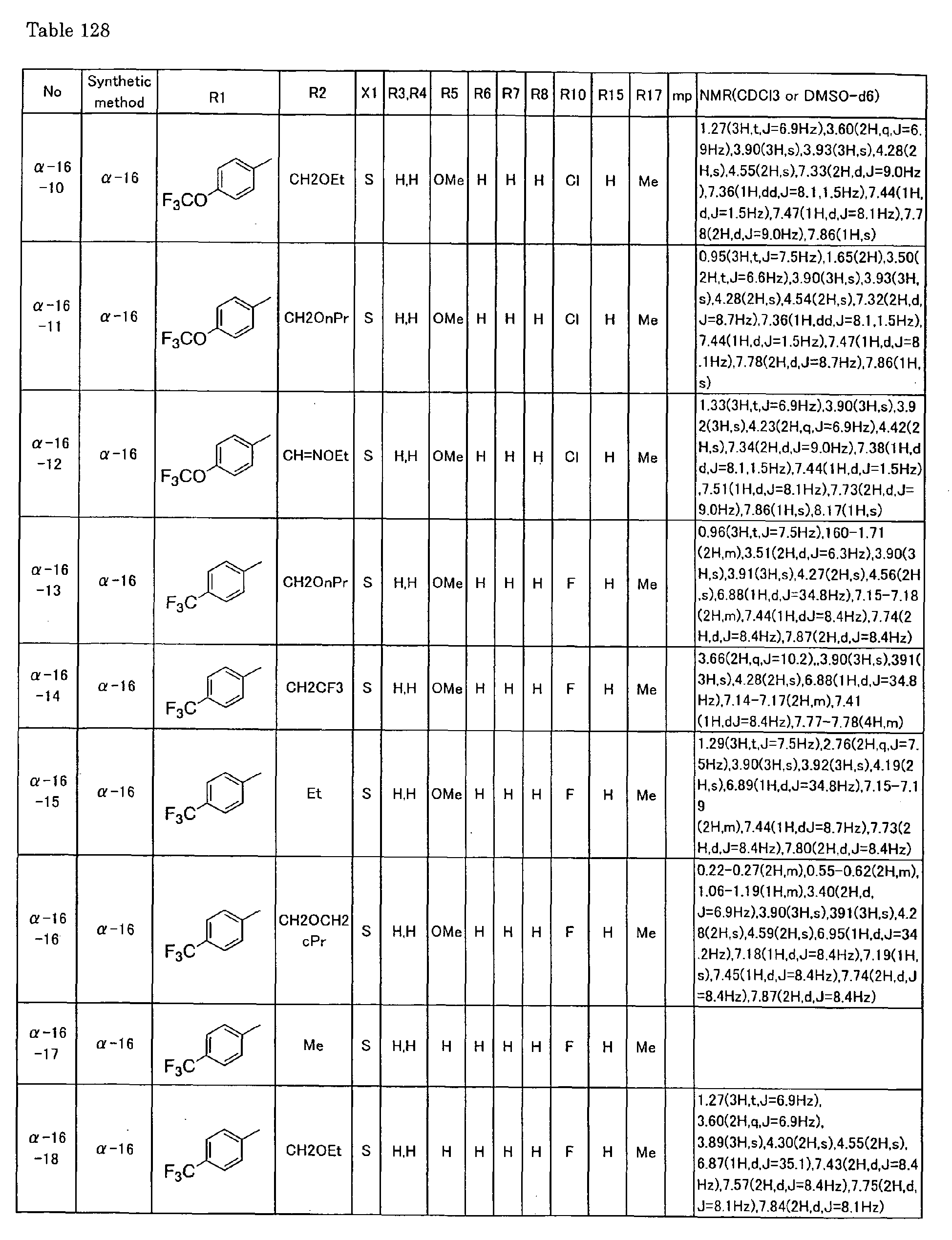

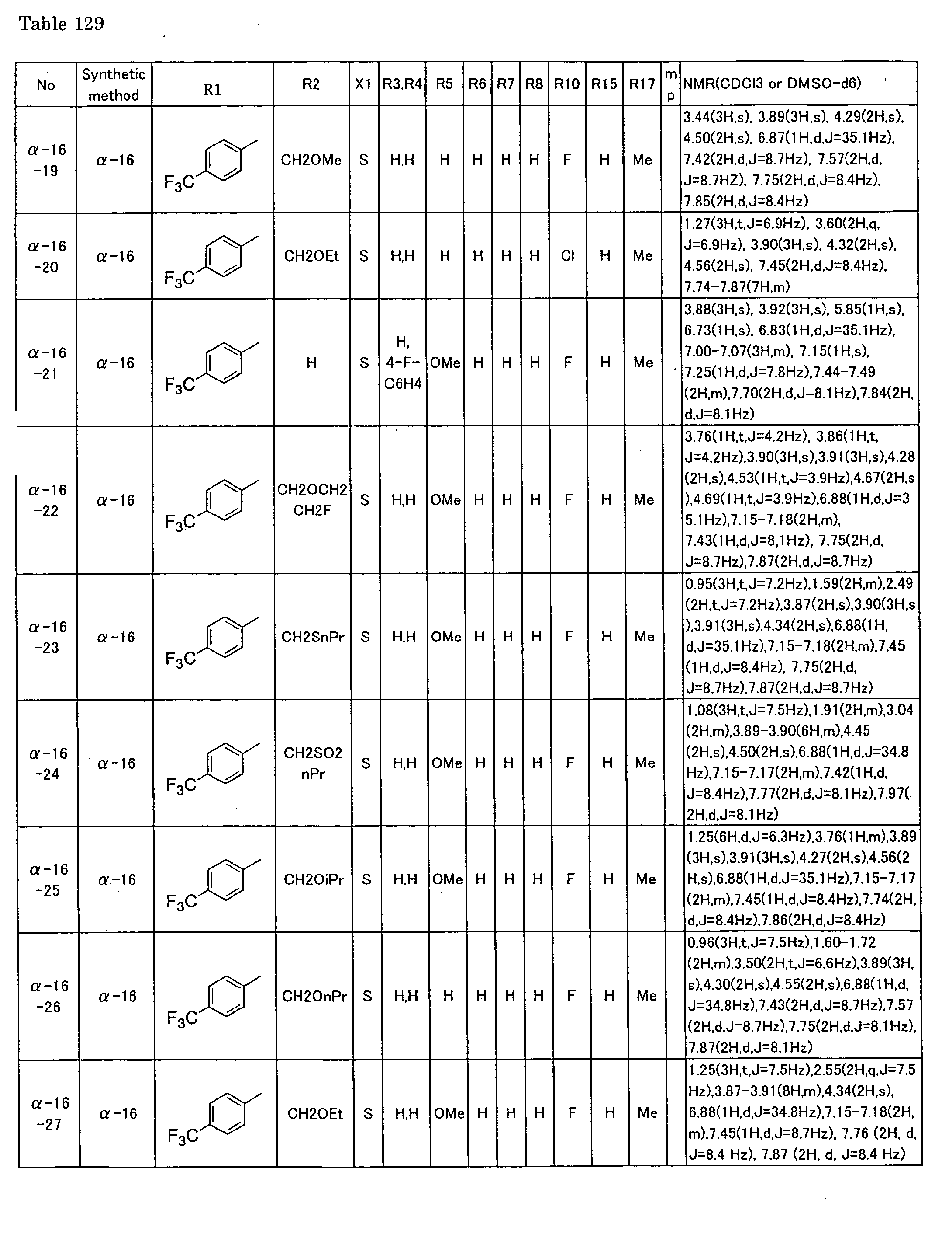

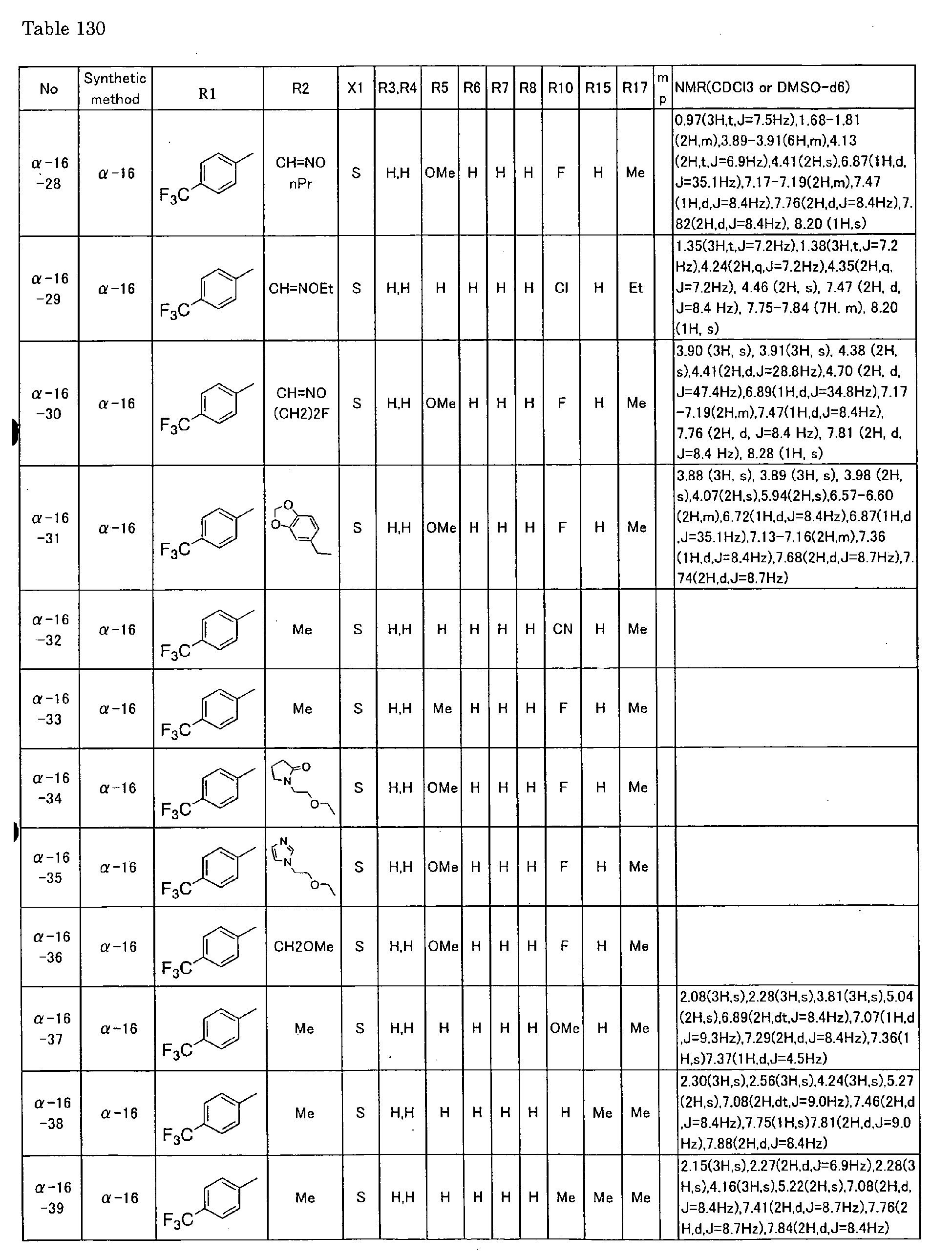

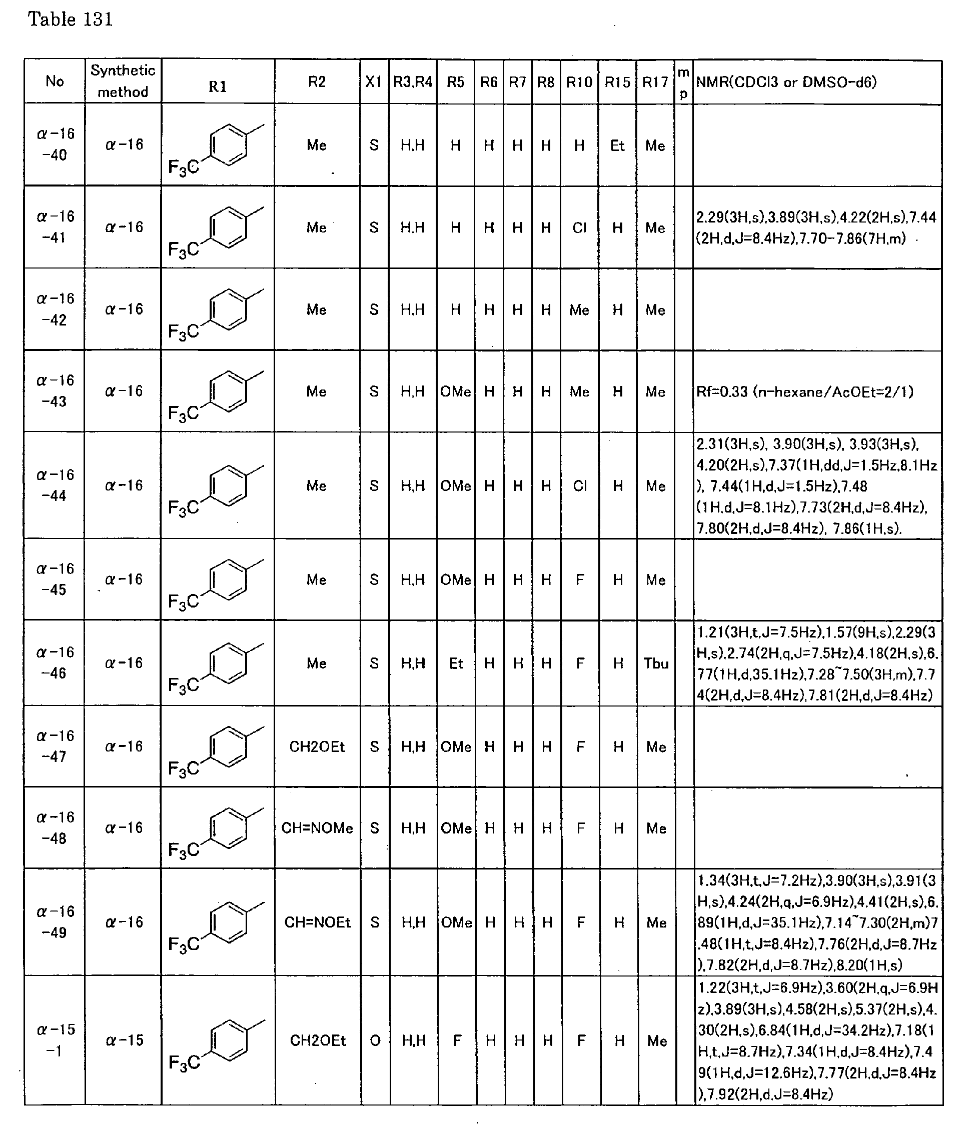

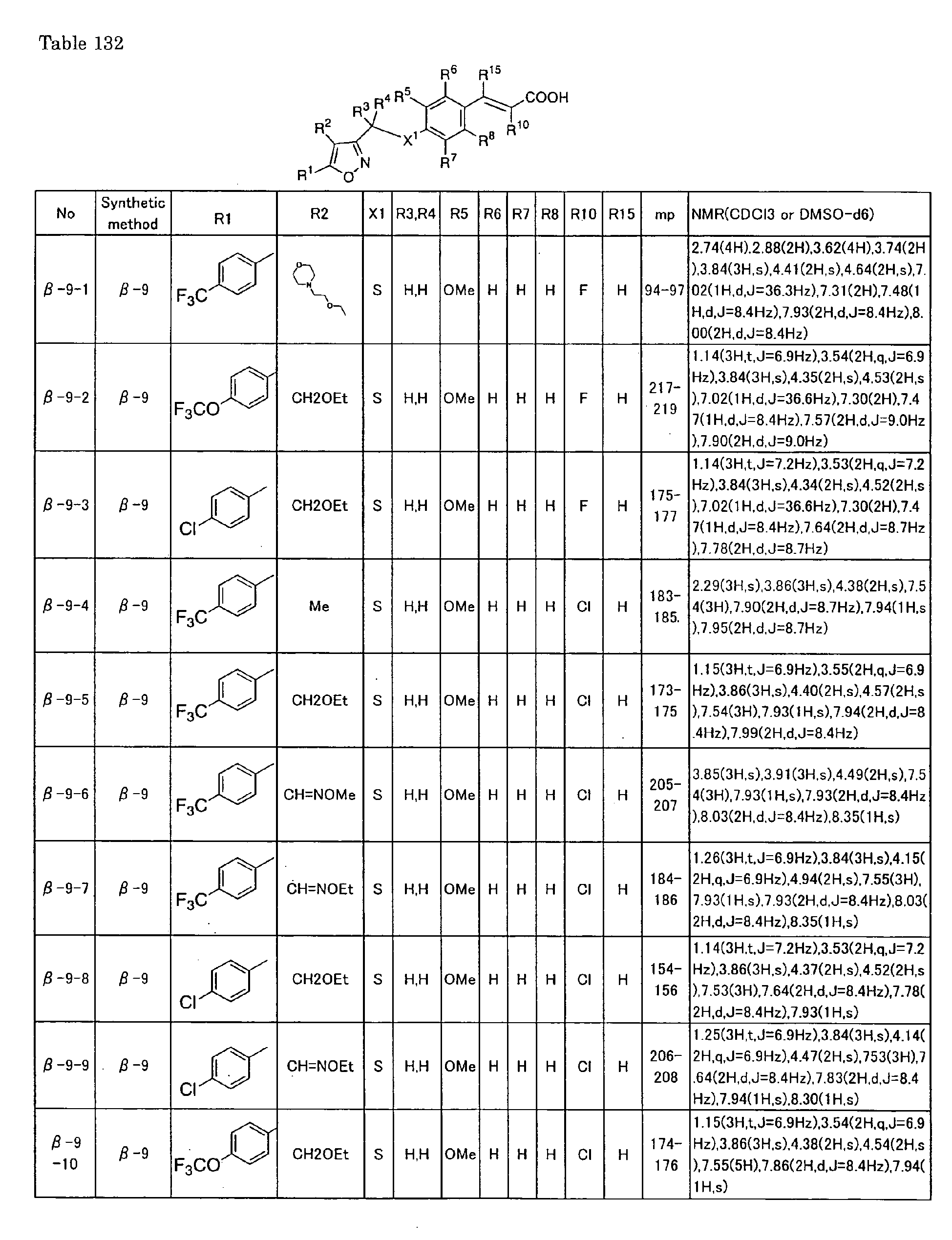

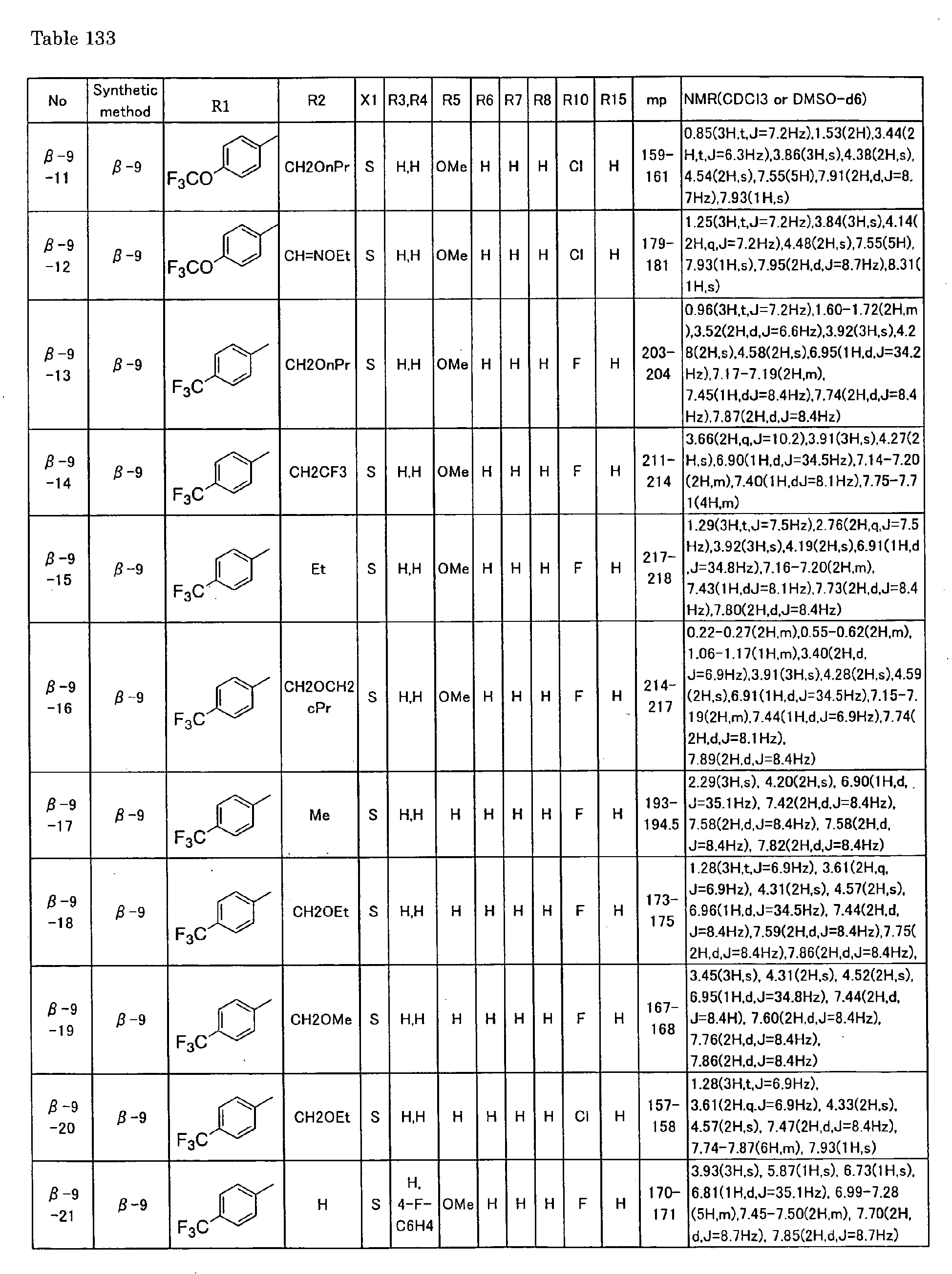

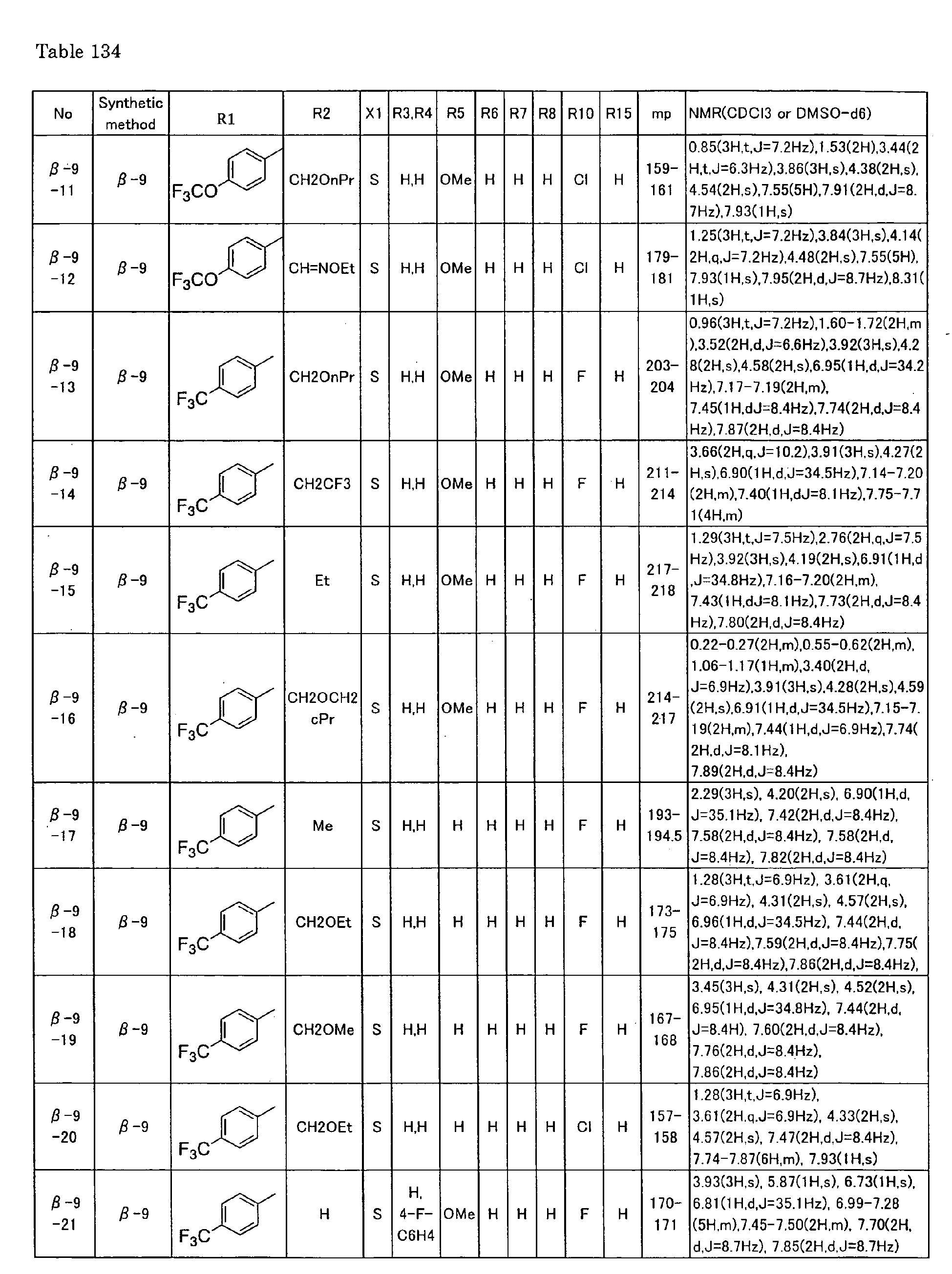

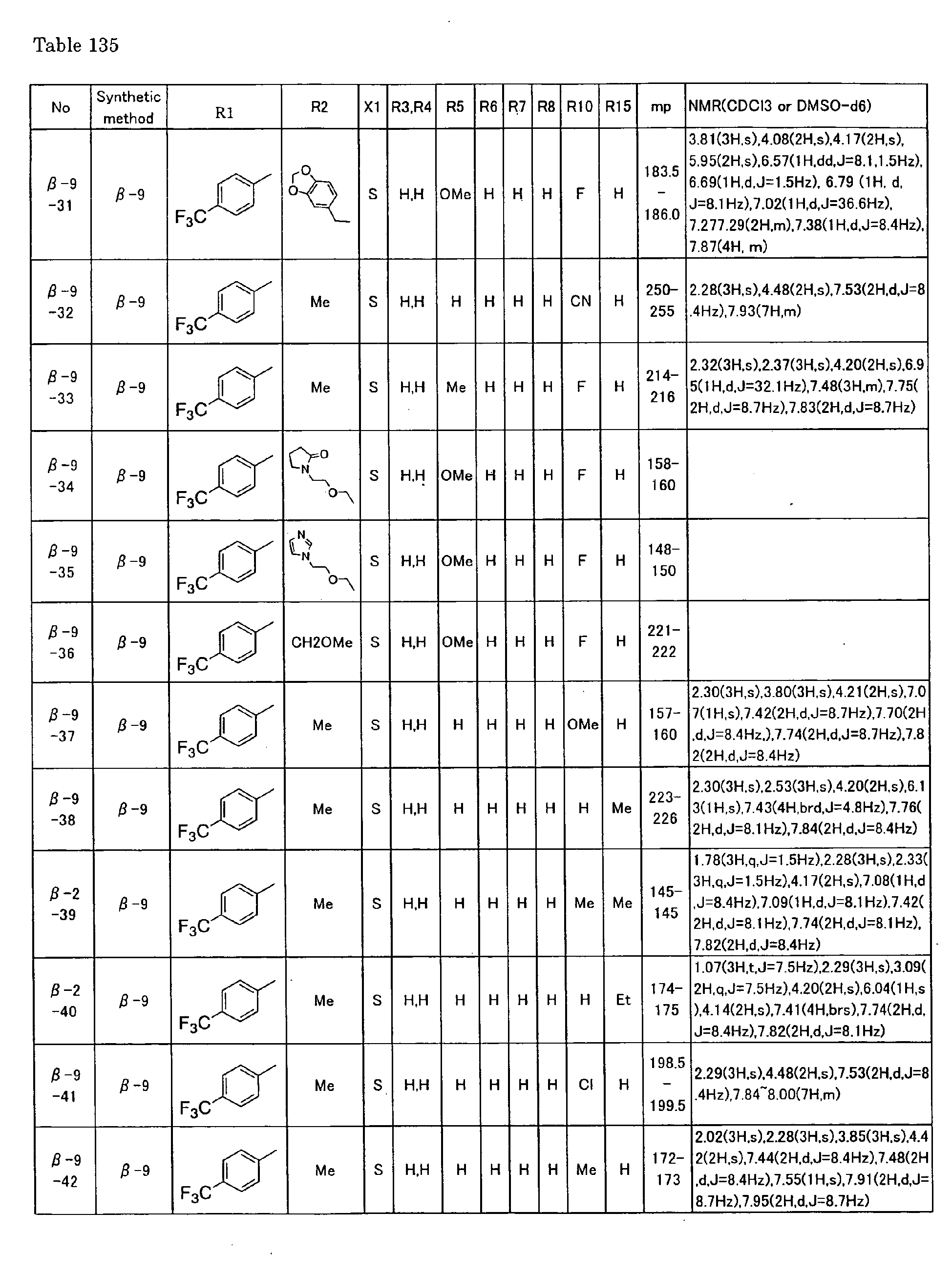

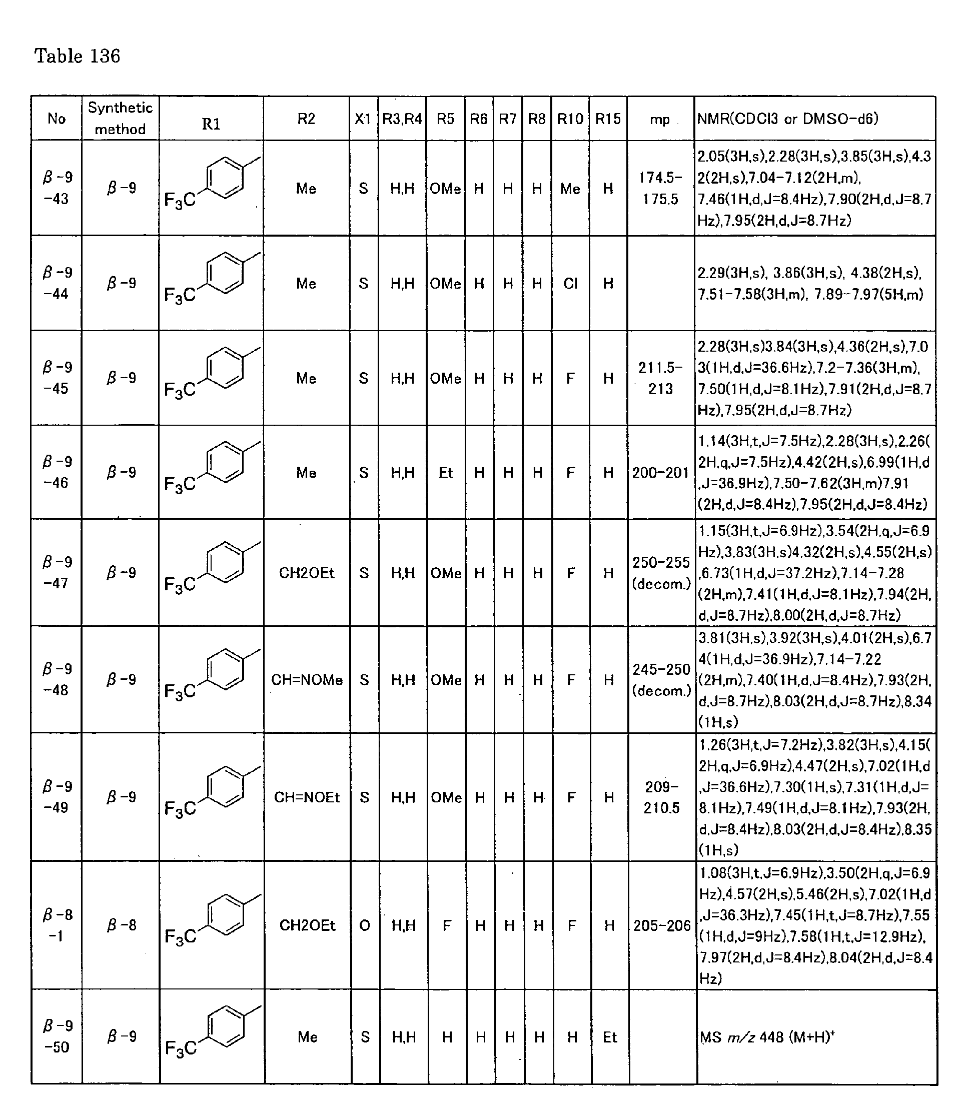

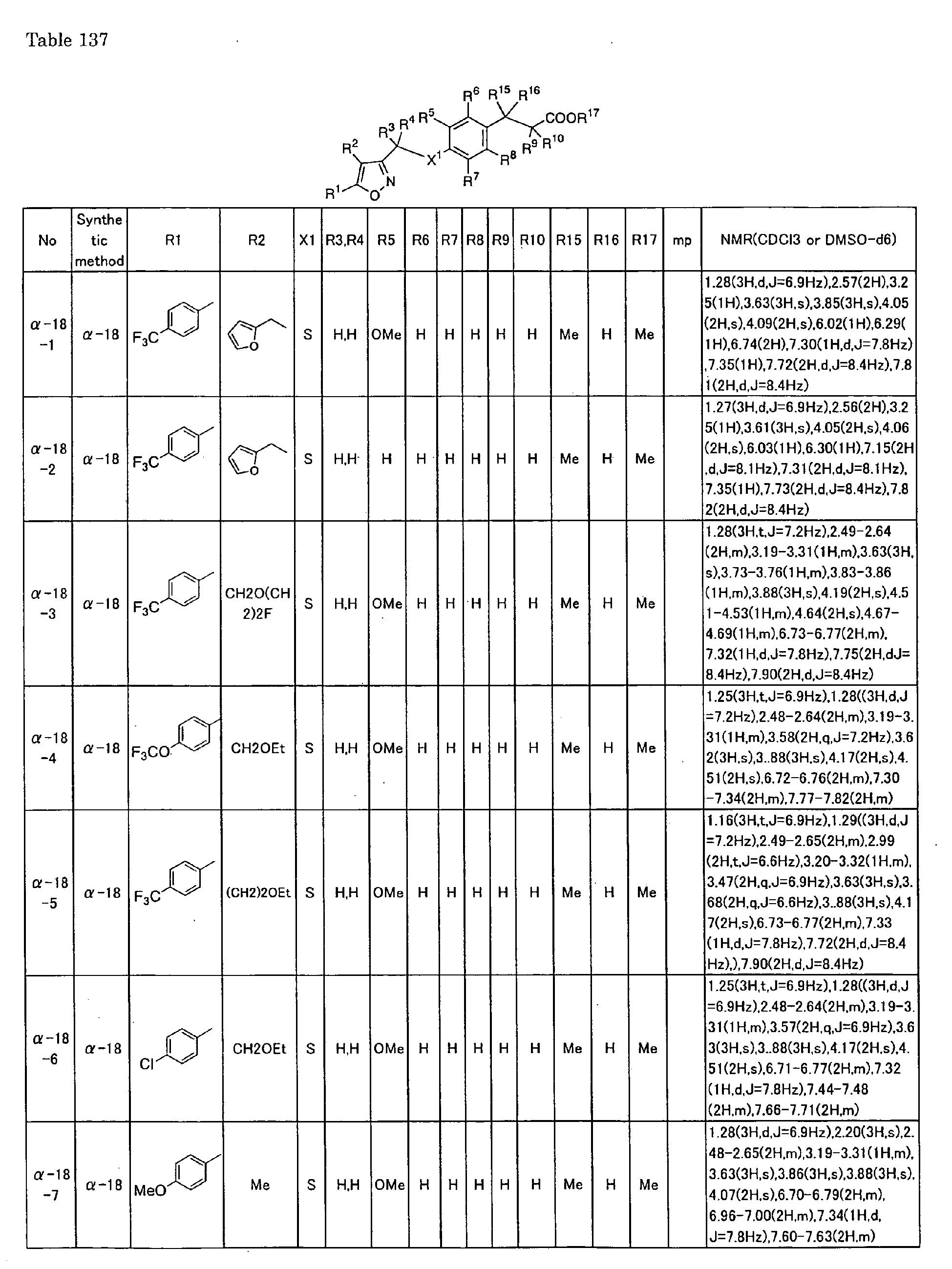

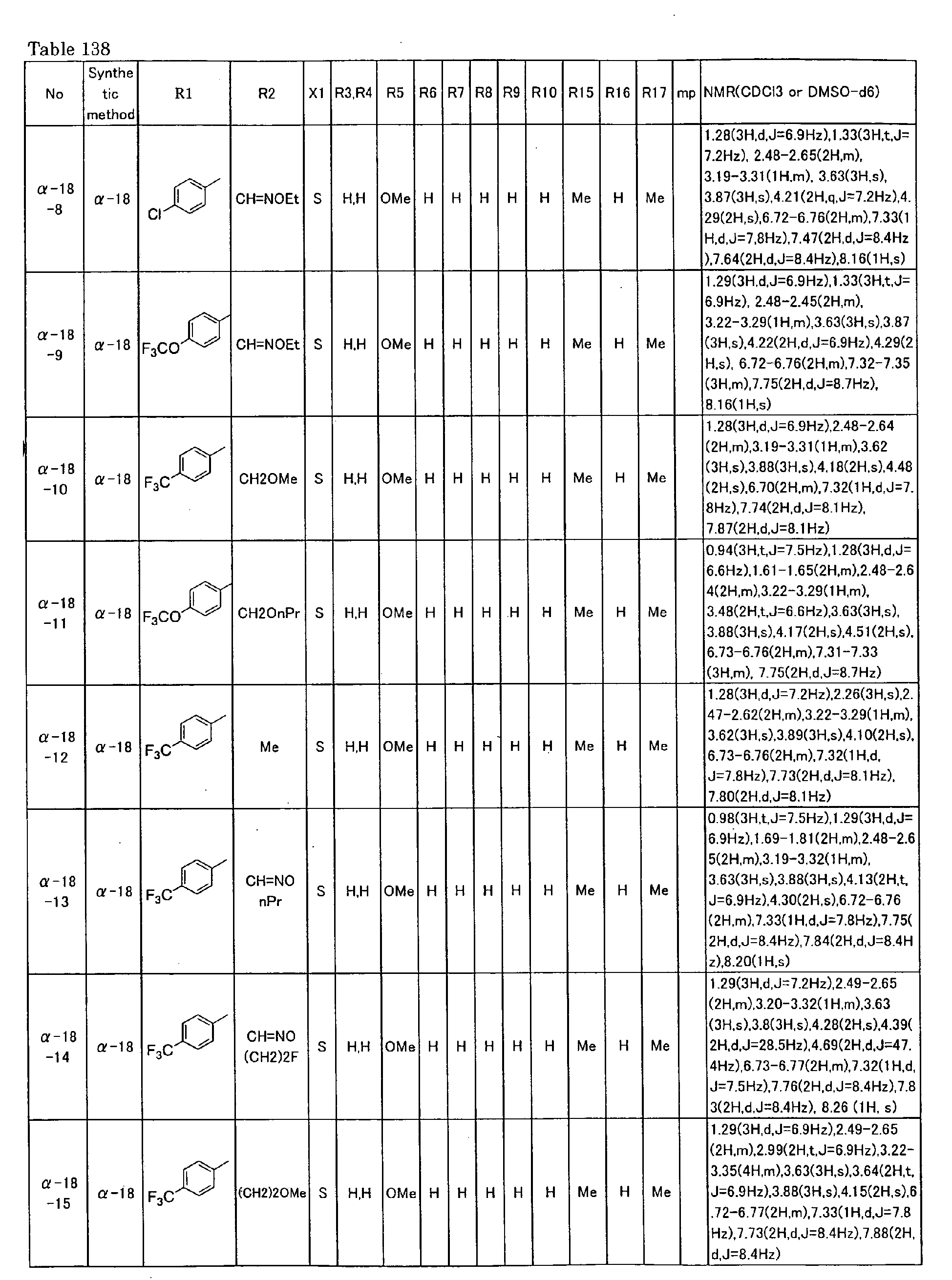

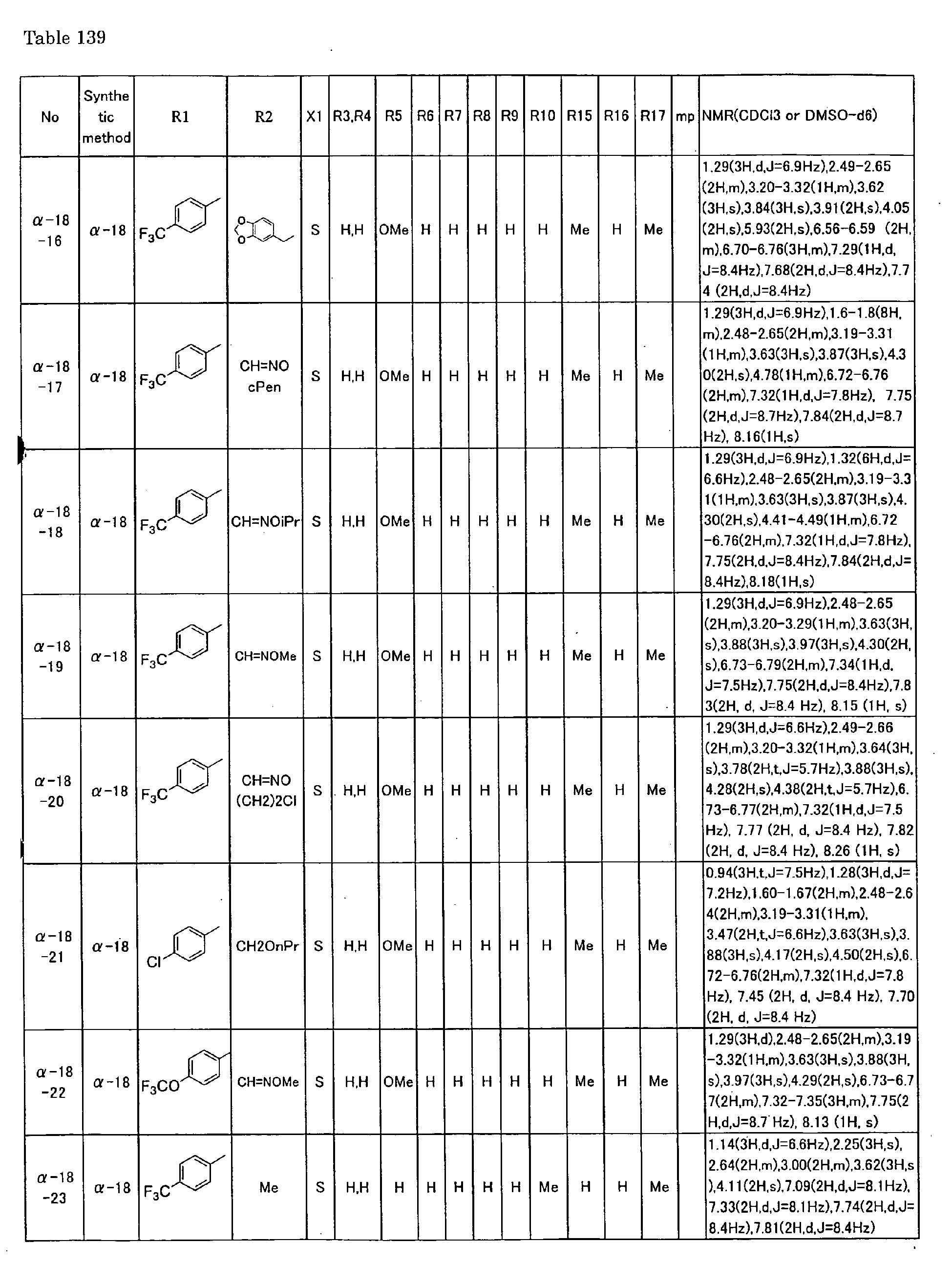

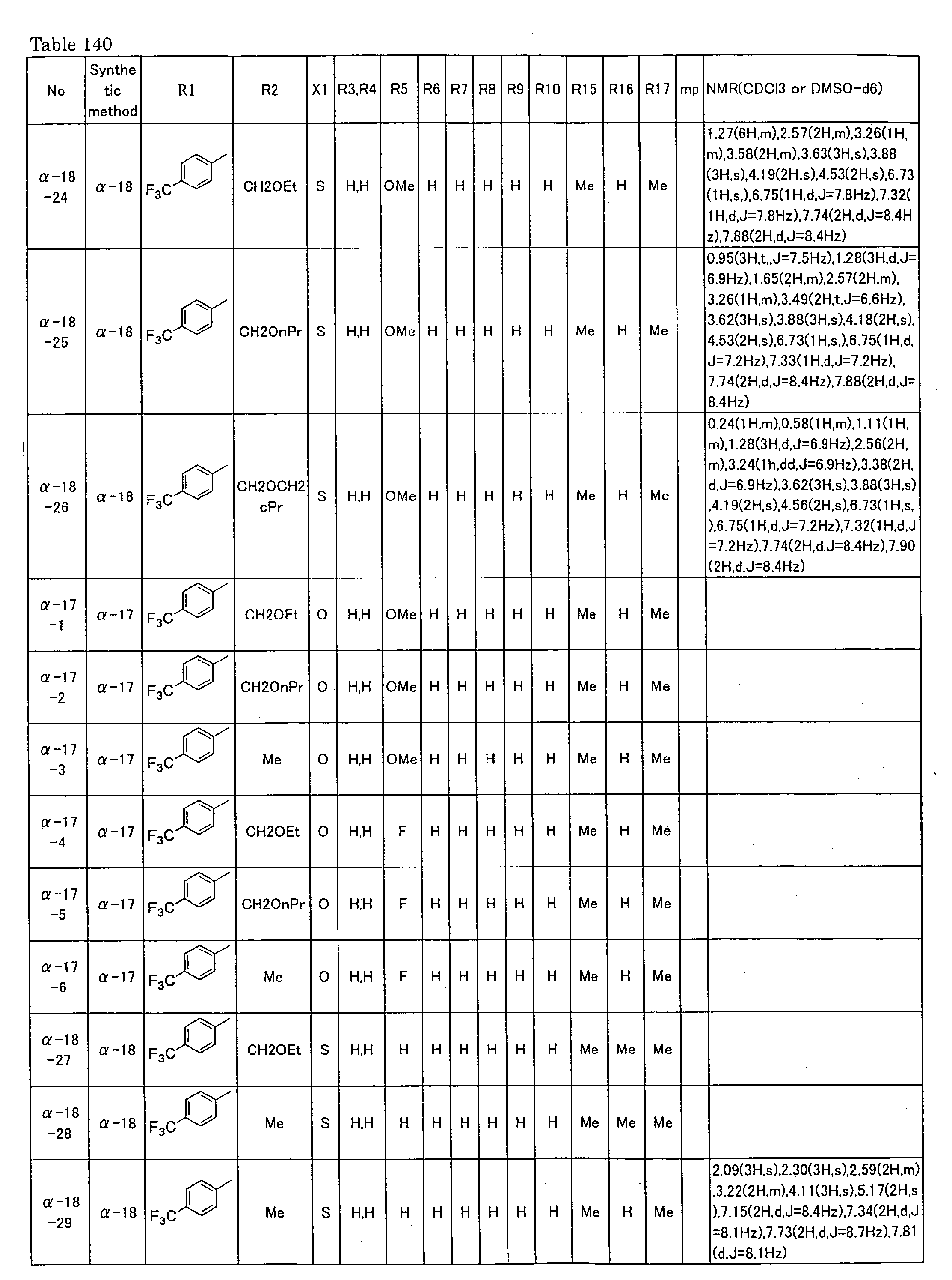

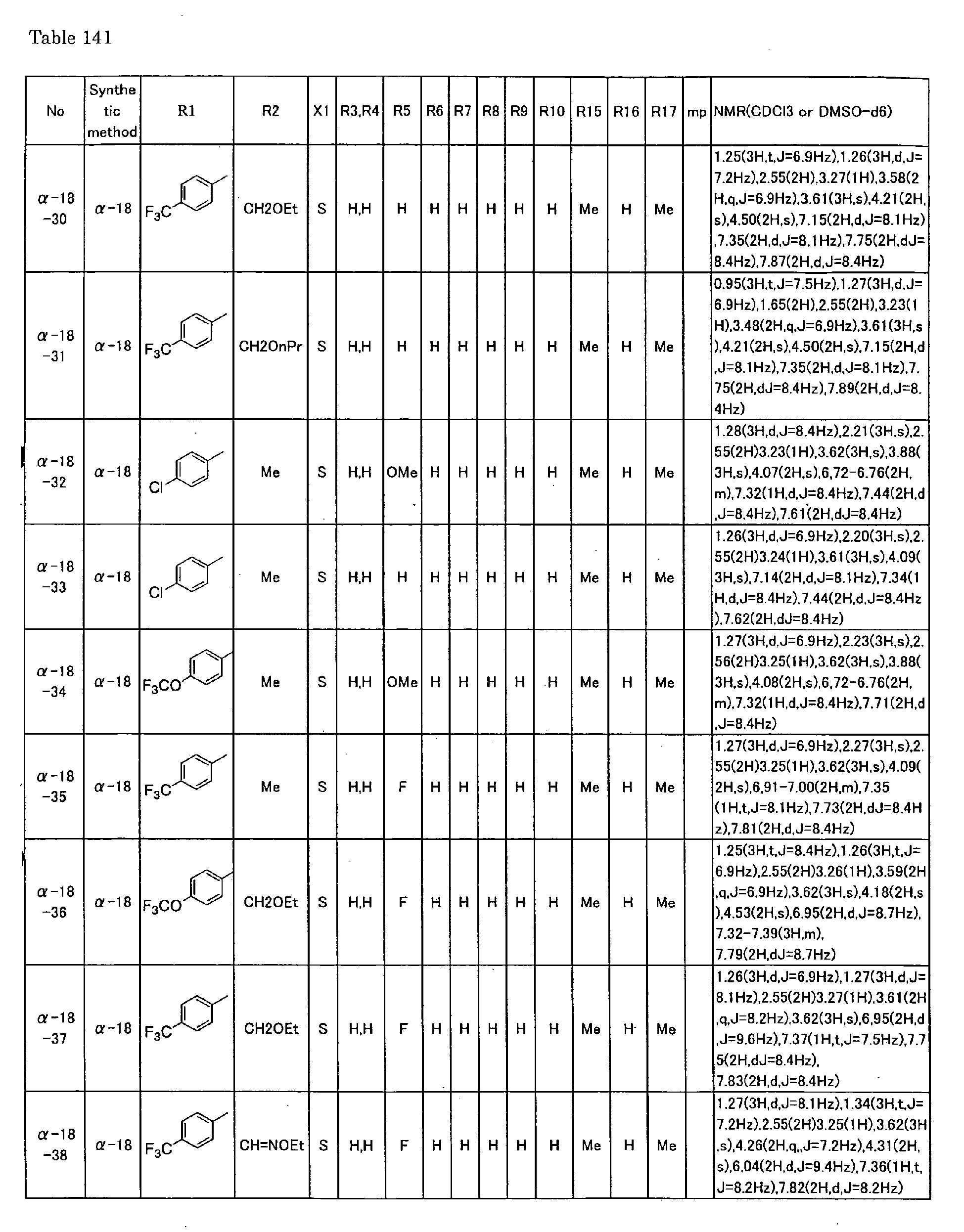

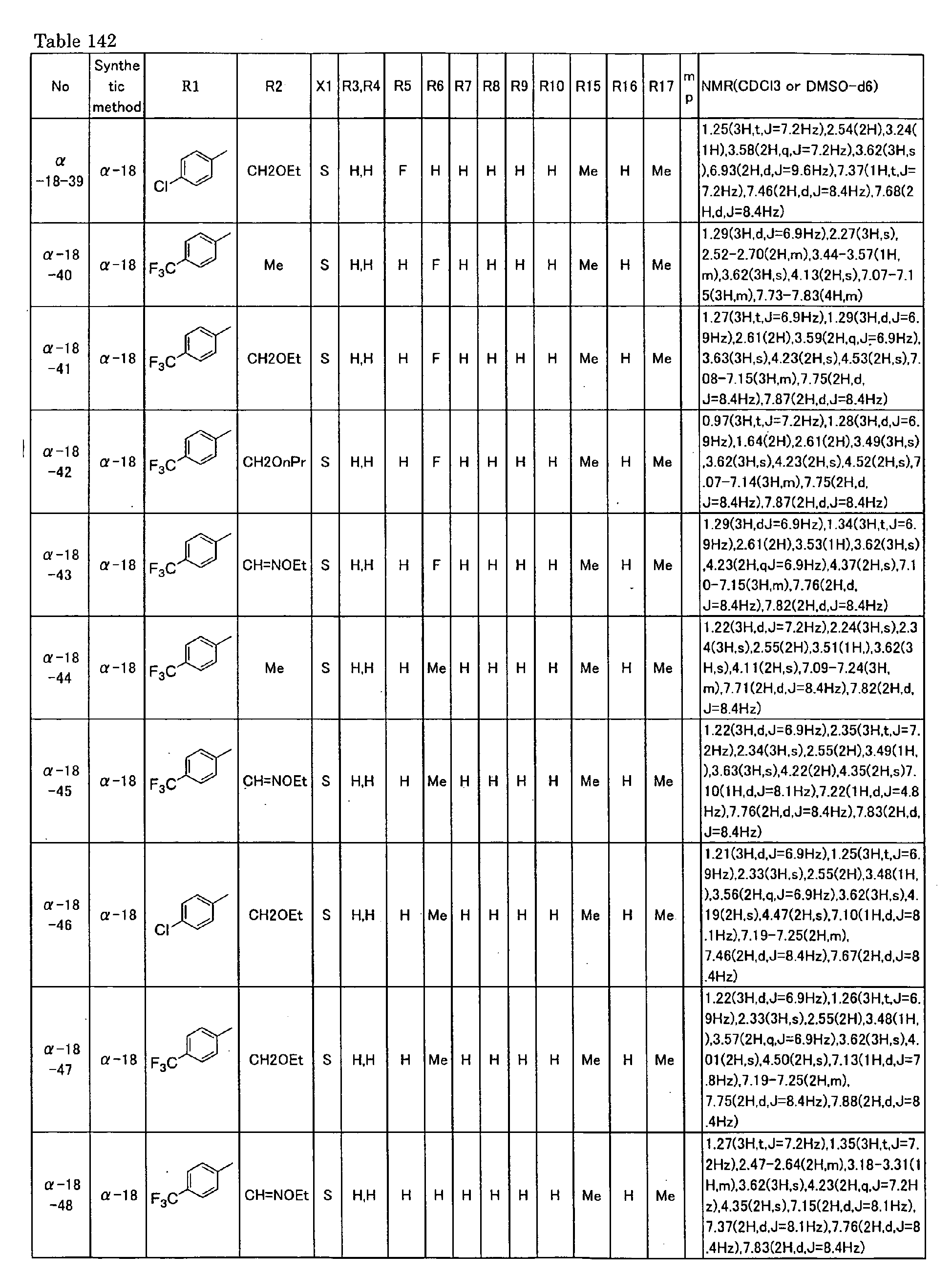

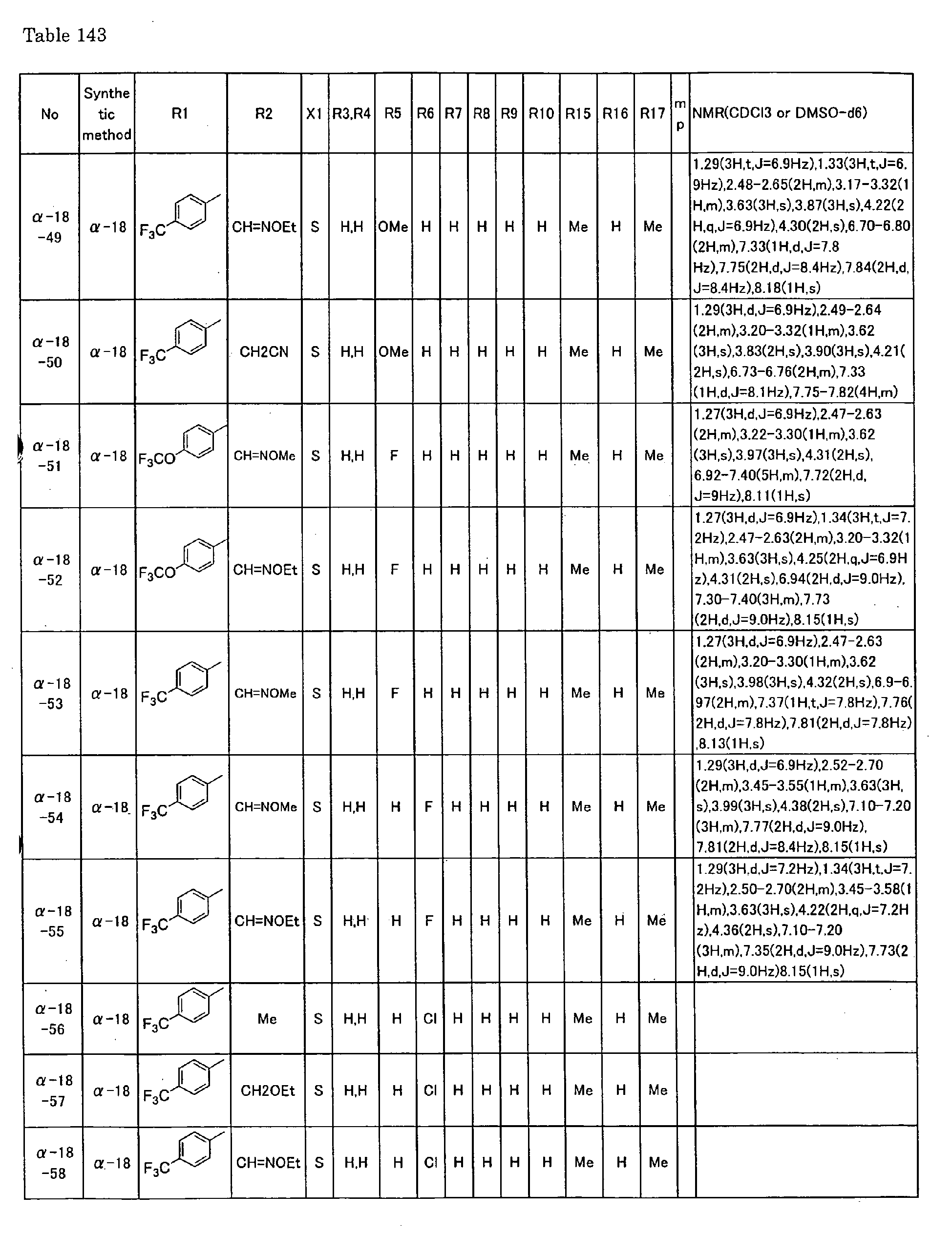

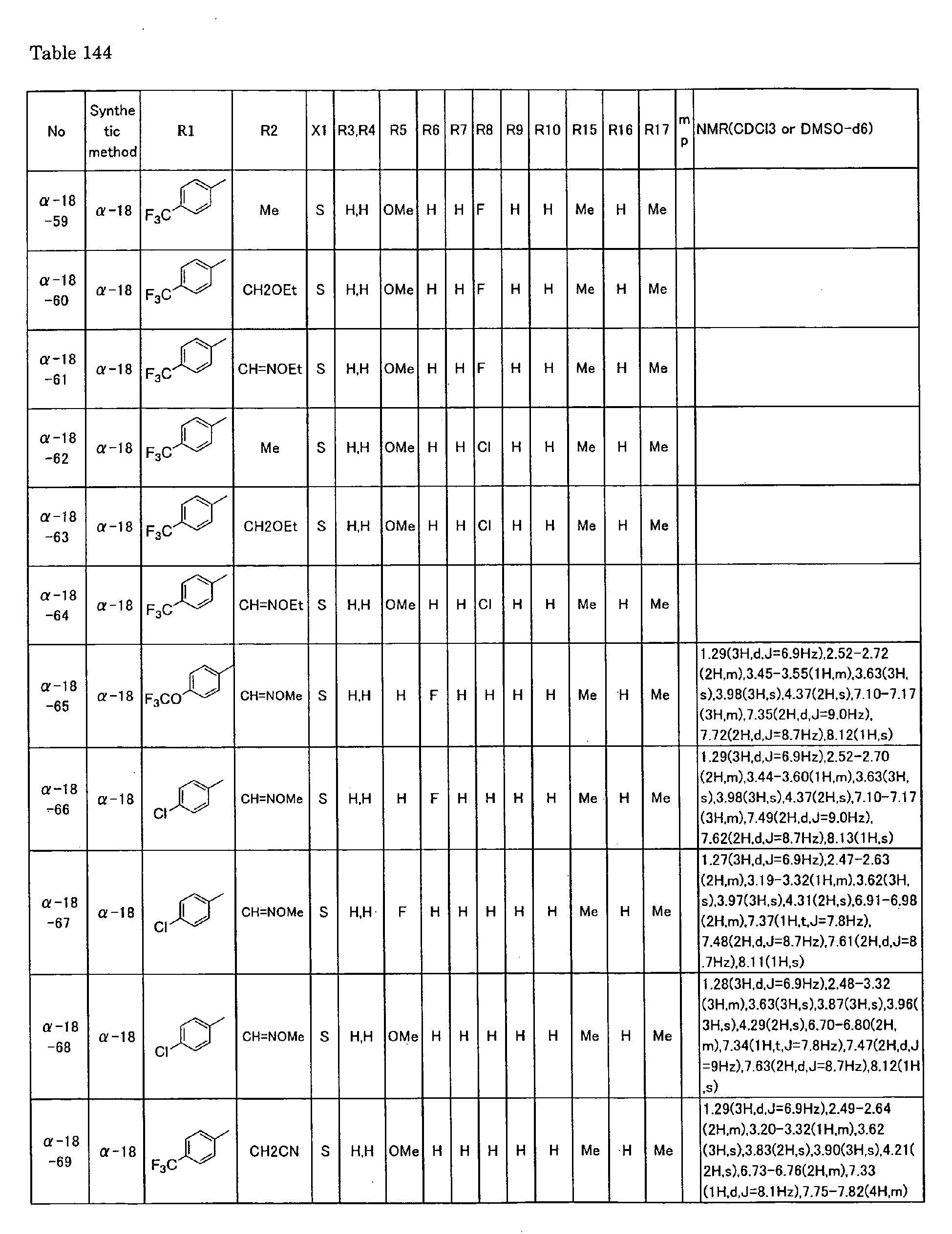

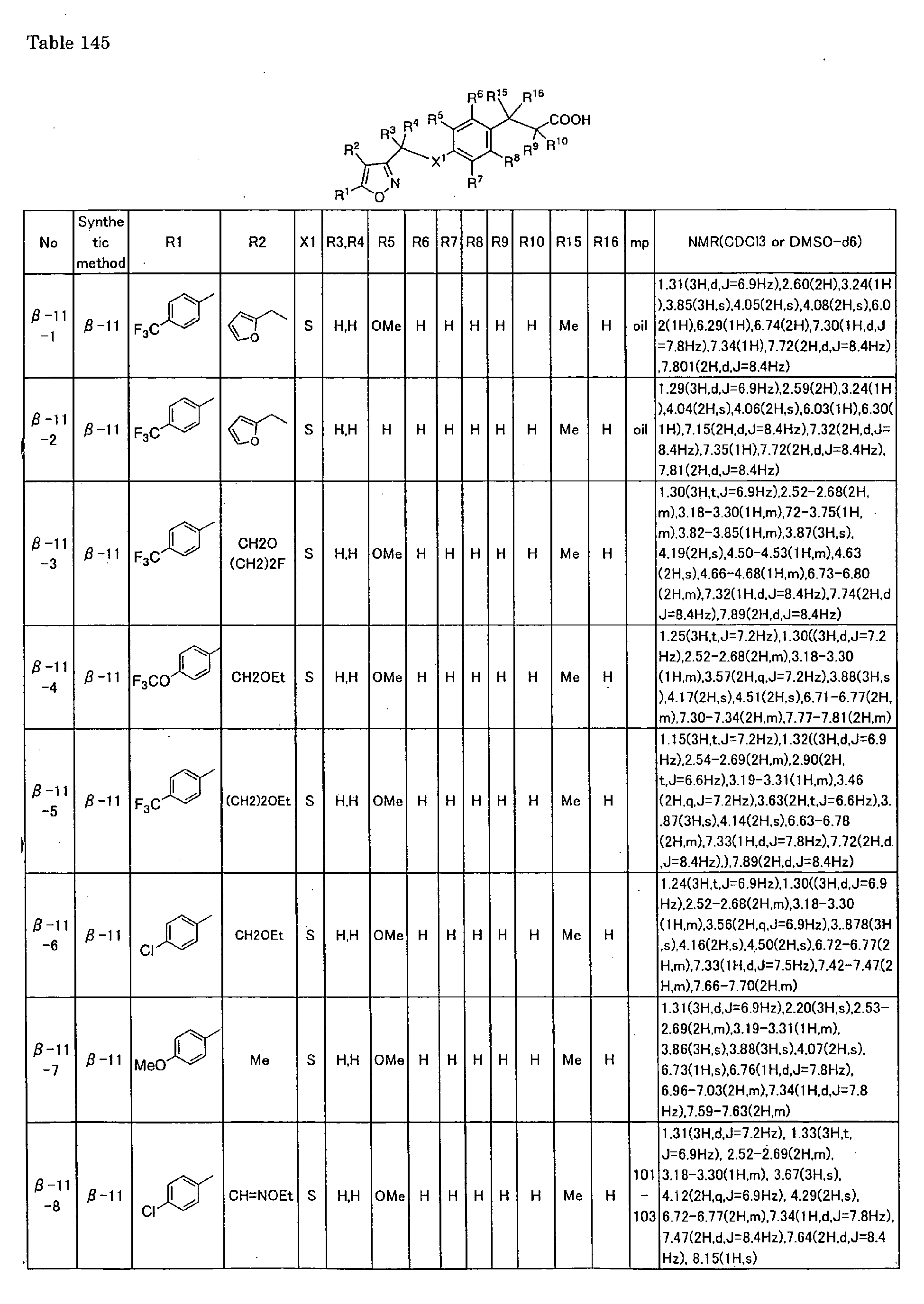

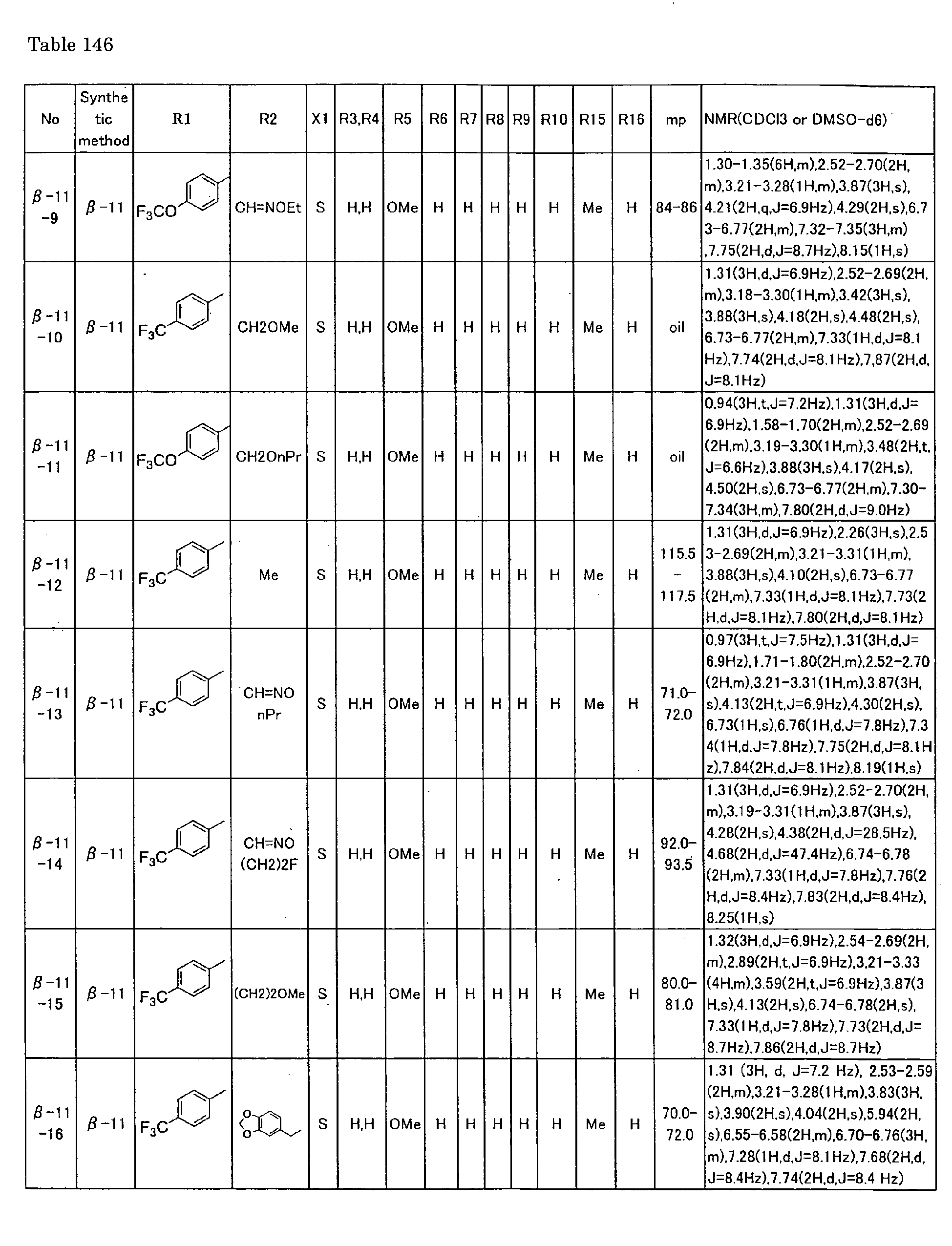

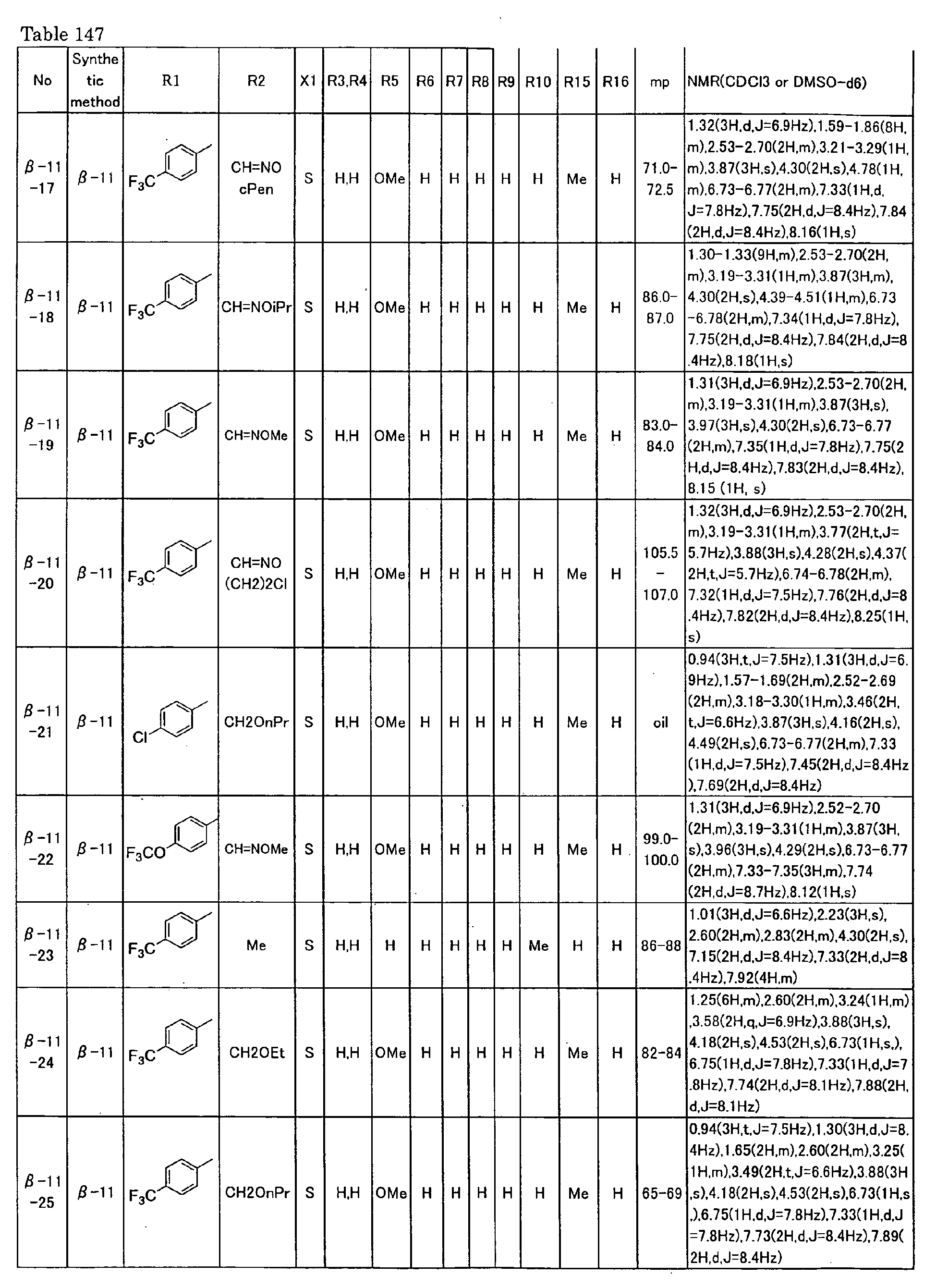

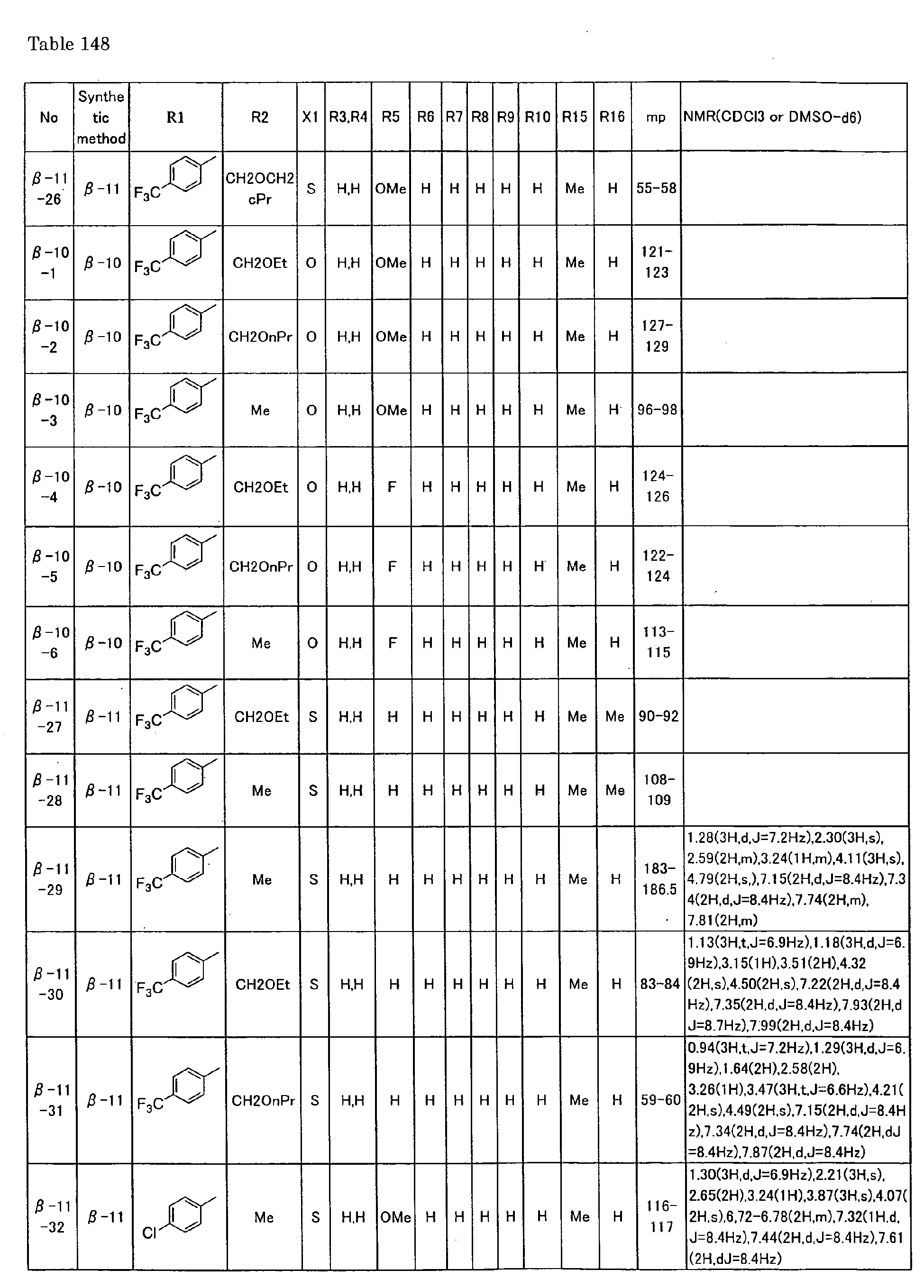

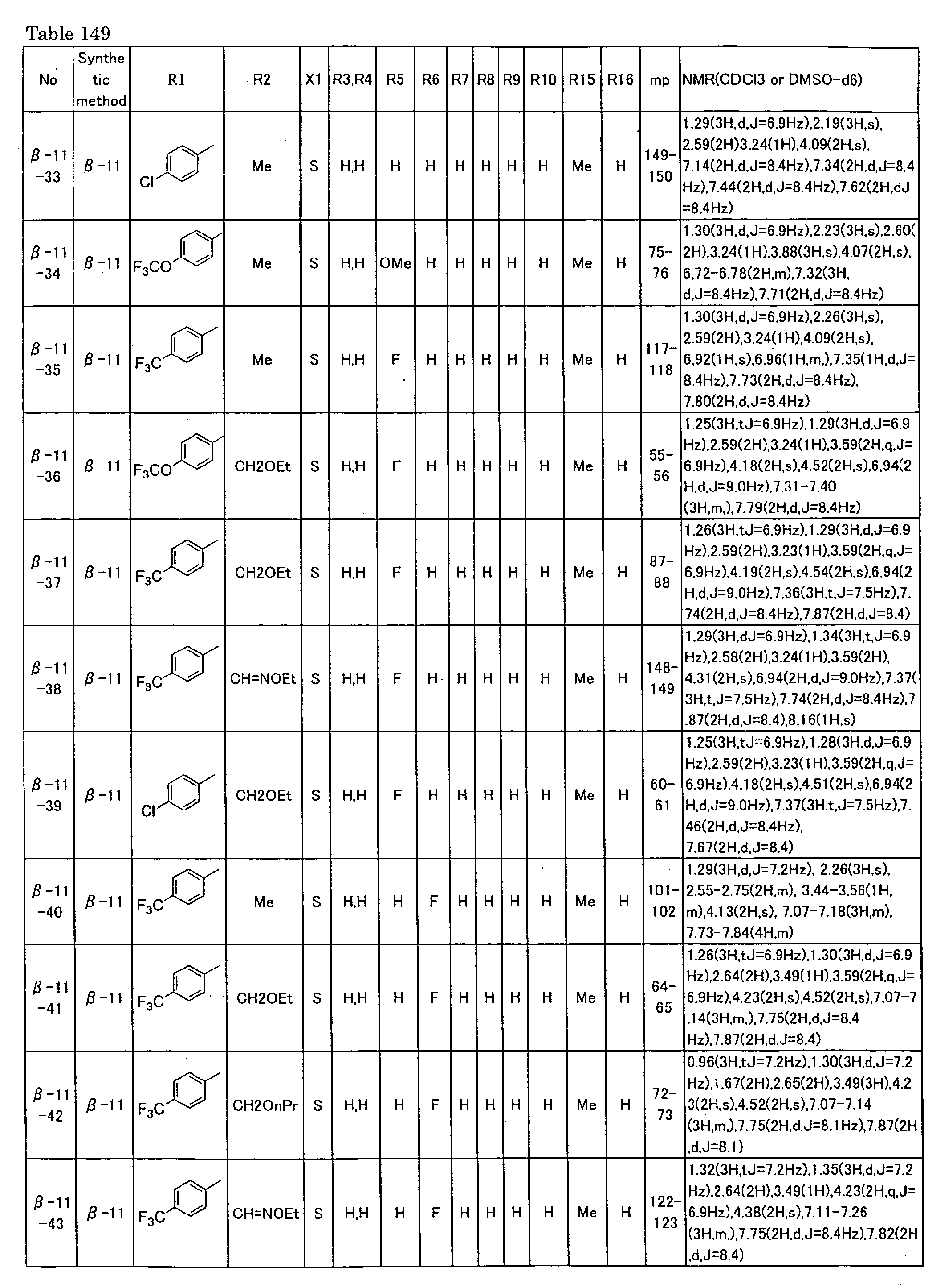

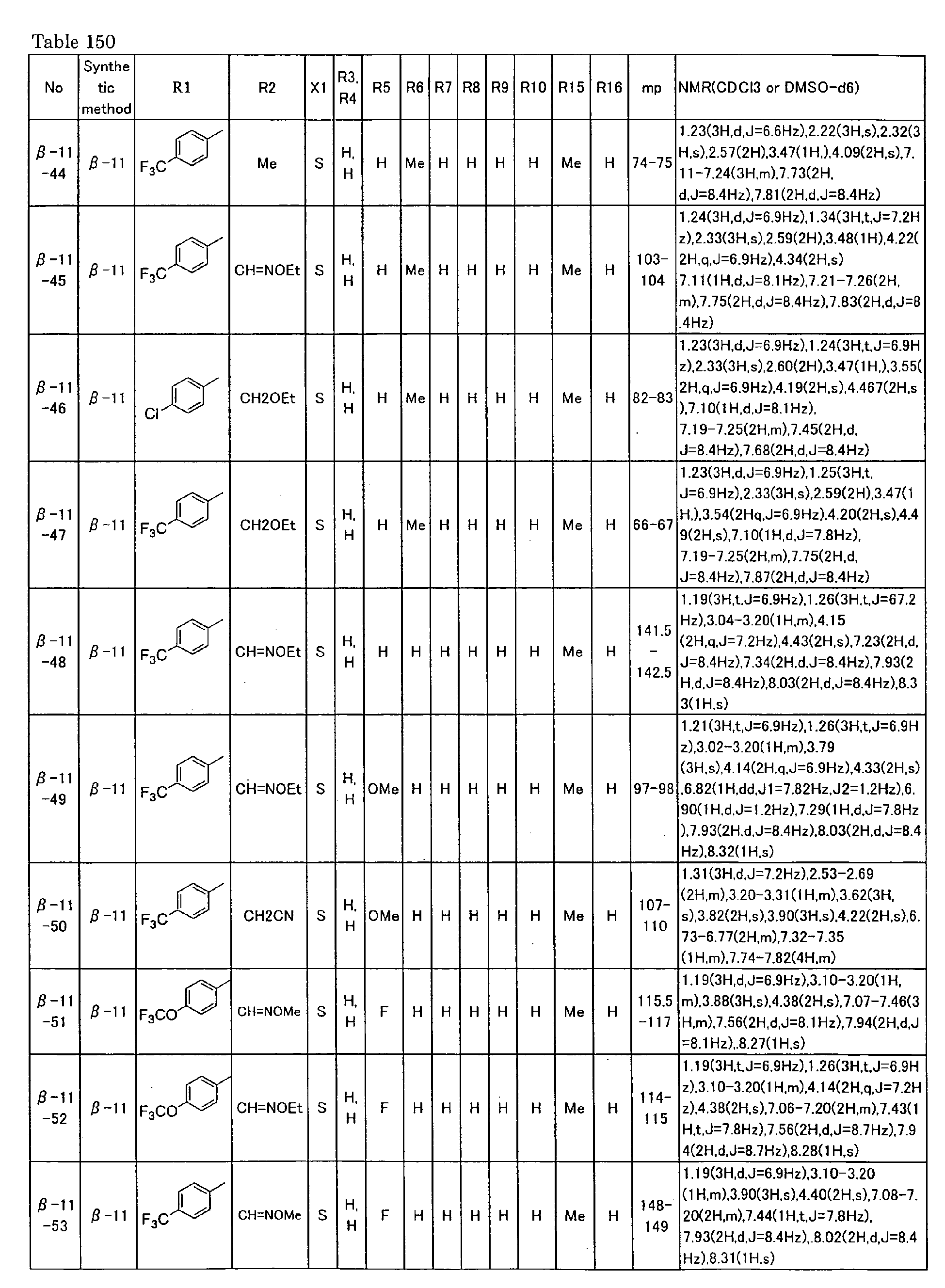

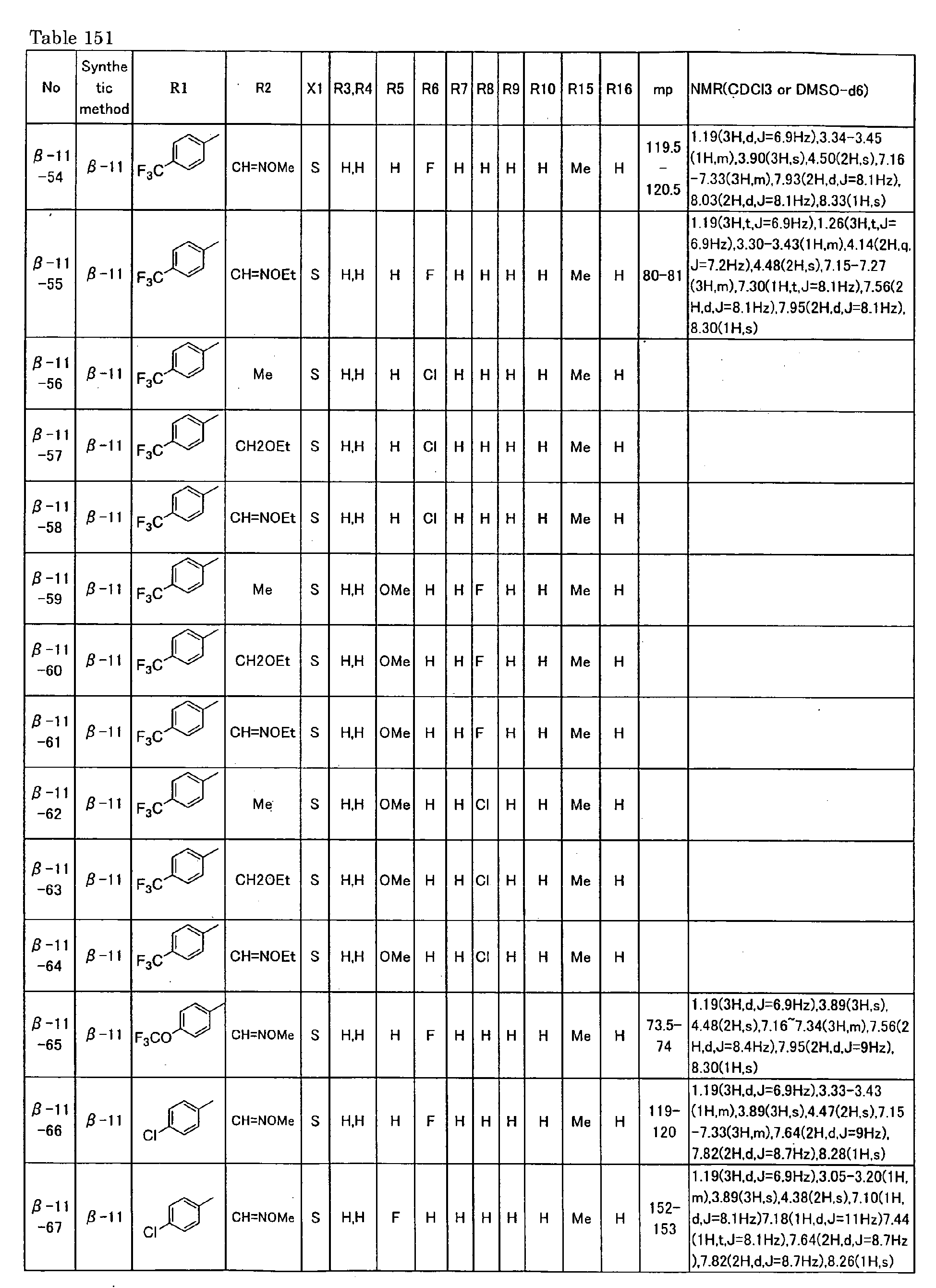

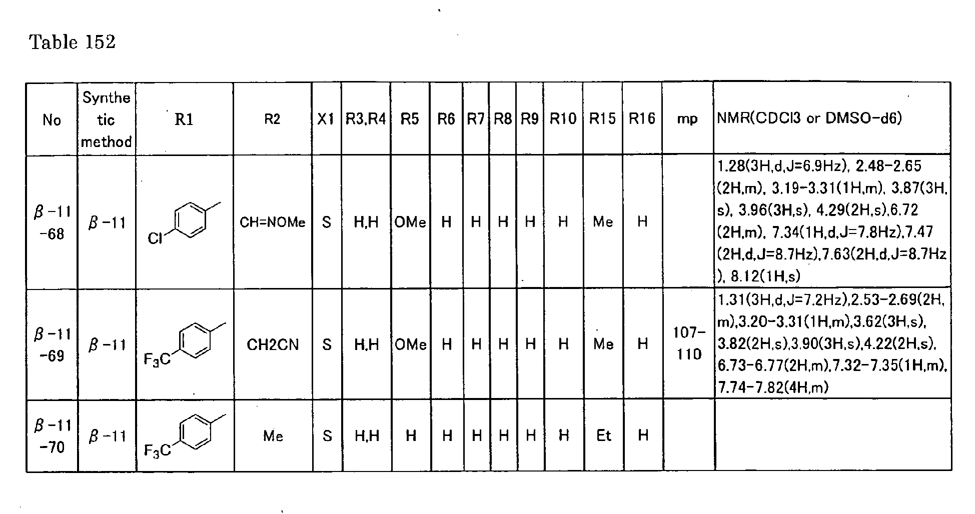

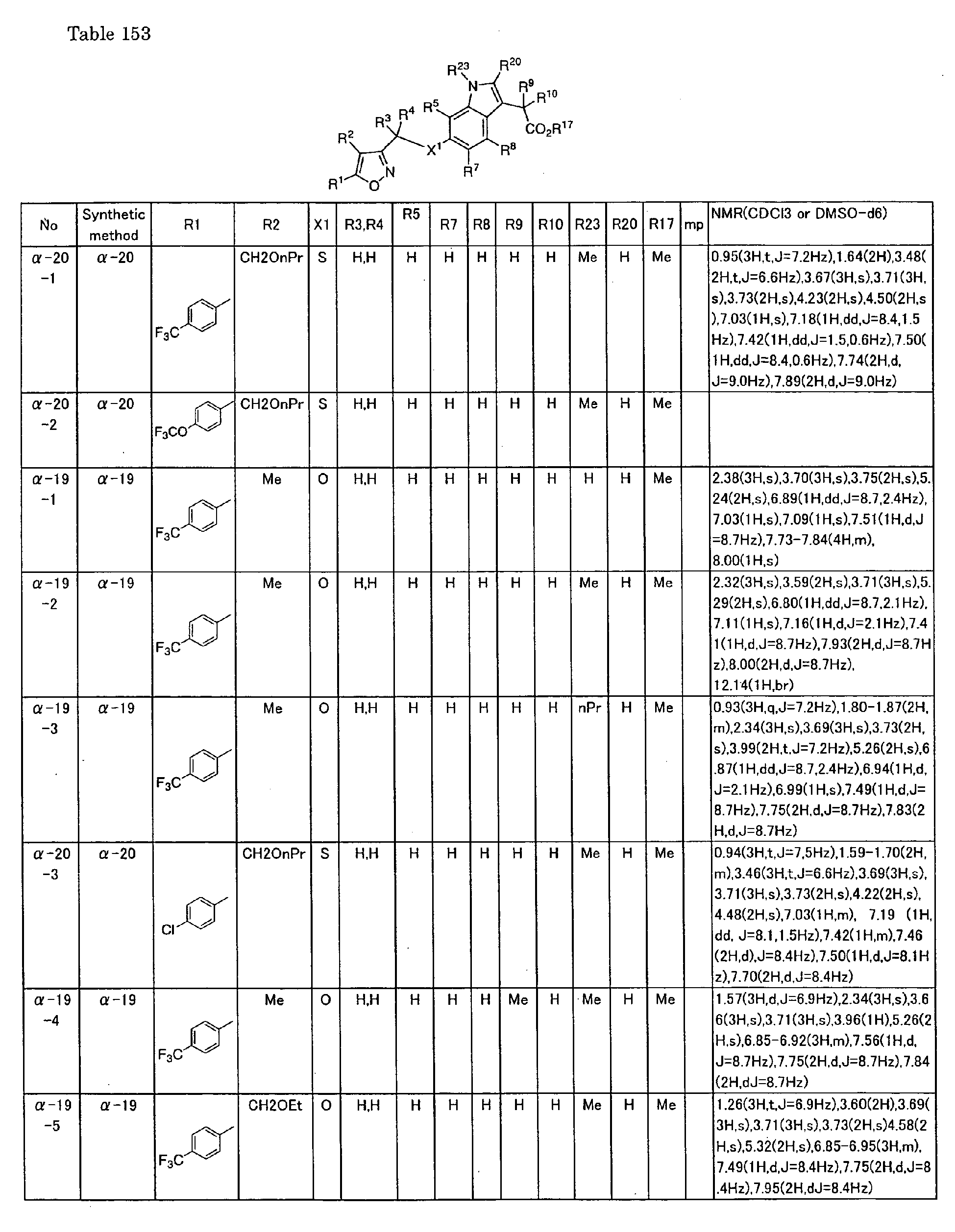

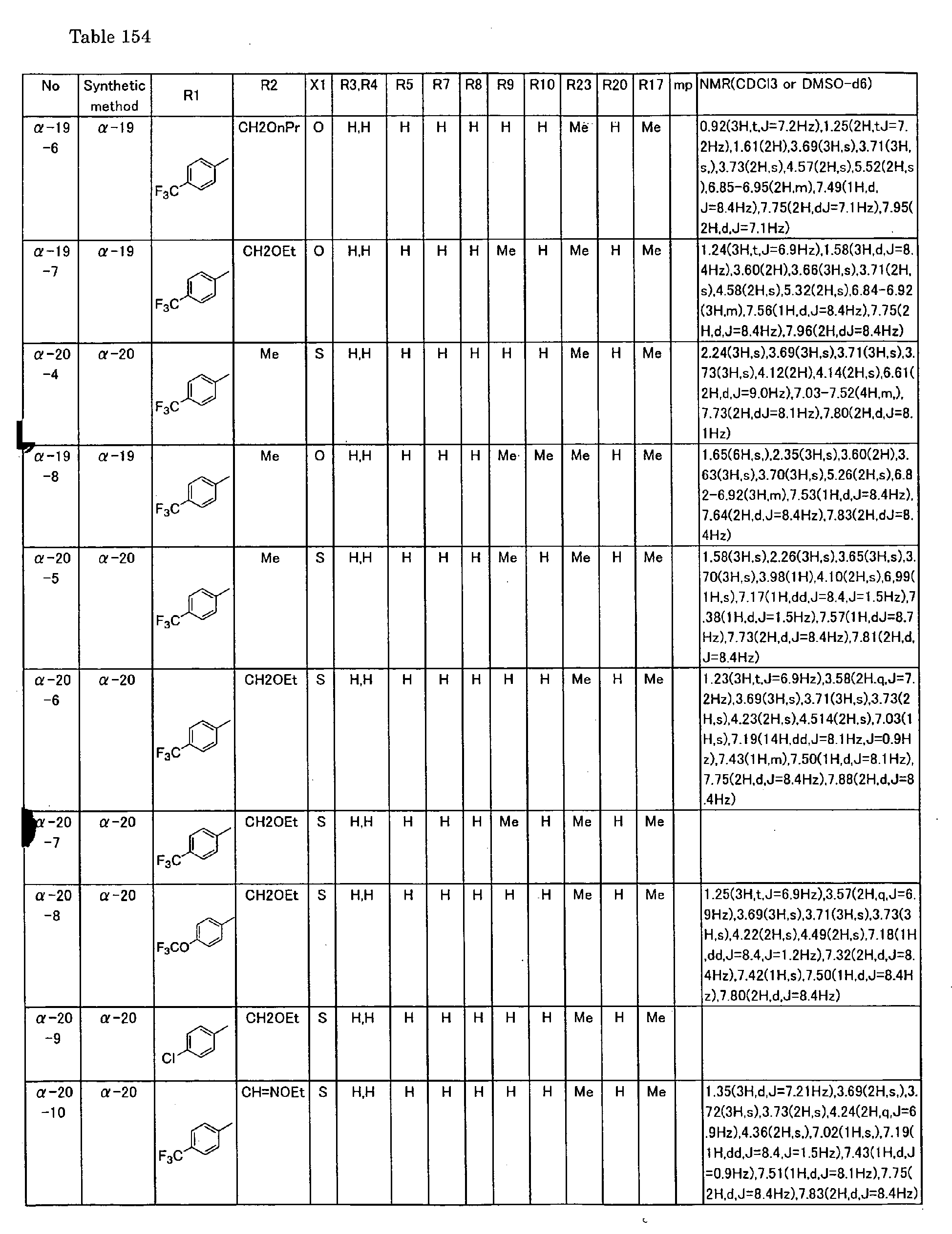

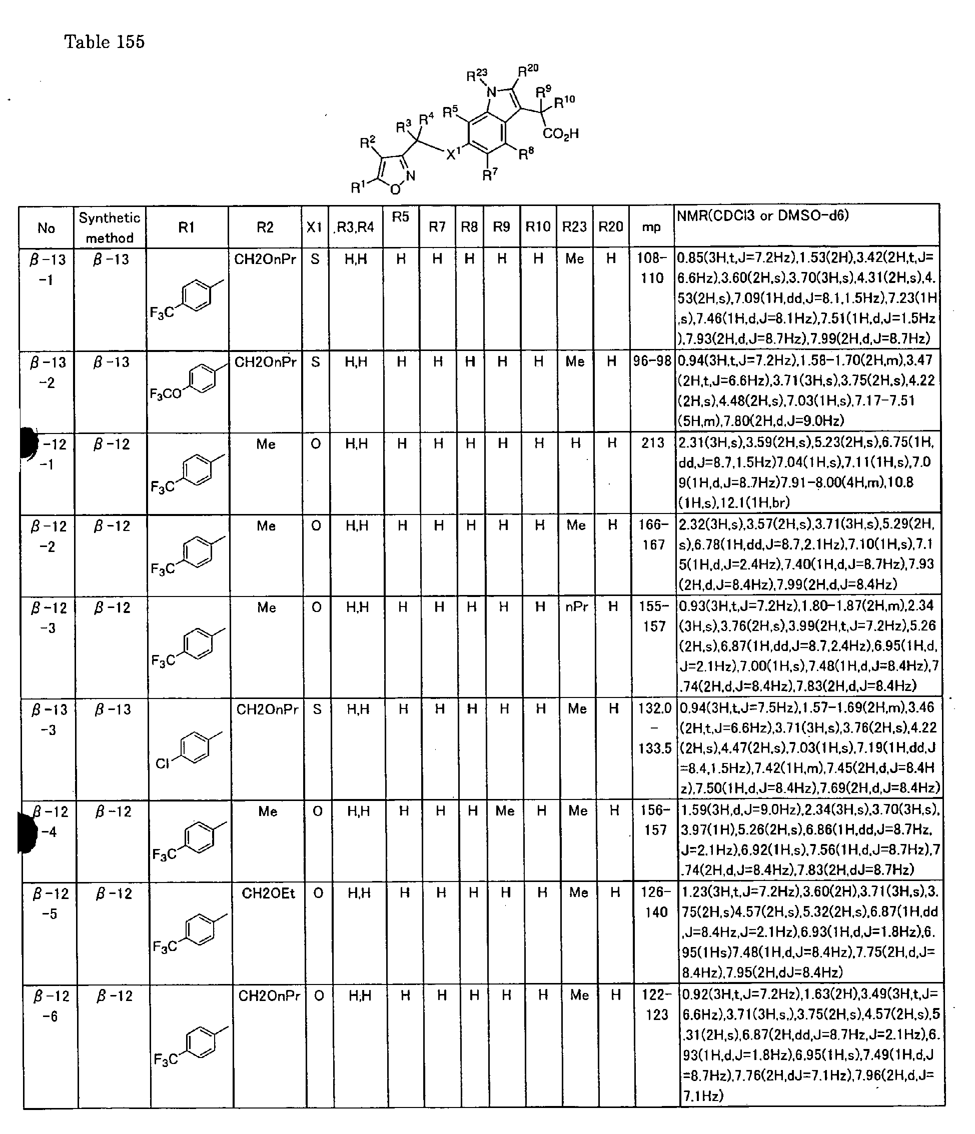

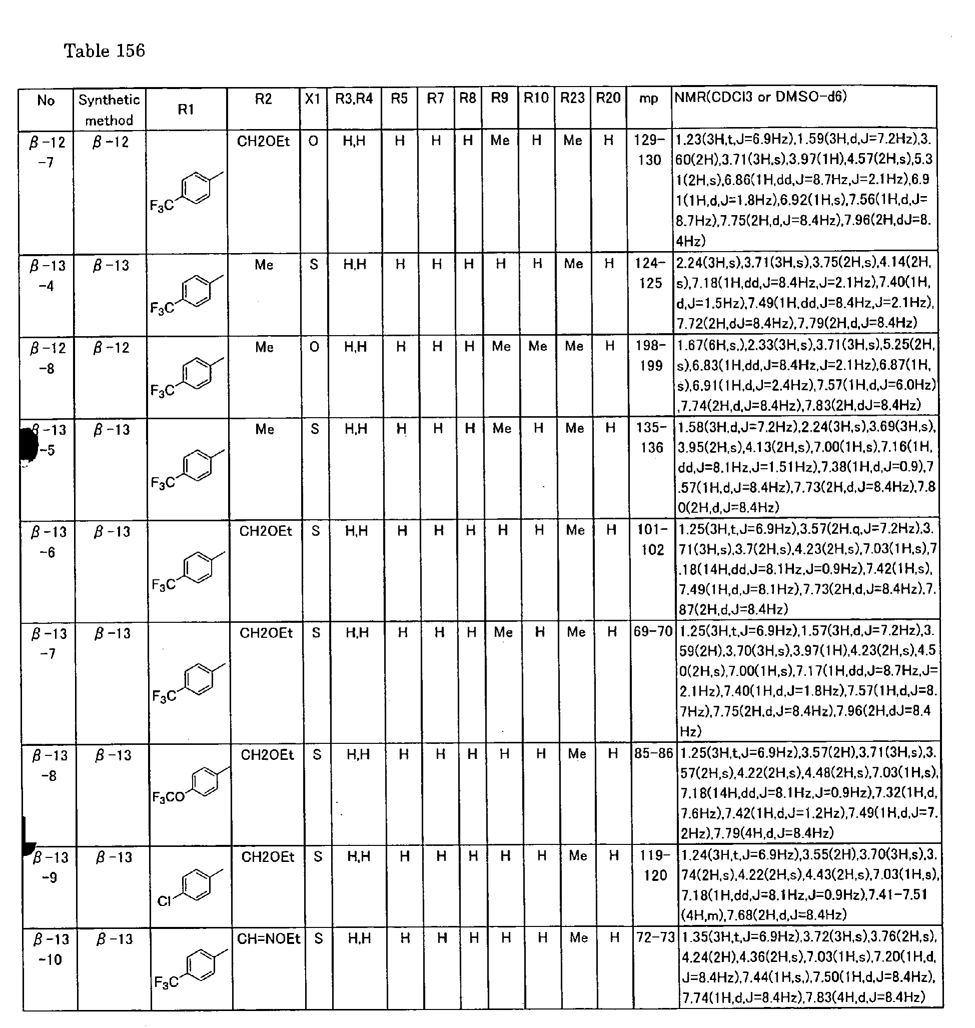

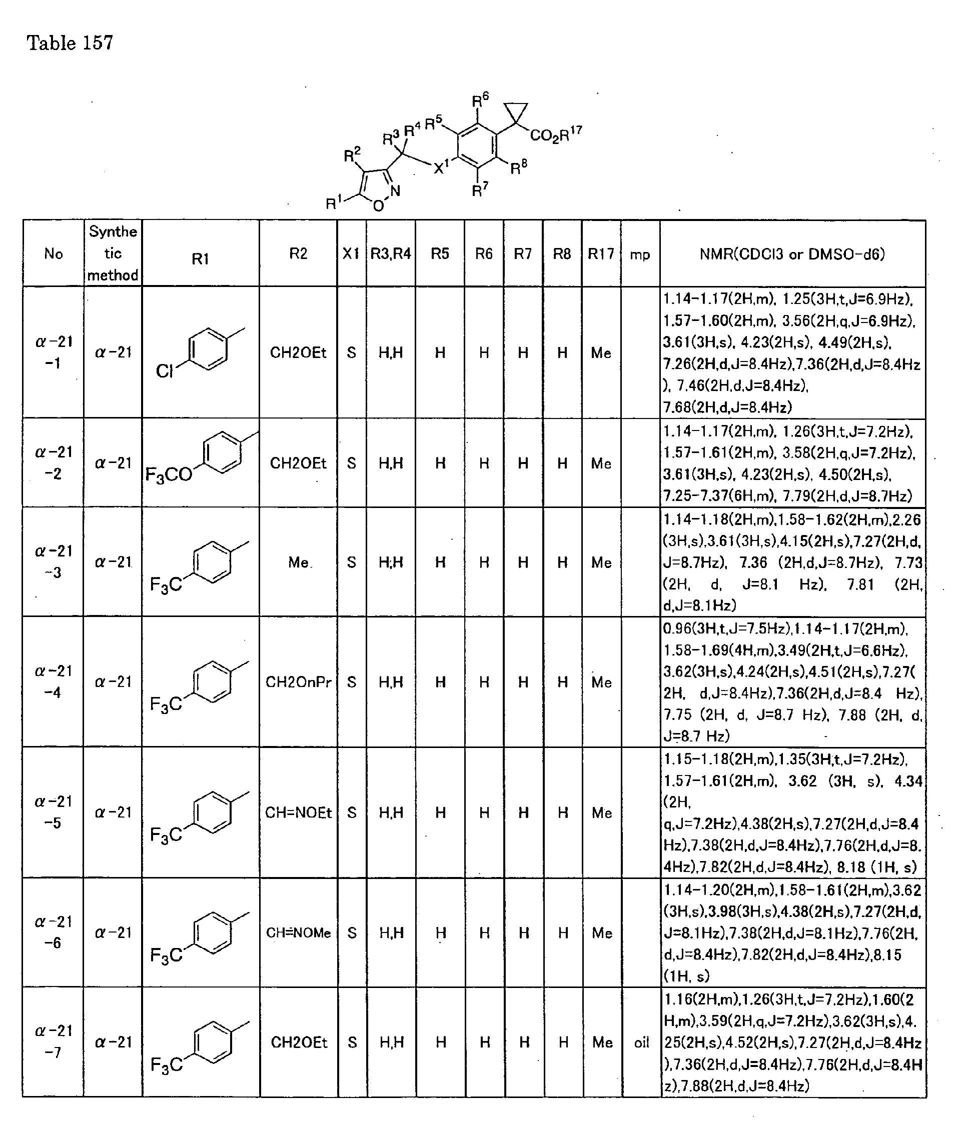

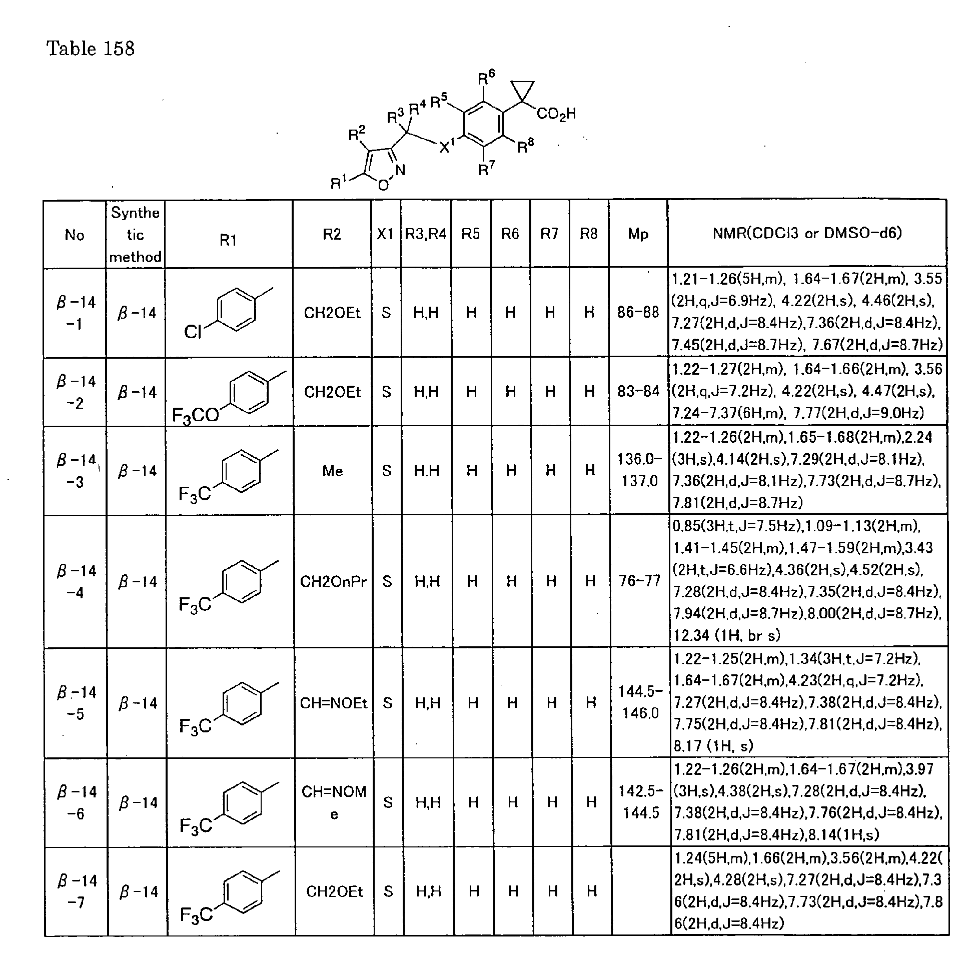

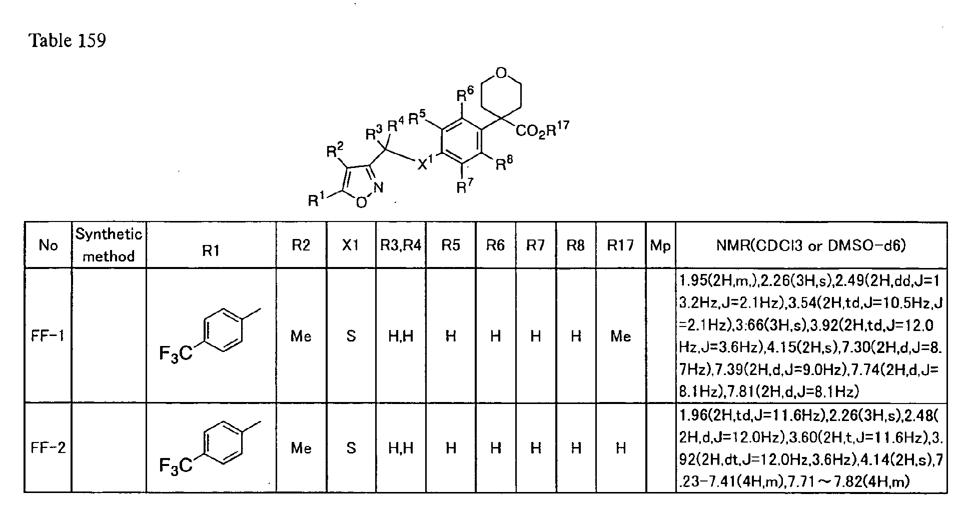

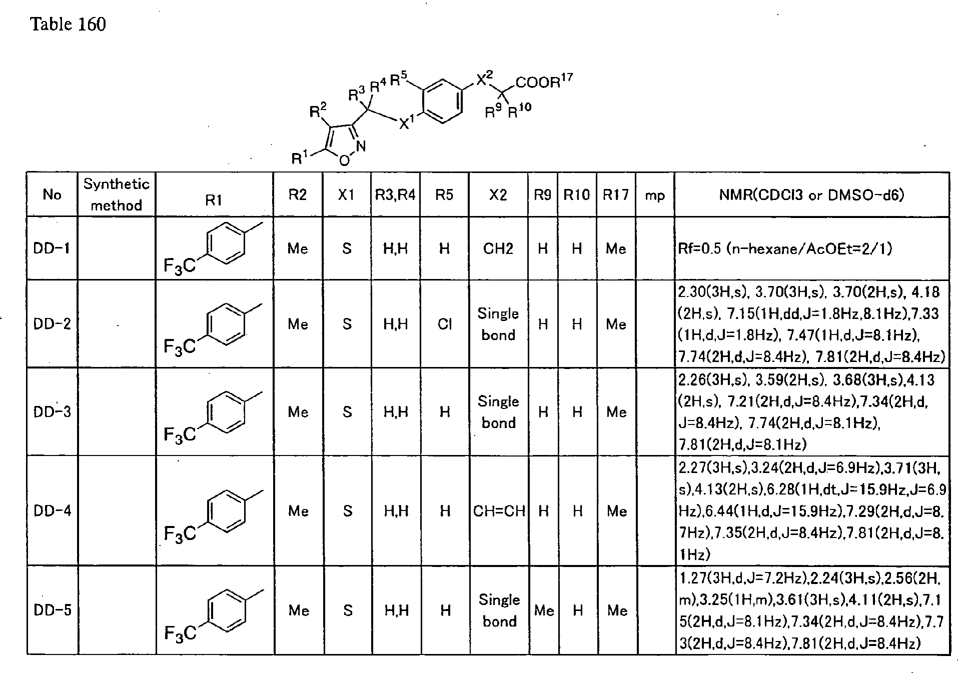

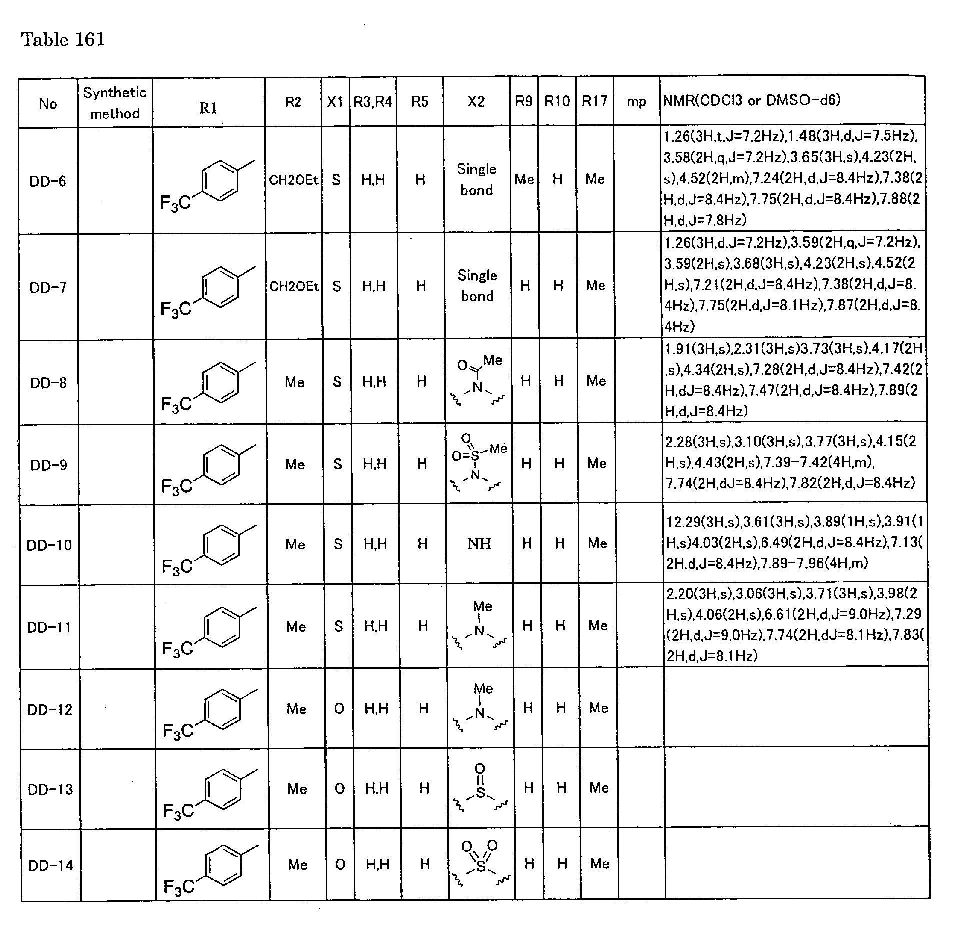

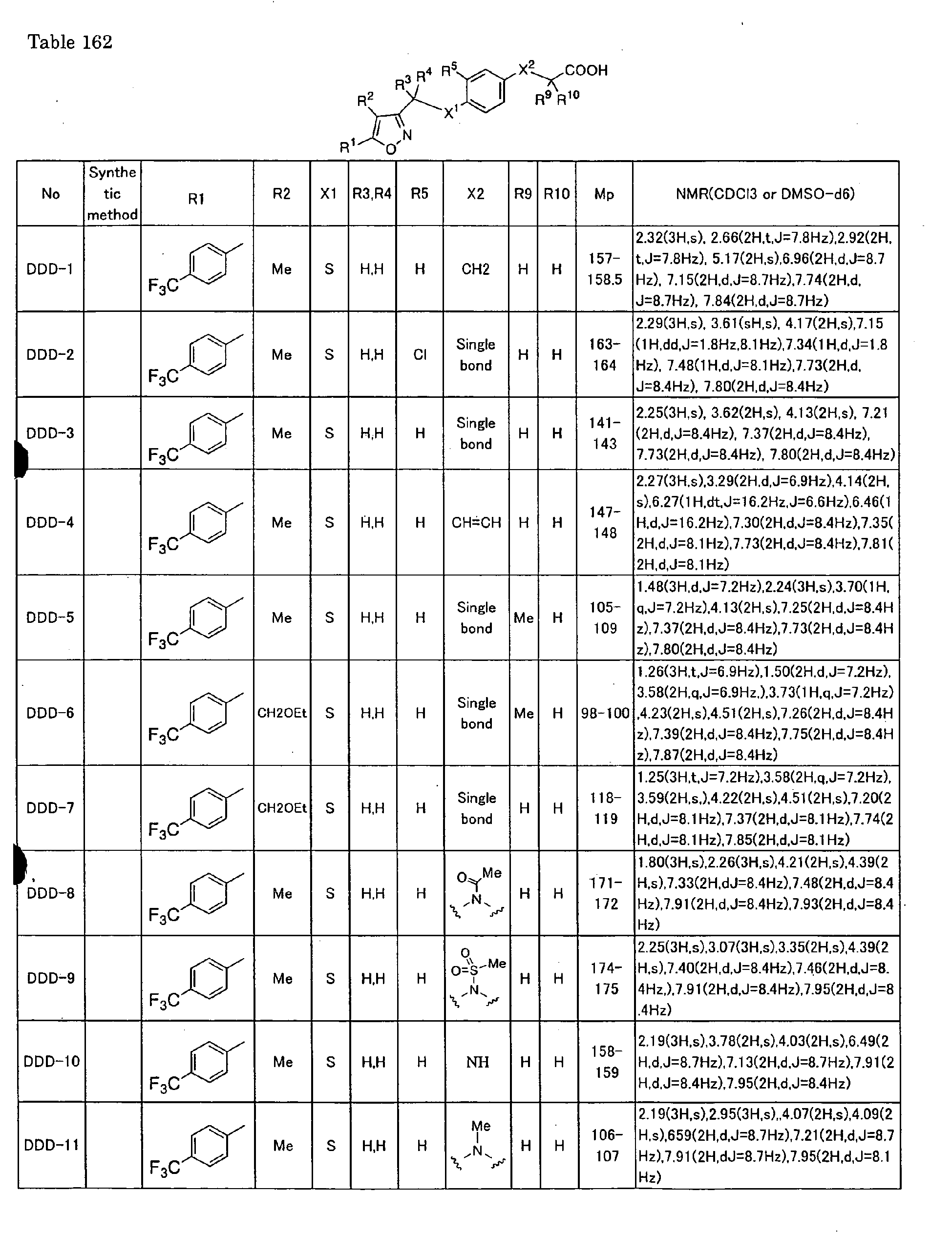

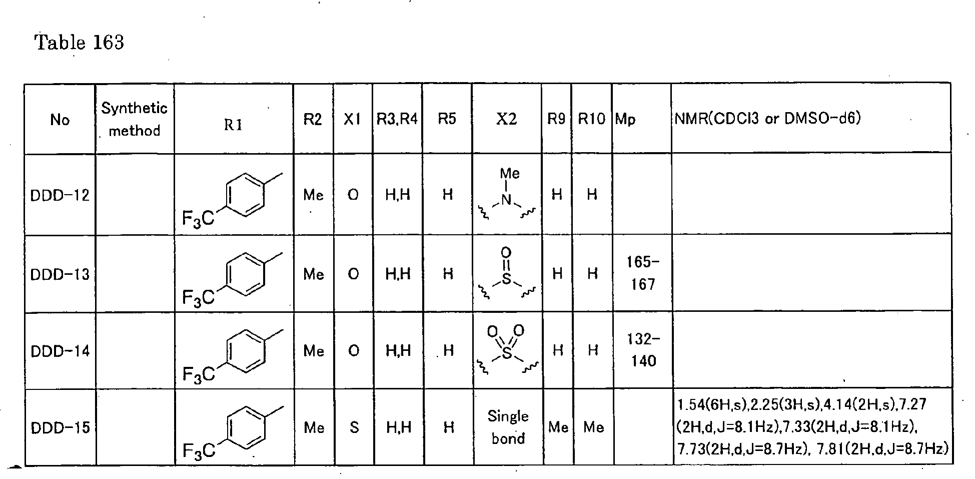

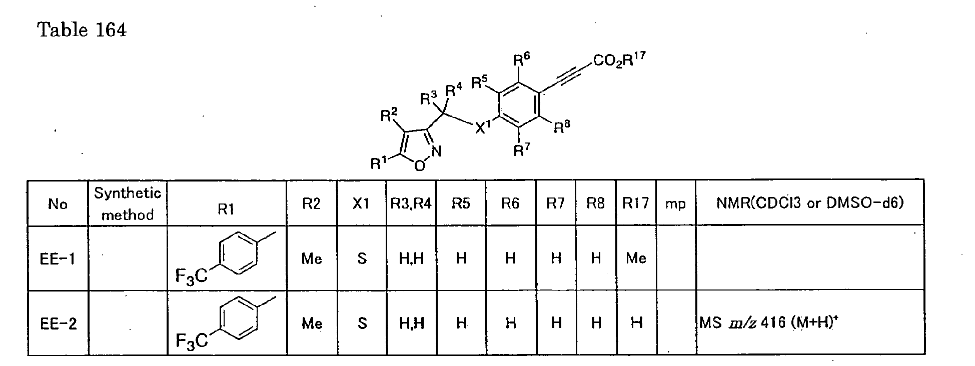

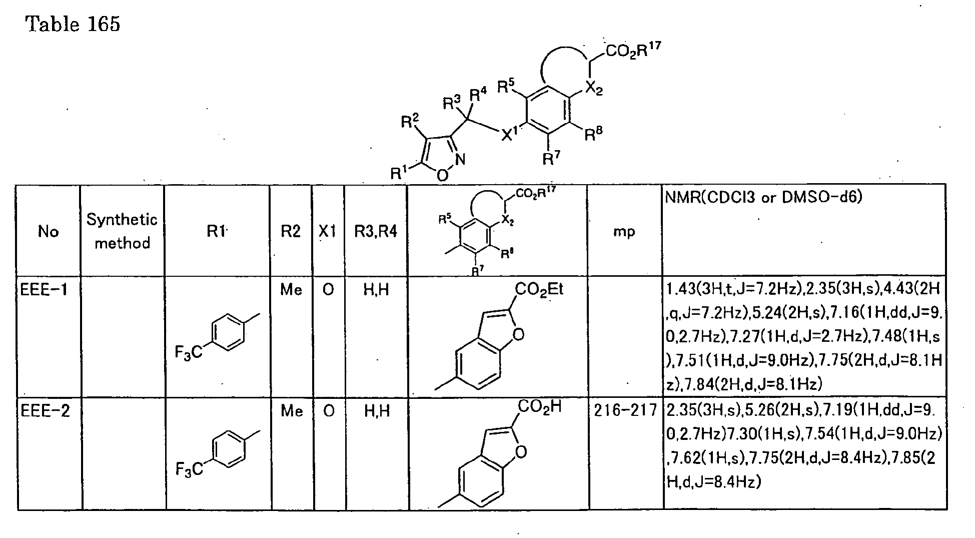

### Test Example 1 Test for transcriptional activity of PPARδ and α

A chimeric transcription factor assay, which is commonly used to detect nuclear receptor activity, was employed to measure PPAR transcriptional activity. Specifically, two plasmids, one that expresses the fusion protein of DNA binding domain of yeast transcription factor GAL4 and a ligand binding domain of a receptor, and a reporter plasmid were transiently transfected to CHO cells. The activity of the promoter containing a recognition sequence of GAL4 coded on the reporter plasmid was used as a parameter to estimate the activity of the receptor.
Plasmid: The ligand binding domain of human PPARδ (hPPARδ) or α (hPPARα) (δ: aa 139 - C-end;α: aa 167 - C-end) is obtained by PCR amplification using Human Universal Quick-Clone cDNA (CLONTECH). Each amplified cDNA was subcloned into pCR2.1-TOPO vector (Invitrogen) and the identity of the cDNA clones was confirmed by the DNA sequence. Then, each obtained cDNA fragment was subcloned into pBIND vector (Promega) to construct a plasmid expressing the fusion protein with DNA binding domain of yeast transcription factor GAL4. pG5luc vector (Promega) was used as a reporter plasmid.
Cell culturing and transfection: CHO cells were cultured in 10% FBS-αMEM. With a 96-well plate (Costar), CHO cells, that were dispersed with trypsin treatment, 20000 cells per well and the two plasmids obtained by the above procedure, 25 ng per well, were transfected with FuGene Reagent (Roche) by following the instruction of the manufacture.
Measurement of the transcriptional activity: CHO cells 100 µl per well, which were transfected as above, were dispensed into the wells in which a test compound dissolved in DMSO 0.5 µl was spotted in advance. After the cells and a test compound were cultured together for 24 hours in a CO₂ incubator, the luciferase activity was measured by adding luciferase substrates, PicaGene LT2.0 (Toyo ink) 100 µl per well. LUMINOUS CT-9000D (DIA-IATRON) is used to measure the activity.

As to PPARδ, the concentration of a test compound which shows 1/2 of maximum luciferase activity was calculated using an Excel program to obtain the EC₅₀ value for PPARδ activity of a test compound. The result is shown in Table 166.

As to PPARα, the proportionate increase of luciferase activity in the concentration of a test compound 1 µM and 10 µM in contrast to DMSO was calculated. The result is shown in Table 167.

**Table 166**

| No. | EC₅₀ (nM) |
|---|---|
| | hPPARδ |
| Reference compound 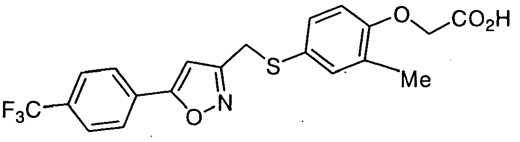 | 37 |
| α-7-3-1 | 9.5 |
| β-1-3 | 9.9 |
| β-1-15 | 1.5 |
| β-1-8 | 11 |
| β-4-1 | 16 |
| β-5-1 | 14 |

**Table 167**

| No. | HPPARα | |
|---|---|---|
| | 1 µM | 10 µM |
| β-1-32 | 22.9 | 44.5 |
| β-1-33 | 18.4 | 40.7 |

### Test Example 2 Test for inhibition of CYP2C9 enzyme

The test for inhibition of CYP2C9 enzyme is carried out with human liver microsomes and hydration activity of 4-position of tolbutamide that is a typical reaction of CYP2C9 as a parameter.

The reaction condition is as below. : A substrate, 5 µM Tolbutamide (¹⁴C labeled compound);the reaction time, 30 minutes; the reaction temperature, 37°C; the protein concentration, 0.25mg /ml (human liver microsomes, 15 pol, Lot. 210296, XenoTech).

To the HEPES Buffer (pH 7.4), is added the protein (human liver microsomes), a drug solution and a substrate with the composition as the above. NADPH, which is a coenzyme of the reaction, is added thereto to start the reaction. After reacting for the fixed hours, 2N hydrochloric acid solution is added thereto and the reaction is stopped by removing protein. The remaining substrate drug and the generating metabolite are extracted with chloroform. The solvent is removed and the residue is redissolved in methanol. This solution was spotted on TLC, developed with chloroform: methanol: acetic acid = 90: 10: 1, contacted on the imaging plate for about 14-20 hours and analyzed by BAS2000. As to the generation activity of the metabolite, Tolbutamide 4-potition hydration body, the activity in case that the solvent dissolving a drug is added to the reaction assay is used as a control (100 %). The residual activity (%) in case that the test drug solution is added to the reaction is calculated.

**Table 168**

| No. | EC₅₀ (nM) HPPARδ | Residual activity (%) CYP2C9 |
|---|---|---|
| Reference compound 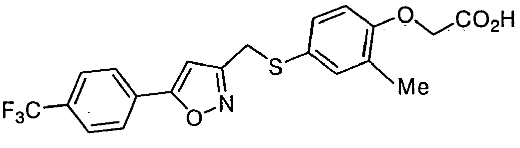 | 37 | 28 |
| β-2-38 | 35 | 47 |

## Claims

1. A compound of the formula (I): 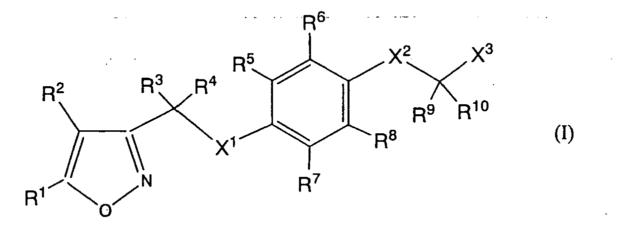 (wherein
R¹ is halogen, hydroxy, optionally substituted lower alkyl, optionally substituted lower alkenyl, optionally substituted lower alkynyl, optionally substituted lower alkoxy, carboxy, optionally substituted lower alkoxycarbonyl, optionally substituted lower alkylthio, optionally substituted acyl, optionally substituted amino, optionally substituted carbamoyl, optionally substituted thiocarbamoyl, optionally substituted carbamoyloxy, optionally substituted thiocarbamoyloxy, optionally substituted hydrazinocarbonyl, optionally substituted lower alkylsulfonyloxy, optionally substituted arylsulfonyloxy, optionally substituted aryl, optionally substituted aryloxy, optionally substituted arylthio or optionally substituted heterocycle,
R² is hydrogen, halogen, hydroxy, optionally substituted lower alkyl, optionally substituted lower alkenyl, optionally substituted lower alkynyl, optionally substituted lower alkoxy, carboxy, optionally substituted lower alkoxycarbonyl, optionally substituted lower alkylthio, optionally substituted acyl, optionally substituted amino, optionally substituted carbamoyl, optionally substituted thiocarbamoyl, optionally substituted carbamoyloxy, optionally substituted thiocarbamoyloxy, optionally substituted hydrazinocarbonyl, optionally substituted lower alkylsulfonyloxy, optionally substituted arylsulfonyloxy, optionally substituted aryl, optionally substituted aryloxy, optionally substituted arylthio or optionally substituted heterocycle,
R³ and R⁴ are each independently hydrogen, halogen, optionally substituted lower alkyl, optionally substituted lower alkenyl, optionally substituted lower alkynyl, optionally substituted aryl or optionally substituted heterocycle,
R⁵, R⁶, R⁷ and R⁸ are each independently hydrogen, halogen, hydroxy, optionally substituted lower alkyl, optionally substituted lower alkenyl, optionally substituted lower alkynyl, optionally substituted lower alkoxy, optionally substituted lower alkylthio, optionally substituted acyl, optionally substituted amino, optionally substituted aryl, optionally substituted aryloxy, optionally substituted arylthio or optionally substituted heterocycle,
R⁹ and R¹⁰ are each independently hydrogen, halogen, cyano, optionally substituted lower alkyl, optionally substituted lower alkoxy, optionally substituted amino or optionally substituted aryl,
X¹ is -O-, -S-, -NR¹¹- (wherein R¹¹ is hydrogen, optionally substituted lower alkyl, optionally substituted acyl, optionally substituted lower alkylsulfonyl or optionally substituted arylsulfonyl), -CR¹²R¹³CO-, -(CR¹²R¹³)mO-, -(CR¹²R¹³)mS- or -O(CR¹²R¹³)m- (wherein R¹² and R¹³ are each independently hydrogen or lower alkyl and m is an integer between 1 and 3),
X² is a bond, -O-, -S-, -SO-, -SO₂-, -CR²⁶=CR²⁷- (wherein R²⁶ and R²⁷ are each independently hydrogen or lower alkyl), -NR¹⁴- (wherein R¹⁴ is hydrogen, optionally substituted lower alkyl, optionally substituted acyl, optionally substituted lower alkylsulfonyl or optionally substituted arylsulfonyl), -CR¹⁵R¹⁶- (wherein R¹⁵ and R¹⁶ are each independently hydrogen or lower alkyl) or -COCR²⁴R²⁵- (wherein R²⁴ and R²⁵ are each independently hydrogen or lower alkyl), and
X³ is COOR¹⁷, C(=NR¹⁷)NR¹⁸OR¹⁹, 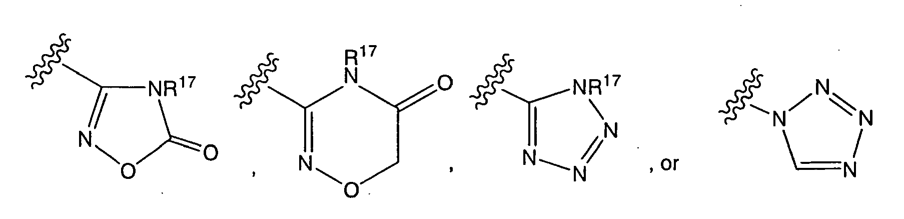 (wherein R¹⁷ - R¹⁹ are each independently hydrogen or lower alkyl),
provided that,
R⁶ and R¹⁴ can be taken together with the neighboring atom to form a ring,
R⁶, R⁹ and R¹⁰ can be taken together with the neighboring carbon atom to form a ring,
R⁶ and R⁹ can be taken together with the neighboring carbon atom to form a ring,
R⁶, R¹⁵ and R¹⁶ can be taken together with the neighboring carbon atom to form a ring, R⁶ and R²⁴ can be taken together with the neighboring carbon atom to form a ring,
R⁹ and R¹⁶ can be joined together to form a bond,
R⁹ and R¹⁰ can be taken together to form a ring,
R⁹ and R²⁵ can be joined together to form a bond,
R⁹, R¹⁰ and R¹⁵ can be taken together with the neighboring carbon atom to form a ring, R¹⁰ and R¹⁵ can be joined together to form a bond, and
R¹⁰ and R¹⁵ can be taken together with the neighboring carbon atom to form a ring)
(provided that, a compound wherein R¹ is an unsubstituted lower alkyl, R⁵ and R⁷ are bromo and X¹ is -O-, a compound wherein R¹ is an unsubstituted lower alkyl and X² is -CH₂- and a compound wherein R² is hydrogen and X² is -O- are excluded.),
a pharmaceutically acceptable salt or a solvate thereof.

2. The compound of claim 1 wherein R¹ is halogen, optionally substituted lower alkyl, optionally substituted aryl or optionally substituted heterocycle, a pharmaceutically acceptable salt or a solvate thereof.

3. The compound of claim 1 wherein R² is halogen, optionally substituted lower alkyl, optionally substituted lower alkenyl, optionally substituted alkynyl, optionally substituted lower alkoxy, optionally substituted acyl, optionally substituted carbamoyl, optionally substituted aryl or optionally substituted arylthio, a pharmaceutically acceptable salt or a solvate thereof.

4. The compound of claim 1 wherein R² is hydrogen, halogen, optionally substituted lower alkyl, optionally substituted lower alkenyl, optionally substituted alkynyl, optionally substituted lower alkoxy, optionally substituted acyl, optionally substituted carbamoyl, optionally substituted aryl or optionally substituted arylthio, a pharmaceutically acceptable salt or a solvate thereof.

5. The compound of claim 1 wherein R³ and R⁴ are each independently hydrogen, lower alkyl or optionally substituted aryl, a pharmaceutically acceptable salt or a solvate thereof.

6. The compound of claim 1 wherein R⁵, R⁶, R⁷ and R⁸ are each independently hydrogen, halogen, optionally substituted lower alkyl or optionally substituted lower alkoxy,
provided that,
R⁶ and R¹⁴ can be taken together with the neighboring atom to form a ring,
R⁶, R⁹ and R¹⁰ can be taken together with the neighboring carbon atom to form a ring,
R⁶ and R⁹ can be taken together with the neighboring carbon atom to form a ring,
R⁶, R¹⁵ and R¹⁶ can be taken together with the neighboring carbon atom to form a ring,
and R⁶ and R²⁴ can be taken together with the neighboring carbon atom to form a ring,
a pharmaceutically acceptable salt or a solvate thereof.

7. The compound of claim 1 wherein R⁹ and R¹⁰ are each independently hydrogen, halogen, cyano, optionally substituted lower alkyl or optionally substituted lower alkoxy,
provided that,
R⁹, R¹⁰ and R⁶ can be taken together with the neighboring carbon atom to form a ring,
R⁹ and R⁶ can be taken together with the neighboring carbon atom to form a ring,
R9 and R¹⁶ can be joined together to form a bond,
R⁹ and R¹⁰ can be taken together to form a ring,
R⁹ and R²⁵ can be joined together to form a bond,
R⁹, R¹⁰ and R¹⁵ can be taken together with the neighboring carbon atom to form a ring, R¹⁰ and R¹⁵ can be joined together to form a bond, and
R¹⁰ and R¹⁵ can be taken together with the neighboring carbon atom to form a ring,
a pharmaceutically acceptable salt or a solvate thereof.

8. The compound of claim 1 wherein X¹ is O, S, NR¹¹ (wherein R¹¹ is hydrogen or optionally substituted lower alkyl) or CH₂CO, a pharmaceutically acceptable salt or a solvate thereof.

9. The compound of claim 1 wherein X³ is COOR¹⁷ (wherein R¹⁷ is hydrogen or lower alkyl), a pharmaceutically acceptable salt or a solvate thereof.

10. The compound of claim 1 wherein R¹ is lower alkyl, optionally substituted aryl (the substituent is halogen, optionally substituted lower alkyl or optionally substituted lower alkoxy) or heterocycle,
R² is hydrogen, halogen, optionally substituted lower alkyl (the substituent is halogen, hydroxy, optionally substituted lower alkoxy, lower alkylamino, optionally substituted imino, lower alkylsulfonyl, optionally substituted aryl or heterocycle), optionally substituted lower alkynyl (the substituent is aryl), optionally substituted lower alkoxy (the substituent is halogen), alkoxycarbonyl, acyl, carbamoyl, optionally substituted aryl (the substituent is optionally substituted lower alkyl or optionally substituted lower alkoxy) or arylthio,
R³ and R⁴ are each independently, hydrogen, lower alkyl or optionally substituted aryl (the substituent is halogen),
R⁵, R⁶, R⁷ and R⁸ are each independently, hydrogen, halogen, optionally substituted lower alkyl (the substituent is halogen) or optionally substituted lower alkoxy (the substituent is halogen),
R⁹ and R¹⁰ are each independently hydrogen, halogen, cyano, lower alkyl or lower alkoxy,
X¹ is O, S, NH or CH₂CO, and
X³ is COOR¹⁷, C(=NR¹⁷)NR¹⁸OR¹⁹, 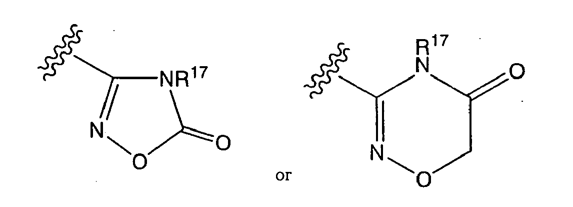 (wherein R¹⁷ - R¹⁹ are each independently hydrogen or lower alkyl),
provided that,
R⁶ and R¹⁴ can be taken together with the neighboring atom to form a ring,
R6, R⁹ and R¹⁰ can be taken together with the neighboring carbon atom to form a ring,
R⁶ and R⁹ can be taken together with the neighboring carbon atom to form a ring,
R6, R¹⁵ and R¹⁶ can be taken together with the neighboring carbon atom to form a ring,
R⁶ and R²⁴ can be taken together with the neighboring carbon atom to form a ring,
R⁹ and R¹⁶ can be joined together to form a bond,
R⁹ and R¹⁰ can be taken together to form a ring,
R⁹ and R²⁵ can be joined together to form a bond,
R⁹, R¹⁰ and R¹⁵ can be taken together with the neighboring carbon atom to form a ring, R¹⁰ and R¹⁵ can be joined together to form a bond, and
R¹⁰ and R¹⁵ can be taken together with the neighboring carbon atom to form a ring,
a pharmaceutically acceptable salt or a solvate thereof.

11. The compound of any one of claim 1 - 10 wherein X² is a bond, -O-, -SO-, -SO₂- or -CR²⁶=CR²⁷- (wherein R²⁶ and R²⁷ are each independently hydrogen or lower alkyl), a pharmaceutically acceptable salt or a solvate thereof.

12. The compound of any one of claim 1 - 10 wherein X² is -CR¹⁵R¹⁶- (wherein R¹⁵ is hydrogen or lower alkyl and R¹⁶ and R⁹ are joined together to form a bond or wherein R¹⁶ and R⁹ are joined together to form a bond and R¹⁵ and R¹⁰ are joined together to form a bond), a pharmaceutically acceptable salt or a solvate thereof.

13. The compound of any one of claim 1 - 10 wherein X² is -NR¹⁴- (wherein R¹⁴ is hydrogen, lower alkyl, acyl or lower alkylsulfonyl or wherein R¹⁴ and R⁶ are taken together with the neighboring atom to form a ring), -CR¹⁵R¹⁶- (wherein R¹⁵, R¹⁶ and R⁶ are taken together with the neighboring carbon atom to form a ring, wherein R⁹, R¹⁰ and R¹⁵ can be taken together with the neighboring carbon atom to form a ring or wherein R¹⁵ and R¹⁰ are taken together with the neighboring carbon atom to form a ring and R¹⁶ and R⁹ are joined together to form a bond) or -COCR²⁴R²⁵- (wherein R²⁴ and R⁶ are taken together with the neighboring carbon atom to form a ring and R²⁵ and R⁹ are joined together to form a bond), a pharmaceutically acceptable salt or a solvate thereof.

14. The compound of claim 1 wherein R² is halogen, hydroxy, optionally substituted lower alkyl, optionally substituted lower alkenyl, optionally substituted lower alkynyl, optionally substituted lower alkoxy, carboxy, optionally substituted lower alkoxycarbonyl, optionally substituted lower alkylthio, optionally substituted acyl, optionally substituted amino, optionally substituted carbamoyl, optionally substituted thiocarbamoyl, optionally substituted carbamoyloxy, optionally substituted thiocarbamoyloxy, optionally substituted hydrazinocarbonyl, optionally substituted lower alkylsulfonyloxy, optionally substituted arylsulfonyloxy, optionally substituted aryl, optionally substituted aryloxy, optionally substituted arylthio or optionally substituted heterocycle,
R⁹ and R¹⁰ are each independently hydrogen,
X¹ is -O-, -S-, -(CR¹²R¹³)mO- or -(CR¹²R¹³)mS- (wherein R¹² and R¹³ are each independently hydrogen or lower alkyl and m is an integer between 1 and 3),
X² is -O-, and
X³ is COOR¹⁷ (wherein R¹⁷ is hydrogen or lower alkyl),
a pharmaceutically acceptable salt or a solvate thereof.

15. The compound of claim 1 wherein R⁹ and R¹⁶ are joined together to form a bond,
R¹⁰ is hydrogen, halogen, lower alkyl, lower alkoxy or cyano,
X¹ is -O-, -S-, -(CR¹²R¹³)mO- or -(CR¹²R¹³)mS- (wherein R¹² and R¹³ are each independently hydrogen or lower alkyl and m is an integer between 1 and 3),
X² is -CR¹⁵R¹⁶- (wherein R¹⁵ is hydrogen or lower alkyl and R¹⁶ and R⁹ are joined together to form a bond), and
X³ is COOR¹⁷ (wherein R¹⁷ is hydrogen or lower alkyl), a pharmaceutically acceptable salt or a solvate thereof.

16. The compound of claim 1 wherein R¹ is halogen, a substituted lower alkyl, optionally substituted aryl or optionally substituted heterocycle,
R⁹ and R¹⁰ are each independently hydrogen or lower alkyl,
X¹ is -O-, -S-, -(CR¹²R¹³)mO- or -(CR¹²R¹³)mS- (wherein R¹² and R¹³ are each independently hydrogen or lower alkyl and m is an integer between 1 and 3),
X² is a bond or -CR¹⁵R¹⁶- (wherein R¹⁵ and R¹⁶ are each independently hydrogen or lower alkyl), and
X³ is COOR¹⁷ (wherein R¹⁷ is hydrogen or lower alkyl), a pharmaceutically acceptable salt or a solvate thereof.

17. The compound of claim 1 wherein R⁹ and R¹⁰ are each independently hydrogen,
X¹ is -O- or -S-,
X² is -NR¹⁴- (wherein R¹⁴ and R⁶ are taken together with the neighboring atom to form a ring), -CR¹⁵R¹⁶- (wherein R¹⁵, R¹⁶ and R⁶ are taken together with the neighboring carbon atom to form a ring), or -COCR²⁴R²⁵- (wherein R²⁴ and R⁶ are taken together with the neighboring carbon atom to form a ring and R²⁵ and R⁹ are joined together to form a bond), and
X³ is COOR¹⁷ (wherein R¹⁷ is hydrogen or lower alkyl), a pharmaceutically acceptable salt or a solvate thereof.

18. The compound of claim 1 wherein R⁹ and R¹⁶ are joined together to form a bond,
X¹ is -O- or -S-,
X² is -CR¹⁵R¹⁶- (wherein R¹⁵ and R¹⁰ are taken together with the neighboring carbon atom to form a ring and R¹⁶ and R⁹ are joined together to form a bond or wherein R⁹, R¹⁰ and R¹⁵ are taken together with the neighboring carbon atom to form a ring), and
X³ is COOR¹⁷ (wherein R¹⁷ is hydrogen or lower alkyl), a pharmaceutically acceptable salt or a solvate thereof.

19. The compound of claim 1 wherein R⁹ and R¹⁰ are taken together to form a ring,
X¹ is -O- or -S-,
X² is a bond or -CR¹⁵R¹⁶- (wherein R¹⁵ and R¹⁶ are each independently hydrogen or lower alkyl), and
X³ is COOR¹⁷ (wherein R¹⁷ is hydrogen or lower alkyl), a pharmaceutically acceptable salt or a solvate thereof.

20. A compound of the formula: 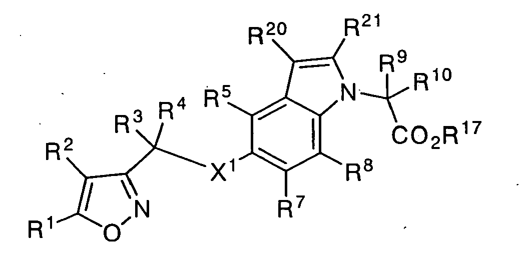 (wherein
R¹ is halogen, hydroxy, optionally substituted lower alkyl, optionally substituted lower alkenyl, optionally substituted lower alkynyl, optionally substituted lower alkoxy, carboxy, optionally substituted lower alkoxycarbonyl, optionally substituted lower alkylthio, optionally substituted acyl, optionally substituted amino, optionally substituted carbamoyl, optionally substituted thiocarbamoyl, optionally substituted carbamoyloxy, optionally substituted thiocarbamoyloxy, optionally substituted hydrazinocarbonyl, optionally substituted lower alkylsulfonyloxy, optionally substituted arylsulfonyloxy, optionally substituted aryl, optionally substituted aryloxy, optionally substituted arylthio or optionally substituted heterocycle,
R² is hydrogen, halogen, hydroxy, optionally substituted lower alkyl, optionally substituted lower alkenyl, optionally substituted lower alkynyl, optionally substituted lower alkoxy, carboxy, optionally substituted lower alkoxycarbonyl, optionally substituted lower alkylthio, optionally substituted acyl, optionally substituted amino, optionally substituted carbamoyl, optionally substituted thiocarbamoyl, optionally substituted carbamoyloxy, optionally substituted thiocarbamoyloxy, optionally substituted hydrazinocarbonyl, optionally substituted lower alkylsulfonyloxy, optionally substituted arylsulfonyloxy, optionally substituted aryl, optionally substituted aryloxy, optionally substituted arylthio or optionally substituted heterocycle,
R³ and R⁴ are each independently, hydrogen, halogen, optionally substituted lower alkyl, optionally substituted lower alkenyl, optionally substituted lower alkynyl, optionally substituted aryl or optionally substituted heterocycle,
R⁵, R⁷ and R⁸ are each independently hydrogen, halogen, hydroxy, optionally substituted lower alkyl, optionally substituted lower alkenyl, optionally substituted lower alkynyl, optionally substituted lower alkoxy, optionally substituted lower alkylthio, optionally substituted acyl, optionally substituted amino, optionally substituted aryl, optionally substituted aryloxy, optionally substituted arylthio or optionally substituted heterocycle,
R⁹ and R¹⁰ are each independently hydrogen, halogen, cyano, optionally substituted lower alkyl, optionally substituted lower alkoxy, optionally substituted amino or optionally substituted aryl,
R²⁰ and R²¹ are each independently hydrogen, halogen, hydroxy, cyano, optionally substituted lower alkyl, optionally substituted lower alkenyl, optionally substituted lower alkynyl, optionally substituted lower alkoxy, optionally substituted lower alkylthio, optionally substituted acyl, optionally substituted amino, optionally substituted imino, optionally substituted aryl, optionally substituted aryloxy, optionally substituted arylthio or optionally substituted heterocycle,
X¹ is -O-, -S-, -NR¹¹- (wherein R¹¹ is hydrogen, optionally substituted lower alkyl, optionally substituted acyl, optionally substituted lower alkylsulfonyl or optionally substituted arylsulfonyl), -CR¹²R¹³CO-, -(CR¹²R¹³)mO-, -(CR¹²R¹³)mS- or -O(CR¹²R¹³)m-(wherein R¹² and R¹³ are each independently hydrogen or lower alkyl and m is an integer between 1 and 3), and
R¹⁷ is hydrogen or lower alkyl), a pharmaceutically acceptable salt or a solvate thereof.

21. The compound of claim 20 wherein R¹ is optionally substituted aryl,
R² is optionally substituted lower alkyl,
R³and R⁴ are each independently hydrogen or optionally substituted aryl,
R⁵, R⁷ and R⁸ are each independently hydrogen, optionally substituted lower alkyl or optionally substituted lower alkoxy,
R⁹ and R¹⁰ are each independently hydrogen or optionally substituted lower alkyl,
R²⁰ and R²¹ are each independently hydrogen, cyano, optionally substituted lower alkyl or optionally substituted lower alkoxy, and
X¹ is -O- or -S-,
a pharmaceutically acceptable salt or a solvate thereof.

22. A compound of the formula: 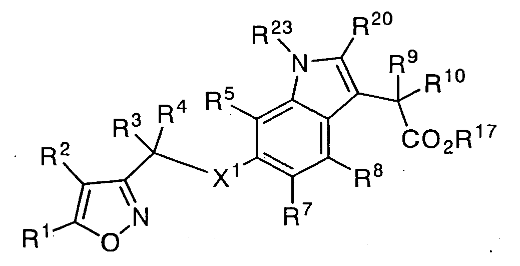 (wherein
R¹ is halogen, hydroxy, optionally substituted lower alkyl, optionally substituted lower alkenyl, optionally substituted lower alkynyl, optionally substituted lower alkoxy, carboxy, optionally substituted lower alkoxycarbonyl, optionally substituted lower alkylthio, optionally substituted acyl, optionally substituted amino, optionally substituted carbamoyl, optionally substituted thiocarbamoyl, optionally substituted carbamoyloxy, optionally substituted thiocarbamoyloxy, optionally substituted hydrazinocarbonyl, optionally substituted lower alkylsulfonyloxy, optionally substituted arylsulfonyloxy, optionally substituted aryl, optionally substituted aryloxy, optionally substituted arylthio or optionally substituted heterocycle,
R² is hydrogen, halogen, hydroxy, optionally substituted lower alkyl, optionally substituted lower alkenyl, optionally substituted lower alkynyl, optionally substituted lower alkoxy, carboxy, optionally substituted lower alkoxycarbonyl, optionally substituted lower alkylthio, optionally substituted acyl, optionally substituted amino, optionally substituted carbamoyl, optionally substituted thiocarbamoyl, optionally substituted carbamoyloxy, optionally substituted thiocarbamoyloxy, optionally substituted hydrazinocarbonyl, optionally substituted lower alkylsulfonyloxy, optionally substituted arylsulfonyloxy, optionally substituted aryl, optionally substituted aryloxy, optionally substituted arylthio or optionally substituted heterocycle,
R³ and R⁴ are each independently hydrogen, halogen, optionally substituted lower alkyl, optionally substituted lower alkenyl, optionally substituted lower alkynyl, optionally substituted aryl or optionally substituted heterocycle,
R⁵, R⁷, R⁸ and R²⁰ are each independently hydrogen, halogen, hydroxy, optionally substituted lower alkyl, optionally substituted lower alkenyl, optionally substituted lower alkynyl, optionally substituted lower alkoxy, optionally substituted lower alkylthio, optionally substituted acyl, optionally substituted amino, optionally substituted aryl, optionally substituted aryloxy, optionally substituted arylthio or optionally substituted heterocycle,
R²³ is hydrogen, optionally substituted lower alkyl, optionally substituted lower alkenyl, optionally substituted lower alkynyl, optionally substituted acyl, optionally substituted lower alkylsulfonyl or optionally substituted arylsulfonyl, optionally substituted amino, optionally substituted aryl or optionally substituted heterocycle,
R⁹ and R¹⁰ are each independently hydrogen, halogen, cyano, optionally substituted lower alkyl, optionally substituted lower alkoxy, optionally substituted amino or optionally substituted aryl,
X¹ is -O-, -S-, -NR¹¹- (wherein R¹¹ is hydrogen, optionally substituted lower alkyl, optionally substituted acyl, optionally substituted lower alkylsulfonyl or optionally substituted arylsulfonyl), -CR¹²R¹³CO-, -(CR¹²R¹³)mO-, -(CR¹²R¹³)mS- or -O(CR¹²R¹³)m-(wherein R¹² and R¹³ are each independently hydrogen or lower alkyl and m is an integer between 1 and 3), and
R¹⁷ is hydrogen or lower alkyl),
a pharmaceutically acceptable salt or a solvate thereof.

23. The compound of claim 22 wherein R¹ is optionally substituted aryl,
R² is optionally substituted lower alkyl,
R³ and R⁴ are hydrogen,
R⁵, R⁷ and R⁸ are hydrogen,
R⁹ and R¹⁰ are each independently hydrogen or optionally substituted lower alkyl,
R²⁰ and R²³ are each independently hydrogen or optionally substituted lower alkyl, and
X¹ is -O- or -S-, a pharmaceutically acceptable salt or a solvate thereof.

24. A compound of the formula: 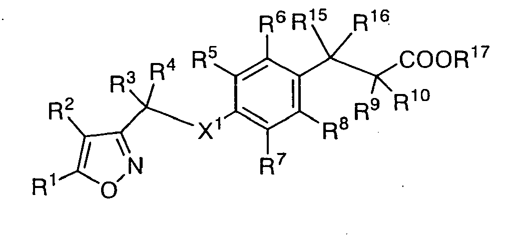 (wherein
R¹ is halogen, hydroxy, optionally substituted lower alkyl, optionally substituted lower alkenyl, optionally substituted lower alkynyl, optionally substituted lower alkoxy, carboxy, optionally substituted lower alkoxycarbonyl, optionally substituted lower alkylthio, optionally substituted acyl, optionally substituted amino, optionally substituted carbamoyl, optionally substituted thiocarbamoyl, optionally substituted carbamoyloxy, optionally substituted thiocarbamoyloxy, optionally substituted hydrazinocarbonyl, optionally substituted lower alkylsulfonyloxy, optionally substituted arylsulfonyloxy, optionally substituted aryl, optionally substituted aryloxy, optionally substituted arylthio or optionally substituted heterocycle,
R² is hydrogen, halogen, hydroxy, optionally substituted lower alkyl, optionally substituted lower alkenyl, optionally substituted lower alkynyl, optionally substituted lower alkoxy, carboxy, optionally substituted lower alkoxycarbonyl, optionally substituted lower alkylthio, optionally substituted acyl, optionally substituted amino, optionally substituted carbamoyl, optionally substituted thiocarbamoyl, optionally substituted carbamoyloxy, optionally substituted thiocarbamoyloxy, optionally substituted hydrazinocarbonyl, optionally substituted lower alkylsulfonyloxy, optionally substituted arylsulfonyloxy, optionally substituted aryl, optionally substituted aryloxy, optionally substituted arylthio or optionally substituted heterocycle,
R³ and R⁴ are each independently hydrogen, halogen, optionally substituted lower alkyl, optionally substituted lower alkenyl, optionally substituted lower alkynyl, optionally substituted aryl or optionally substituted heterocycle,
R⁵, R⁶, R⁷ and R⁸ are each independently hydrogen, halogen, hydroxy, optionally substituted lower alkyl, optionally substituted lower alkenyl, optionally substituted lower alkynyl, optionally substituted lower alkoxy, optionally substituted lower alkylthio, optionally substituted acyl, optionally substituted amino, optionally substituted aryl, optionally substituted aryloxy, optionally substituted arylthio or optionally substituted heterocycle,
R⁹ and R¹⁰ are hydrogen,
X¹ is -O-, -S-, -NR¹¹- (wherein R¹¹ is hydrogen, optionally substituted lower alkyl, optionally substituted acyl, optionally substituted lower alkylsulfonyl or optionally substituted arylsulfonyl), -CR¹²R¹³CO-, -(CR¹²R¹³)mO-, -(CR¹²R¹³)mS- or -O(CR¹²R¹³)m-(wherein R¹² and R¹³ are each independently hydrogen or lower alkyl and m is an integer between 1 and 3),
R¹⁵ is lower alkyl,
R¹⁶ is hydrogen, and
R¹⁷ is hydrogen or lower alkyl)
a pharmaceutically acceptable salt or a solvate thereof.

25. The compound of claim 24 wherein R¹ is optionally substituted aryl,
R² is optionally substituted lower alkyl,
R³ and R⁴ are hydrogen,
R⁵, R⁶, R⁷ and R⁸ are each independently hydrogen, halogen, optionally substituted lower alkyl or optionally substituted lower alkoxy, and
X¹ is -O- or -S-,
a pharmaceutically acceptable salt or a solvate thereof.

26. A pharmaceutical composition comprising a compound, a pharmaceutically acceptable salt or a solvate thereof of any one of claims 1-25.

27. A pharmaceutical composition as peroxisome proliferator-activated receptors agonists, which comprises a compound, a pharmaceutically acceptable salt or a solvate thereof of any one of claims 1-25 as active ingredient.
